# EUROPEAN PATENT APPLICATION

(11) **EP 4 227 412 A1**
(43) Date of publication of application: **16.08.2023**
(21) Application number: 21878066.6
(22) Date of filing: 08.10.2021
(51) Int. Cl.: C12N 15/113, C12N 15/10, C12N 9/22, C12N 15/63, C12N 15/90

(54) **ENGINEERED GUIDE RNA FOR INCREASING EFFICIENCY OF CRISPR/CAS12F1 (CAS14A1) SYSTEM, AND USE THEREOF**

(30) Priority: 08.10.2020 KR 20200129937; 29.12.2020 KR 20200185528; 05.04.2021 KR 20210044152
(71) Applicant: Genkore Inc, Yuseong-gu, Daejeon 34141 (KR)
(72) Inventor: KIM, Yong-Sam, Daejeon 34118 (KR); KIM, Do Yon, Daejeon 34069 (KR); LEE, Jeong Mi, Daejeon 34116 (KR); MOON, Su Bin, Daejeon 34116 (KR)
(74) Representative: Dehns
(86) International application number: PCT/KR2021/013933
(87) International publication number: WO 2022/075816

(57) **Abstract**

Provided is an engineered CRISPR/Cas12f1 (Cas14a1) system. Provided are an engineered guide RNA for more effectively performing cleavage, editing, or modifying of a target nucleic acid, and an engineered CRISPR/Cas12f1 (Cas14a1) system comprising the same. In addition, provided is a method of performing cleavage, editing, or modifying of a target nucleic acid by using the engineered guide RNA and the engineered CRISPR/Cas12f1 (Cas14a1) system comprising the same.

## Description

### TECHNICAL FIELD

### Related Applications

The present disclosure claims priority to Korean Patent Application No. 10-2020-0129937 filed on October 8, 2020, Korean Patent Application No. 10-2020-0185528 filed on December 29, 2020, and Korean Patent Application No. 10-2021-0044152 filed on April 5, 2021, the disclosure of each of which is incorporated herein by reference in its entirety.

The present disclosure relates to an engineered CRISPR/Cas12f1 (Cas14a1) system. More specifically, the present disclosure relates to an engineered guide RNA for more effectively performing cleavage, editing, or modifying of a target nucleic acid, an engineered CRISPR/Cas12f1 (Cas14a1) system comprising the same, and a use thereof.

### BACKGROUND ART

A CRISPR/Cas12f1 system is a CRISPR/Cas system classified as Class 2, Type V. A previous study (Harrington et al., Science 362, 839-842 (2018)) has reported for the first time the CRISPR/Cas14a system which is a CRISPR/Cas system derived from Archaea. Since then, a subsequent study (Tautvydas Karvelis et al., Nucleic Acids Research 48, 5016-5023 (2020)), classified the CRISPR/Cas14 system as a CRISPR/Cas12f system. The CRISPR/Cas12f1 system belongs to a V-F1 system, which is a variant of the CRISPR/Cas system classified as Class 2, Type V, and includes the CRISPR/Cas14a1 system having Cas14a1 as an effector protein. The CRISPR/Cas12f1 system is characterized in that a size of its effector protein is significantly smaller than a CRISPR/Cas9 system. However, as revealed in a previous study (Harrington et al., Science 362, 839-842 (2018), US 2020/0190494 A1), the CRISPR/Cas12f1 system, particularly the CRISPR/Cas14a1 system, has no or extremely low double-strand DNA cleavage activity (Karvelis, T. et al., bioRxiv, 654897 (2019)), which limits its application to gene editing technology.

### TECHNICAL PROBLEM

An object of the present disclosure is to provide an engineered guide RNA for a CRISPR/Cas12f1 (Cas14a1) system.

Another object of the present disclosure is to provide an engineered CRISPR/Cas12f1 (Cas14a1) system.

Yet another object of the present disclosure is to provide a composition comprising the engineered CRISPR/Cas12f1 (Cas14a1) system.

Still yet another object of the present disclosure is to provide a method of modifying a target nucleic acid or target gene using the engineered CRISPR/Cas12f1 (Cas14a1) system.

### SOLUTION TO PROBLEM

The present disclosure provides an engineered guide RNA for a CRISPR/Cas12f1 (Cas14a1) system. The engineered guide RNA comprises an engineered trans-activating CRISPR RNA (tracrRNA) and a CRISPR RNA (crRNA). Here, the engineered tracrRNA is a tracrRNA modified not to comprise a sequence of five or more consecutive uridines. Alternatively, the engineered tracrRNA may be a tracrRNA modified so that it does not comprise a sequence of five or more consecutive uridines and has a length shorter than a wildtype tracrRNA.

In an embodiment, the engineered guide RNA may comprise an engineered trans-activating CRISPR RNA (tracrRNA) and a CRISPR RNA (crRNA).

The engineered tracrRNA may be a tracrRNA modified not to comprise a sequence of five or more consecutive uridines.

The engineered tracrRNA may comprise a first sequence, a second sequence, a third sequence, a fourth sequence, and a fifth sequence in a 3' to 5' direction.

Here, the first sequence may be 5'-CAAAUUCANNNVNCCUCUCCAAUUCUGCACAA-3' (SEQ ID NO: 194) or a part of SEQ ID NO: 194. N may each independently be A, C, G or U. V may be A, C or G. The part of SEQ ID NO: 194 may be a sequence that comprises 5'-CAAAUUCANNNVN-3' (SEQ ID NO: 193) and does not comprise a partial sequence at the 3' end of SEQ ID NO: 194. In an embodiment, the first sequence may be 5'-CAAAUUCANNNCNCCUCUCCAAUUCUGCACAA-3' (SEQ ID NO: 111) or a part of SEQ ID NO: 111. The part of SEQ ID NO: 111 may be 5'-CAAAUUCANNNCNCCUCUCCAAUUCUGCACA-3' (SEQ ID NO: 273), 5'-CAAAUUCANNNCNCCUCUCCAAUUCUGCAC-3' (SEQ ID NO: 274), 5'-CAAAUUCANNNCNCCUCUCCAAUUCUGCA-3' (SEQ ID NO: 275), 5'-CAAAUUCANNNCNCCUCUCCAAUUCUGC-3' (SEQ ID NO: 276), 5'-CAAAUUCANNNCNCCUCUCCAAUUCUG-3' (SEQ ID NO: 277), 5'-CAAAUUCANNNCNCCUCUCCAAUUCU-3' (SEQ ID NO: 278), 5'-CAAAUUCANNNCNCCUCUCCAAUUC-3' (SEQ ID NO: 279), 5'-CAAAUUCANNNCNCCUCUCCAAUU-3' (SEQ ID NO: 280), 5'-CAAAUUCANNNCNCCUCUCCAAU-3' (SEQ ID NO: 281), 5'-CAAAUUCANNNCNCCUCUCCAA-3' (SEQ ID NO: 282), 5'-CAAAUUCANNNCNCCUCUCCA-3' (SEQ ID NO: 283), 5'-CAAAUUCANNNCNCCUCUCC-3' (SEQ ID NO: 284), 5'-CAAAUUCANNNCNCCUCUC-3' (SEQ ID NO: 285), 5'-CAAAUUCANNNCNCCUCU-3' (SEQ ID NO: 286), 5'-CAAAUUCANNNCNCCUC-3' (SEQ ID NO: 287), 5'-CAAAUUCANNNCNCCU-3' (SEQ ID NO: 288), 5'-CAAAUUCANNNCNCC-3' (SEQ ID NO: 289), 5'-CAAAUUCANNNCNC-3' (SEQ ID NO: 290), or 5'-CAAAUUCANNNCN-3' (SEQ ID NO: 272). Here, N may be each independently A, C, G or U. As an example, the sequence 5'-NNNCN-3' present in SEQ ID NO: 111 or the part thereof may be 5'-UUUCU-3', 5'-GUUCU-3', 5'-UCUCU-3', 5'-UUGCU-3', 5'-UUUCC-3', 5'-GCUCU-3', 5'-GUUCC-3', 5'-UCGCU-3', 5'-UCUCC-3', 5'-UUGCC-3', 5'-GCGCU-3', 5'-GCUCC-3', 5'-GUGCC-3', 5'-UCGCC-3', 5'-GCGCC-3', or 5'-GUGCU-3'.

Here, the second sequence is 5'-AUGUCGAGAAGUGCUUUCUUCGGAAAGUAACCCUCGAAA-3' (SEQ ID NO: 211) or a sequence having sequence identity or sequence similarity of at least 70%, 80%, or 90% or more to SEQ ID NO: 211.

Here, the third sequence may be 5'-GGCUGCUUGCAUCAGCCUA-3' (SEQ ID NO: 212) or a sequence having sequence identity or sequence similarity of at least 70%, 80%, or 90% or more to SEQ ID NO: 212.

Here, the fourth sequence may be 5'-CCGCUUCACCAAAAGCUGUCCCUUAGGGGAUUAGAACUUGAGUGAAGGU G-3' (SEQ ID NO: 213) or a part of SEQ ID NO: 213. The part of SEQ ID NO: 213 may be a sequence obtained by deleting at least one pair of nucleotides forming a complementary base pair and/or at least one nucleotide not involved in forming a complementary base pair from SEQ ID NO: 213. The part of SEQ ID NO: 213 may be 5'-CCGCUUCACCAAAAGCUGUCCUUAGGGAUUAGAACUUGAGUGAAGGUG-3' (SEQ ID NO: 214), 5'-CCGCUUCACCAAAAGCUGUCUUAGGAUUAGAACUUGAGUGAAGGUG-3' (SEQ ID NO: 215), 5'-CCGCUUCACCAAAAGCUGUUUAGAUUAGAACUUGAGUGAAGGUG-3' (SEQ ID NO: 216), 5'-CCGCUUCACCAAAAGCUGUUAGUUAGAACUUGAGUGAAGGUG-3' (SEQ ID NO: 217), 5'-CCGCUUCACCAAAAGCUGUUAGUAGAACUUGAGUGAAGGUG-3' (SEQ ID NO: 231), 5'-CCGCUUCACCAAAAGCUUUAGAGAACUUGAGUGAAGGUG-3' (SEQ ID NO: 232), 5'-CCGCUUCACCAAAAGCUUAGGAACUUGAGUGAAGGUG-3' (SEQ ID NO: 233), 5'-CCGCUUCACCAAAAGUUAGAACUUGAGUGAAGGUG-3' (SEQ ID NO: 234), 5'-CCGCUUCACCAAAAUUAGACUUGAGUGAAGGUG-3' (SEQ ID NO: 235), 5'-CCGCUUCACCAAAUUAGCUUGAGUGAAGGUG-3' (SEQ ID NO: 236), 5'-CCGCUUCACCAAUUAGUUGAGUGAAGGUG-3' (SEQ ID NO: 237), 5'-CCGCUUCACCAUUAGUGAGUGAAGGUG-3' (SEQ ID NO: 238), 5'-CCGCUUCACCUUAGGAGUGAAGGUG-3' (SEQ ID NO:239), 5'-CCGCUUCACUUAGAGUGAAGGUG-3' (SEQ ID NO: 240), 5'-CCGCUUCACUUAGGUGAAGGUG-3' (SEQ ID NO: 241), 5'-CCGCUUCAUUAGUGAAGGUG-3' (SEQ ID NO: 242), 5'-CCGCUUCUUAGGAAGGUG-3' (SEQ ID NO: 243), 5'-CCGCUUUUAGAAGGUG-3' (SEQ ID NO: 244), 5'-CCGCUUUAGAGGUG-3' (SEQ ID NO: 245), 5'-CCGCUUAGGGUG-3' (SEQ ID NO: 246), 5'-CCGUUAGGUG-3' (SEQ ID NO: 247), 5'-CCUUAGGUG-3', 5'-CUUAGUG-3', 5'-CUUAGG-3', or 5'-UUAG-3'. Here, 5'-UUAG-3' included in the part of SEQ ID NO: 213 may be optionally substituted with 5'-GAAA-3'.

Here, the fifth sequence may be 5'-CUUCACUGAUAAAGUGGAGAA-3' (SEQ ID NO: 248) or a part of SEQ ID NO: 248. The part of SEQ ID NO: 248 may be a sequential partial sequence at the 3' end of SEQ ID NO: 248. The part of SEQ ID NO: 248 may be 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', 5'-AAGUGGAGAA-3' (SEQ ID NO: 249), 5'-AAAGUGGAGAA-3' (SEQ ID NO: 250), 5'-UAAAGUGGAGAA-3' (SEQ ID NO: 251), 5'-AUAAAGUGGAGAA-3' (SEQ ID NO: 252), 5'-GAUAAAGUGGAGAA-3' (SEQ ID NO: 253), 5'-UGAUAAAGUGGAGAA-3' (SEQ ID NO: 254), 5'-CUGAUAAAGUGGAGAA-3' (SEQ ID NO: 255), 5'-ACUGAUAAAGUGGAGAA-3' (SEQ ID NO: 256), 5'-CACUGAUAAAGUGGAGAA-3' (SEQ ID NO: 257), 5'-UCACUGAUAAAGUGGAGAA-3' (SEQ ID NO: 258), or 5'-UUCACUGAUAAAGUGGAGAA-3' (SEQ ID NO: 259).

The crRNA may be a wildtype crRNA or an engineered crRNA.

Here, the wildtype crRNA may comprise a wildtype repeat sequence and a guide sequence in a 5' to 3' direction. The wildtype repeat sequence may be 5'-GUUGCAGAACCCGAAUAGACGAAUGAAGGAAUGCAAC-3' (SEQ ID NO: 312).

Here, the engineered crRNA may comprise a sixth sequence, a seventh sequence, and a guide sequence in a 5' to 3' direction.

Here, the sixth sequence may be 5'-GUUGCAGAACCCGAAUAGNBNNNUGAAGGA-3' (SEQ ID NO: 373), or a part of SEQ ID NO: 373. N may each independently be A, C, G or U. B may be U, C or G. The part of SEQ ID NO: 373 may be a sequence that comprises 5'-NBNNNUGAAGGA-3' (SEQ ID NO: 372) and does not comprise a partial sequence at the 3' end of SEQ ID NO: 373. In an embodiment, the sixth sequence may be 5'-GUUGCAGAACCCGAAUAGNGNNNUGAAGGA-3' (SEQ ID NO: 408) or a part of SEQ ID NO: 408. The part of SEQ ID NO: 408 may be 5'-UUGCAGAACCCGAAUAGNGNNNUGAAGGA-3' (SEQ ID NO: 413), 5'-UGCAGAACCCGAAUAGNGNNNUGAAGGA-3' (SEQ ID NO: 414), 5'-GCAGAACCCGAAUAGNGNNNUGAAGGA-3' (SEQ ID NO: 415), 5'-CAGAACCCGAAUAGNGNNNUGAAGGA-3' (SEQ ID NO: 416), 5'-AGAACCCGAAUAGNGNNNUGAAGGA-3' (SEQ ID NO: 417), 5'-GAACCCGAAUAGNGNNNUGAAGGA-3' (SEQ ID NO: 418), 5'-AACCCGAAUAGNGNNNUGAAGGA-3' (SEQ ID NO: 419), 5'-ACCCGAAUAGNGNNNUGAAGGA-3' (SEQ ID NO: 420), 5'-CCCGAAUAGNGNNNUGAAGGA-3' (SEQ ID NO: 421), 5'-CCGAAUAGNGNNNUGAAGGA-3' (SEQ ID NO: 422), 5'-CGAAUAGNGNNNUGAAGGA-3' (SEQ ID NO: 423), 5'-GAAUAGNGNNNUGAAGGA-3' (SEQ ID NO: 424), 5'-AAUAGNGNNNUGAAGGA-3' (SEQ ID NO: 425), 5'-AUAGNGNNNUGAAGGA-3' (SEQ ID NO: 426), 5'-UAGNGNNNUGAAGGA-3' (SEQ ID NO: 427), 5'-AGNGNNNUGAAGGA-3' (SEQ ID NO: 428), or 5'-NGNNNUGAAGGA-3' (SEQ ID NO: 412). Here, N may each independently be A, C, G or U. As an example, the sequence 5'-NGNNN-3' present in SEQ ID NO: 408 or the part thereof may be 5'-AGGAA-3', 5'-AGCAA-3', 5'-AGAAA-3', 5'-AGCAU-3', 5'-AGCAG-3', 5'-AGCAC-3', 5'-AGCUA-3', 5'-AGCGA-3', 5'-AGCCA-3', 5'-UGCAA-3', 5'-UGCUA-3', 5'-UGCGA-3', 5'-UGCCA-3', 5'-GGCAA-3', 5'-GGCUA-3', 5'-GGCGA-3', 5'-GGCCA-3', 5'-CGCAA-3', 5'-CGCUA-3', 5'-CGCGA-3', or 5'-CGCCA-3'.

Here, the seventh sequence may be a sequence having sequence identity or sequence similarity of at least 70%, 80%, or 90% or more to 5'-AUGCAAC-3' or 5'-AUGCAAC-3'.

Here, the guide sequence may be a sequence capable of hybridizing with a target sequence or a sequence forming a complementary bond to the target sequence, and may have 15 to 30 nucleotides (nts).

The crRNA may further comprise a U-rich tail sequence. Here, the U-rich tail sequence may be located at the 3' end of the crRNA. Here, the U-rich tail sequence may be 5'-(UₐN)_{d}Uₑ-3', 5'-UₐVUₐVUₑ-3', or 5'-UₐVUₐVUₐVUₑ-3'. N may be A, C, G or U. V may each independently be A, C or G. a may be an integer of 0 to 4. d may be an integer of 0 to 3. e may be an integer from 0 to 10.

The engineered guide RNA may be a dual guide RNA in which a modified tracrRNA and a crRNA are present as two independent molecules, or a single guide RNA in which a modified tracrRNA and a crRNA are linked to each other to exist as one molecule. When the engineered guide RNA is a single guide RNA, the engineered guide RNA may further comprise a linker sequence. Here, the linker sequence may be located between the modified tracrRNA and the crRNA.

In an embodiment, the engineered tracrRNA may comprise 5'-CUUCACUGAUAAAGUGGAGAACCGCUUCACCAAAAGCUGUCCCUUAGGG GAUUAGAACUUGAGUGAAGGUGGGCUGCUUGCAUCAGCCUAAUGUCGA GAAGUGCUUUCUUCGGAAAGUAACCCUCGAAACAAAUUCANNNCNCCUC UCCAAUUCUGCACAA-3' (SEQ ID NO: 269), 5'-ACCGCUUCACCAAAAGCUGUCCCUUAGGGGAUUAGAACUUGAGUGAAGG UGGGCUGCUUGCAUCAGCCUAAUGUCGAGAAGUGCUUUCUUCGGAAAG UAACCCUCGAAACAAAUUCANNNCNCCUCUCCAAUUCUGCACAA-3' (SEQ ID NO: 304), 5'-CUUCACUGAUAAAGUGGAGAACCGCUUCACCAAUUAGUUGAGUGAAGGU GGGCUGCUUGCAUCAGCCUAAUGUCGAGAAGUGCUUUCUUCGGAAAGU AACCCUCGAAACAAAUUCANNNCNCCUCUCCAAUUCUGCACAA-3' (SEQ ID NO: 590), or 5'-ACCGCUUCACCAAUUAGUUGAGUGAAGGUGGGCUGCUUGCAUCAGCCU AAUGUCGAGAAGUGCUUUCUUCGGAAAGUAACCCUCGAAACAAAUUCAN NNCNCCUCUCCAAUUCUGCACAA-3' (SEQ ID NO: 301). The engineered crRNA may comprise 5'-GUUGCAGAACCCGAAUAGNGNNNUGAAGGAAUGCAAC-3' (SEQ ID NO: 591) and a guide sequence. Here, N may each independently be A, C, G, or U. As an example, the engineered tracrRNA may comprise 5'-CUUCACUGAUAAAGUGGAGAACCGCUUCACCAAAAGCUGUCCCUUAGGG GAUUAGAACUUGAGUGAAGGUGGGCUGCUUGCAUCAGCCUAAUGUCGA GAAGUGCUUUCUUCGGAAAGUAACCCUCGAAACAAAUUCAGUGCUCCUC UCCAAUUCUGCACAA-3' (SEQ ID NO: 270), 5'-ACCGCUUCACCAAAAGCUGUCCCUUAGGGGAUUAGAACUUGAGUGAAGG UGGGCUGCUUGCAUCAGCCUAAUGUCGAGAAGUGCUUUCUUCGGAAAG UAACCCUCGAAACAAAUUCAGUGCUCCUCUCCAAUUCUGCACAA-3' (SEQ ID NO: 308), 5'-CUUCACUGAUAAAGUGGAGAACCGCUUCACCAAUUAGUUGAGUGAAGGU GGGCUGCUUGCAUCAGCCUAAUGUCGAGAAGUGCUUUCUUCGGAAAGU AACCCUCGAAACAAAUUCAGUGCUCCUCUCCAAUUCUGCACAA-3' (SEQ ID NO: 595), or 5'-ACCGCUUCACCAAUUAGUUGAGUGAAGGUGGGCUGCUUGCAUCAGCCU AAUGUCGAGAAGUGCUUUCUUCGGAAAGUAACCCUCGAAACAAAUUCAG UGCUCCUCUCCAAUUCUGCACAA-3' (SEQ ID NO: 306). The engineered crRNA may comprise 5'-GUUGCAGAACCCGAAUAGAGCAAUGAAGGAAUGCAAC-3' (SEQ ID NO: 539) and a guide sequence.

In another embodiment, the engineered tracrRNA may comprise 5'-CUUCACUGAUAAAGUGGAGAACCGCUUCACCAAAAGCUGUCCCUUAGGG GAUUAGAACUUGAGUGAAGGUGGGCUGCUUGCAUCAGCCUAAUGUCGA GAAGUGCUUUCUUCGGAAAGUAACCCUCGAAACAAAUUCANNNCNCCUC UC-3' (SEQ ID NO: 592), 5'-ACCGCUUCACCAAAAGCUGUCCCUUAGGGGAUUAGAACUUGAGUGAAGG UGGGCUGCUUGCAUCAGCCUAAUGUCGAGAAGUGCUUUCUUCGGAAAG UAACCCUCGAAACAAAUUCANNNCNCCUCUC-3' (SEQ ID NO: 305), 5'-CUUCACUGAUAAAGUGGAGAACCGCUUCACCAAUUAGUUGAGUGAAGGU GGGCUGCUUGCAUCAGCCUAAUGUCGAGAAGUGCUUUCUUCGGAAAGU AACCCUCGAAACAAAUUCANNNCNCCUCUC-3' (SEQ ID NO: 593), or 5'-ACCGCUUCACCAAUUAGUUGAGUGAAGGUGGGCUGCUUGCAUCAGCCU AAUGUCGAGAAGUGCUUUCUUCGGAAAGUAACCCUCGAAACAAAUUCAN NNCNCCUCUC-3' (SEQ ID NO: 300). The engineered crRNA may comprise 5'-GAAUAGNGNNNUGAAGGAAUGCAAC-3' (SEQ ID NO: 594) and a guide sequence. Here, N may each independently be A, C, G or U. As an example, the engineered tracrRNA may comprise 5'-CUUCACUGAUAAAGUGGAGAACCGCUUCACCAAAAGCUGUCCCUUAGGG GAUUAGAACUUGAGUGAAGGUGGGCUGCUUGCAUCAGCCUAAUGUCGA GAAGUGCUUUCUUCGGAAAGUAACCCUCGAAACAAAUUCGUGCUCCUCU C-3' (SEQ ID NO: 596), 5'-ACCGCUUCACCAAAAGCUGUCCCUUAGGGGAUUAGAACUUGAGUGAAGG UGGGCUGCUUGCAUCAGCCUAAUGUCGAGAAGUGCUUUCUUCGGAAAG UAACCCUCGAAACAAAUUCAGUGCUCCUCUC-3' (SEQ ID NO: 309), 5'-CUUCACUGAUAAAGUGGAGAACCGCUUCACCAAUUAGUUGAGUGAAGGU GGGCUGCUUGCAUCAGCCUAAUGUCGAGAAGUGCUUUCUUCGGAAAGU AACCCUCGAAACAAAUUCAGUGCUCCUCUC-3' (SEQ ID NO: 597), or 5'-ACCGCUUCACCAAUUAGUUGAGUGAAGGUGGGCUGCUUGCAUCAGCCU AAUGUCGAGAAGUGCUUUCUUCGGAAAGUAACCCUCGAAACAAAUUCAG UGCUCCUCUC-3' (SEQ ID NO: 307). The engineered crRNA may comprise 5'-GAAUAGAGCAAUGAAGGAAUGCAAC-3' (SEQ ID NO: 598) and a guide sequence.

The present disclosure provides a vector for an engineered CRISPR/Cas12f1 (Cas14a1) system. The vector comprises a nucleic acid encoding an engineered guide RNA.

In an embodiment, the vector may comprise a nucleic acid encoding an engineered guide RNA.

The engineered guide RNA may comprise an engineered tracrRNA and a crRNA. Here, the engineered guide RNA may have the same configuration as the embodiment of the engineered guide RNA described above.

The vector may further comprise a promoter for the nucleic acid encoding the engineered guide RNA. Here, the promoter may be a U6 promoter, an H1 promoter, or a 7SK promoter.

The vector may be a plasmid, a PCR amplicon or a viral vector. Here, the viral vector may be at least one viral vector selected from the group consisting of a retroviral (retrovirus) vector, a lentiviral (lentivirus) vector, an adenoviral (adenovirus vector), an adeno-associated viral (adeno-associated virus; AAV) vector, a vaccinia viral (vaccinia virus) vector, a poxviral (poxvirus) vector, and a herpes simplex viral (herpes simplex virus) vector).

The present disclosure provides a composition comprising an engineered CRISPR/Cas12f1 (Cas14a1) system. The engineered CRISPR/Cas12f1 (Cas14a1) system comprises an engineered guide RNA or a nucleic acid encoding the same; and a Cas14a1 protein or a nucleic acid encoding the same.

In an embodiment, the composition may comprise an engineered guide RNA or a nucleic acid encoding the same; and a Cas14a1 protein or a nucleic acid encoding the same.

The engineered guide RNA may comprise an engineered tracrRNA and a crRNA. Here, the engineered guide RNA may have the same configuration as the embodiment of the engineered guide RNA described above.

The composition may be in a form of a vector. Here, the composition may comprise a nucleic acid encoding an engineered guide RNA; and a nucleic acid encoding a Cas14a1 protein. Here, the vector may be a plasmid, mRNA (transcript), PCR amplicon, or viral vector. Here, the viral vector may be at least one viral vector selected from the group consisting of a retroviral (retrovirus) vector, a lentiviral (lentivirus) vector, an adenoviral (adenovirus vector), an adeno-associated viral (adeno-associated virus; AAV) vector, a vaccinia viral (vaccinia virus) vector, a poxviral (poxvirus) vector, and a herpes simplex viral (herpes simplex virus) vector).

Here, the composition may comprise a vector comprising both a nucleic acid encoding an engineered guide RNA; and a nucleic acid encoding a Cas14a1 protein. The vector may further comprise a promoter for the nucleic acid encoding the engineered guide RNA, and the promoter may be a U6 promoter, an H1 promoter, or a 7SK promoter. In addition, the vector may further comprise a promoter for the nucleic acid encoding the Cas14a1 protein, and the promoter may be a CMV promoter, an LTR promoter, an Ad MLP promoter, an HSV promoter, an SV40 promoter, a CBA promoter, or an RSV promoter. Alternatively, the composition may comprise a first vector comprising a nucleic acid encoding an engineered guide RNA and a second vector comprising a nucleic acid encoding a Cas14a1 protein. The first vector may further comprise a promoter for the nucleic acid encoding the engineered guide RNA, and the promoter may be a U6 promoter, H1 promoter, or 7SK promoter. In addition, the second vector may further comprise a promoter for the nucleic acid encoding the Cas14a1 protein, and the promoter may be a CMV promoter, an LTR promoter, an Ad MLP promoter, an HSV promoter, an SV40 promoter, a CBA promoter or an RSV promoter.

Alternatively, the composition may be in a form in which a nucleic acid and a protein are mixed. Here, the composition may comprise an engineered guide RNA and a Cas14a1 protein. The composition may be in a form of a ribonucleoprotein (RNP) which is an engineered CRISPR/Cas14a1 complex.

In addition, the present disclosure provides a method of modifying a target nucleic acid or target gene present in a target cell using an engineered CRISPR/Cas12f1 (Cas14a1) system. The method comprises treating the target cell, in which the target nucleic acid or target gene is present, with a composition comprising the engineered CRISPR/Cas12f1 (Cas14a1) system.

In an embodiment, the method may comprise treating a eukaryotic cell, in which a target nucleic acid or target gene is present, with a composition comprising the engineered CRISPR/Cas12f1 (Cas14a1) system.

The eukaryotic cell may be yeast, a plant cell, a non-human-animal cell or a human cell.

The target nucleic acid or target gene may be double-stranded DNA or single-stranded DNA comprising a target strand having a target sequence.

The composition comprising the engineered CRISPR/Cas12f1 (Cas14a1) system may be the same as the embodiment of the composition described above.

The method may be performed in vitro, ex vivo, or in vivo in a non-human animal.

The method may optionally further comprise culturing the eukaryotic cell treated with the composition comprising the engineered CRISPR/Cas12f1 (Cas14a1) system.

By using the method, the target nucleic acid or target gene may comprise a modification caused by the engineered CRISPR/Cas12f1 (Cas14a1) system. Here, the modification may be deletion, substitution, and/or insertion of at least one nucleotide in the target nucleic acid or target gene.

### ADVANTAGEOUS EFFECTS OF DISCLOSURE

The present disclosure relates to an engineered CRISPR/Cas12f1 (Cas14a1) system. Through the technology disclosed in the present specification, it is possible to provide an engineered guide RNA and an engineered CRISPR/Cas12f1 (Cas14a1) system comprising the same. In addition, it is possible to provide an effective method of cleaving, editing, or modifying a target nucleic acid using the engineered guide RNA and the engineered CRISPR/Cas12f1 (Cas14a1) system comprising the same.

### BRIEF DESCRIPTION OF DRAWINGS

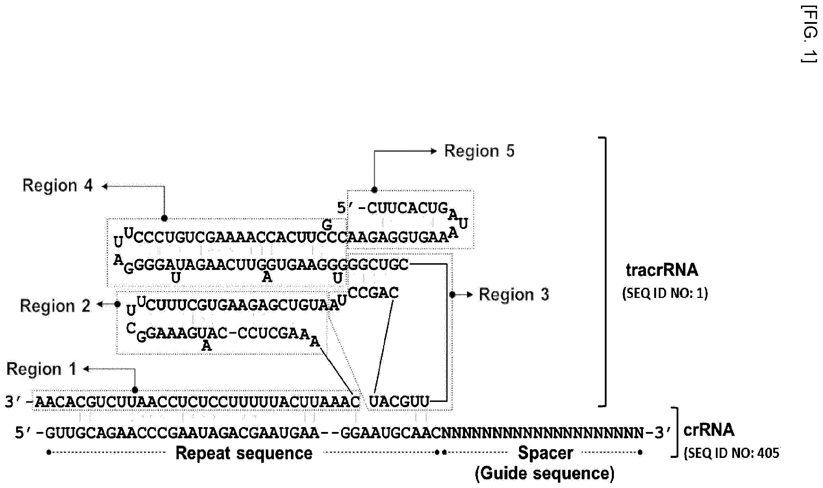

FIG. 1 shows a wildtype tracrRNA (SEQ ID NO: 1) and a wildtype crRNA (SEQ ID NO: 405), in which their respective regions are indicated. The wildtype tracrRNA is divided into region 1, region 2, region 3, region 4, and region 5 according to the stem formation. The wildtype crRNA is divided into a repeat sequence (repeat seq.) and a guide sequence (guide seq. or a spacer sequence). Here, the guide sequence varies in sequence and length depending on the target, and N may each independently be A, C, G, or U.

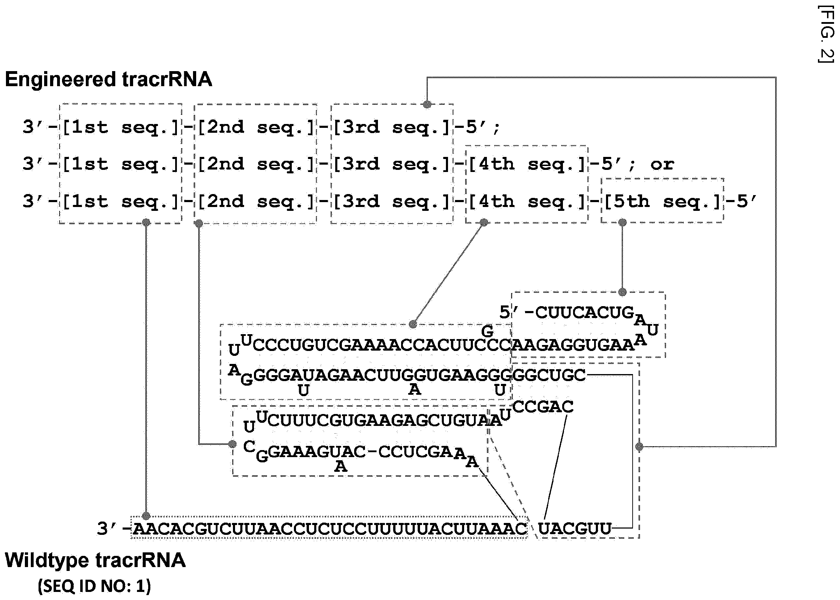

FIG. 2 concisely shows structures of engineered tracrRNAs. Each sequence was shown by comparative matching to the wildtype tracrRNA (SEQ ID NO: 1) for comparison. The engineered tracrRNA comprises a first sequence (1st seq.), second sequence (2nd seq.), and third sequence (3rd seq.) sequentially in a 3' to 5' direction. In addition, the engineered tracrRNA may optionally further comprise a fourth sequence (4th seq.) and/or a fifth sequence (5th seq.) at the 5' end.

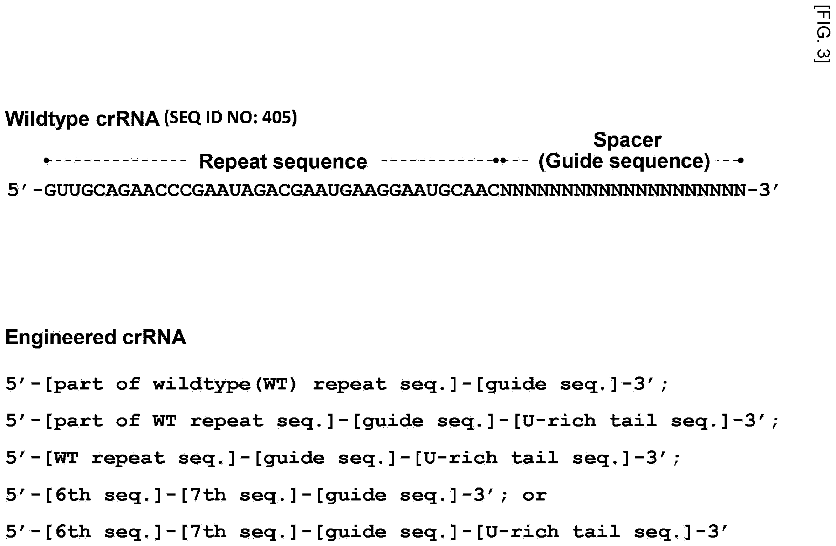

FIG. 3 concisely shows structures of engineered crRNAs. Each sequence is shown by comparison to the wildtype crRNA (SEQ ID NO: 405). The engineered crRNA may further comprise i) a part of a wildtype repeat sequence and a guide sequence; ii) a part of a wildtype repeat sequence, a guide sequence and a U-rich tail sequence (U-rich tail seq.); iii) a wildtype repeat sequence, a guide sequence and a U-rich tail sequence; iv) a sixth sequence (6th seq.), a seventh sequence (7th seq.) and a guide sequence; or v) a sixth sequence, a seventh sequence, a guide sequence and a U-rich tail sequence. Here, the sixth sequence and seventh sequence are associated with a repeat sequence of the wildtype crRNA. Here, the guide sequence varies in sequence and length depending on the target, and N may each independently be A, C, G, or U.

FIGS. 4 to 10 are schematic diagrams of various examples of gRNA engineering. Here, the guide sequence may vary in sequence and length depending on the target. N may each independently be A, C, G or U, each V may be A, C or G, and each B may be U, C or G.

FIG. 4 is a schematic diagram of various examples of gRNA engineering.

FIG. 5 is a schematic diagram of various examples of gRNA engineering.

FIG. 6 is a schematic diagram of various examples of gRNA engineering.

FIG. 7 is a schematic diagram of various examples of gRNA engineering.

FIG. 8 is a schematic diagram of various examples of gRNA engineering.

FIG. 9 is a schematic diagram of various examples of gRNA engineering.

FIG. 10 is a schematic diagram of various examples of gRNA engineering.

FIG. 11 shows results obtained by substituting any one of five consecutive uridines present in the wildtype tracrRNA with an adenosine, cytidine or guanosine, respectively, and identifying indel (%).

FIG. 12 shows results obtained by fixedly substituting the 141 st uridine with a cytidine based on the results of FIG. 11, substituting each of the four remaining uridines with a cytidine or guanosine, and identifying indel (%). (n=3, *, p<0.05)

FIG. 13 shows results obtained by substituting each of 5'-ACGAA-3' of the crRNA ", which corresponds to a modified sequence of the tracrRNA, with an adenosine, uridine, cytidine or guanosine based on the results of FIG. 12, and identifying indel (%).

FIG. 14 shows results obtained by substituting each of cytidine and guanosine in 5'-ACGAA-3' of the crRNA of with an adenosine, uridine, cytidine or guanosine based on the results of FIG. 13, and identifying indel (%). (n=3, *, p<0.05)

FIG. 15 shows results obtained by substituting each adenosine in 5'-AGCAA-3' of the crRNA of with a uridine, cytidine or guanosine based on the results of FIG. 14, and identifying indel (%). (n=3, n.s., not significant)

FIG. 16 shows results obtained by identifying indel (%) in a case where an engineered tracrRNA and/or an engineered crRNA comprising M1-1 is used. An increase of indel (%) was identified when the engineered crRNA (M1-1) was used in combination with the engineered tracrRNA (M1-1). (n=3, *, p<0.05, n.s., not significant)

FIG. 17 shows results obtained by identifying indel (%) using an engineered gRNA comprising M1-1 and M1-2. Here, M1-2 is a variant obtained by deleting each of 13 nts, 25 nts, and 37 nts from MS1 (i.e., a region constituting stem 5). (n=3)

FIG. 18 shows results obtained by identifying indel (%) using an engineered gRNA comprising M1-1 and M2. Here, M2 is a variant obtained by deleting each of 6 nts, 13 nts, and 21 nts from MS2 (i.e., a region constituting stem 2). (n=3)

FIG. 19 shows results obtained by identifying indel (%) using an engineered gRNA comprising M1-1 and M3. Here, M3 is a variant obtained by deleting each of 7 nts, 14 nts, and 20 nts is deleted from MS3 (i.e., a region constituting stem 1). (n=3)

FIG. 20 shows results obtained by identifying indel (%) using an engineered gRNA comprising M1-1 and M4. (n=3, n.s., not significant)

FIG. 21 shows results obtained identifying indel (%) using an engineered gRNA comprising M1-1 and M4. Here, M4 was designed as U₄VU₆ based on the results of FIG. 20 wherein V is A, C or G. (n=3, n.s., not significant)

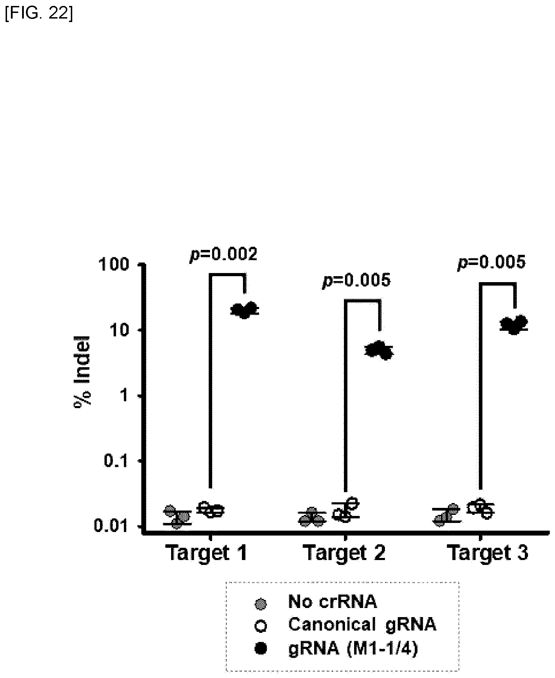

FIG. 22 shows results obtained identifying indel (%) using an engineered gRNA comprising M1-1 and M4.

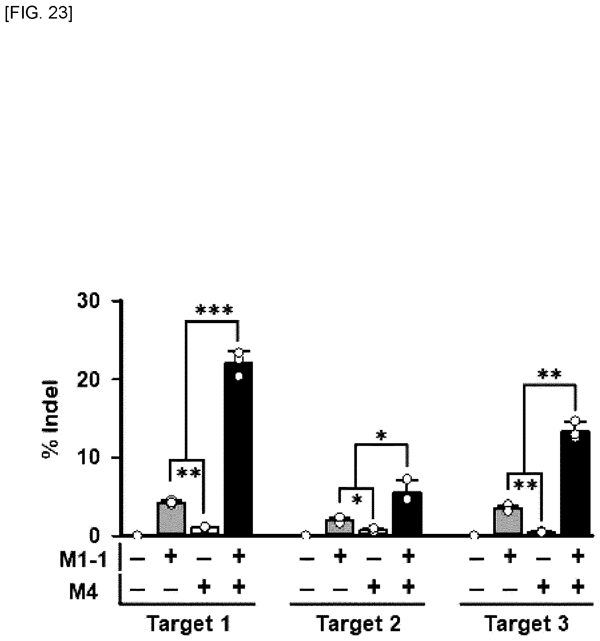

FIG. 23 shows results obtained identifying indel (%) using an engineered gRNA comprising M1-1 and/or M4. An increase of indel (%) was identified when an engineered gRNA comprising both M1-1 and M4 was used. (n=3, *, p<0.05; **, p<0.01; ***, p<0.001. n.s., not significant)

FIG. 24 shows results obtained identifying indel (%) using an engineered gRNA comprising M1-1, M3, and M4. Here, an effect of an engineered gRNA comprising M1-1 and M4 was compared to one to which M3 was added Here, M3 is a variant obtained by each of 12 nts, 13 nts, 14 nts, 15 nts, 16 nts, 17 nts, 18 nts, 19 nts, 20 nts and 21 nts from MS3 (i.e., a region constituting stem 1). (n=3)

FIG. 25 shows results obtained identifying indel (%) using an engineered gRNA comprising M1-1, M1-2, M3, and M4. Here, an effect of an engineered gRNA comprising M1-1, M3 and M4 was compared to one to which M1-2 was added. Here, M1-2 is a variant obtained by deleting each of 10 bp, 12 bp, and 18 bp from MS1 (i.e., a region constituting stem 5). (n=3, n.s., not significant)

FIG. 26 shows results obtained identifying indel (%) using an engineered gRNA comprising M1-1, M1-2, M3, and M4. Here, an effect of an engineered gRNA comprising M1-1, M3 and M4 was compared to one to which M1-2 was added. Here, M1-2 is a variant obtained by each of 1 nts, 3 nts, 5 nts, 7 nts, 9 nts, 11 nts, 13 nts, 15 nts, 17 nts, 19 nts, 21 nts, 23 nts, 25 nts, 27 nts, 29 nts, 31 nts, 33 nts, and 37 nts from MS1 (i.e., a region constituting stem 5). (n=3)

FIG. 27 shows results obtained by additionally modifying an engineered gRNA comprising M1-1, M1-2, M3 and M4, and identifying indel (%). Here, the additional modification is achieved by linking the 3' end of an engineered tracrRNA and the 5' end of an engineered crRNA using a hammerhead ribozyme nucleotide sequence. In the engineered gRNA comprising the hammerhead ribozyme nucleotide sequence, the hammerhead ribozyme nucleotide sequence linked to the 3' end of the engineered tracrRNA is self-cleaved by a hammerhead ribozyme, which allows the engineered gRNA to act as a dual gRNA. (n=3)

FIG. 28 shows results obtained by additionally modifying an engineered gRNA comprising M1-1, M1-2, M2, M3 and/or M4, and identifying indel (%). (n=3)
FIG. 29 shows results obtained by identifying indel pattern using a geCas14a1_3.0 system that is an engineered CRISPR/Cas14a1 system using an engineered gRNA comprising M1-1, M1-2, M3 and M4.

FIG. 30 shows a schematic diagram (A) of an experimental design for investigating the molecular mechanism underlying effects of gRNA engineering (M1-M4) and its results (B and C). For the details of A, reference is made to the section "8. Quantitative analysis of gRNA expression" of Experimental method as described below. B and C show that gRNA engineering (M1-M4) results in a sharp increase in expression of gRNA having a complete sequence.

FIG. 31 shows results obtained by analyzing in vitro digestion of double-stranded DNA (dsDNA) caused by gRNA engineering (M1-M4). In particular, the results indicate that there is a difference in digestion effect of double-stranded DNA (dsDNA) according to the presence or absence of M3.

FIG. 32 shows a table summarizing protospacer sequences at 88 endogenous gene loci randomly selected by searching in silico endogenous targets having the sequence 5'-TTTR-N20-NGG-3' that can be edited by Cas9, Cas12a, and Cas12f1.

FIG. 33 shows a table summarizing protospacer sequences at 88 endogenous gene loci randomly selected by searching in silico endogenous targets having the sequence 5'-TTTR-N20-NGG-3' that can be edited by Cas9, Cas12a, and Cas12f1.

FIG. 34 shows a table summarizing protospacer sequences at 88 endogenous gene loci randomly selected by searching in silico endogenous targets having the sequence 5'-TTTR-N20-NGG-3' that can be edited by Cas9, Cas12a, and Cas12f1.

FIG. 35 shows results obtained by identifying indel (%) at the 88 endogenous loci selected in FIGS. 32 to 34 using CRISPR/SpCas9 system, CRISPR/AsCas12a system, canonical CRISPR/Cas14a1 system, or geCas14a1_3.0 system. A is a schematic diagram of each guide RNA of CRISPR/SpCas9 system, CRISPR/AsCas12a system, canonical CRISPR/Cas14a1 system, and geCas14a1_3.0 system. Here, DR is the rest of the guide RNA except for a spacer sequence. B shows results obtained by identifying indel (%) at the 88 endogenous loci using each of the CRISPR/Cas systems.

FIG. 36 shows diagrams summarizing the results of FIG. 35 in various ways. A is a box-and-whisker plot for indel (%) at the 88 endogenous loci caused by each of the CRISPR/Cas systems, in which the cyan horizontal line represents an average value of each of the CRISPR/Cas systems. B is a table summarizing distribution of the number of targets per a specific indel range which is obtained by subdividing the indel (%) caused by each of the CRISPR/Cas systems. C is a heat map for the indel (%) per target which is obtained by each of the CRISPR/Cas systems.

FIG. 37 shows results of in vitro cleavage assay. Experiments were conducted using a plasmid comprising Intergenic22 (5'-TTTAAGAACACATACCCCTGGGCC-3' (SEQ ID NO: 490)) that is a protospacer sequence, and the plasmid also comprises an Apal restriction enzyme site (5'-GGGCCC-3') for a control. The sequence shown in the drawing is 5'-GGGCGAATTGGGCCCTCTAGATGCATGCTCGAGCGGCCGCCAGTGTGAT GGATATCTGCAGAATTCGCCCTTTGCATTTAAGAACACATACCCCTGGGCC AGGATGCATGCATGCATGCATG-3' (SEQ ID NO: 737), and the Apal restriction enzyme site and the protospacer sequence are underlined.

### MODE OF DISCLOSURE

### Definition of terms

Definitions of terms used in the present specification are as follows.

### Nucleic acid, nucleotide, nucleoside and base

"Nucleic acid" is a biomolecule (or biopolymer) composed of nucleotide units and is also called a polynucleotide. The nucleic acid comprises both DNA and RNA. "Nucleotide" is a unit composed of phosphoric acid, a pentose sugar, and a base (or nucleobase). In RNA (ribonucleic acid), the pentose sugar is ribose, and in DNA (deoxyribonucleic acid), the pentose sugar is deoxyribose. The nucleotide has one selected from adenine (A), guanine (G), cytosine (C), thymine (T) and uracil (U) as a nucleobase. Here, adenine, guanine, and cytosine exist both in RNA and DNA, thymine exists only in DNA, and uracil exists only in RNA. A nucleotide may also be said to be composed of phosphoric acid and a nucleoside. Here, the "nucleoside" consists of a pentose sugar and a nucleobase. The nucleoside is classified into adenosine, thymidine, cytidine, guanosine, and uridine according to the type of nucleobase. Each nucleoside is abbreviated as U (uridine), A (adenosine), T (thymidine), C (cytidine) and G (guanosine). In addition, the nucleotide is abbreviated as U (uridine monophosphate), A (adenosine monophosphate), T (thymidine monophosphate), C (cytidine monophosphate) and G (guanosine monophosphate). In addition, the terms include all meanings recognized by those skilled in the art, and may be appropriately interpreted according to the context.

In the present specification, bases, nucleosides, nucleotides, nucleic acids, RNA, and DNA are abbreviated as A, T, G, C, and U depending on the type of base. The above abbreviation may be appropriately interpreted depending on the context. For example, the sequence 5'-UUUUU-3' may be a sequence of five consecutive bases (uracil), a sequence of five consecutive nucleosides (uridine) and/or a sequence of five consecutive nucleotides (uridine monophosphate). In addition, in the case of a nucleic acid, RNA, and DNA, nucleotides constituting the nucleic acid, RNA, and DNA are abbreviated as uridine, adenosine, thymidine, cytidine and guanosine according to the type of nucleoside. The above abbreviation may be appropriately interpreted depending on the context. For example, RNA comprising a sequence of four consecutive uridines may be interpreted as RNA comprising four consecutive uridine monophosphate nucleotides.

### RNA duplex

"RNA duplex" refers to a double-stranded RNA structure formed by complementary bonds (or bonds between paired bases) between two RNA fragments. Here, the two RNA fragments may exist on different strands or may exist on one strand (single strand). When the two RNA fragments exist on a single strand, some regions (i.e., two RNA fragments) within the single strand form bonds between paired bases to form a stem-loop or hairpin structure. The RNA duplex is also called "RNA stem" or "stem", and is used interchangeably with RNA stem or stem hereinafter. The term includes all meanings recognized by those skilled in the art, and may be appropriately interpreted according to the context.

### Target nucleic acid or target gene

"Target nucleic acid" or "target gene" means a nucleic acid or gene that is a subject to be edited by a CRISPR/Cas12f1 system (or CRISPR/Cas14a1 system). The target nucleic acid or target gene may be a nucleic acid or gene which is present in a cell or artificially synthesized. When present in a cell, the target nucleic acid or target gene may refer to both an endogenous gene or nucleic acid that belongs to the cell, and an exogenous gene or nucleic acid, and is not particularly limited as long as it can be a target to be edited by a CRISPR/Cas12f1 system (or CRISPR/Cas14a1 system). The target nucleic acid or target gene may be single-stranded DNA, double-stranded DNA, single-stranded RNA, double-stranded RNA, and/or DNA-RNA hybrid. The target nucleic acid or target gene may be used interchangeably, and may refer to the same subject. In addition, the term includes all meanings recognized by those skilled in the art, and may be appropriately interpreted according to the context.

### Target sequence, target strand and non-target strand

"Target sequence", which is a sequence present in a target nucleic acid or target gene, refers to a sequence recognized by a guide RNA of a CRISPR/Cas12f1 system (or CRISPR/Cas14a1 system) or a target sequence to be modified by a CRISPR/Cas12f1 system (or CRISPR/Cas14a1 system). Specifically, the target sequence refers to a sequence having complementarity to a guide sequence included in the guide RNA or a sequence complementarily binding to the guide sequence.

"Target strand" refers to a strand comprising a target sequence. When the target nucleic acid or target gene is single-stranded, the strand may be a target strand. Alternatively, when the target nucleic acid or target gene is double-stranded, one of the double-strand may be a target strand, and the other strand may be a strand complementary to the target strand. Here, the strand complementary to the target strand is referred to as a "non-target strand".

The non-target strand comprises a protospacer adjacent motif (PAM) sequence and a protospacer sequence. The PAM sequence is a sequence recognized by a Cas12f1 (or Cas14a1) protein of a CRISPR/Cas12f1 system (or CRISPR/Cas14a1 system). The protospacer sequence, which is located at the 5' end or the 3' end of the PAM sequence, is a sequence having complementarity to a target sequence or a sequence complementarily binding to a target sequence. Correlation between the protospacer sequence and the target sequence is similar to correlation between the target sequence and the guide sequence. Due to these characteristics, in general, a protospacer sequence may be used to design a guide sequence. That is, when designing a guide sequence complementarily binding to a target sequence, the guide sequence may be designed as a nucleotide sequence having the same nucleotide sequence as the protospacer sequence. Here, the guide sequence is designed by replacing T with U in the nucleotide sequence of the protospacer sequence.

### Vector

"Vector", unless otherwise specified, refers collectively to any material capable of transporting a genetic material into a cell. For example, a vector may be a DNA molecule comprising a genetic material of interest, for example, a nucleic acid encoding an effector protein (Cas protein) of a CRISPR/Cas system, and/or a nucleic acid encoding a guide RNA, however, the vector is not limited thereto. The term includes all meanings recognized by those skilled in the art, and may be appropriately interpreted according to the context.

### Operably linked

The term "operably linked" means that a particular component is arranged with another component in a functional relationship, that is, a particular component is linked to another component so that the particular component can perform its intended function. For example, in a case where a promoter sequence is operably linked to a sequence encoding an A protein, the promoter is linked to the sequence encoding the A protein so that the promoter transcribes and/or expresses the sequence encoding the A protein in a cell. In addition, the term includes all meanings recognized by those skilled in the art, and may be appropriately interpreted according to the context.

### Engineered

The term "engineered" is used to distinguish it from a material, a molecule, or the like whose configuration already exists in nature, and this means that the material, a molecule, or the like has undergone artificial modification For example, the "engineered guide RNA" refers to a guide RNA obtained by applying artificial modification to the configuration of a naturally occurring guide RNA. In addition, the term includes all meanings recognized by those skilled in the art, and may be appropriately interpreted according to the context.

### Nuclear localization sequence or signal (NLS) and nuclear export sequence or signal (NES)

In a case where a substance outside the cell's nucleus is transported into the nucleus by nuclear transport, "nuclear localization sequence or signal (NLS)" refers to a peptide of a certain length or a sequence thereof, wherein the peptide is attached to a protein to be transported and acts as a type of "tag". In a case where a substance inside the cell's nucleus is transported outside the nucleus by nuclear transport, "nuclear export sequence or signal (NES)" refers to a peptide of a certain length or a sequence thereof, wherein the peptide is attached to a protein to be transported and acts as a type of "tag". As used herein, the terms "NLS" and "NES" include all meanings recognized by those skilled in the art, and may be appropriately interpreted according to the context.

### Tag

The term "tag" refers collectively to a functional domain added to facilitate tracking and/or separation and purification of a peptide or protein. Specifically, the tag includes, but is not limited to, tag proteins such as a histidine (His) tag, a V5 tag, a FLAG tag, an influenza hemagglutinin (HA) tag, an Myc tag, a VSV-G tag, and a thioredoxin (Trx) tag; fluorescent proteins such as a green fluorescent protein (GFP), a yellow fluorescent protein (YFP), a cyan fluorescent protein (CFP), a blue fluorescent protein (BFP), HcRED, and DsRed; and reporter proteins (enzymes) such as a glutathione-S-transferase (GST), a horseradish peroxidase (HRP), a chloramphenicol acetyltransferase (CAT) beta-galactosidase, a beta-glucuronidase, and a luciferase. As used herein, the term "tag" includes all meanings recognized by those skilled in the art, and may be appropriately interpreted according to the context.

Unless defined otherwise, all technical and scientific terms used herein have the same meanings as commonly understood by those skilled in the art to which the present disclosure belongs. Although methods and materials similar or equivalent to those described herein may be used in practice or experimentation of the present disclosure, suitable methods and materials are described below. All publications, patents, and other references mentioned herein are incorporated by reference in their entireties. Additionally, the materials, methods, and examples are illustrative only and not intended to limit the present disclosure.

### Hereinafter, the present disclosure will be described.

### CRISPR/Cas12f1 system (or CRISPR/Cas14a1 system), its limitations and solutions

A CRISPR/Cas12f1 system is a CRISPR/Cas system classified as Class 2, Type V. A previous study (Harrington et al., Science 362, 839-842 (2018)) has reported for the first time the CRISPR/Cas14 system which is a CRISPR/Cas system derived from Archaea. A subsequent study (Tautvydas Karvelis et al., Nucleic Acids Research 48, 5016-5023 (2020)) classified the CRISPR/Cas14 system as a CRISPR/Cas12f1 system. The CRISPR/Cas12f1 system belongs to a V-F1 system, which is an ortholog of the CRISPR/Cas system classified as Class 2, Type V, and includes the CRISPR/Cas14a1 system having Cas14a1 as an effector protein.

The CRISPR/Cas12f1 system disclosed herein relates to a CRISPR/Cas14a1 system. The CRISPR/Cas12f1 system described herein refers to a CRISPR/Cas14a1 system, and the terms "CRISPR/Cas12f1 system" and "CRISPR/Cas14a1 system" are used interchangeably. The CRISPR/Cas12f1 system is also referred to as a CRISPR/Cas12f1 (Cas14a1) system or a CRISPR/Cas14a1 system.

The CRISPR/Cas12f1 system is characterized in that a size of its effector protein is significantly smaller than a CRISPR/Cas9 system. This characteristic makes it possible to solve the difficulty of loading adeno-associated virus (AAV) caused by a large size of most of previously studied Cas nucleases and the resulting difficulty of application as a gene therapy agent. However, despite these advantages, as revealed in previous studies (Harrington et al., Science 362, 839-842 (2018), Tautvydas Karvelis et al., Nucleic Acids Research 48, 5016-5023 (2020)), the CRISPR/Cas12f1 system, especially the CRISPR/Cas14a1 system, does not show cleavage activity on double-stranded DNA in a cell, or shows cleavage activity with extremely low efficiency, which imposes limitations that it is difficult to actively apply the system for gene editing.

The engineered guide RNA disclosed herein and the CRISPR/Cas14a1 system using the same solve this problem by increasing editing or modifying efficiency of a target nucleic acid or target gene. More specifically, the present inventors artificially engineered a guide RNA to solve a sequence-related problem of a wildtype tracrRNA. The wildtype tracrRNA comprises a sequence of five consecutive uridines (the wildtype tracrRNA has 5'-CUUCACUGAUAAAGUGGAGAACCGCUUCACCAAAAGCUGUCCCUUAGGG GAUUAGAACUUGAGUGAAGGUGGGCUGCUUGCAUCAGCCUAAUGUCGA GAAGUGCUUUCUUCGGAAAGUAACCCUCGAAACAAAUUCAUUUUUCCUC UCCAAUUCUGCACAA-3' (SEQ ID NO: 1)). When the wildtype tracrRNA is expressed in a cell using a vector or the like, a sequence of five consecutive uridines of the wildtype tracrRNA are included in the vector as a sequence of five consecutive thymidines corresponding thereto. Here, the sequence of five consecutive thymidines may act as a transcription termination signal under certain conditions. Such an action as a termination signal inhibits normal expression of the tracrRNA and also inhibits normal formation of a guide RNA. As a result, editing or modifying efficiency of the CRISPR/Cas14a1 system on a target nucleic acid or target gene may be ultimately reduced.

Accordingly, the present inventors developed an engineered tracrRNA in which a sequence of five consecutive uridines of a wildtype tracrRNA is artificially modified. In addition, the present inventors optimized a guide RNA by reducing lengths of the tracrRNA and the crRNA which constitute the guide RNA. The engineered guide RNA of the present disclosure, of which expression and length have been optimized, has advantages such as reduced costs for synthesis of the guide RNA or additional space (capacity) created when inserted into a viral vector, and is effective for gene therapy applications. In addition, a CRISPR/Cas14a1 system using the optimized guide RNA of the present disclosure exhibits increased editing or modifying efficiency on a target nucleic acid or target gene.

Hereinafter, the engineered guide RNA and the engineered CRISPR/Cas14a1 system are described in detail.

### <Engineered guide RNA>

An aspect disclosed by the present specification relates to an engineered guide RNA for a CRISPR/Cas12f1 (Cas14a1) system. The engineered guide RNA allows the CRISPR/Cas12f1 (Cas14a1) system to more effectively perform cleavage, editing or modifying of a target nucleic acid or target gene. In particular, the engineered guide RNA may bring about the following effects:
optimized length of the guide RNA, which in turn creates additional space (capacity) when it is inserted into a viral vector;
reduced synthesis costs caused by optimized length of the guide RNA ;
normal expression of a tracrRNA in cells;
increased expression of the guide RNA that is functional (operable);
increased stability of the guide RNA;
increased stability of a guide RNA-Cas12f1 protein complex;
effective induction of formation of a guide RNA-Cas12f1 protein complex;
increased cleavage efficiency of a CRISPR/Cas12f1 (Cas14a1) system on a target nucleic acid ; and
increased editing or modifying efficiency of a CRISPR/Cas12f1 (Cas14a1) system on a target nucleic acid.

More specifically, the engineered guide RNA is a guide RNA modified not to comprise a sequence of five or more consecutive uridines, and/or modified to have a length shorter than a wildtype tracrRNA, and/or modified to comprise a U-rich tail sequence, and comprises an engineered trans-activating CRISPR RNA (tracrRNA) and a CRISPR RNA (crRNA). Here, the crRNA is a wildtype crRNA or an engineered crRNA. That is, the engineered guide RNA comprises an engineered tracrRNA and a wildtype crRNA, or comprises an engineered tracrRNA and an engineered crRNA.

The engineered tracrRNA does not comprise a sequence of five or more consecutive uridines. Alternatively, the engineered tracrRNA does not comprise a sequence of five or more consecutive uridines and does not comprise a part of a nucleotide sequence at the 5' end and/or the 3' end of a wildtype tracrRNA.

The engineered crRNA comprises an engineered repeat sequence, wherein the engineered repeat sequence is a sequence which is modified to have a shorter length than a wildtype repeat sequence or in which a part of a nucleotide sequence of a wildtype repeat sequence is modified. The wildtype crRNA and the engineered crRNA may optionally further comprise a U-rich tail sequence at the 3' end.

In addition, the engineered guide RNA may optionally further comprise a linker.

The respective components will be described in detail below.

### 1. Engineered tracrRNA

An engineered tracrRNA is an engineered form of a wildtype tracrRNA of which a part of a nucleotide sequence is artificially modified and/or which is modified to have a shorter length.

More specifically, the engineered tracrRNA is a tracrRNA modified not to comprise a sequence of five or more consecutive uridines. Alternatively, the engineered tracrRNA is a tracrRNA that is modified not to comprise a sequence of five or more consecutive uridines and modified to have a shorter length than a wildtype tracrRNA. Here, the engineered tracrRNA comprises a sequence of four or less consecutive uridines. Alternatively, the engineered tracrRNA does not comprise a sequence of five or more consecutive uridines.

The engineered tracrRNA is one selected from:
i) a tracrRNA modified not to comprise a sequence of five or more consecutive uridines;
ii) a tracrRNA that is modified not to comprise a sequence of five or more consecutive uridines and modified not to comprise a part of a nucleotide sequence at the 5' end of a wildtype tracrRNA;
iii) a tracrRNA that is modified not to comprise a sequence of five or more consecutive uridines and modified not to comprise a part of a nucleotide sequence at the 3' end of a wildtype tracrRNA;
iv) a tracrRNA that is modified not to comprise a sequence of five or more consecutive uridines and not to comprise a part of nucleotide sequence in the middle of a wildtype tracrRNA; and
v) a tracrRNA that is modified not to comprise a sequence of five or more consecutive uridines, and modified not to comprise a part of a nucleotide sequence at the 5' end of a wildtype tracrRNA, and/or modified not to comprise a part of nucleotide sequence in the middle of a wildtype tracrRNA, and/or modified not to comprise a part of a nucleotide sequence at the 3' end of a wildtype tracrRNA.

The engineered tracrRNA may interact with a crRNA and/or interact with Cas12f1 (Cas14a1).

The engineered tracrRNA may form at least two or more RNA stems. Here, the RNA stem may interact with a Cas12f1 (Cas14a1) protein.

The engineered tracrRNA is an engineered form of a wildtype tracrRNA of which a region is modified. Here, the region of the wildtype tracrRNA may be one or more of the regions to be modified in the wildtype tracrRNA shown in FIG. 1. In the present specification, the regions to be modified in the wildtype tracrRNA are set by dividing them based on regions forming an RNA duplex (FIG. 1).

The engineered tracrRNA comprises at least three or more sequences selected from a first sequence, a second sequence, a third sequence, a fourth sequence, and a fifth sequence. Here, the at least three or more sequences are a first sequence, a second sequence, and a third sequence. Here, the first sequence, the second sequence, and the third sequence are sequentially located in the engineered tracrRNA in a 3' to 5' direction. Alternatively, the third sequence, the second sequence, and the first sequence are sequentially located in the engineered tracrRNA in a 5' to 3' direction.

The first sequence, the second sequence, the third sequence, the fourth sequence, and the fifth sequence are divided based on the regions to be modified in the wildtype tracrRNA, and each of the sequences forms an RNA duplex or participates in forming an RNA duplex. Accordingly, the engineered tracrRNA may comprise at least two or more RNA duplexes (FIG. 2). Hereinafter, the regions to be modified in the wildtype tracrRNA and each of the sequences thereof will be described in detail.

### Region to be modified in wildtype tracrRNA

The engineered tracrRNA described herein is an engineered form of a wildtype trancrRNA of which a region is modified. The region of the wildtype tracrRNA is at least one of the regions to be modified in the wildtype tracrRNA. Here, the regions to be modified in the wildtype tracrRNA are set by dividing them based on regions, which form an RNA duplex or participate in forming an RNA duplex, in the wildtype tracrRNA. The wildtype tracrRNA forms four RNA duplexes and participates in forming one RNA duplex. That is, the wildtype tracrRNA is involved in formation of a total of five RNA duplexes.

In the present specification, the regions to be modified in the wildtype tracrRNA are divided into a total of 5 regions. The five regions are: region 1 to be modified, region 2 to be modified, region 3 to be modified, region 4 to be modified, and region 5 to be modified.

Here, the region 1 to be modified may be a region from the 130th cytidine (C) to the 161st adenosine (A) from the 5' end of the wildtype tracrRNA. Here, the region 2 to be modified may be a region from the 91st adenosine (A) to the 129th adenosine (A) from the 5' end of the wildtype tracrRNA. Here, the region 3 to be modified may be a region from the 72nd guanosine (G) to the 90th adenosine (A) from the 5' end of the wildtype tracrRNA. Here, the region 4 to be modified may be a region from the 22nd cytidine (C) to the 71st guanosine (G) from the 5' end of the wildtype tracrRNA. Here, the region 5 to be modified may be a region from the 1st cytidine (C) to the 21st adenosine (A) from the 5' end of the wildtype tracrRNA (FIG. 1). These regions to be modified in the wildtype tracrRNA are set to describe respective sequences (a first sequence, a second sequence, a third sequence, a fourth sequence, and a fifth sequence) included in the engineered tracrRNA.

### 1-1) First sequence (one strand for stem 5) - essential sequence

The engineered tracrRNA comprises a first sequence. The first sequence corresponds to the region 1 to be modified in the wildtype tracrRNA (FIG. 2). The first sequence is an essential sequence included in the engineered tracrRNA. The first sequence is a sequence complementarily binding to a partial sequence of a crRNA. Here, the first sequence forms an RNA duplex by complementarily binding to the partial sequence of the crRNA. Here, the RNA duplex formed by the first sequence and the crRNA is referred to as stem 5 in the present specification. At least one nucleotide forming stem 5 may interact with a WED domain and/or a ZF domain of a Cas12f1 protein.

The first sequence does not comprise a sequence of five or more consecutive uridines.

The first sequence comprises a sequence of four or less consecutive uridines. Here, the first sequence may comprise a sequence of four consecutive uridines. Alternatively, the first sequence may comprise a sequence of three consecutive uridines. Alternatively, the first sequence may comprise a sequence of two consecutive uridines. Alternatively, the first sequence may comprise a sequence of one uridine. Alternatively, the first sequence may not comprise a sequence of uridine.

The first sequence comprises at least one nucleotide that forms a complementary bond with a crRNA. Here, the first sequence may comprise at least one nucleotide that does not form a complementary bond with a crRNA. Here, the first sequence may form at least one complementary bond with a crRNA.

The first sequence comprises a nucleotide forming at least one base pairing with a crRNA. Here, the first sequence may comprise at least one nucleotide that does not form base pairing with a crRNA. Here, the first sequence may form at least one base pairing with a crRNA.

The first sequence is located at the 3' end of the engineered tracrRNA.

The first sequence may be one selected from the following sequences:
5'-CAAAUUCA(N)ₐCCUCUCCAAUUCUGCACAA-3' (SEQ ID NO: 2);
5'-CAAAUUCAVNNNN(V)ₐCCUCUCCAAUUCUGCACAA-3' (SEQ ID NO: 3);
5'-CAAAUUCANVNNN(N)_{b}CCUCUCCAAUUCUGCACAA-3' (SEQ ID NO: 4);
5'-CAAAUUCANNVNN(N)_{c}CCUCUCCAAUUCUGCACAA-3' (SEQ ID NO: 5);
5'-CAAAUUCANNNVN(N)_{d}CCUCUCCAAUUCUGCACAA-3' (SEQ ID NO: 6);
5'-CAAAUUCANNNNV(N)ₐCCUCUCCAAUUCUGCACAA-3' (SEQ ID NO: 7); and
a part of a sequence selected from SEQ ID NOS: 2 to 7.

Here, N may be each independently A, C, G or U, and each V may be independently A, C or G.

Here, a may be an integer from 0 to 4, b may be an integer from 0 to 1, c may be an integer from 0 to 2, and d may be an integer from 0 to 3.

Here, in (N)ₐ, (N)_{c} or (N)_{d}, when a, c and d are integers other than 0 or 1, N in (N)ₐ, (N)_{c} or (N)_{d} may each independently be A, C, G or U. For example, when d is 3, (N)₃ refers to 5'-NNN-3' wherein N may each independently be A, C, G, or U.

Here, in (V)ₐ, when a is an integer other than 0 or 1, each V in (V)ₐ may be independently A, C, or G. For example, when a is 4, (V)₄ refers to 5'-VVVV-3 wherein each V may independently be A, C or G.

Here, 5'-CAAAUUCA-3' located at the 5' end of SEQ ID NOS: 2 to 7 and/or 5'-CCUCUCCAAUUCUGCACAA-3' (SEQ ID NO: 8) located at the 3' end thereof may be selectively modified. The modification may be one in which at least one nucleotide in 5'-CAAAUUCA-3' and/or 5'-CCUCUCCAAUUCUGCACAA-3' (SEQ ID NO: 8) is deleted or substituted with another nucleotide. Alternatively, the modification may be one in which one or more nucleotides are inserted into 5'-CAAAUUCA-3' and/or 5'-CCUCUCCAAUUCUGCACAA-3' (SEQ ID NO: 8).

### i) Sequence 5'-CAAAUUCA(N)ₐCCUCUCCAAUUCUGCACAA-3' (SEQ ID NO: 2)

In an embodiment, the first sequence may be 5'-CAAAUUCA(N)ₐCCUCUCCAAUUCUGCACAA-3' (SEQ ID NO: 2).
N may each independently be A, C, G or U.
a may be an integer of 0 to 4.

In an embodiment, the first sequence may be 5'-CAAAUUCACCUCUCCAAUUCUGCACAA-3' (SEQ ID NO: 9).

In another embodiment, the first sequence may be 5'-CAAAUUCANCCUCUCCAAUUCUGCACAA-3' (SEQ ID NO: 10). Here, N may be A, C, G or U. For example, the first sequence may be 5'-CAAAUUCAACCUCUCCAAUUCUGCACAA-3' (SEQ ID NO: 11), 5'-CAAAUUCACCCUCUCCAAUUCUGCACAA-3' (SEQ ID NO: 12), 5'-CAAAUUCAGCCUCUCCAAUUCUGCACAA-3' (SEQ ID NO: 13), or 5'-CAAAUUCAUCCUCUCCAAUUCUGCACAA-3' (SEQ ID NO: 14).

In another embodiment, the first sequence may be 5'-CAAAUUCANNCCUCUCCAAUUCUGCACAA-3' (SEQ ID NO: 15). Here, N may each independently be A, C, G or U. For example, the first sequence may be 5'-CAAAUUCAAUCCUCUCCAAUUCUGCACAA-3' (SEQ ID NO: 16), 5'-CAAAUUCACUCCUCUCCAAUUCUGCACAA-3' (SEQ ID NO: 17), 5'-CAAAUUCAGUCCUCUCCAAUUCUGCACAA-3' (SEQ ID NO: 18), or 5'-CAAAUUCAUUCCUCUCCAAUUCUGCACAA-3' (SEQ ID NO: 19). The examples are for an illustrative purpose only, and the scope of the present disclosure is not limited thereto.

In yet another embodiment, the first sequence may be 5'-CAAAUUCANNNCCUCUCCAAUUCUGCACAA-3' (SEQ ID NO: 20). Here, N may each independently be A, C, G or U. For example, the first sequence may be 5'-CAAAUUCAUUUCCUCUCCAAUUCUGCACAA-3' (SEQ ID NO: 21), 5'-CAAAUUCAGUGCCUCUCCAAUUCUGCACAA-3' (SEQ ID NO: 22), 5'-CAAAUUCAGUUCCUCUCCAAUUCUGCACAA-3' (SEQ ID NO: 23), or 5'-CAAAUUCAUAUCCUCUCCAAUUCUGCACAA-3' (SEQ ID NO: 24). The examples are for an illustrative purpose only, and the scope of the present disclosure is not limited thereto.

In still yet another embodiment, the first sequence may be 5'-CAAAUUCANNNNCCUCUCCAAUUCUGCACAA-3' (SEQ ID NO: 25). Here, N may each independently be A, C, G or U. For example, the first sequence may be 5'-CAAAUUCAUUUUCCUCUCCAAUUCUGCACAA-3' (SEQ ID NO: 26), 5'-CAAAUUCAGUGCCCUCUCCAAUUCUGCACAA-3' (SEQ ID NO: 27), 5'-CAAAUUCAGUUCCCUCUCCAAUUCUGCACAA-3' (SEQ ID NO: 28), 5'-CAAAUUCAGUGUCCUCUCCAAUUCUGCACAA-3' (SEQ ID NO: 29), 5'-CAAAUUCAUUGCCCUCUCCAAUUCUGCACAA-3' (SEQ ID NO: 30), or 5'-CAAAUUCAUUUCCCUCUCCAAUUCUGCACAA-3' (SEQ ID NO: 31). The examples are for an illustrative purpose only, and the scope of the present disclosure is not limited thereto.

### ii) Sequence 5'-CAAAUUCAVNNNN(V)ₐCCUCUCCAAUUCUGCACAA-3' (SEQ ID NO: 3)

In an embodiment, the first sequence may be 5'-CAAAUUCAVNNNN(V)ₐCCUCUCCAAUUCUGCACAA-3' (SEQ ID NO: 3).
N may each independently be A, C, G or U.
Each V can be independently A, C or G.
a may be an integer of 0 to 4.

In an embodiment, the first sequence may be 5'-CAAAUUCAANNNN(V)ₐCCUCUCCAAUUCUGCACAA-3' (SEQ ID NO: 32). Here, N may each independently be A, C, G or U. Each V may be independently A, C or G. a may be an integer of 0 to 4. Here, when a is 0, the first sequence may be 5'-CAAAUUCAANNNNCCUCUCCAAUUCUGCACAA-3' (SEQ ID NO: 33). Alternatively, when a is 1, the first sequence may be 5'-CAAAUUCAANNNNVCCUCUCCAAUUCUGCACAA-3' (SEQ ID NO: 34). Alternatively, when a is 2, the first sequence may be 5'-CAAAUUCAANNNNVVCCUCUCCAAUUCUGCACAA-3' (SEQ ID NO: 35). Alternatively, when a is 3, the first sequence may be 5'-CAAAUUCAANNNNVVVCCUCUCCAAUUCUGCACAA-3' (SEQ ID NO: 36). Alternatively, when a is 4, the first sequence may be 5'-CAAAUUCAANNNNVVVVCCUCUCCAAUUCUGCACAA-3' (SEQ ID NO: 37).

For example, the first sequence may be 5'-CAAAUUCAAUUUUCCUCUCCAAUUCUGCACAA-3' (SEQ ID NO: 38), 5'-CAAAUUCAAUUCUCCUCUCCAAUUCUGCACAA-3' (SEQ ID NO: 39), 5'-CAAAUUCAAUGCUCCUCUCCAAUUCUGCACAA-3' (SEQ ID NO: 40), or 5'-CAAAUUCAAUGCUACCUCUCCAAUUCUGCACAA-3' (SEQ ID NO: 41). The examples are for an illustrative purpose only, and the scope of the present disclosure is not limited thereto.

In another embodiment, the first sequence may be 5'-CAAAUUCACNNNN(V)ₐCCUCUCCAAUUCUGCACAA-3' (SEQ ID NO: 42). Here, N may each independently be A, C, G or U. Each V may be independently A, C or G. a may be an integer of 0 to 4. Here, when a is 0, the first sequence may be 5'-CAAAUUCACNNNNCCUCUCCAAUUCUGCACAA-3' (SEQ ID NO: 43). Alternatively, when a is 1, the first sequence may be 5'-CAAAUUCACNNNNVCCUCUCCAAUUCUGCACAA-3' (SEQ ID NO: 44). Alternatively, when a is 2, the first sequence may be 5'-CAAAUUCACNNNNVVCCUCUCCAAUUCUGCACAA-3' (SEQ ID NO: 45). Alternatively, when a is 3, the first sequence may be 5'-CAAAUUCACNNNNVVVCCUCUCCAAUUCUGCACAA-3' (SEQ ID NO: 46). Alternatively, when a is 4, the first sequence may be 5'-CAAAUUCACNNNNVVVVCCUCUCCAAUUCUGCACAA-3' (SEQ ID NO: 47).

For example, the first sequence may be 5'-CAAAUUCACUUUUCCUCUCCAAUUCUGCACAA-3' (SEQ ID NO: 48), 5'-CAAAUUCACUUCUCCUCUCCAAUUCUGCACAA-3' (SEQ ID NO: 49), 5'-CAAAUUCACUGCUCCUCUCCAAUUCUGCACAA-3' (SEQ ID NO: 50), or 5'-CAAAUUCACUGCUCCCUCUCCAAUUCUGCACAA-3' (SEQ ID NO: 51). The examples are for an illustrative purpose only, and the scope of the present disclosure is not limited thereto.

In another embodiment, the first sequence may be 5'-CAAAUUCAGNNNN(V)ₐCCUCUCCAAUUCUGCACAA-3' (SEQ ID NO: 52). Here, N may each independently be A, C, G or U. Each V may be independently A, C or G. a may be an integer of 0 to 4. Here, when a is 0, the first sequence may be 5'-CAAAUUCAGNNNNCCUCUCCAAUUCUGCACAA-3' (SEQ ID NO: 53). Alternatively, when a is 1, the first sequence may be 5'-CAAAUUCAGNNNNVCCUCUCCAAUUCUGCACAA-3' (SEQ ID NO: 54). Alternatively, when a is 2, the first sequence may be 5'-CAAAUUCAGNNNNVVCCUCUCCAAUUCUGCACAA-3' (SEQ ID NO: 55). Alternatively, when a is 3, the first sequence may be 5'-CAAAUUCAGNNNNVVVCCUCUCCAAUUCUGCACAA-3' (SEQ ID NO: 56). Alternatively, when a is 4, the first sequence may be 5'-CAAAUUCAGNNNNVVVVCCUCUCCAAUUCUGCACAA-3' (SEQ ID NO: 57).

For example, the first sequence may be 5'-CAAAUUCAGUUUUCCUCUCCAAUUCUGCACAA-3' (SEQ ID NO: 58), 5'-CAAAUUCAGUUCUCCUCUCCAAUUCUGCACAA-3' (SEQ ID NO: 59), 5'-CAAAUUCAGUGCUCCUCUCCAAUUCUGCACAA-3' (SEQ ID NO: 60), or 5'-CAAAUUCAGUGCUGCCUCUCCAAUUCUGCACAA-3' (SEQ ID NO: 61). The examples are for an illustrative purpose only, and the scope of the present disclosure is not limited thereto.

### iii) Sequence 5'-CAAAUUCANVNNN(N)_{b}CCUCUCCAAUUCUGCACAA-3' (SEQ ID NO: 4)

In an embodiment, the first sequence may be 5'-CAAAUUCANVNNN(N)_{b}CCUCUCCAAUUCUGCACAA-3' (SEQ ID NO: 4).
N may each independently be A, C, G or U.
V may be A, C or G.
b may be an integer of 0 to 1.

In an embodiment, the first sequence may be 5'-CAAAUUCANANNN(N)_{b}CCUCUCCAAUUCUGCACAA-3' (SEQ ID NO: 62). Here, N may each independently be A, C, G or U. b may be an integer of 0 or 1. Here, when b is 0, the first sequence may be 5'-CAAAUUCANANNNCCUCUCCAAUUCUGCACAA-3' (SEQ ID NO: 63). Alternatively, when b is 1, the first sequence may be 5'-CAAAUUCANANNNNCCUCUCCAAUUCUGCACAA-3' (SEQ ID NO: 64).

For example, the first sequence may be 5'-CAAAUUCAUAUUUCCUCUCCAAUUCUGCACAA-3' (SEQ ID NO: 65), 5'-CAAAUUCAUAUCUCCUCUCCAAUUCUGCACAA-3' (SEQ ID NO: 66), or 5'-CAAAUUCAUAGCUCCUCUCCAAUUCUGCACAA-3' (SEQ ID NO: 67). The examples are for an illustrative purpose only, and the scope of the present disclosure is not limited thereto.

In another embodiment, the first sequence may be 5'-CAAAUUCANCNNN(N)_{b}CCUCUCCAAUUCUGCACAA-3' (SEQ ID NO: 68). Here, N may each independently be A, C, G or U. b may be an integer of 0 or 1. Here, when b is 0, the first sequence may be 5'-CAAAUUCANCNNNCCUCUCCAAUUCUGCACAA-3' (SEQ ID NO: 69). Alternatively, when b is 1, the first sequence may be 5'-CAAAUUCANCNNNNCCUCUCCAAUUCUGCACAA-3' (SEQ ID NO: 70).

For example, the first sequence may be 5'-CAAAUUCAGCUCUCCUCUCCAAUUCUGCACAA-3' (SEQ ID NO: 71), 5'-CAAAUUCAUCUCCCCUCUCCAAUUCUGCACAA-3' (SEQ ID NO: 72), 5'-CAAAUUCAUCUCUCCUCUCCAAUUCUGCACAA-3' (SEQ ID NO: 73), or 5'-CAAAUUCAUCGCUCCUCUCCAAUUCUGCACAA-3' (SEQ ID NO: 74). The examples are for an illustrative purpose only, and the scope of the present disclosure is not limited thereto.

In another embodiment, the first sequence may be 5'-CAAAUUCANGNNN(N)_{b}CCUCUCCAAUUCUGCACAA-3' (SEQ ID NO: 75). Here, N may each independently be A, C, G or U. b may be an integer of 0 or 1. Here, when b is 0, the first sequence may be 5'-CAAAUUCANGNNNCCUCUCCAAUUCUGCACAA-3' (SEQ ID NO: 76). Alternatively, when b is 1, the first sequence may be 5'-CAAAUUCANGNNNNCCUCUCCAAUUCUGCACAA-3' (SEQ ID NO: 77).

For example, the first sequence may be 5'-CAAAUUCAUGUUUCCUCUCCAAUUCUGCACAA-3' (SEQ ID NO: 78), 5'-CAAAUUCAUGUCUCCUCUCCAAUUCUGCACAA-3' (SEQ ID NO: 79), or 5'-CAAAUUCAUGGCUCCUCUCCAAUUCUGCACAA-3' (SEQ ID NO: 80). The examples are for an illustrative purpose only, and the scope of the present disclosure is not limited thereto.

### iv) Sequence CAAAUUCANNVNN(N)_{c}CCUCUCCAAUUCUGCACAA-3' (SEQ ID NO: 5)

In an embodiment, the first sequence may be 5'-CAAAUUCANNVNN(N)_{c}CCUCUCCAAUUCUGCACAA-3' (SEQ ID NO: 5).
N may each independently be A, C, G or U.
V may be A, C or G.
c may be an integer of 0 to 2.

In an embodiment, the first sequence may be 5'-CAAAUUCANNANN(N)_{c}CCUCUCCAAUUCUGCACAA-3' (SEQ ID NO: 81). Here, N may each independently be A, C, G or U. c may be an integer of 0 to 2. Here, when c is 0, the first sequence may be 5'-CAAAUUCANNANNCCUCUCCAAUUCUGCACAA-3' (SEQ ID NO: 82). Alternatively, when c is 1, the first sequence may be 5'-CAAAUUCANNANNNCCUCUCCAAUUCUGCACAA-3' (SEQ ID NO: 83). Alternatively, when c is 2, the first sequence may be 5'-CAAAUUCANNANNNNCCUCUCCAAUUCUGCACAA-3' (SEQ ID NO: 84).

For example, the first sequence may be 5'-CAAAUUCAUUAUUCCUCUCCAAUUCUGCACAA-3' (SEQ ID NO: 85), 5'-CAAAUUCAUUACUCCUCUCCAAUUCUGCACAA-3' (SEQ ID NO: 86), or 5'-CAAAUUCAUCACUCCUCUCCAAUUCUGCACAA-3' (SEQ ID NO: 87). The examples are for an illustrative purpose only, and the scope of the present disclosure is not limited thereto.

In another embodiment, the first sequence may be 5'-CAAAUUCANNCNN(N)_{c}CCUCUCCAAUUCUGCACAA-3' (SEQ ID NO: 88). Here, N may each independently be A, C, G or U. c may be an integer of 0 to 2. Here, when c is 0, the first sequence may be 5'-CAAAUUCANNCNNCCUCUCCAAUUCUGCACAA-3' (SEQ ID NO: 89). Alternatively, when c is 1, the first sequence may be 5'-CAAAUUCANNCNNNCCUCUCCAAUUCUGCACAA-3' (SEQ ID NO: 90). Alternatively, when c is 2, the first sequence may be 5'-CAAAUUCANNCNNNNCCUCUCCAAUUCUGCACAA-3' (SEQ ID NO: 91).

For example, the first sequence may be 5'-CAAAUUCAUUCUUCCUCUCCAAUUCUGCACAA-3' (SEQ ID NO: 92), 5'-CAAAUUCAUUCCUCCUCUCCAAUUCUGCACAA-3' (SEQ ID NO: 93), or 5'-CAAAUUCAGUCUUCCUCUCCAAUUCUGCACAA-3' (SEQ ID NO: 94). The examples are for an illustrative purpose only, and the scope of the present disclosure is not limited thereto.

In another embodiment, the first sequence may be 5'-CAAAUUCANNGNN(N)_{c}CCUCUCCAAUUCUGCACAA-3' (SEQ ID NO: 95). Here, N may each independently be A, C, G or U. c may be an integer of 0 to 2. Here, when c is 0, the first sequence may be 5'-CAAAUUCANNGNNCCUCUCCAAUUCUGCACAA-3' (SEQ ID NO: 96). Alternatively, when c is 1, the first sequence may be 5'-CAAAUUCANNGNNNCCUCUCCAAUUCUGCACAA-3' (SEQ ID NO: 97). Alternatively, when c is 2, the first sequence may be 5'-CAAAUUCANNGNNNNCCUCUCCAAUUCUGCACAA-3' (SEQ ID NO: 98).

For example, the first sequence may be 5'-CAAAUUCAUUGCUCCUCUCCAAUUCUGCACAA-3' (SEQ ID NO: 99), 5'-CAAAUUCAUCGCCCCUCUCCAAUUCUGCACAA-3' (SEQ ID NO: 100), or 5'-CAAAUUCAGCGCCCCUCUCCAAUUCUGCACAA-3' (SEQ ID NO: 101). The examples are for an illustrative purpose only, and the scope of the present disclosure is not limited thereto.

### v) Sequence of 5'-CAAAUUCANNNVN(N)_{d}CCUCUCCAAUUCUGCACAA-3' (SEQ ID NO: 6)

In an embodiment, the first sequence may be 5'-CAAAUUCANNNVN(N)_{d}CCUCUCCAAUUCUGCACAA-3' (SEQ ID NO: 6).
N may each independently be A, C, G or U.
V may be A, C or G.
d may be an integer of 0 to 3.

In an embodiment, the first sequence may be 5'-CAAAUUCANNNAN(N)_{d}CCUCUCCAAUUCUGCACAA-3' (SEQ ID NO: 102). Here, N may each independently be A, C, G or U. d may be an integer of 0 to 3. Here, when d is 0, the first sequence may be 5'-CAAAUUCANNNANCCUCUCCAAUUCUGCACAA-3' (SEQ ID NO: 103). Alternatively, when d is 1, the first sequence may be 5'-CAAAUUCANNNANNCCUCUCCAAUUCUGCACAA-3' (SEQ ID NO: 104). Alternatively, when d is 2, the first sequence may be 5'-CAAAUUCANNNANNNCCUCUCCAAUUCUGCACAA-3' (SEQ ID NO: 105). Alternatively, when d is 3, the first sequence may be 5'-CAAAUUCANNNANNNNCCUCUCCAAUUCUGCACAA-3' (SEQ ID NO: 106).

For example, the first sequence may be 5'-CAAAUUCAUUUAUCCUCUCCAAUUCUGCACAA-3' (SEQ ID NO: 107), 5'-CAAAUUCAGUUAUCCUCUCCAAUUCUGCACAA-3' (SEQ ID NO: 108), or 5'-CAAAUUCAUUGAUCCUCUCCAAUUCUGCACAA-3' (SEQ ID NO: 109). The examples are for an illustrative purpose only, and the scope of the present disclosure is not limited thereto.

In another embodiment, the first sequence may be 5'-CAAAUUCANNNCN(N)_{d}CCUCUCCAAUUCUGCACAA-3' (SEQ ID NO: 110). Here, N may each independently be A, C, G or U. d may be an integer of 0 to 3. Here, when d is 0, the first sequence may be 5'-CAAAUUCANNNCNCCUCUCCAAUUCUGCACAA-3' (SEQ ID NO: 111). Alternatively, when d is 1, the first sequence may be 5'-CAAAUUCANNNCNNCCUCUCCAAUUCUGCACAA-3' (SEQ ID NO: 112). Alternatively, when d is 2, the first sequence may be 5'-CAAAUUCANNNCNNNCCUCUCCAAUUCUGCACAA-3' (SEQ ID NO: 113). Alternatively, when d is 3, the first sequence may be 5'-CAAAUUCANNNCNNNNCCUCUCCAAUUCUGCACAA-3' (SEQ ID NO: 114).

For example, the first sequence may be 5'-CAAAUUCAUUUCUCCUCUCCAAUUCUGCACAA-3' (SEQ ID NO: 115), 5'-CAAAUUCAGUGCCCCUCUCCAAUUCUGCACAA-3' (SEQ ID NO: 116), 5'-CAAAUUCAGCGCUCCUCUCCAAUUCUGCACAA-3' (SEQ ID NO: 117), 5'-CAAAUUCAUUGCCCCUCUCCAAUUCUGCACAA-3' (SEQ ID NO: 118), or 5'-CAAAUUCAGCUCCCCUCUCCAAUUCUGCACAA-3' (SEQ ID NO: 119). The examples are for an illustrative purpose only, and the scope of the present disclosure is not limited thereto.

In another embodiment, the first sequence may be 5'-CAAAUUCANNNGN(N)_{d}CCUCUCCAAUUCUGCACAA-3' (SEQ ID NO: 120). Here, N may each independently be A, C, G or U. d may be an integer of 0 to 3. Here, when d is 0, the first sequence may be 5'-CAAAUUCANNNGNCCUCUCCAAUUCUGCACAA-3' (SEQ ID NO: 121). Alternatively, when d is 1, the first sequence may be 5'-CAAAUUCANNNGNNCCUCUCCAAUUCUGCACAA-3' (SEQ ID NO: 122). Alternatively, when d is 2, the first sequence may be 5'-CAAAUUCANNNGNNNCCUCUCCAAUUCUGCACAA-3' (SEQ ID NO: 123). Alternatively, when d is 3, the first sequence may be 5'-CAAAUUCANNNGNNNNCCUCUCCAAUUCUGCACAA-3' (SEQ ID NO: 124).

For example, the first sequence may be 5'-CAAAUUCAUUUGUCCUCUCCAAUUCUGCACAA-3' (SEQ ID NO: 125), 5'-CAAAUUCAUUGGUCCUCUCCAAUUCUGCACAA-3' (SEQ ID NO: 126), or 5'-CAAAUUCAGUCGUCCUCUCCAAUUCUGCACAA-3' (SEQ ID NO: 127). The examples are for an illustrative purpose only, and the scope of the present disclosure is not limited thereto.

### vi) Sequence of 5'-CAAAUUCANNNNV(N)ₐCCUCUCCAAUUCUGCACAA-3' (SEQ ID NO: 7)

In an embodiment, the first sequence may be 5'-CAAAUUCANNNNV(N)ₐCCUCUCCAAUUCUGCACAA-3' (SEQ ID NO: 7).
N may each independently be A, C, G or U.
V may be A, C or G.
a may be an integer of 0 to 4.

In an embodiment, the first sequence may be 5'-CAAAUUCANNNNA(N)ₐCCUCUCCAAUUCUGCACAA-3' (SEQ ID NO: 128). Here, N may each independently be A, C, G or U. a may be an integer of 0 to 4. Here, when a is 0, the first sequence may be 5'-CAAAUUCANNNNACCUCUCCAAUUCUGCACAA-3' (SEQ ID NO: 129). Alternatively, when a is 1, the first sequence may be 5'-CAAAUUCANNNNANCCUCUCCAAUUCUGCACAA-3' (SEQ ID NO: 130). Alternatively, when a is 2, the first sequence may be 5'-CAAAUUCANNNNANNCCUCUCCAAUUCUGCACAA-3' (SEQ ID NO: 131). Alternatively, when a is 3, the first sequence may be 5'-CAAAUUCANNNNANNNCCUCUCCAAUUCUGCACAA-3' (SEQ ID NO: 132). Alternatively, when a is 4, the first sequence may be 5'-CAAAUUCANNNNANNNNCCUCUCCAAUUCUGCACAA-3' (SEQ ID NO: 133).

For example, the first sequence may be 5'-CAAAUUCAUUUUACCUCUCCAAUUCUGCACAA-3' (SEQ ID NO: 134), 5'-CAAAUUCAUUUCACCUCUCCAAUUCUGCACAA-3' (SEQ ID NO: 135), or 5'-CAAAUUCAUUGCACCUCUCCAAUUCUGCACAA-3' (SEQ ID NO: 136). The examples are for an illustrative purpose only, and the scope of the present disclosure is not limited thereto.

In another embodiment, the first sequence may be 5'-CAAAUUCANNNNC(N)ₐCCUCUCCAAUUCUGCACAA-3' (SEQ ID NO: 137). Here, N may each independently be A, C, G or U. a may be an integer of 0 to 4. Here, when a is 0, the first sequence may be 5'-CAAAUUCANNNNCCCUCUCCAAUUCUGCACAA-3' (SEQ ID NO: 138). Alternatively, when a is 1, the first sequence may be 5'-CAAAUUCANNNNCNCCUCUCCAAUUCUGCACAA-3' (SEQ ID NO: 139). Alternatively, when a is 2, the first sequence may be 5'-CAAAUUCANNNNCNNCCUCUCCAAUUCUGCACAA-3' (SEQ ID NO: 140). Alternatively, when a is 3, the first sequence may be 5'-CAAAUUCANNNNCNNNCCUCUCCAAUUCUGCACAA-3' (SEQ ID NO: 141). Alternatively, when a is 4, the first sequence may be 5'-CAAAUUCANNNNCNNNNCCUCUCCAAUUCUGCACAA-3' (SEQ ID NO: 142).

For example, the first sequence may be 5'-CAAAUUCAUCUCCCCUCUCCAAUUCUGCACAA-3' (SEQ ID NO: 143), 5'-CAAAUUCAGUUCCCCUCUCCAAUUCUGCACAA-3' (SEQ ID NO: 144), or 5'-CAAAUUCAUUUCCCCUCUCCAAUUCUGCACAA-3' (SEQ ID NO: 145). The examples are for an illustrative purpose only, and the scope of the present disclosure is not limited thereto.

In another embodiment, the first sequence may be 5'-CAAAUUCANNNNG(N)ₐCCUCUCCAAUUCUGCACAA-3' (SEQ ID NO: 146). Here, N may each independently be A, C, G or U. a may be an integer of 0 to 4. Here, when a is 0, the first sequence may be 5'-CAAAUUCANNNNGCCUCUCCAAUUCUGCACAA-3' (SEQ ID NO: 147). Alternatively, when a is 1, the first sequence may be 5'-CAAAUUCANNNNGNCCUCUCCAAUUCUGCACAA-3' (SEQ ID NO: 148). Alternatively, when a is 2, the first sequence may be 5'-CAAAUUCANNNNGNNCCUCUCCAAUUCUGCACAA-3' (SEQ ID NO: 149). Alternatively, when a is 3, the first sequence may be 5'-CAAAUUCANNNNGNNNCCUCUCCAAUUCUGCACAA-3' (SEQ ID NO: 150). Alternatively, when a is 4, the first sequence may be 5'-CAAAUUCANNNNGNNNNCCUCUCCAAUUCUGCACAA-3' (SEQ ID NO: 151).

For example, the first sequence may be 5'-CAAAUUCAUUUCGCCUCUCCAAUUCUGCACAA-3' (SEQ ID NO: 152), 5'-CAAAUUCAGUGCGCCUCUCCAAUUCUGCACAA-3' (SEQ ID NO: 153), or 5'-CAAAUUCAGUUCGCCUCUCCAAUUCUGCACAA-3' (SEQ ID NO: 154). The examples are for an illustrative purpose only, and the scope of the present disclosure is not limited thereto.

### vii) Part of any one sequence selected from SEQ ID NOS:2 to 7

In an embodiment, the first sequence may be a part of 5'-CAAAUUCA(N)ₐCCUCUCCAAUUCUGCACAA-3' (SEQ ID NO: 2), and may comprise 5'-CAAAUUCA(N)ₐ-3' in SEQ ID NO: 2 while not comprising a sequential partial sequence at the 3' end of SEQ ID NO: 2. Here, N may each independently be A, C, G or U. a may be an integer of 0 to 4.

In an embodiment, the first sequence may be a part of 5'-CAAAUUCANNNNCCUCUCCAAUUCUGCACAA-3' (SEQ ID NO: 25), and may comprise 5'-CAAAUUCANNNN-3' (SEQ ID NO: 155) while not comprising a partial sequence at the 3' end of SEQ ID NO: 25. For example, the first sequence may be 5'-CAAAUUCANNNNCCUCUCCAAUUCUGCAC-3' (SEQ ID NO: 156), 5'-CAAAUUCANNNNCCUCUCCAAUUCUGC-3' (SEQ ID NO: 157), 5'-CAAAUUCANNNNCCUCUCCAAUUCU-3' (SEQ ID NO: 158), 5'-CAAAUUCANNNNCCUCUCCAAUU-3' (SEQ ID NO: 159), 5'-CAAAUUCANNNNCCUCUCCAA-3' (SEQ ID NO: 160), 5'-CAAAUUCANNNNCCUCUCC-3' (SEQ ID NO: 161), 5'-CAAAUUCANNNNCCUCU-3' (SEQ ID NO: 162), 5'-CAAAUUCANNNNCCU-3' (SEQ ID NO: 163), 5'-CAAAUUCANNNNC-3' (SEQ ID NO: 164), or 5'-CAAAUUCANNNN-3' (SEQ ID NO: 155). Here, N may each independently be A, C, G or U. The examples are for an illustrative purpose only, and the scope of the present disclosure is not limited thereto.

In another embodiment, the first sequence may be a part of 5'-CAAAUUCAVNNNN(V)ₐCCUCUCCAAUUCUGCACAA-3' (SEQ ID NO: 3), and may comprise 5'-CAAAUUCAVNNNN-3' (SEQ ID NO: 165) in SEQ ID NO: 3 while not comprising a sequential partial sequence at the 3' end of SEQ ID NO: 3. Here, N may each independently be A, C, G or U. V may be A, C or G. a may be an integer of 0 to 4.

In an embodiment, the first sequence may be a part of 5'-CAAAUUCAVNNNNCCUCUCCAAUUCUGCACAA-3' (SEQ ID NO: 166), and may comprise 5'-CAAAUUCAVNNNN-3' (SEQ ID NO: 165) while not comprising a partial sequence at the 3' end of SEQ ID NO: 166. For example, the first sequence may be 5'-CAAAUUCAVNNNNCCUCUCCAAUUCUGCA-3' (SEQ ID NO: 167), 5'-CAAAUUCAVNNNNCCUCUCCAAUUCUG-3' (SEQ ID NO: 168), 5'-CAAAUUCAVNNNNCCUCUCCAAUUCU-3' (SEQ ID NO: 169), 5'-CAAAUUCAVNNNNCCUCUCCAAUU-3' (SEQ ID NO: 170), 5'-CAAAUUCAVNNNNCCUCUCCAA-3' (SEQ ID NO: 171), 5'-CAAAUUCAVNNNNCCUCUCC-3' (SEQ ID NO: 172), 5'-CAAAUUCAVNNNNCCUCU-3' (SEQ ID NO: 173), 5'-CAAAUUCAVNNNNCCU-3' (SEQ ID NO: 174), or 5'-CAAAUUCAVNNNN-3' (SEQ ID NO: 165). Here, N may each independently be A, C, G or U. The examples are for an illustrative purpose only, and the scope of the present disclosure is not limited thereto.

In another embodiment, the first sequence may be a part of 5'-CAAAUUCANVNNN(N)_{b}CCUCUCCAAUUCUGCACAA -3' (SEQ ID NO: 4), and may comprise 5'-CAAAUUCANVNNN-3' (SEQ ID NO: 175) in SEQ ID NO: 4 while not comprising a sequential partial sequence at the 3' end of SEQ ID NO: 4. Here, N may each independently be A, C, G or U. V may be A, C or G. b may be an integer of 0 to 1.

In an embodiment, the first sequence may be a part of 5'-CAAAUUCANVNNNCCUCUCCAAUUCUGCACAA -3' (SEQ ID NO: 176), and may comprise 5'-CAAAUUCANVNNN-3' (SEQ ID NO: 175) while not comprising a partial sequence at the 3' end of SEQ ID NO: 176. For example, the first sequence may be 5'-CAAAUUCANVNNNCCUCUCCAAUUCUGCA-3' (SEQ ID NO: 177), 5'-CAAAUUCANVNNNCCUCUCCAAUUCU-3' (SEQ ID NO: 178), 5'-CAAAUUCANVNNNCCUCUCCAAUU-3' (SEQ ID NO: 179), 5'-CAAAUUCANVNNNCCUCUCCAA-3' (SEQ ID NO: 180), 5'-CAAAUUCANVNNNCCUCUCC-3' (SEQ ID NO: 181), 5'-CAAAUUCANVNNNCCUCU-3' (SEQ ID NO: 182), 5'-CAAAUUCANVNNNCC-3' (SEQ ID NO: 183), or 5'-CAAAUUCANVNNN-3' (SEQ ID NO: 175). Here, N may each independently be A, C, G or U. The examples are for an illustrative purpose only, and the scope of the present disclosure is not limited thereto.

In another embodiment, the first sequence may be a part of 5'-CAAAUUCANNVNN(N)_{c}CCUCUCCAAUUCUGCACAA-3' (SEQ ID NO: 5), and may comprise 5'-CAAAUUCANNVNN-3' (SEQ ID NO: 184) in SEQ ID NO: 5 while not comprising a sequential partial sequence at the 3' end of SEQ ID NO: 5. Here, N may each independently be A, C, G or U. V may be A, C or G. c may be an integer of 0 to 2.

In an embodiment, the first sequence may be a part of 5'-CAAAUUCANNVNNCCUCUCCAAUUCUGCACAA-3' (SEQ ID NO: 185), and may comprise 5'-CAAAUUCANNVNN-3' (SEQ ID NO: 184) while not comprising a partial sequence at the 3' end of SEQ ID NO: 185. For example, the first sequence may be 5'-CAAAUUCANNVNNCCUCUCCAAUUCUGCA-3' (SEQ ID NO: 186), 5'-CAAAUUCANNVNNCCUCUCCAAUUCU-3' (SEQ ID NO: 187), 5'-CAAAUUCANNVNNCCUCUCCAAUU-3' (SEQ ID NO: 188), 5'-CAAAUUCANNVNNCCUCUCCAA-3' (SEQ ID NO: 189), 5'-CAAAUUCANNVNNCCUCUCC-3' (SEQ ID NO: 190), 5'-CAAAUUCANNVNNCCUCU-3' (SEQ ID NO: 191), 5'-CAAAUUCANNVNNCC-3' (SEQ ID NO: 192), or 5'-CAAAUUCANNVNN-3' (SEQ ID NO: 184). Here, N may each independently be A, C, G or U. The examples are for an illustrative purpose only, and the scope of the present disclosure is not limited thereto.

In another embodiment, the first sequence may be a part of 5'-CAAAUUCANNNVN(N)_{d}CCUCUCCAAUUCUGCACAA-3' (SEQ ID NO: 6), and may comprise 5'-CAAAUUCANNNVN-3' (SEQ ID NO: 193) in SEQ ID NO: 6 while not comprising a sequential partial sequence at the 3' end of SEQ ID NO: 6. Here, N may each independently be A, C, G or U. V may be A, C or G. d may be an integer of 0 to 3.

In an embodiment, the first sequence may be a part of 5'-CAAAUUCANNNVNCCUCUCCAAUUCUGCACAA-3' (SEQ ID NO: 194), and may comprise 5'-CAAAUUCANNNVN-3' (SEQ ID NO: 193) while not comprising a partial sequence at the 3' end of SEQ ID NO: 194. For example, the first sequence may be 5'-CAAAUUCANNNVNCCUCUCCAAUUCUGCA-3' (SEQ ID NO: 195), 5'-CAAAUUCANNNVNCCUCUCCAAUUCU-3' (SEQ ID NO: 196), 5'-CAAAUUCANNNVNCCUCUCCAAUU-3' (SEQ ID NO: 197), 5'-CAAAUUCANNNVNCCUCUCCAA-3' (SEQ ID NO: 198), 5'-CAAAUUCANNNVNCCUCUCC-3' (SEQ ID NO: 199), 5'-CAAAUUCANNNVNCCUCU-3' (SEQ ID NO: 200), 5'-CAAAUUCANNNVNCC-3' (SEQ ID NO: 201), or 5'-CAAAUUCANNNVN-3' (SEQ ID NO: 193). Here, N may each independently be A, C, G or U. The examples are for an illustrative purpose only, and the scope of the present disclosure is not limited thereto.

In another embodiment, the first sequence may be a part of 5'-CAAAUUCANNNNV(N)aCCUCUCCAAUUCUGCACAA-3' (SEQ ID NO: 7), and may comprise 5'-CAAAUUCANNNNV-3' (SEQ ID NO: 202) in SEQ ID NO: 7 while not comprising a sequential partial sequence at the 3' end of SEQ ID NO: 7. Here, N may each independently be A, C, G or U. V may be A, C or G. a may be an integer of 0 to 4.

In an embodiment, the first sequence may be a part of 5'-CAAAUUCANNNNVCCUCUCCAAUUCUGCACAA-3' (SEQ ID NO: 203), and may comprise 5'-CAAAUUCANNNNV-3' (SEQ ID NO: 202) while not comprising a partial sequence at the 3' end of SEQ ID NO: 203. For example, the first sequence may be 5'-CAAAUUCANNNNVCCUCUCCAAUUCUGCA-3' (SEQ ID NO: 204), 5'-CAAAUUCANNNNVCCUCUCCAAUUCU-3' (SEQ ID NO: 205), 5'-CAAAUUCANNNNVCCUCUCCAAUU-3' (SEQ ID NO: 206), 5'-CAAAUUCANNNNVCCUCUCCAA-3' (SEQ ID NO: 207), 5'-CAAAUUCANNNNVCCUCUCC-3' (SEQ ID NO: 208), 5'-CAAAUUCANNNNVCCUCU-3' (SEQ ID NO: 209), 5'-CAAAUUCANNNNVCC-3' (SEQ ID NO: 210), or 5'-CAAAUUCANNNNV-3' (SEQ ID NO: 202). Here, N may each independently be A, C, G or U. The examples are for an illustrative purpose only, and the scope of the present disclosure is not limited thereto.

### 1-2) Second sequence (stem 4) - essential sequence

The engineered tracrRNA comprises a second sequence. The first sequence corresponds to the region 2 to be modified in the wildtype tracrRNA (FIG. 2). The second sequence is an essential sequence included in the engineered tracrRNA. The second sequence forms an RNA duplex through complementary binding therein. Here, the RNA duplex formed in the second sequence is referred to as stem 4 in the present specification. At least one nucleotide forming the stem 4 may interact with a REC domain and/or a RuvC domain of a Cas12f1 protein.

The second sequence does not comprise a sequence of five or more consecutive uridines.

The second sequence is located at the 5' end of the first sequence.

The second sequence is covalently linked to the 5' end of the first sequence.

In an embodiment, the second sequence may be 5'-AUGUCGAGAAGUGCUUUCUUCGGAAAGUAACCCUCGAAA-3' (SEQ ID NO: 211).

In another embodiment, the second sequence may be a sequence at least 70% identical or similar to SEQ ID NO: 211. Alternatively, the second sequence may be a sequence having sequence identity or sequence similarity of at least 70% or more to SEQ ID NO: 211.

In another embodiment, the second sequence may be a sequence identical or similar to SEQ ID NO: 211 by at least 70% to 75%, at least 70% to 80%, at least 70% to 85%, at least 70% to 90%, at least 70% to 95%, at least 70% to 100%, at least 75% to 80%, at least 75% to 85%, at least 75% to 90%, at least 75% to 95%, or at least 75% to 100%. Alternatively, the second sequence may be a sequence having sequence identity or sequence similarity of at least 70% to 75%, at least 70% to 80%, at least 70% to 85%, at least 70% to 90%, at least 70% to 95%, at least 70% to 100%, at least 75% to 80%, at least 75% to 85%, at least 75% to 90%, at least 75% to 95%, or at least 75% to 100% to SEQ ID NO: 211. Alternatively, the second sequence may be a sequence identical or similar to SEQ ID NO: 211 by at least 80% to 85%, at least 80% to 90%, at least 80% to 95%, at least 80% to 100%, at least 85% to 90%, at least 85% to 95%, or at least 85% to 100%. Alternatively, the second sequence may be a sequence having sequence identity or sequence similarity of at least 80% to 85%, at least 80% to 90%, at least 80% to 95%, at least 80% to 100%, at least 85% to 90%, at least 85% to 95%, or at least 85% to 100% to SEQ ID NO: 211. Alternatively, the second sequence may be a sequence identical or similar to SEQ ID NO: 211 by at least 90% to 95%, at least 90% to 100%, or at least 95% to 100%. Alternatively, the second sequence may be a sequence having sequence identity or sequence similarity of at least 90% to 95%, at least 90% to 100%, or at least 95% to 100% to SEQ ID NO: 211.

In another embodiment, the second sequence may be a sequence identical or similar to SEQ ID NO: 211 by at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100%. Alternatively, the second sequence may have sequence identity or sequence similarity of 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% to SEQ ID NO: 211.

### 1-3) Third sequence (stem 3) - essential sequence

The engineered tracrRNA comprises a third sequence. The first sequence corresponds to the region 3 to be modified in the wildtype tracrRNA (FIG. 2). The third sequence is an essential sequence included in the engineered tracrRNA. The third sequence is a sequence binding complementarily to a part of a crRNA. Here, the third sequence forms an RNA duplex by binding complementarily to a part of a crRNA. In addition, the third sequence forms an RNA duplex through complementary binding therein. Here, the RNA duplex formed by complementary binding between the third sequence and the part of the crRNA and complementary binding in the third sequence is named stem 3 in the present specification. At least one nucleotide forming stem 3 may interact with a WED domain and/or a RuvC domain of a Cas12f1 protein.

The third sequence comprises at least one nucleotide that forms a complementary bond to a crRNA. Here, the third sequence may comprise at least one nucleotide that does not form a complementary bond to a crRNA. Here, the third sequence may form at least one complementary bond with a crRNA.

The third sequence comprises a nucleotide forming at least one base pair with a crRNA. Here, the third sequence may comprise a nucleotide not involved in forming at least one base pair with a crRNA. Here, the third sequence may form at least one base pairing with a crRNA.

The third sequence does not comprise a sequence of five or more consecutive uridines.

The third sequence is located at the 5' end of the second sequence.

The third sequence is covalently bonded to the 5' end of the second sequence.

In an embodiment, the third sequence may be 5'-GGCUGCUUGCAUCAGCCUA-3' (SEQ ID NO: 212).

In another embodiment, the third sequence may be a sequence at least 70% or more identical or similar to SEQ ID NO: 212. Alternatively, the third sequence may be a sequence having sequence identity or sequence similarity of at least 70% or more to SEQ ID NO: 212.

In another embodiment, the third sequence may be a sequence identical or similar to SEQ ID NO: 212 by at least 70% to 75%, at least 70% to 80%, at least 70% to 85%, at least 70% to 90%, at least 70% to 95%, at least 70% to 100%, at least 75% to 80%, at least 75% to 85%, at least 75% to 90%, at least 75% to 95% or at least 75% to 100%. Alternatively, the third sequence may be a sequence having sequence identity or sequence similarity to SEQ ID NO: 212 of at least 70% to 75%, at least 70% to 80%, at least 70% to 85%, at least 70% to 90%, at least 70% to 95%, at least 70% to 100%, at least 75% to 80%, at least 75% to 85%, at least 75% to 90%, at least 75% to 95% or at least 75% to 100%. Alternatively, the third sequence may be a sequence identical or similar to SEQ ID NO: 212 by at least 80% to 85%, at least 80% to 90%, at least 80% to 95%, at least 80% to 100%, at least 85% to 90%, at least 85% to 95%, or at least 85% to 100%. Alternatively, the third sequence may be a sequence having sequence identity or sequence similarity to SEQ ID NO: 212 of at least 80% to 85%, at least 80% to 90%, at least 80% to 95%, at least 80% to 100%, at least 85% to 90%, at least 85% to 95%, or at least 85% to 100%. Alternatively, the third sequence may be a sequence identical or similar to SEQ ID NO: 212 by at least 90% to 95%, at least 90% to 100%, or at least 95% to 100%. Alternatively, the third sequence may be a sequence having sequence identity or sequence similarity to SEQ ID NO: 212 of at least 90% to 95%, at least 90% to 100%, or at least 95% to 100%.

In another embodiment, the third sequence may be a sequence identical or similar to SEQ ID NO: 212 by at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100%. Alternatively, the third sequence is a sequence having sequence identity or sequence similarity of 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% to SEQ ID NO: 212.

### 1-4) Fourth sequence (stem 2)

The engineered tracrRNA may comprise a fourth sequence. The first sequence corresponds to the region 4 to be modified in the wildtype tracrRNA (FIG. 2). The fourth sequence is a nucleotide sequence that may be modified in various ways, and may be a sequence of 1 to 50 nucleotides (a sequence of 1 to 50 nts). The fourth sequence is located at the 5' end of the third sequence, which is an essential sequence of the engineered tracrRNA. The fourth sequence forms an RNA duplex through complementary binding therein. Here, the RNA duplex formed in the fourth sequence is referred to as stem 2 in the present specification. At least one nucleotide forming stem 2 may interact with a RuvC domain of a Cas12f1 protein.

The fourth sequence does not comprise a sequence of five or more consecutive uridines.

The fourth sequence is covalently linked to the 5' end of the third sequence.

In an embodiment, the fourth sequence may be 5'-CCGCUUCACCAAAAGCUGUCCCUUAGGGGAUUAGAACUUGAGUGAAGGU G-3' (SEQ ID NO: 213). Here, the fourth sequence is capable of forming an RNA duplex through complementary binding therein, and the formed RNA duplex is referred to as wildtype stem 2.

In another embodiment, the fourth sequence is a part of SEQ ID NO: 213, and may be a sequence modified so that it has shorter wildtype stem 2 than that formed by SEQ ID NO: 213. Here, the fourth sequence may comprise modified stem 2, which is shorter than the wildtype stem 2 or may not form an RNA duplex. In an embodiment, the fourth sequence may be a sequence obtained by deleting at least one pair of nucleotides forming a complementary bond from SEQ ID NO: 213. For example, the fourth sequence may be 5'-CCGCUUCACCAAAAGCUGUCCUUAGGGAUUAGAACUUGAGUGAAGGUG-3' (SEQ ID NO: 214), 5'-CCGCUUCACCAAAAGCUGUCUUAGGAUUAGAACUUGAGUGAAGGUG-3' (SEQ ID NO: 215), 5'-CCGCUUCACCAAAAGCUGUUUAGAUUAGAACUUGAGUGAAGGUG-3' (SEQ ID NO: 216), 5'-CCGCUUCACCAAAAGCUGUUAGUUAGAACUUGAGUGAAGGUG-3' (SEQ ID NO: 217), 5'-CCGCUUCACCAAAAGCGUUAGUUGAACUUGAGUGAAGGUG-3' (SEQ ID NO: 218), 5'-CCGCUUCACCAAAAGGUUAGUUAACUUGAGUGAAGGUG-3' (SEQ ID NO: 219), 5'-CCGCUUCACCAAAGGUUAGUUAACUGAGUGAAGGUG-3' (SEQ ID NO: 220), 5'-CCGCUUCACCAAGGUUAGUUAACGAGUGAAGGUG-3' (SEQ ID NO: 221), 5'-CCGCUUCACAAGGUUAGUUAACAGUGAAGGUG-3' (SEQ ID NO: 222), 5'-CCGCUUCAAAGGUUAGUUAACAUGAAGGUG-3' (SEQ ID NO: 223), 5'-CCGCUUCAAGGUUAGUUAACAGAAGGUG-3' (SEQ ID NO: 224), 5'-CCGCUUAAGGUUAGUUAACAAAGGUG-3' (SEQ ID NO: 225), 5'-CCGCUAAGGUUAGUUAACAAGGUG-3' (SEQ ID NO: 226), 5'-CCGCAAGGUUAGUUAACAGGUG-3' (SEQ ID NO: 227), 5'-CCGAAGGUUAGUUAACAGUG-3' (SEQ ID NO: 228), 5'-CGAAGGUUAGUUAACAUG-3' (SEQ ID NO: 229), or 5'-GAAGGUUAGUUAACAU-3' (SEQ ID NO: 230), wherein 5'-UUAG-3' included in SEQ ID NOS: 214 to 230 may be substituted with 5'-GAAA-3'. In another embodiment, the fourth sequence may be a sequence obtained by deleting at least one pair of nucleotides forming a complementary base pair and/or at least one nucleotide not involved in forming a complementary base pair from SEQ ID NO: 213. For example, the fourth sequence may be 5'-CCGCUUCACCAAAAGCUGUCCUUAGGGAUUAGAACUUGAGUGAAGGUG-3' (SEQ ID NO: 214), 5'-CCGCUUCACCAAAAGCUGUCUUAGGAUUAGAACUUGAGUGAAGGUG-3' (SEQ ID NO: 215), 5'-CCGCUUCACCAAAAGCUGUUUAGAUUAGAACUUGAGUGAAGGUG-3' (SEQ ID NO: 216), 5'-CCGCUUCACCAAAAGCUGUUAGUUAGAACUUGAGUGAAGGUG-3' (SEQ ID NO: 217), 5'-CCGCUUCACCAAAAGCUGUUAGUAGAACUUGAGUGAAGGUG-3' (SEQ ID NO: 231), 5'-CCGCUUCACCAAAAGCUUUAGAGAACUUGAGUGAAGGUG-3' (SEQ ID NO: 232), 5'-CCGCUUCACCAAAAGCUUAGGAACUUGAGUGAAGGUG-3' (SEQ ID NO: 233), 5'-CCGCUUCACCAAAAGUUAGAACUUGAGUGAAGGUG-3' (SEQ ID NO: 234), 5'-CCGCUUCACCAAAAUUAGACUUGAGUGAAGGUG-3' (SEQ ID NO: 235), 5'-CCGCUUCACCAAAUUAGCUUGAGUGAAGGUG-3' (SEQ ID NO: 236), 5'-CCGCUUCACCAAUUAGUUGAGUGAAGGUG-3' (SEQ ID NO: 237), 5'-CCGCUUCACCAUUAGUGAGUGAAGGUG-3' (SEQ ID NO: 238), 5'-CCGCUUCACCUUAGGAGUGAAGGUG-3' (SEQ ID NO:239), 5'-CCGCUUCACUUAGAGUGAAGGUG-3' (SEQ ID NO: 240), 5'-CCGCUUCACUUAGGUGAAGGUG-3' (SEQ ID NO: 241), 5'-CCGCUUCAUUAGUGAAGGUG-3' (SEQ ID NO: 242), 5'-CCGCUUCUUAGGAAGGUG-3' (SEQ ID NO: 243), 5'-CCGCUUUUAGAAGGUG-3' (SEQ ID NO: 244), 5'-CCGCUUUAGAGGUG-3' (SEQ ID NO: 245), 5'-CCGCUUAGGGUG-3' (SEQ ID NO: 246), 5'-CCGUUAGGUG-3' (SEQ ID NO: 247), 5'-CCUUAGGUG-3', 5'-CUUAGUG-3', 5'-CUUAGG-3', or 5'-UUAG-3', wherein 5'-UUAG-3' included in the sequences may be substituted with 5'-GAAA-3'. In another embodiment, the fourth sequence may be a sequence of SEQ ID NO: 213 from which a sequence of one or more arbitrarily selected nucleotides is deleted. Here, the sequence of one or more arbitrarily selected nucleotides may be a randomly selected sequence regardless of an order or direction in SEQ ID NO: 213, and when the sequence of one or more arbitrarily selected nucleotides is a sequence of two or more nucleotides, the sequence may be a sequence of two or more consecutive nucleotides or a sequence of two or more non-consecutive nucleotides.

In another embodiment, the fourth sequence may be an arbitrary nucleotide sequence or an arbitrarily arranged nucleotide sequence. Here, the arbitrary nucleotide or the arbitrarily arranged sequence may be a sequence of 1 to 50 nts.

In another embodiment, the fourth sequence may be a sequence having sequence identity or sequence similarity of at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% to a sequence selected from SEQ ID NOS: 214 to 230.

### 1-5) Fifth sequence (stem 1)

The engineered tracrRNA may comprise a fifth sequence. The first sequence corresponds to the region 5 to be modified in the wildtype tracrRNA (FIG. 2). The fifth sequence is a nucleotide sequence that may be modified in various ways, and may be a sequence of 1 to 21 nucleotides (1 to 21 nts). The fifth sequence forms an RNA duplex through complementary binding therein. Here, the RNA duplex formed in the fifth sequence is referred to as stem 1 in the present specification.

The fifth sequence does not comprise a sequence of five or more consecutive uridines.

The fifth sequence is located at the 5' end of the fourth sequence.

The fifth sequence is covalently linked to the 5' end of the fourth sequence.

In an embodiment, the fifth sequence may be 5'-CUUCACUGAUAAAGUGGAGAA-3' (SEQ ID NO: 248).

In another embodiment, the fifth sequence may be a part of SEQ ID NO: 248, and may be a sequential partial sequence at the 3' end of SEQ ID NO: 248. In an embodiment, the fifth sequence may be 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', 5'-AAGUGGAGAA-3' (SEQ ID NO: 249), 5'-AAAGUGGAGAA-3' (SEQ ID NO: 250), 5'-UAAAGUGGAGAA-3' (SEQ ID NO: 251), 5'-AUAAAGUGGAGAA-3' (SEQ ID NO: 252), 5'-GAUAAAGUGGAGAA-3' (SEQ ID NO: 253), 5'-UGAUAAAGUGGAGAA-3' (SEQ ID NO: 254), 5'-CUGAUAAAGUGGAGAA-3' (SEQ ID NO: 255), 5'-ACUGAUAAAGUGGAGAA-3' (SEQ ID NO: 256), 5'-CACUGAUAAAGUGGAGAA-3' (SEQ ID NO: 257), 5'-UCACUGAUAAAGUGGAGAA-3' (SEQ ID NO: 258), or 5'-UUCACUGAUAAAGUGGAGAA-3' (SEQ ID NO: 259).

In another embodiment, the fifth sequence may be a sequence of SEQ ID NO: 248 from which a sequence of one or more arbitrarily selected nucleotides is deleted. Here, the sequence of one or more arbitrarily selected nucleotides may be a randomly selected sequence regardless of an order or direction in SEQ ID NO: 248, and when the sequence of one or more arbitrarily selected nucleotides is a sequence of two or more nucleotides, the sequence may be a sequence of two or more consecutive nucleotides or a sequence of two or more non-consecutive nucleotides.

In another embodiment, the fifth sequence may be an arbitrary nucleotide sequence or an arbitrarily arranged nucleotide sequence. Here, the arbitrary nucleotide sequence or the arbitrarily arranged nucleotide sequence may be a sequence of 1 to 21 nts.

In another embodiment, the fifth sequence may be a sequence having sequence identity or sequence similarity of at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% to a sequence selected from SEQ ID NOS: 248 to 259.

### 1-6) Additional sequence

The engineered tracrRNA may optionally further comprise an additional sequence. The additional sequence may be located at the 3' end of the engineered tracrRNA. The additional sequence may be located at the 3' end of the first sequence. The additional sequence may be located at the 5' end of the engineered tracrRNA. The additional sequence may be located at the 5' end of the fifth sequence.

The additional sequence may be a sequence of 1 to 40 nucleotides (a sequence of 1 to 40 nts).

In an embodiment, the additional sequence may be a sequence of 1 to 5, 1 to 10, 1 to 15, 1 to 20, 1 to 25, 1 to 30, 1 to 35, 1 to 40, 5 to 10, 5 to 15, 5 to 20, 5 to 25, 5 to 30, 5 to 35 or 5 to 40 nucleotides. Alternatively, the additional sequence may be a sequence of 10 to 15, 10 to 20, 10 to 25, 10 to 30, 10 to 35, 10 to 40, 15 to 20, 15 to 25, 15 to 30, 15 to 35 or 15 to 40 nucleotides. Alternatively, the additional sequence may be a sequence of 20 to 25, 20 to 30, 20 to 35, 20 to 40, 25 to 30, 25 to 35 or 25 to 40 nucleotides. Alternatively, the additional sequence may be a sequence of 30 to 35, 30 to 40 or 35 to 40 nucleotides.

In another embodiment, the additional sequence may be a sequence of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39 or 40 nucleotides.

The additional sequence may be an arbitrary nucleotide sequence or an arbitrarily arranged nucleotide sequence. For example, the additional sequence may be 5'-AUAAAGGUGA-3' (SEQ ID NO: 260). The examples are for an illustrative purpose only, and the scope of the present disclosure is not limited thereto.

The additional sequence may be a known nucleotide sequence. For example, the additional sequence may be a hammerhead ribozyme nucleotide sequence. Here, the hammerhead ribozyme nucleotide sequence may be 5'-CUGAUGAGUCCGUGAGGACGAAACGAGUAAGCUCGUC-3' (SEQ ID NO: 261) or 5'-CUGCUCGAAUGAGCAAAGCAGGAGUGCCUGAGUAGUC-3' (SEQ ID NO: 262). Alternatively, the hammerhead ribozyme nucleotide sequence may be a sequence having sequence identity or sequence similarity of at least 85%, 86%, 87%, 88%, 89%, 90%, 91%, 92%, 93%, 94%, 95%, 96%, 97%, 98%, 99% or 100% to each of the above-mentioned sequences.

The examples are for an illustrative purpose only, and the scope of the present disclosure is not limited thereto.

### 1-7) Chemical modification

The engineered tracrRNA described above may optionally comprise a chemical modification of at least one nucleotide. Here, the chemical modification may be a modification of any of various covalent bonds that may occur in a nucleotide base and/or sugar. For example, the chemical modification may include methylation, halogenation, acetylation, phosphorylation, phosphorothioate linkage, locked nucleic acid (LNA), 2'-O-methyl 3'phosphorothioate(MS), or 2'-O-methyl 3'thioPACE(MSP), or may include all modifications of a nucleic acid described in WO 2019/089820 A1, but is not limited thereto.

### 1-8) Examples of engineered tracrRNA

Based on the previous description, examples of engineered tracrRNA are described. Specific descriptions of the components included in the examples below are the same as those described above for the corresponding components. The examples are for an illustrative purpose only, and the scope of the present disclosure is not limited thereto.

In an embodiment, the engineered tracrRNA may be a sequence that does not comprise a sequence of five or more consecutive uridines, and may comprise a sequence of four or less consecutive uridines.

In an embodiment, the engineered tracrRNA may comprise a first sequence, a second sequence and a third sequence. Here, the first sequence, the second sequence, and the third sequence may be sequentially located in the engineered tracrRNA in a 3 to 5' direction (5'-[3rd sequence]-[2nd sequence]-[1st sequence]-3'). Here, the first sequence, the second sequence, and the third sequence may be sequences that do not comprise a sequence of five or more consecutive uridines, and may be sequences that comprise a sequence of four or less consecutive uridines. Here, the first sequence may be at least one sequence selected from the following sequences: 5'-CAAAUUCA(N)ₐCCUCUCCAAUUCUGCACAA-3' (SEQ ID NO: 2); 5'-CAAAUUCAVNNNN(V)ₐCCUCUCCAAUUCUGCACAA-3' (SEQ ID NO: 3); 5'-CAAAUUCANVNNN(N)_{b}CCUCUCCAAUUCUGCACAA-3' (SEQ ID NO: 4); 5'-CAAAUUCANNVNN(N)_{c}CCUCUCCAAUUCUGCACAA-3' (SEQ ID NO: 5); 5'-CAAAUUCANNNVN(N)_{d}CCUCUCCAAUUCUGCACAA-3' (SEQ ID NO: 6); and 5'-CAAAUUCANNNNV(N)ₐCCUCUCCAAUUCUGCACAA-3' (SEQ ID NO: 7). Here, N may each independently A, C, G or U, and each V may be independently A, C or G. a may be an integer from 0 to 4, b may be an integer from 0 to 1, c may be an integer from 0 to 2, and d may be an integer from 0 to 3. In (N)ₐ, (N)_{c}, or (N)a, when a, c and d are integers other than 0 or 1, N in (N)ₐ, (N)_{c}, or (N)_{d} may each independently A, C, G or U. In (V)ₐ, when a is an integer other than 0 or 1, each V in (V)ₐ may independently be A, C or G. Here, the second sequence may be 5'-AUGUCGAGAAGUGCUUUCUUCGGAAAGUAACCCUCGAAA-3' (SEQ ID NO: 211) or a sequence having sequence identity of at least 70% to SEQ ID NO: 211. Here, the third sequence may be 5'-GGCUGCUUGCAUCAGCCUA-3' (SEQ ID NO: 212), or a sequence having sequence identity of at least 70% to SEQ ID NO: 212. The engineered tracrRNA may optionally further comprise an additional sequence. Here, the additional sequence may be a sequence of 1 to 40 nucleotides (a sequence of 1 to 40 nts). The additional sequence may be located at the 3' end of the engineered tracrRNA (5'-[3rd sequence]-[2nd sequence]-[1st sequence]-[additional sequence]-3').

For a specific example, the engineered tracrRNA may comprise a first sequence, a second sequence and a third sequence as described below. Here, the first sequence may be 5'-CAAAUUCANNNCNCCUCUCCAAUUCUGCACAA-3' (SEQ ID NO: 111) or a sequence having sequence identity of at least 70% or more to SEQ ID NO: 111. Here, the sequence having sequence identity of at least 70% or more to SEQ ID NO: 111 may be a sequence having sequence identity of at least 70% or more to the remaining sequence thereof except for 5'-CANNNCNC-3'. Here, N may each independently be A, C, G or U. Here, the second sequence may be SEQ ID NO: 211 or a sequence having sequence identity of at least 70% or more to SEQ ID NO: 211. Here, the third sequence may be SEQ ID NO: 212 or a sequence having sequence identity of at least 70% to SEQ ID NO: 212. The engineered tracrRNA may have the first sequence, the second sequence, and the third sequence sequentially in a 3' to 5' direction (5'-[SEQ ID NO: 212 or a sequence having sequence identity of at least 70% or more thereto]-[SEQ ID NO: 211 or a sequence having sequence identity of at least 70% or more thereto]-[SEQ ID NO: 111 or a sequence having sequence identity of at least 70% or more thereto]-3'). The engineered tracrRNA may optionally further comprise an additional sequence. Here, the additional sequence may be a hammerhead ribozyme nucleotide sequence. The hammerhead ribozyme nucleotide sequence may be SEQ ID NO: 261 or a SEQ ID NO: 262. The additional sequence may be located at the 3' end of the engineered tracrRNA. As an example, the engineered tracrRNA may comprise 5'-GGCUGCUUGCAUCAGCCUAAUGUCGAGAAGUGCUUUCUUCGGAAAGUA ACCCUCGAAACAAAUUCANNNCNCCUCUCCAAUUCUGCACAA-3' (SEQ ID NO: 263). Here, N may each independently be A, C, G or U. As another example, the engineered tracrRNA may comprise 5'-GGCUGCUUGCAUCAGCCUAAUGUCGAGAAGUGCUUUCUUCGGAAAGUA ACCCUCGAAACAAAUUCAGUGCUCCUCUCCAAUUCUGCACAA-3' (SEQ ID NO: 264). As yet another example, the engineered tracrRNA may comprise 5'-GGCUGCUUGCAUCAGCCUAAUGUCGAGAAGUGCUUUCUUCGGAAAGUA ACCCUCGAAACAAAUUCAUUUCUCCUCUCCAAUUCUGCACAA-3' (SEQ ID NO: 265).

In another embodiment, the engineered tracrRNA may comprise a first sequence, a second sequence, a third sequence, and a fourth sequence. Here, the first sequence, the second sequence, the third sequence, and the fourth sequence may be sequentially located in the engineered tracrRNA in a 3' to 5' direction (5'-[4th sequence]-[3rd sequence]-[2nd sequence]-[1st sequence]-3'). Here, the first sequence, the second sequence, the third sequence, and the fourth sequence may be sequences that do not comprise a sequence of five or more consecutive uridines, and may be a sequence that comprises a sequence of four or less consecutive uridines. Here, the first sequence may be at least one sequence selected from the following sequences: 5'-CAAAUUCA(N)ₐCCUCUCCAAUUCUGCACAA-3' (SEQ ID NO: 2); 5'-CAAAUUCAVNNNN(V)ₐCCUCUCCAAUUCUGCACAA-3' (SEQ ID NO: 3); 5'-CAAAUUCANVNNN(N)_{b}CCUCUCCAAUUCUGCACAA-3' (SEQ ID NO: 4); 5'-CAAAUUCANNVNN(N)_{c}CCUCUCCAAUUCUGCACAA-3' (SEQ ID NO: 5); 5'-CAAAUUCANNNVN(N)_{d}CCUCUCCAAUUCUGCACAA-3' (SEQ ID NO: 6); and 5'-CAAAUUCANNNNV(N)ₐCCUCUCCAAUUCUGCACAA-3' (SEQ ID NO: 7). Here, N may be each independently A, C, G or U, and each V may be independently A, C or G. a may be an integer from 0 to 4, b may be an integer from 0 to 1, c may be an integer from 0 to 2, and d may be an integer from 0 to 3. In (N)ₐ, (N)_{c}, or (N)_{d}, when a, c and d are integers other than 0 or 1, N in (N)ₐ, (N)_{c}, or (N)_{d} may be each independently A, C, G or U. In (V)ₐ, when a is an integer other than 0 or 1, each V in (V)ₐ may be independently A, C or G. Here, the second sequence may be SEQ ID NO: 211 or a sequence having sequence identity of at least 70% or more to SEQ ID NO: 211. Here, the third sequence may be SEQ ID NO: 212 or a sequence having sequence identity of at least 70% or more to SEQ ID NO: 212. Here, the fourth sequence may be 5'-CCGCUUCACCAAAAGCUGUCCCUUAGGGGAUUAGAACUUGAGUGAAGGU G-3' (SEQ ID NO: 213) or a sequence having sequence identity of at least 70% or more to SEQ ID NO: 213. The engineered tracrRNA may optionally further comprise an additional sequence. Here, the additional sequence may be a sequence of 1 to 40 nucleotides (a sequence of 1 to 40 nts). The additional sequence may be located at the 3' end of the engineered tracrRNA (5'-[4th sequence]-[3rd sequence]-[2nd sequence]-[1st sequence]-[additional sequence]-3').

For a specific example, the engineered tracrRNA may comprise a first sequence, a second sequence, a third sequence, and a fourth sequence as described below. Here, the first sequence may be SEQ ID NO: 111, or a sequence having sequence identity of at least 70% or more to SEQ ID NO: 111. Here, the sequence having sequence identity of at least 70% or more to SEQ ID NO: 111 may be a sequence having sequence identity of at least 70% or more to the remaining sequence thereof except for 5'-CANNNCNC-3'. Here, N may each independently be A, C, G or U. Here, the second sequence may be SEQ ID NO: 211 or a sequence having sequence identity of at least 70% or more to SEQ ID NO: 211. Here, the third sequence may be SEQ ID NO: 212 or a sequence having sequence identity of at least 70% or more to SEQ ID NO: 212. Here, the fourth sequence may be SEQ ID NO: 213 or a sequence having sequence identity of at least 70% or more to SEQ ID NO: 213. The engineered tracrRNA may have the first sequence, the second sequence, the third sequence, and the fourth sequence sequentially in a 3' to 5' direction (5'-[SEQ ID NO: 213 or a sequence having sequence identity of at least 70% or more thereto]-[SEQ ID NO: 212 or a sequence having sequence identity of at least 70% or more thereto]-[SEQ ID NO: 211 or a sequence having sequence identity of at least 70% or more thereto]-[SEQ ID NO: 111 or a sequence having sequence identity of at least 70% or more thereto]-3'). The engineered tracrRNA may optionally further comprise an additional sequence. Here, the additional sequence may be a hammerhead ribozyme nucleotide sequence. The hammerhead ribozyme nucleotide sequence may be SEQ ID NO: 261 or SEQ ID NO: 262. The additional sequence may be located at the 3' end of the engineered tracrRNA. As an example, the engineered tracrRNA may comprise 5'-CCGCUUCACCAAAAGCUGUCCCUUAGGGGAUUAGAACUUGAGUGAAGGU GGGCUGCUUGCAUCAGCCUAAUGUCGAGAAGUGCUUUCUUCGGAAAGU AACCCUCGAAACAAAUUCANNNCNCCUCUCCAAUUCUGCACAA-3' (SEQ ID NO: 266). Here, N may each independently be A, C, G or U. As another example, the engineered tracrRNA may comprise 5'-CCGCUUCACCAAAAGCUGUCCCUUAGGGGAUUAGAACUUGAGUGAAGGU GGGCUGCUUGCAUCAGCCUAAUGUCGAGAAGUGCUUUCUUCGGAAAGU AACCCUCGAAACAAAUUCAGUGCUCCUCUCCAAUUCUGCACAA-3' (SEQ ID NO: 267). As yet another example, the engineered tracrRNA may comprise a sequence 5'-CCGCUUCACCAAAAGCUGUCCCUUAGGGGAUUAGAACUUGAGUGAAGGU GGGCUGCUUGCAUCAGCCUAAUGUCGAGAAUUUCUUUCUUCGGAAAGU AACCCUCGAAACAAAUUCAGUGCUCCUCUCCAAUUCUGCACAA-3' (SEQ ID NO: 268).

For another embodiment, the engineered tracrRNA may comprise a first sequence, a second sequence, a third sequence, a fourth sequence, and a fifth sequence. Here, the first sequence, the second sequence, the third sequence, the fourth sequence, and the fifth sequence may be sequentially located in the engineered tracrRNA in a 3' to 5' direction (5'-[5th sequence]-[4th sequence]-[3rd sequence]-[2nd sequence]-[1st sequence]-3'). Here, the first sequence, the second sequence, the third sequence, the fourth sequence, and the fifth sequence may be sequences that do not comprise a sequence of five or more consecutive uridines, and may be a sequence that comprises a sequence of four or less consecutive uridines. Here, the first sequence may be at least one sequence selected from the following sequences: 5'-CAAAUUCA(N)ₐCCUCUCCAAUUCUGCACAA-3' (SEQ ID NO: 2); 5'-CAAAUUCAVNNNN(V)ₐCCUCUCCAAUUCUGCACAA-3' (SEQ ID NO: 3); 5'-CAAAUUCANVNNN(N)_{b}CCUCUCCAAUUCUGCACAA-3' (SEQ ID NO: 4); 5'-CAAAUUCANNVNN(N)_{c}CCUCUCCAAUUCUGCACAA-3' (SEQ ID NO: 5); 5'-CAAAUUCANNNVN(N)_{d}CCUCUCCAAUUCUGCACAA-3' (SEQ ID NO: 6); and 5'-CAAAUUCANNNNV(N)ₐCCUCUCCAAUUCUGCACAA-3' (SEQ ID NO: 7). Here, N may be each independently A, C, G or U, and each V may be independently A, C or G. a may be an integer from 0 to 4, b may be an integer from 0 to 1, c may be an integer from 0 to 2, and d may be an integer from 0 to 3. In (N)ₐ, (N)_{c} or (N)_{d}, when a, c and d are integers other than 0 or 1, N in (N)ₐ, (N)_{c} or (N)_{d} may each independently be A, C, G or U. In (V)ₐ, when a is an integer other than 0 or 1, each V in (V)ₐ may be independently A, C or G. Here, the second sequence may be SEQ ID NO: 211 or a sequence having sequence identity of at least 70% or more to SEQ ID NO: 211. Here, the third sequence may be SEQ ID NO: 212 or a sequence having sequence identity of at least 70% or more to SEQ ID NO: 212. Here, the fourth sequence may be SEQ ID NO: 213 or a sequence having sequence identity of at least 70% or more to SEQ ID NO: 213. Here, the fifth sequence may be 5'-CUUCACUGAUAAAGUGGAGAA-3'(SEQ ID NO: 248), or a sequence having sequence identity of at least 70% to SEQ ID NO: 248. The engineered tracrRNA may optionally further comprise an additional sequence. Here, the additional sequence may be a sequence of 1 to 40 nucleotides (a sequence of 1 to 40 nts). The additional sequence may be located at the 3' end of the engineered tracrRNA (5'-[5th sequence]-[4th sequence]-[3rd sequence]-[2nd sequence]-[1st sequence]-[additional sequence]-3').

In a specific example, the engineered tracrRNA may comprise a first sequence, a second sequence, a third sequence, a fourth sequence, and a fifth sequence as described below. Here, the first sequence may be SEQ ID NO: 111, or a sequence having sequence identity of at least 70% or more to SEQ ID NO: 111. Here, the sequence having sequence identity of at least 70% or more to SEQ ID NO: 111 may be a sequence having sequence identity of at least 70% or more to the remaining sequence thereof except for 5'-CANNNCNC-3'. Here, N may each independently be A, C, G or U. Here, the second sequence may be SEQ ID NO: 211 or a sequence having sequence identity of at least 70% or more to SEQ ID NO: 211. Here, the third sequence may be SEQ ID NO: 212 or a sequence having sequence identity of at least 70% or more to SEQ ID NO: 212. Here, the fourth sequence may be SEQ ID NO: 213 or a sequence having sequence identity of at least 70% or more to SEQ ID NO: 213. Here, the fifth sequence may be SEQ ID NO: 248 or a sequence having sequence identity of at least 70% or more to SEQ ID NO: 248. The engineered tracrRNA may have the first sequence, the second sequence, the third sequence, the fourth sequence, and the fifth sequence sequentially in a 3' to 5' direction (5'-[SEQ ID NO: 248 or a sequence having sequence identity of at least 70% or more thereto]-[SEQ ID NO: 213 or a sequence having sequence identity of at least 70% or more thereto]-[SEQ ID NO: 212 or a sequence having sequence identity of at least 70% or more thereto]-[SEQ ID NO: 211 or a sequence having sequence identity of at least 70% or more thereto]-[SEQ ID NO: 111 or a sequence having sequence identity of at least 70% or more thereto]-3'). The engineered tracrRNA may optionally further comprise an additional sequence. Here, the additional sequence may be a hammerhead ribozyme nucleotide sequence. The hammerhead ribozyme nucleotide sequence may be SEQ ID NO: 261 or SEQ ID NO: 262. The additional sequence may be located at the 3' end of the engineered tracrRNA. As an example, the engineered tracrRNA may be 5'-CUUCACUGAUAAAGUGGAGAACCGCUUCACCAAAAGCUGUCCCUUAGGG GAUUAGAACUUGAGUGAAGGUGGGCUGCUUGCAUCAGCCUAAUGUCGA GAAGUGCUUUCUUCGGAAAGUAACCCUCGAAACAAAUUCANNNCNCCUC UCCAAUUCUGCACAA-3' (SEQ ID NO: 269). Here, N may each independently be A, C, G or U. As another example, the engineered tracrRNA may be 5'-CUUCACUGAUAAAGUGGAGAACCGCUUCACCAAAAGCUGUCCCUUAGGG GAUUAGAACUUGAGUGAAGGUGGGCUGCUUGCAUCAGCCUAAUGUCGA GAAGUGCUUUCUUCGGAAAGUAACCCUCGAAACAAAUUCAGUGCUCCUC UCCAAUUCUGCACAA-3' (SEQ ID NO: 270). As yet another example, the engineered tracrRNA may be 5'-CUUCACUGAUAAAGUGGAGAACCGCUUCACCAAAAGCUGUCCCUUAGGG GAUUAGAACUUGAGUGAAGGUGGGCUGCUUGCAUCAGCCUAAUGUCGA GAAGUGCUUUCUUCGGAAAGUAACCCUCGAAACAAAUUCAUUUCUCCUC UCCAAUUCUGCACAA-3' (SEQ ID NO: 271).

In another embodiment, the engineered tracrRNA may be a sequence modified so that it does not comprise a sequence of five or more consecutive uridines and has a length shorter than a wildtype tracrRNA. Here, the engineered tracrRNA may comprise a sequence of four less consecutive uridines, and may not comprise a part of the wildtype tracrRNA.

In an embodiment, the engineered tracrRNA may comprise a first sequence, a second sequence and a third sequence. Here, the first sequence, the second sequence, and the third sequence may be sequentially located in the engineered tracrRNA in a 3' to 5' direction (5'-[3rd sequence]-[2nd sequence]-[1st sequence]-3'). Here, the first sequence, the second sequence, and the third sequence may be sequences that do not comprise a sequence of five or more consecutive uridines, and may be sequences that comprise a sequence of four or less consecutive uridines. Here, the first sequence may be at least one sequence selected from the following sequences: a part of 5'-CAAAUUCA(N)ₐCCUCUCCAAUUCUGCACAA-3' (SEQ ID NO: 2); a part of 5'-CAAAUUCAVNNNN(V)ₐCCUCUCCAAUUCUGCACAA-3' (SEQ ID NO: 3); a part of 5'-CAAAUUCANVNNN(N)_{b}CCUCUCCAAUUCUGCACAA-3' (SEQ ID NO: 4); a part of 5'-CAAAUUCANNVNN(N)_{c}CCUCUCCAAUUCUGCACAA-3' (SEQ ID NO: 5); a part of 5'-CAAAUUCANNNVN(N)_{d}CCUCUCCAAUUCUGCACAA-3' (SEQ ID NO: 6); and a part of 5'-CAAAUUCANNNNV(N)ₐCCUCUCCAAUUCUGCACAA-3' (SEQ ID NO: 7). Here, N may be each independently A, C, G or U, and each V may be independently A, C or G. a may be an integer from 0 to 4, b may be an integer from 0 to 1, c may be an integer from 0 to 2, and d may be an integer from 0 to 3. In (N)ₐ, (N)_{c} or (N)_{d}, when a, c and d are integers other than 0 or 1, N in (N)ₐ, (N)_{c} or (N)_{d} may each independently be A, C, G or U. In (V)ₐ, when a is an integer other than 0 or 1, each V in (V)ₐ may be independently A, C or G. Here, the second sequence may be SEQ ID NO: 211 or a sequence having sequence identity of at least 70% or more to SEQ ID NO: 211. Here, the third sequence may be SEQ ID NO: 212 or a sequence having sequence identity of at least 70% or more to SEQ ID NO: 212. The engineered tracrRNA may optionally further comprise an additional sequence. Here, the additional sequence may be a sequence of 1 to 40 nucleotides (a sequence of 1 to 40 nts). The additional sequence may be located at the 3' end of the engineered tracrRNA (5'-[3rd sequence]-[2nd sequence]-[1st sequence]-[additional sequence]-3').

For a specific example, the engineered tracrRNA may comprise a first sequence, a second sequence and a third sequence as described below. Here, the first sequence may be a part of SEQ ID NO: 111. Here, the part of SEQ ID NO: 111 may comprise 5'-CAAAUUCANNNCN-3' (SEQ ID NO: 272) while not comprising a partial sequence at the 3' end of SEQ ID NO: 111. Here, the part SEQ ID NO: 111 may be 5'-CAAAUUCANNNCNCCUCUCCAAUUCUGCACA-3' (SEQ ID NO: 273), 5'-CAAAUUCANNNCNCCUCUCCAAUUCUGCAC-3' (SEQ ID NO: 274), 5'-CAAAUUCANNNCNCCUCUCCAAUUCUGCA-3' (SEQ ID NO: 275), 5'-CAAAUUCANNNCNCCUCUCCAAUUCUGC-3' (SEQ ID NO: 276), 5'-CAAAUUCANNNCNCCUCUCCAAUUCUG-3' (SEQ ID NO: 277), 5'-CAAAUUCANNNCNCCUCUCCAAUUCU-3' (SEQ ID NO: 278), 5'-CAAAUUCANNNCNCCUCUCCAAUUC-3' (SEQ ID NO: 279), 5'-CAAAUUCANNNCNCCUCUCCAAUU-3' (SEQ ID NO: 280), 5'-CAAAUUCANNNCNCCUCUCCAAU-3' (SEQ ID NO: 281), 5'-CAAAUUCANNNCNCCUCUCCAA-3' (SEQ ID NO: 282), 5'-CAAAUUCANNNCNCCUCUCCA-3' (SEQ ID NO: 283), 5'-CAAAUUCANNNCNCCUCUCC-3' (SEQ ID NO: 284), 5'-CAAAUUCANNNCNCCUCUC-3' (SEQ ID NO: 285), 5'-CAAAUUCANNNCNCCUCU-3' (SEQ ID NO: 286), 5'-CAAAUUCANNNCNCCUC-3' (SEQ ID NO: 287), 5'-CAAAUUCANNNCNCCU-3' (SEQ ID NO: 288), 5'-CAAAUUCANNNCNCC-3' (SEQ ID NO: 289), 5'-CAAAUUCANNNCNC-3' (SEQ ID NO: 290), or 5'-CAAAUUCANNNCN-3' (SEQ ID NO: 272). Here, N may each independently be A, C, G or U. Here, the second sequence may be SEQ ID NO: 211 or a sequence having sequence identity of at least 70% or more to SEQ ID NO: 211. Here, the third sequence may be SEQ ID NO: 212 or a sequence having sequence identity of at least 70% or more to SEQ ID NO: 212. The engineered tracrRNA may have the first sequence, the second sequence, and the third sequence sequentially in a 3' to 5' direction (5'-[SEQ ID NO: 212 or a sequence having sequence identity of at least 70% or more thereto]-[SEQ ID NO: 211 or a sequence having sequence identity of at least 70% or more thereto]-[a sequence selected from SEQ ID NOS: 272 to 290]-3'). The engineered tracrRNA may optionally further comprise an additional sequence. Here, the additional sequence may be a hammerhead ribozyme nucleotide sequence. The hammerhead ribozyme nucleotide sequence may be SEQ ID NO: 261 or SEQ ID NO: 262. The additional sequence may be located at the 3' end of the engineered tracrRNA. As an example, the engineered tracrRNA may comprise 5'-GGCUGCUUGCAUCAGCCUAAUGUCGAGAAGUGCUUUCUUCGGAAAGUA ACCCUCGAAACAAAUUCANNNCNCCUCUC-3' (SEQ ID NO: 291). Here, N may each independently be A, C, G or U. As another example. the engineered tracrRNA may comprise 5'-GGCUGCUUGCAUCAGCCUAAUGUCGAGAAGUGCUUUCUUCGGAAAGUA ACCCUCGAAACAAAUUCAGUGCUCCUCUC-3' (SEQ ID NO: 292). As yet another example, the engineered tracrRNA may comprise 5'-GGCUGCUUGCAUCAGCCUAAUGUCGAGAAGUGCUUUCUUCGGAAAGUA ACCCUCGAAACAAAUUCAUUUCUCCUCUC-3' (SEQ ID NO: 293).

In another embodiment, the engineered tracrRNA may comprise a first sequence, a second sequence, a third sequence, and a fourth sequence. Here, the first sequence, the second sequence, the third sequence, and the fourth sequence may be sequentially located in the engineered tracrRNA in a 3' to 5' direction (5'-[4th sequence]-[3rd sequence]-[2nd sequence]-[1st sequence]-3'). Here, the first sequence, the second sequence, the third sequence, and the fourth sequence may be sequences that do not comprise a sequence of five or more consecutive uridines, and may be a sequence that comprises a sequence of four or less consecutive uridines. Here, the first sequence may be at least one sequence selected from the following sequences: 5'-CAAAUUCA(N)ₐCCUCUCCAAUUCUGCACAA-3' (SEQ ID NO: 2); 5'-CAAAUUCAVNNNN(V)ₐCCUCUCCAAUUCUGCACAA-3' (SEQ ID NO: 3); 5'-CAAAUUCANVNNN(N)_{b}CCUCUCCAAUUCUGCACAA-3' (SEQ ID NO: 4); 5'-CAAAUUCANNVNN(N)_{c}CCUCUCCAAUUCUGCACAA-3' (SEQ ID NO: 5); 5'-CAAAUUCANNNVN(N)_{d}CCUCUCCAAUUCUGCACAA-3' (SEQ ID NO: 6); 5'-CAAAUUCANNNNV(N)ₐCCUCUCCAAUUCUGCACAA-3' (SEQ ID NO: 7); and a part of a sequence selected from SEQ ID NOS: 2 to 7. Here, N may be each independently A, C, G or U, and each V may be independently A, C or G. a may be an integer from 0 to 4, b may be an integer from 0 to 1, c may be an integer from 0 to 2, and d may be an integer from 0 to 3. In (N)ₐ, (N)_{c} or (N)_{d}, when a, c and d are integers other than 0 or 1, N in (N)ₐ, (N)_{c} or (N)_{d} may each independently be A, C, G or U. In (V)ₐ, when a is an integer other than 0 or 1, each V in (V)ₐ may be independently A, C or G. Here, the second sequence may be SEQ ID NO: 211 or a sequence having sequence identity of at least 70% or more to SEQ ID NO: 211. Here, the third sequence may be SEQ ID NO: 212 or a sequence having sequence identity of at least 70% or more to SEQ ID NO: 212. Here, the fourth sequence may be SEQ ID NO: 213 or a part of SEQ ID NO: 213. The engineered tracrRNA may optionally further comprise an additional sequence. Here, the additional sequence may be a sequence of 1 to 40 nucleotides (a sequence of 1 to 40 nts). The additional sequence may be located at the 3' end of the engineered tracrRNA (5'-[4th sequence]-[3rd sequence]-[2nd sequence]-[1st sequence]-[additional sequence]-3').

For a specific example, the engineered tracrRNA may comprise a first sequence, a second sequence, a third sequence, and a fourth sequence as described below. Here, the first sequence may be SEQ ID NO: 111 or a part of SEQ ID NO: 111. Here, the part of SEQ ID NO: 111 may comprise SEQ ID NO: 272 while not comprising a partial sequence at the 3' end of SEQ ID NO: 111. Here, the part SEQ ID NO: 111 may be a sequence selected from SEQ ID NOS: 272 to 290. Here, N may each independently be A, C, G or U. Here, the second sequence may be SEQ ID NO: 211 or a sequence having sequence identity of at least 70% or more to SEQ ID NO: 211. Here, the third sequence may be SEQ ID NO: 212 or a sequence having sequence identity of at least 70% or more to SEQ ID NO: 212. Here, the fourth sequence may be SEQ ID NO: 213 or a part of SEQ ID NO: 213. Here, the part of SEQ ID NO: 213 may be a sequence obtained by deleting at least one pair of nucleotides forming a complementary base pair and/or at least one nucleotide not involved in forming a complementary base pair from SEQ ID NO: 213. Here, the part of SEQ ID NO: 213 may be a sequence selected from the group consisting of SEQ ID NOS: 214 to 217, SEQ ID NO: 231 to 247, 5'-CCUUAGGUG-3', 5'-CUUAGUG-3', 5'-CUUAGG-3', and 5'-UUAG-3', wherein 5'-UUAG-3' included in the selected sequence may be substituted with 5'-GAAA-3'. The first sequence, the second sequence, the third sequence, and the fourth sequence may be sequentially located in the engineered tracrRNA in a 3' to 5' direction (5'-[SEQ ID NO: 213 or a sequence having sequence identity of at least 70% or more thereto]-[SEQ ID NO: 212 or a sequence having sequence identity of at least 70% or more thereto]-[SEQ ID NO: 211 or a sequence having sequence identity of at least 70% or more thereto]-[SEQ ID NO: 111 or a part thereof]-3'). The engineered tracrRNA may optionally further comprise an additional sequence. Here, the additional sequence may be a hammerhead ribozyme nucleotide sequence. The hammerhead ribozyme nucleotide sequence may be SEQ ID NO: 261 or SEQ ID NO: 262. The additional sequence may be located at the 3' end of the engineered tracrRNA. As an example, the engineered tracrRNA may comprise 5'-CCGCUUCACCUUAGGAGUGAAGGUGGGCUGCUUGCAUCAGCCUAAUGU CGAGAAGUGCUUUCUUCGGAAAGUAACCCUCGAAACAAAUUCANNNCNC CUCUC-3' (SEQ ID NO: 294), or 5'-CCGCUUCACCGAAAGAGUGAAGGUGGGCUGCUUGCAUCAGCCUAAUGU CGAGAAGUGCUUUCUUCGGAAAGUAACCCUCGAAACAAAUUCANNNCNC CUCUC-3' (SEQ ID NO: 295). Here, N may each independently be A, C, G or U. As an example, the engineered tracrRNA may comprise 5'-CCGCUUUUAGAAGGUGGGCUGCUUGCAUCAGCCUAAUGUCGAGAAGUG CUUUCUUCGGAAAGUAACCCUCGAAACAAAUUCANNNCNCCUCUCCAAU UCUGCACAA-3' (SEQ ID NO: 296), or 5'-CCGCUUGAAAAAGGUGGGCUGCUUGCAUCAGCCUAAUGUCGAGAAGUG CUUUCUUCGGAAAGUAACCCUCGAAACAAAUUCANNNCNCCUCUCCAAU UCUGCACAA-3' (SEQ ID NO: 297). Here, N may each independently be A, C, G or U. As another example, the engineered tracrRNA may comprise 5'-CCGCUUCACCAAUUAGUUGAGUGAAGGUGGGCUGCUUGCAUCAGCCUA AUGUCGAGAAGUGCUUUCUUCGGAAAGUAACCCUCGAAACAAAUUCAGU GCUCCUCUCCAAUUCUGCACAA-3' (SEQ ID NO: 298), or 5'-CCGCUUCACCAAGAAAUUGAGUGAAGGUGGGCUGCUUGCAUCAGCCUAA UGUCGAGAAGUGCUUUCUUCGGAAAGUAACCCUCGAAACAAAUUCAGUG CUCCUCUCCAAUUCUGCACAA-3' (SEQ ID NO: 299).

For another embodiment, the engineered tracrRNA may comprise a first sequence, a second sequence, a third sequence, a fourth sequence and a fifth sequence. Here, the first sequence, the second sequence, the third sequence, the fourth sequence, and the fifth sequence may be sequentially located in the engineered tracrRNA in a 3' to 5' direction (5'-[5th sequence]-[4th sequence]-[3rd sequence]-[2nd sequence]-[1st sequence]-3'). Here, the first sequence, the second sequence, the third sequence, the fourth sequence, and the fifth sequence may be sequences that do not comprise a sequence of five or more consecutive uridines, and may be a sequence that comprises a sequence of four or less consecutive uridines. Here, the first sequence may be at least one sequence selected from the following sequences: 5'-CAAAUUCA(N)ₐCCUCUCCAAUUCUGCACAA-3' (SEQ ID NO: 2); 5'-CAAAUUCAVNNNN(V)ₐCCUCUCCAAUUCUGCACAA-3' (SEQ ID NO: 3); 5'-CAAAUUCANVNNN(N)_{b}CCUCUCCAAUUCUGCACAA-3' (SEQ ID NO: 4); 5'-CAAAUUCANNVNN(N)_{c}CCUCUCCAAUUCUGCACAA-3' (SEQ ID NO: 5); 5'-CAAAUUCANNNVN(N)_{d}CCUCUCCAAUUCUGCACAA-3' (SEQ ID NO: 6); 5'-CAAAUUCANNNNV(N)ₐCCUCUCCAAUUCUGCACAA-3' (SEQ ID NO: 7); and a part of a sequence selected from SEQ ID NOS: 2 to 7. Here, N may be each independently A, C, G or U, and each V may be independently A, C or G. a may be an integer from 0 to 4, b may be an integer from 0 to 1, c may be an integer from 0 to 2, and d may be an integer from 0 to 3. In (N)ₐ, (N)_{c} or (N)_{d}, when a, c and d are integers other than 0 or 1, N in (N)ₐ, (N)_{c} or (N)_{d} may each independently be A, C, G or U. In (V)ₐ, when a is an integer other than 0 or 1, each V in (V)ₐ may be independently A, C or G. Here, the second sequence may be SEQ ID NO: 211 or a sequence having sequence identity of at least 70% or more to SEQ ID NO: 211. Here, the third sequence may be SEQ ID NO: 212 or a sequence having sequence identity of at least 70% or more to SEQ ID NO: 212. Here, the fourth sequence may be SEQ ID NO: 213 or a part of SEQ ID NO: 213. Here, the fifth sequence may be SEQ ID NO: 248, or a part of SEQ ID NO: 248. The engineered tracrRNA may optionally further comprise an additional sequence. Here, the additional sequence may be a sequence of 1 to 40 nucleotides (a sequence of 1 to 40 nts). The additional sequence may be located at the 3' end of the engineered tracrRNA (5'-[5th sequence]-[4th sequence]-[3rd sequence]-[2nd sequence]-[1st sequence]-[additional sequence]-3').

For a specific example, the engineered tracrRNA may comprise a first sequence, a second sequence, a third sequence, a fourth sequence, and a fifth sequence which are described below. Here, the first sequence may be 5'-CAAAUUCANNNCNCCUCUCCAAUUCUGCACAA-3' (SEQ ID NO: 111) or a part of SEQ ID NO: 111. Here, the part of SEQ ID NO: 111 may comprise SEQ ID NO: 272 while not comprising a partial sequence at the 3' end of SEQ ID NO: 111. Here, the part of SEQ ID NO: 111 may be a sequence selected from SEQ ID NOS: 272 to 290. Here, N may each independently be A, C, G or U. Here, the second sequence may be SEQ ID NO: 211 or a sequence having sequence identity of at least 70% or more to SEQ ID NO: 211. Here, the third sequence may be SEQ ID NO: 212 or a sequence having sequence identity of at least 70% or more to SEQ ID NO: 212. Here, the fourth sequence may be SEQ ID NO: 213 or a part of SEQ ID NO: 213. Here, the part of SEQ ID NO: 213 may be a sequence obtained by deleting at least one pair of nucleotides forming a complementary base pair and/or at least one nucleotide not involved in forming a complementary base pair from SEQ ID NO: 213. Here, the part of SEQ ID NO: 213 may be a sequence selected from SEQ ID NOS: 214 to 217, SEQ ID NO: 231 to 247, 5'-CCUUAGGUG-3', 5'-CUUAGUG-3', 5'-CUUAGG-3', and 5'-UUAG-3', wherein 5'-UUAG-3' included in the selected sequence may be substituted with 5'-GAAA-3'. Here, the fifth sequence may be SEQ ID NO: 248, or a part of SEQ ID NO: 248. Here, the part of the SEQ ID NO: 248 may be a sequential partial sequence at the 3' end of SEQ ID NO: 248, and may be a sequence selected from the group consisting of 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', and SEQ ID NOS: 249 to 259. The first sequence, the second sequence, the third sequence, the fourth sequence, and the fifth sequence may be sequentially located in the engineered tracrRNA in a 3' to 5' direction (5'-[SEQ ID NO: 248 or a part thereof]-[SEQ ID NO: 213 or a part thereof]-[SEQ ID NO: 212 or a sequence having sequence identity of at least 70% or more thereto]-[SEQ ID NO: 211 or a sequence having sequence identity of at least 70% or more thereto]-[SEQ ID NO: 111 or a part thereof]-3'). The engineered tracrRNA may optionally further comprise an additional sequence. Here, the additional sequence may be a hammerhead ribozyme nucleotide sequence. The hammerhead ribozyme nucleotide sequence may be SEQ ID NO: 261 or SEQ ID NO: 262. The additional sequence may be located at the 3' end of the engineered tracrRNA. As an example, the engineered tracrRNA may be 5'-ACCGCUUCACCAAUUAGUUGAGUGAAGGUGGGCUGCUUGCAUCAGCCU AAUGUCGAGAAGUGCUUUCUUCGGAAAGUAACCCUCGAAACAAAUUCAN NNCNCCUCUC-3' (SEQ ID NO: 300), 5'-ACCGCUUCACCAAUUAGUUGAGUGAAGGUGGGCUGCUUGCAUCAGCCU AAUGUCGAGAAGUGCUUUCUUCGGAAAGUAACCCUCGAAACAAAUUCAN NNCNCCUCUCCAAUUCUGCACAA-3' (SEQ ID NO: 301), 5'-ACCGCUUCACCAAGAAAUUGAGUGAAGGUGGGCUGCUUGCAUCAGCCUA AUGUCGAGAAGUGCUUUCUUCGGAAAGUAACCCUCGAAACAAAUUCANN NCNCCUCUC-3' (SEQ ID NO: 302), or 5'-ACCGCUUCACCAAGAAAUUGAGUGAAGGUGGGCUGCUUGCAUCAGCCUA AUGUCGAGAAGUGCUUUCUUCGGAAAGUAACCCUCGAAACAAAUUCANN NCNCCUCUCCAAUUCUGCACAA-3' (SEQ ID NO: 303). Here, N may each independently be A, C, G or U. As another example, the engineered tracrRNA may be 5'-ACCGCUUCACCAAAAGCUGUCCCUUAGGGGAUUAGAACUUGAGUGAAGG UGGGCUGCUUGCAUCAGCCUAAUGUCGAGAAGUGCUUUCUUCGGAAAG UAACCCUCGAAACAAAUUCANNNCNCCUCUCCAAUUCUGCACAA-3' (SEQ ID NO: 304), or 5'-ACCGCUUCACCAAAAGCUGUCCCUUAGGGGAUUAGAACUUGAGUGAAGG UGGGCUGCUUGCAUCAGCCUAAUGUCGAGAAGUGCUUUCUUCGGAAAG UAACCCUCGAAACAAAUUCANNNCNCCUCUC-3' (SEQ ID NO: 305). Here, N may each independently be A, C, G or U. As yet another example, the engineered tracrRNA may be 5'-ACCGCUUCACCAAUUAGUUGAGUGAAGGUGGGCUGCUUGCAUCAGCCU AAUGUCGAGAAGUGCUUUCUUCGGAAAGUAACCCUCGAAACAAAUUCAG UGCUCCUCUCCAAUUCUGCACAA-3' (SEQ ID NO: 306), 5'-ACCGCUUCACCAAUUAGUUGAGUGAAGGUGGGCUGCUUGCAUCAGCCU AAUGUCGAGAAGUGCUUUCUUCGGAAAGUAACCCUCGAAACAAAUUCAG UGCUCCUCUC-3' (SEQ ID NO: 307), 5'-ACCGCUUCACCAAAAGCUGUCCCUUAGGGGAUUAGAACUUGAGUGAAGG UGGGCUGCUUGCAUCAGCCUAAUGUCGAGAAGUGCUUUCUUCGGAAAG UAACCCUCGAAACAAAUUCAGUGCUCCUCUCCAAUUCUGCACAA-3' (SEQ ID NO: 308), or 5'-ACCGCUUCACCAAAAGCUGUCCCUUAGGGGAUUAGAACUUGAGUGAAGG UGGGCUGCUUGCAUCAGCCUAAUGUCGAGAAGUGCUUUCUUCGGAAAG UAACCCUCGAAACAAAUUCAGUGCUCCUCUC-3' (SEQ ID NO: 309). As still yet another example, the engineered tracrRNA may be 5'-ACCGCUUCACCAAGAAAUUGAGUGAAGGUGGGCUGCUUGCAUCAGCCUA AUGUCGAGAAGUGCUUUCUUCGGAAAGUAACCCUCGAAACAAAUUCAGU GCUCCUCUCCAAUUCUGCACAA-3' (SEQ ID NO: 310), or 5'-ACCGCUUCACCAAGAAAUUGAGUGAAGGUGGGCUGCUUGCAUCAGCCUA AUGUCGAGAAGUGCUUUCUUCGGAAAGUAACCCUCGAAACAAAUUCAGU GCUCCUCUC-3' (SEQ ID NO: 311).

### 2. Wildtype crRNA or engineered crRNA

The crRNA (CRISPR RNA) of the present disclosure comprises a sequence that interacts with a target nucleic acid, and a sequence that interacts with a Cas12f1 (Cas14a1) protein (a sequence that interacts with a tracrRNA). The crRNA may recognize, bind to, or target a target nucleic acid, and the crRNA may recognize or bind to a tracrRNA. In addition, the crRNA may recognize or bind to a Cas12f1 (Cas14a1) protein. The crRNA described herein comprises both a wildtype crRNA and an engineered crRNA.

The wildtype crRNA comprises a wildtype repeat sequence and a guide sequence. Here, the wildtype repeat sequence and the guide sequence are sequentially located in the wildtype crRNA in a 5' to 3' direction. Here, the wildtype repeat sequence is 5'-GUUGCAGAACCCGAAUAGACGAAUGAAGGAAUGCAAC-3' (SEQ ID NO: 312).

The engineered crRNA of the present disclosure is an engineered form of a wildtype crRNA, of which a part of a nucleotide sequence is artificially modified, which is modified to have a shorter length, and/or to which a U-rich tail sequence is added to the 3' end (FIG. 3). Here, the engineered crRNA is modified to improve an interaction with the engineered tracrRNA described above, or modified to further improve a function of an engineered guide RNA (or an engineered CRISPR/Cas12f1 (Cas14a1) system).

In an embodiment, the engineered crRNA comprises a sixth sequence, a seventh sequence, and a guide sequence. Here, the sixth sequence, the seventh sequence, and the guide sequence may be sequentially located in the engineered crRNA in a 5' to 3' direction.

In an embodiment, the engineered crRNA comprises a sixth sequence, a seventh sequence, a guide sequence, and a U-rich tail sequence. Here, the sixth sequence, the seventh sequence, the guide sequence, and the U-rich tail sequence may be sequentially located in the engineered crRNA in a 5' to 3' direction. Here, the sixth sequence and the seventh sequence are each divided based on the regions binding to the engineered tracrRNA (FIG. 3).

In an embodiment, the engineered crRNA comprises a wildtype repeat sequence, a guide sequence, and a U-rich tail sequence. Here, the wildtype repeat sequence, the guide sequence, and the U-rich tail sequence may be sequentially located in the engineered crRNA in a 5' to 3' direction. Here, the wildtype repeat sequence is 5'-GUUGCAGAACCCGAAUAGACGAAUGAAGGAAUGCAAC-3' (SEQ ID NO: 312).

In an embodiment, the engineered crRNA comprises a part of the wildtype repeat sequence and a guide sequence, or a part of the wildtype repeat sequence, a guide sequence, and a U-rich tail sequence. Here, the part of the wildtype repeat sequence and the guide sequence; or the part of the wildtype repeat sequence, the guide sequence, and the U-rich tail sequence may be sequentially located in the engineered crRNA in a 5' to 3' direction. Here, the part of the wildtype repeat sequence is a part of 5'-GUUGCAGAACCCGAAUAGACGAAUGAAGGAAUGCAAC-3' (SEQ ID NO: 312) and does not comprise a sequential partial sequence at the 5' end of SEQ ID NO: 312.

Hereinafter, the sixth sequence, the seventh sequence, the guide sequence, and the U-rich tail sequence will be described in detail.

### 2-1) Sixth sequence (other strand for stem 5 / part of repeat sequence)

The engineered crRNA comprises a sixth sequence. The sixth sequence is an essential sequence included in the engineered crRNA. The sixth sequence is a sequence binding complementarily to a part of the engineered tracrRNA. Here, the sixth sequence forms an RNA duplex by binding complementarily to the first sequence of the engineered tracrRNA. Here, the RNA duplex formed by the sixth sequence and the first sequence is stem 5. At least one nucleotide forming stem 5 may interact with a WED domain and/or a ZF domain of a Cas12f1 protein.

The sixth sequence does not comprise 5'-ACGAA-3'.

The sixth sequence comprises at least one nucleotide forming a complementary bond to the first sequence. Here, the sixth sequence may comprise at least one nucleotide not involved in forming a complementary bond to the first sequence. That is, the sixth sequence may form at least one complementary bond with the first sequence.

The sixth sequence comprises a nucleotide forming at least one base pair with the first sequence. Here, the sixth sequence may comprise a nucleotide not involved in forming at least one base pair with the first sequence. That is, the sixth sequence may form at least one base pair with the first sequence.

The sixth sequence is located at the 5' end of the engineered crRNA.

The sixth sequence may be a sequence that is modified in various ways according to the first sequence of the engineered tracrRNA described above. That is, the sixth sequence may be a sequence that is variously modified according to N and/or V present in the first sequence. Here, the sixth sequence may comprise a nucleotide forming a complementary bond to at least one N and/or V present in the first sequence.

The sixth sequence may be a sequence selected from the following sequences:
5'-GUUGCAGAACCCGAAUAG(N)ₐUGAAGGA-3' (SEQ ID NO: 313);
5'-GUUGCAGAACCCGAAUAG(B)ₐNNNNBUGAAGGA-3' (SEQ ID NO: 314);
5'-GUUGCAGAACCCGAAUAG(N)_{b}NNNBNUGAAGGA-3' (SEQ ID NO: 315);
5'-GUUGCAGAACCCGAAUAG(N)_{c}NNBNNUGAAGGA-3' (SEQ ID NO: 316);
5'-GUUGCAGAACCCGAAUAG(N)_{d}NBNNNUGAAGGA-3' (SEQ ID NO: 317);
5'-GUUGCAGAACCCGAAUAG(N)ₐBNNNNUGAAGGA-3' (SEQ ID NO: 318); and
a part of a sequence selected from SEQ ID NOS: 313 to 318.

Here, N may be each independently A, C, G or U, and each B may be independently U, C or G.

Here, a may be an integer from 0 to 4, b may be an integer from 0 to 1, c may be an integer from 0 to 2, and d may be an integer from 0 to 3.

Here, in (N)ₐ, (N)_{c} or (N)_{d}, when a, c and d are integers other than 0 or 1, N in (N)ₐ, (N)_{c} or (N)_{d} may each independently be A, C, G or U. For example, when d is 3, (N)₃ refers to 5'-NNN-3' wherein N may each independently be A, C, G, or U.

Here, for (B)ₐ, when a is an integer other than 0 or 1, each B in (B)ₐ may be independently U, C, or G. For example, when a is 4, (B)₄ represents 5'-BBBB-3' wherein each B may independently be U, C or G.

Here, 5'-GUUGCAGAACCCGAAUAG-3' (SEQ ID NO: 319) located at the 5' end of SEQ ID NOS: 313 to 318 and/or 5'-UGAAGGA-3' located at the 3' end thereof may be optionally modified. The modification may be one in which at least one nucleotide in SEQ ID NO: 319 and/or 5'-UGAAGGA-3' is deleted or substituted with another nucleotide. Alternatively, the modification may be one in which one or more nucleotides are inserted into SEQ ID NO: 319 and/or 5'-UGAAGGA-3' .

The sixth sequence may be a sequence selected from SEQ ID NOS: 313 to 318 according to the first sequence of the engineered tracrRNA. Here, the sixth sequence may be in a close relationship with the first sequence. The sixth sequence may be designed in various ways according to the first sequence. That is, the sixth sequence may be designed in various ways according to N and/or V present in the first sequence. Here, the sixth sequence may comprise a nucleotide forming a complementary bond to at least one N and/or V present in the first sequence.

### i) Sequence 5'-GUUGCAGAACCCGAAUAG(N)ₐUGAAGGA-3' (SEQ ID NO: 313)

In an embodiment, when the first sequence is 5'-CAAAUUCA(N)ₐCCUCUCCAAUUCUGCACAA-3' (SEQ ID NO: 2), the sixth sequence may be 5'-GUUGCAGAACCCGAAUAG(N)ₐUGAAGGA-3' (SEQ ID NO: 313). Here, N may each independently be A, C, G or U. a may be an integer of 0 to 4. Here, at least one N of (N)ₐ of the sixth sequence may form a complementary bond to at least one N of (N)ₐ of the first sequence. For example, when the first sequence is 5'-CAAAUUCAUUCUCCUCUCCAAUUCUGCACAA-3' (SEQ ID NO: 320), the sixth sequence may be 5'-GUUGCAGAACCCGAAUAGUGCCUGAAGGA-3' (SEQ ID NO: 321) (among the nucleotides corresponding to respective (N)ₐ, the nucleotides that form a complementary bond to each other are each underlined). The examples are for an illustrative purpose only, and the scope of the present disclosure is not limited thereto.

### ii) Sequence 5'-GUUGCAGAACCCGAAUAG(B)ₐNNNNBUGAAGGA-3' (SEQ ID NO: 314)

In an embodiment, when the first sequence is 5'-CAAAUUCAVNNNN(V)ₐCCUCUCCAAUUCUGCACAA-3' (SEQ ID NO: 3), the sixth sequence may be 5'-GUUGCAGAACCCGAAUAG(B)ₐNNNNBUGAAGGA-3' (SEQ ID NO: 314). Here, N may be each independently A, C, G or U, and each B may be U, C or G. a may be an integer of 0 to 4. Here, at least one N and/or B in (B)ₐNNNNB of the sixth sequence may form a complementary bond to at least one N and/or V in VNNNN(V)ₐ of the first sequence. For example, when the first sequence is 5'-CAAAUUCACAUUCCCCUCUCCAAUUCUGCACAA-3' (SEQ ID NO: 322), the sixth sequence may be 5'-GUUGCAGAACCCGAAUAGGAAAGCUGAAGGA-3' (SEQ ID NO: 323) (among the nucleotides corresponding to (B)ₐNNNNB and VNNNN(V)ₐ, the nucleotides that form complementary bonds with each other are respectively underlined). The examples are for an illustrative purpose only, and the scope of the present disclosure is not limited thereto.

### iii) Sequence 5'-GUUGCAGAACCCGAAUAG(N)_{b}NNNBNUGAAGGA-3' (SEQ ID NO: 315)

In an embodiment, when the first sequence is 5'-CAAAUUCANVNNN(N)_{b}CCUCUCCAAUUCUGCACAA-3' (SEQ ID NO: 4), the sixth sequence may be 5'-GUUGCAGAACCCGAAUAG(N)_{b}NNNBNUGAAGGA-3' (SEQ ID NO: 315). Here, N may be each independently A, C, G or U, and B may be U, C or G. b may be an integer of 0 to 1. Here, at least one N and/or B in (N)_{b}NNNBN of the sixth sequence may form a complementary bond to at least one N and/or V in NVNNN(N)_{b} of the first sequence. For example, when the first sequence is 5'-CAAAUUCAGUGCUCCUCUCCAAUUCUGCACAA-3' (SEQ ID NO: 324), the sixth sequence may be 5'-GUUGCAGAACCCGAAUAGAGCAAUGAAGGA-3' (SEQ ID NO: 325) (among the nucleotides corresponding to (N)_{b}NNNBN and NVNNN(N)_{b}, the nucleotides that form complementary bonds with each other are respectively underlined). The examples are for an illustrative purpose only, and the scope of the present disclosure is not limited thereto.

### iv) Sequence of 5'-GUUGCAGAACCCGAAUAG(N)_{c}NNBNNUGAAGGA-3' (SEQ ID NO: 316)

In an embodiment, when the first sequence is 5'-CAAAUUCANNVNN(N)_{c}CCUCUCCAAUUCUGCACAA-3' (SEQ ID NO: 5), the sixth sequence may be 5'-GUUGCAGAACCCGAAUAG(N)_{c}NNBNNUGAAGGA-3' (SEQ ID NO: 316). Here, N may be each independently A, C, G or U, and B may be U, C or G. c may be an integer of 0 to 2. Here, at least one N and/or B in (N)_{c}NNNBN of the sixth sequence may form a complementary bond to at least one N and/or V in NNVNN(N)_{c} of the first sequence. For example, when the first sequence is 5'-CAAAUUCAUUUCUCCUCUCCAAUUCUGCACAA-3' (SEQ ID NO: 326), the sixth sequence may be 5'-GUUGCAGAACCCGAAUAGAGGAAUGAAGGA-3' (SEQ ID NO: 327) (among the nucleotides corresponding to (N)_{c}NNBNN and NNVNN(N)_{c}, the nucleotides that form complementary bonds to each other are respectively underlined). The examples are for an illustrative purpose only, and the scope of the present disclosure is not limited thereto.

### v) Sequence 5'-GUUGCAGAACCCGAAUAG(N)_{d}NBNNNUGAAGGA-3' (SEQ ID NO: 317)

In an embodiment, when the first sequence is 5'-CAAAUUCANNNVN(N)_{d}CCUCUCCAAUUCUGCACAA-3' (SEQ ID NO: 6), the sixth sequence may be 5'-GUUGCAGAACCCGAAUAG(N)_{d}NBNNNUGAAGGA-3' (SEQ ID NO: 317). Here, N may be each independently A, C, G or U, and B may be U, C or G. d may be an integer of 0 to 3. Here, at least one N and/or B in (N)_{d}NBNNN of the sixth sequence may form a complementary bond to at least one N and/or V in NNNVN(N)_{d} of the first sequence. For example, when the first sequence is 5'-CAAAUUCACUGCCUUCCUCUCCAAUUCUGCACAA-3' (SEQ ID NO: 328), the sixth sequence may be 5'-GUUGCAGAACCCGAAUAGAAGGCAGUGAAGGA-3' (SEQ ID NO: 329) (among the nucleotides corresponding to (N)aNBNNN and NNNVN(N)_{d}, the nucleotides that form complementary bonds to each other are respectively underlined). The examples are for an illustrative purpose only, and the scope of the present disclosure is not limited thereto.

### vi) Sequence 5'-GUUGCAGAACCCGAAUAG(N)ₐBNNNNUGAAGGA-3' (SEQ ID NO: 318)

In an embodiment, when the first sequence is 5'-CAAAUUCANNNNV(N)aCCUCUCCAAUUCUGCACAA-3' (SEQ ID NO: 7), the sixth sequence may be 5'-GUUGCAGAACCCGAAUAG(N)ₐBNNNNUGAAGGA-3' (SEQ ID NO: 318). Here, N may be each independently A, C, G or U, and B may be U, C or G. a may be an integer of 0 to 4. Here, at least one N and/or B in (N)ₐBNNNN of the sixth sequence may form a complementary bond to at least one N and/or V in NNNNV(N)ₐ of the first sequence. For example, when the first sequence is 5'-CAAAUUCACAUUGAUCCUCUCCAAUUCUGCACAA-3' (SEQ ID NO: 330), the sixth sequence may be 5'-GUUGCAGAACCCGAAUAGACGAAAGUGAAGGA-3' (SEQ ID NO: 331) (among the nucleotides corresponding to (N)ₐBNNNN and NNNNV(N)ₐ, the nucleotides that form complementary bonds to each other are respectively underlined). The examples are for an illustrative purpose only, and the scope of the present disclosure is not limited thereto.

### vii) Part of sequence selected from SEQ ID NOS: 313 to 318

In an embodiment, the sixth sequence may be a part of 5'-GUUGCAGAACCCGAAUAG(N)ₐUGAAGGA-3' (SEQ ID NO: 313), and may comprise 5'-(N)ₐUGAAGGA-3' in SEQ ID NO: 313while not comprising a sequential partial sequence at the 5' end of SEQ ID NO: 313. Here, N may each independently be A, C, G or U. a may be an integer of 0 to 4.

In an embodiment, the sixth sequence may be a part of 5'-GUUGCAGAACCCGAAUAGNNNNUGAAGGA-3' (SEQ ID NO: 332) and may comprise 5'-NNNNUGAAGGA-3' (SEQ ID NO: 333) while not comprising a partial sequence at the 5' end. For example, the sixth sequence may be 5'-UGCAGAACCCGAAUAGNNNNUGAAGGA-3' (SEQ ID NO: 334), 5'-CAGAACCCGAAUAGNNNNUGAAGGA-3' (SEQ ID NO: 335), 5'-GAACCCGAAUAGNNNNUGAAGGA-3' (SEQ ID NO: 336), 5'-ACCCGAAUAGNNNNUGAAGGA-3' (SEQ ID NO: 337), 5'-CCGAAUAGNNNNUGAAGGA-3' (SEQ ID NO: 338), 5'-GAAUAGNNNNUGAAGGA-3' (SEQ ID NO: 339), 5'-AUAGNNNNUGAAGGA-3' (SEQ ID NO: 340), 5'-AGNNNNUGAAGGA-3' (SEQ ID NO: 341), or 5'-NNNNUGAAGGA-3' (SEQ ID NO: 333). Here, N may each independently be A, C, G or U. The examples are for an illustrative purpose only, and the scope of the present disclosure is not limited thereto.

In another embodiment, the sixth sequence may be a part of 5'-GUUGCAGAACCCGAAUAG(B)ₐNNNNBUGAAGGA-3' (SEQ ID NO: 314), and may comprise 5'-NNNNBUGAAGGA-3' (SEQ ID NO: 342) in SEQ ID NO: 314 while not comprising a sequential partial sequence at the 5' end of SEQ ID NO: 314. Here, N may each independently be A, C, G or U. Each B may independently be U, C or G. a may be an integer of 0 to 4.

In an embodiment, the sixth sequence may be a part of 5'-GUUGCAGAACCCGAAUAGNNNNBUGAAGGA-3' (SEQ ID NO: 343), and may comprise 5'-NNNNBUGAAGGA-3' (SEQ ID NO: 342) while not comprising a partial sequence at the 5' end. For example, the sixth sequence may be 5'-UGCAGAACCCGAAUAGNNNNBUGAAGGA-3' (SEQ ID NO: 344), 5'-CAGAACCCGAAUAGNNNNBUGAAGGA-3' (SEQ ID NO: 345), 5'-GAACCCGAAUAGNNNNBUGAAGGA-3' (SEQ ID NO: 346), 5'-ACCCGAAUAGNNNNBUGAAGGA-3' (SEQ ID NO: 347), 5'-CCGAAUAGNNNNBUGAAGGA-3' (SEQ ID NO: 348), 5'-GAAUAGNNNNBUGAAGGA-3' (SEQ ID NO: 349), 5'-AUAGNNNNBUGAAGGA-3' (SEQ ID NO: 350), 5'-AGNNNNBUGAAGGA-3' (SEQ ID NO: 351), or 5'-NNNNBUGAAGGA-3' (SEQ ID NO: 342). Here, N may each independently be A, C, G or U. B may be U, C or G. The examples are for an illustrative purpose only, and the scope of the present disclosure is not limited thereto.

In another embodiment, the sixth sequence may be a part of 5'-GUUGCAGAACCCGAAUAG(N)_{b}NNNBNUGAAGGA-3' (SEQ ID NO: 315), and may comprise 5'-NNNBNUGAAGGA-3' (SEQ ID NO: 352) in SEQ ID NO: 315 while not comprising a sequential partial sequence at the 5' end of SEQ ID NO: 315. Here, N may each independently be A, C, G or U. B may be U, C or G. b may be an integer of 0 to 1.

In an embodiment, the sixth sequence may be a part of 5'-GUUGCAGAACCCGAAUAGNNNBNUGAAGGA-3' (SEQ ID NO: 353), and may comprise 5'-NNNBNUGAAGGA-3' (SEQ ID NO: 352) while not comprising a partial sequence at the 5' end of SEQ ID NO: 353. For example, the sixth sequence may be 5'-UGCAGAACCCGAAUAGNNNBNUGAAGGA-3' (SEQ ID NO: 354), 5'-CAGAACCCGAAUAGNNNBNUGAAGGA-3' (SEQ ID NO: 355), 5'-GAACCCGAAUAGNNNBNUGAAGGA-3' (SEQ ID NO: 356), 5'-ACCCGAAUAGNNNBNUGAAGGA-3' (SEQ ID NO: 357), 5'-CCGAAUAGNNNBNUGAAGGA-3' (SEQ ID NO: 358), 5'-GAAUAGNNNBNUGAAGGA-3' (SEQ ID NO: 359), 5'-AUAGNNNBNUGAAGGA-3' (SEQ ID NO: 360), 5'-AGNNNBNUGAAGGA-3' (SEQ ID NO: 361), or 5'-NNNBNUGAAGGA-3' (SEQ ID NO: 352). Here, N may each independently be A, C, G or U. B may be U, C or G. The examples are for an illustrative purpose only, and the scope of the present disclosure is not limited thereto.

In another embodiment, the sixth sequence may be a part of 5'-GUUGCAGAACCCGAAUAG(N)_{c}NNBNNUGAAGGA-3' (SEQ ID NO: 316), and may comprise 5'-NNBNNUGAAGGA-3' (SEQ ID NO: 362) in SEQ ID NO: 316 while not comprising a sequential partial sequence at the 5' end of SEQ ID NO: 316. Here, N may each independently be A, C, G or U. B may be U, C or G. c may be an integer of 0 to 2.

In an embodiment, the sixth sequence may be a part of 5'-GUUGCAGAACCCGAAUAGNNBNNUGAAGGA-3' (SEQ ID NO: 363), and may comprise 5'-NNBNNUGAAGGA-3' (SEQ ID NO: 362) while not comprising a partial sequence at the 5' end of SEQ ID NO: 363. For example, the sixth sequence may be 5'-UGCAGAACCCGAAUAGNNBNNUGAAGGA-3' (SEQ ID NO: 364), 5'-CAGAACCCGAAUAGNNBNNUGAAGGA-3' (SEQ ID NO: 365), 5'-GAACCCGAAUAGNNBNNUGAAGGA-3' (SEQ ID NO: 366), 5'-ACCCGAAUAGNNBNNUGAAGGA-3' (SEQ ID NO: 367), 5'-CCGAAUAGNNBNNUGAAGGA-3' (SEQ ID NO: 368), 5'-GAAUAGNNBNNUGAAGGA-3' (SEQ ID NO: 369), 5'-AUAGNNBNNUGAAGGA-3' (SEQ ID NO: 370), 5'-AGNNBNNUGAAGGA-3' (SEQ ID NO: 371), or 5'-NNBNNUGAAGGA-3' (SEQ ID NO: 362). Here, N may each independently be A, C, G or U. B may be U, C or G. The examples are for an illustrative purpose only, and the scope of the present disclosure is not limited thereto.

In another embodiment, the sixth sequence may be a part of 5'-GUUGCAGAACCCGAAUAG(N)_{d}NBNNNUGAAGGA-3' (SEQ ID NO: 317), and may comprise 5'-NBNNNUGAAGGA-3' (SEQ ID NO: 372) in SEQ ID NO: 317 while not comprising a sequential partial sequence at the 5' end of SEQ ID NO: 317. Here, N may each independently be A, C, G or U. B may be U, C or G. d may be an integer of 0 to 3.

In an embodiment, the sixth sequence may be a part of 5'-GUUGCAGAACCCGAAUAGNBNNNUGAAGGA-3' (SEQ ID NO: 373), and may comprise 5'-NBNNNUGAAGGA-3' (SEQ ID NO: 372) while not comprising a partial sequence at the 5' end of SEQ ID NO: 373. For example, the sixth sequence may be 5'-UGCAGAACCCGAAUAGNBNNNUGAAGGA-3' (SEQ ID NO: 374), 5'-CAGAACCCGAAUAGNBNNNUGAAGGA-3' (SEQ ID NO: 375), 5'-GAACCCGAAUAGNBNNNUGAAGGA-3' (SEQ ID NO: 376), 5'-ACCCGAAUAGNBNNNUGAAGGA-3' (SEQ ID NO: 377), 5'-CCGAAUAGNBNNNUGAAGGA-3' (SEQ ID NO: 378), 5'-GAAUAGNBNNNUGAAGGA-3' (SEQ ID NO: 379), 5'-AUAGNBNNNUGAAGGA-3' (SEQ ID NO: 380), 5'-AGNBNNNUGAAGGA-3' (SEQ ID NO: 381), or 5'-NBNNNUGAAGGA-3' (SEQ ID NO: 372). Here, N may each independently be A, C, G or U. B may be U, C or G. The examples are for an illustrative purpose only, and the scope of the present disclosure is not limited thereto.

In another embodiment, the sixth sequence may be a part of 5'-GUUGCAGAACCCGAAUAG(N)ₐBNNNNUGAAGGA-3' (SEQ ID NO: 318), may comprise 5'-BNNNNUGAAGGA-3' (SEQ ID NO: 382) in SEQ ID NO: 318 while not comprising a sequential partial sequence at the 5' end of SEQ ID NO: 318. Here, N may each independently be A, C, G or U. B may be U, C or G. a may be an integer of 0 to 4.

In an embodiment, the sixth sequence may be a part of 5'-GUUGCAGAACCCGAAUAGBNNNNUGAAGGA-3' (SEQ ID NO: 383), and may comprise 5'-BNNNNUGAAGGA-3' (SEQ ID NO: 382) while not comprising a partial sequence at the 5' end of SEQ ID NO: 383. For example, the sixth sequence may be 5'-UGCAGAACCCGAAUAGBNNNNUGAAGGA-3' (SEQ ID NO: 384), 5'-CAGAACCCGAAUAGBNNNNUGAAGGA-3' (SEQ ID NO: 385), 5'-GAACCCGAAUAGBNNNNUGAAGGA-3' (SEQ ID NO: 386), 5'-ACCCGAAUAGBNNNNUGAAGGA-3' (SEQ ID NO: 387), 5'-CCGAAUAGBNNNNUGAAGGA-3' (SEQ ID NO: 388), 5'-GAAUAGBNNNNUGAAGGA-3' (SEQ ID NO: 389), 5'-AUAGBNNNNUGAAGGA-3' (SEQ ID NO: 390), 5'-AGBNNNNUGAAGGA-3' (SEQ ID NO: 391), or 5'-BNNNNUGAAGGA-3' (SEQ ID NO: 382). Here, N may each independently be A, C, G or U. B may be U, C or G. The examples are for an illustrative purpose only, and the scope of the present disclosure is not limited thereto.

### 2-2) Seventh sequence (interaction with the third sequence (or stem 3) /part of repeat sequence)

The engineered crRNA comprises a seventh sequence. The seventh sequence is an essential sequence included in the engineered crRNA. The seventh sequence is a sequence binding complementarily to a part of the engineered tracrRNA. Here, the seventh sequence forms an RNA duplex by binding complementarily to a part of the third sequence of the engineered tracrRNA.

The seventh sequence comprises at least one nucleotide that forms a complementary bond to the third sequence. Here, the seventh sequence may comprise at least one nucleotide that does not form a complementary bond to the third sequence. That is, the seventh sequence may form at least one complementary bond with the third sequence.

The seventh sequence may comprise a nucleotide that forms at least one base pair with the third sequence. Here, the seventh sequence may comprise a nucleotide that does not form at least one base pair with the third sequence. That is, the seventh sequence may form at least one base pair with the third sequence.

The seventh sequence is located at the 3' end of the sixth sequence.

The seventh sequence is covalently linked to the 3' end of the sixth sequence.

In an embodiment, the seventh sequence may be 5'-AUGCAAC-3'.

In another embodiment, the seventh sequence may be a sequence at least 70% or more identical or similar to 5'-AUGCAAC-3'. Alternatively, the seventh sequence may be a sequence having sequence identity or sequence similarity of at least 70% or more to 5'-AUGCAAC-3.

In another embodiment, the seventh sequence may be a sequence identical or similar to 5'-AUGCAAC-3' by at least 70% to 85%, at least 70% to 100%, or at least 85% to 100%. Alternatively, the seventh sequence may be a sequence having sequence identity or sequence similarity to 5'-AUGCAAC-3' of at least 70% to 85%, at least 70% to 100%, or at least 85% to 100%.

In another embodiment, the seventh sequence may be a sequence identical or similar to 5'-AUGCAAC-3' by at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100%. Alternatively, the seventh sequence may be a sequence having sequence identity or sequence similarity to 5'-AUGCAAC-3' of at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100%.

### 2-3) Guide sequence

The wildtype crRNA and the engineered crRNA comprise a guide sequence. The guide sequence is an RNA sequence that recognizes, binds to, or targets a target nucleic acid. More specifically, the guide sequence is an RNA sequence binding complementarily to a target sequence, an RNA sequence capable of forming a complementary bond to a target sequence, or an RNA sequence having complementarity to a target sequence. Alternatively, the guide sequence is an RNA sequence identical or similar to a protospacer sequence. Here, the protospacer sequence is in a close relationship to a target sequence, and a description related thereto is as described in the section "target sequence, target strand, and non-target strand" in definition of terms. The guide sequence is a sequence that is modified according to a target sequence, and the guide sequence may vary depending on the target sequence. In addition, the guide sequence is an RNA sequence. When a target nucleic acid is DNA, the guide sequence comprises uridine (U) capable of forming a complementary bond to adenosine (A) present in the target sequence. Alternatively, when a target nucleic acid is DNA, the guide sequence comprises uridine (U) instead of thymidine (T), for thymidine (T) present in a protospacer sequence. In addition, the guide sequence is also referred to as a spacer sequence. Hereinafter, the terms "guide sequence" and "spacer sequence" are used interchangeably.

The guide sequence is located at the 3' end of the wildtype repeat sequence. Alternatively, the guide sequence is located at the 3' end of the seventh sequence.

The guide sequence is covalently linked to the 3' end of the wildtype repeat sequence. Alternatively, the guide sequence is covalently linked to the 3' end of the seventh sequence.

The guide sequence is a sequence of 15 to 40 nucleotides.

In an embodiment, the guide sequence may be a sequence of 15 to 20, 15 to 25, 15 to 30, 15 to 35 or 15 to 40 nucleotides. Alternatively, the guide sequence may be a sequence of 20 to 25, 20 to 30, 20 to 35 or 20 to 40 nucleotides. Alternatively, the guide sequence may be a sequence of 25 to 30, 25 to 35 or 25 to 40 nucleotides. Alternatively, the guide sequence may be a sequence of 30 to 35 or 30 to 40 nucleotides. Alternatively, the guide sequence may be a sequence of 35 to 40 nucleotides.

In another embodiment, the guide sequence may be a sequence of 15, 16, 17, 18, 19, 20, 21, 22, 23, 24, 25, 26, 27, 28, 29, 30, 31, 32, 33, 34, 35, 36, 37, 38, 39 or 40 nucleotides.

The guide sequence is a sequence binding complementarily to a target sequence. Here, the complementary binding may optionally comprise at least one mismatched binding. For example, the guide sequence is a sequence binding complementarily to a target sequence wherein the complementary binding may comprise 0 to 5 mismatched bindings.

The guide sequence is a sequence complementary to a target sequence. Here, the complementary sequence may comprise a sequence of 0 to 5 mismatched nucleotides to the target sequence. Alternatively, the guide sequence may be a nucleotide sequence that is at least 70% complementary to a target sequence. Here, when the target sequence is DNA and adenosine (A) is present therein, the guide sequence may comprise uridine (U) capable of forming a complementary bond to the adenosine (A).

In an embodiment, the guide sequence may be a sequence complementary to a target sequence by at least 70% to 75%, at least 70% to 80%, at least 70% to 85%, at least 70% to 90%, at least 70% to 95%, at least 70% to 100%, at least 75% to 80%, at least 75% to 85%, at least 75% to 90%, at least 75% to 95% or at least 75% to 100%. Alternatively, the guide sequence may be a sequence complementary to a target sequence by at least 80% to 85%, at least 80% to 90%, at least 80% to 95%, at least 80% to 100%, at least 85% to 90%, at least 85% to 95%, or at least 85% to 100%. Alternatively, the guide sequence may be a sequence complementary to a target sequence by at least 90% to 95%, at least 90% to 100%, or at least 95% to 100%. Alternatively, the guide sequence may be a sequence complementary to a target sequence by at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100%.

The guide sequence is a sequence the same as or similar to the protospacer sequence. That is, the guide sequence is a sequence having sequence identity or sequence similarity to the protospacer sequence. Here, the sequence identity or sequence similarity may be at least 70% or more. Here, when the protospacer sequence is DNA and thymidine (T) is present therein, the guide sequence may comprise uridine (U) instead of thymidine (T).

In an embodiment, the guide sequence may be a sequence identical or similar to the protospacer sequence by at least 70% to 75%, at least 70% to 80%, at least 70% to 85%, at least 70% to 90%, at least 70% to 95%, at least 70% to 100%, at least 75% to 80%, at least 75% to 85%, at least 75% to 90%, at least 75% to 95% or at least 75% to 100%. Alternatively, the guide sequence may be a sequence identical or similar to the protospacer sequence by at least 80% to 85%, at least 80% to 90%, at least 80% to 95%, at least 80% to 100%, at least 85% to 90%, at least 85% to 95%, or at least 85% to 100%. Alternatively, the guide sequence may be a sequence identical or similar to the protospacer sequence by at least 90% to 95%, at least 90% to 100%, or at least 95% to 100%. Alternatively, the guide sequence may be a sequence identical or similar to the protospacer sequence by at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100%.

In another embodiment, the guide sequence may be a sequence having sequence identity or sequence similarity to the protospacer sequence of at least 70% to 75%, at least 70% to 80%, at least 70% to 85%, at least 70% to 90%, at least 70% to 95%, at least 70% to 100%, at least 75% to 80%, at least 75% to 85%, at least 75% to 90%, at least 75% to 95% or at least 75% to 100%. Alternatively, the guide sequence may be a sequence having sequence identity or sequence similarity to the protospacer sequence of at least 80% to 85%, at least 80% to 90%, at least 80% to 95%, at least 80% to 100%, at least 85% to 90%, at least 85% to 95%, or at least 85% to 100%. Alternatively, the guide sequence may be a sequence having sequence identity or sequence similarity to the protospacer sequence of at least 90% to 95%, at least 90% to 100%, or at least 95% to 100%. Alternatively, the guide sequence may be a sequence having sequence identity or sequence similarity to the protospacer sequence of at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100%.

### 2-4) U-rich tail sequence

The engineered crRNA may optionally further comprise a U-rich tail sequence. The U-rich tail sequence is a uridine (U)-rich sequence added to the 3' end of the engineered crRNA. The present inventors' previous study reported that the U-rich tail sequence serves to increase editing efficiency for a target gene (target nucleic acid) using an engineered guide RNA (or the engineered CRISPR/Cas12f1 (Cas14a1) system) (PCT/KR2020/014961).

The U-rich tail sequence may be 5'-(UₐN)_{d}Uₑ-3', 5'-UₐVUₐVUₑ-3', or 5'-UₐVUₐVUₐVUₑ-3'.

Here, N may be A, C, G or U, and each V may be independently A, C or G.

Here, a may be an integer of 0 to 4, d may be an integer of 0 to 3, and e may be an integer of 0 to 10.

In an embodiment, the U-rich tail sequence may be 5'-(UₐU)_{d}Uₑ-3', that is, Uₓ. Here, x may be an integer of 0 to 22. For example, the U-rich tail sequence may be U, UU, UUU, UUUU, UUUUU, UUUUUU, UUUUUUU, UUUUUUUU, UUUUUUUUUU or UUUUUUUUUU (SEQ ID NO: 392).

In another embodiment, the U-rich tail sequence may be 5'-(UₐA)_{d}Uₑ-3'. For example, the U-rich tail sequence may be UAU, UUAUUAU, UUUUAUUUU, UUUAUUUUU or UUUAUUUAUUUAU (SEQ ID NO: 393).

In another embodiment, the U-rich tail sequence may be 5'-(UₐG)_{d}Uₑ-3'. For example, the U-rich tail sequence may be UUG, UUUGU, UUGUUGU, UUUUGUUUU, UGUGUGUUUU (SEQ ID NO: 394) or UUUGUUUUGUUUU (SEQ ID NO: 395).

In another embodiment, the U-rich tail sequence may be 5'-(UₐC)_{d}Uₑ-3'. For example, the U-rich tail sequence may be CUUUUU, UCUCUUU, UUCUU, UUUCU, UUUUCUUUU, or UUUUCUUUUCUUUU (SEQ ID NO: 396).

In another embodiment, the U-rich tail sequence may be 5'-UₐAUₐAUₑ-3', 5'-UaAUaCUe-3', 5'-UaAUaGUe-3', 5'-UaCUaAUe-3', 5'-UaCUaCUe-3', 5'-UaCUaGUe-3', 5'-UₐGUₐAUₑ-3', 5'-UₐGUₐCUₑ-3', or 5'-UₐGUₐGUₑ-3'. For example, the U-rich tail sequence may be UUAUUUAUU, UUUCUAUUUU (SEQ ID NO: 397), UGUCU, UUAUGUUUUU (SEQ ID NO: 398), or UCUUUUGUU.

In another embodiment, the U-rich tail sequence may be 5'-UₐAUₐAUₐAUₑ-3', 5'-UaAUaAUaCUe-3', 5'-UaAUaAUaGUe-3', 5'-UaAUaCUaAUe-3', 5'-UaAUaCUaCUe-3', 5'-UaAUaCUaGUe-3', 5'-UaAUaGUaAUe-3', 5'-UaAUaGUaCUe-3', 5'-UaAUaGUaGUe-3', 5'-UaCUaAUaAUe-3', 5'-UaCUaAUaCUe-3', 5'-UaCUaAUaGUe-3', 5'-UaCUaCUaAUe-3', 5'-UₐCUₐCUₐCUₑ-3', 5'-UₐCUₐCUₐGUₑ-3', 5'-UₐCUₐGUₐAUₑ-3', 5'-UₐCUₐGUₐCUₑ-3', 5'-UₐCUₐGUₐGUₑ-3', 5'-UₐGUₐAUₐAUₑ-3', 5'-UₐGUₐAUₐCUₑ-3', 5'-UₐGUₐAUₐGUₑ-3', 5'-UₐGUₐCUₐAUₑ-3', 5'-UₐGUₐCUₐCUₑ-3', 5'-UₐGUₐCUₐGUₑ-3', 5'-UₐGUₐGUₐAUₑ-3', 5'-UₐGUₐGUₐCUₑ-3', or 5'-UₐGUₐGUₐGUₑ-3'. For example, the U-rich tail sequence may be UUUAUUCUGUU (SEQ ID NO: 399), UUCUCUUUCUUU (SEQ ID NO: 400), UGUUAUUUAUU (SEQ ID NO: 401), UUUCUUUAUGUUU (SEQ ID NO: 402), UAUUUGUUUC (SEQ ID NO: 403) or UUCUUGUUUUAUU (SEQ ID NO: 404).

### 2-5) Additional sequence

The wildtype crRNA and/or the engineered crRNA may optionally further comprise an additional sequence. The additional sequence may be located at the 5' end of the wildtype crRNA and/or the engineered crRNA. The additional sequence may be located at the 5' end of the wildtype repeat sequence or the engineered repeat sequence. The additional sequence may be located at the 5' end of the sixth sequence.

The additional sequence may be a sequence of 1 to 40 nucleotides (a sequence of 1 to 40 nts).

In an embodiment, the additional sequence may be a sequence of 1 to 5, 1 to 10, 1 to 15, 1 to 20, 1 to 25, 1 to 30, 1 to 35, 1 to 40, 5 to 10, 5 to 15, 5 to 20, 5 to 25, 5 to 30, 5 to 35 or 5 to 40 nucleotides. Alternatively, the additional sequence may be a sequence of 10 to 15, 10 to 20, 10 to 25, 10 to 30, 10 to 35, 10 to 40, 15 to 20, 15 to 25, 15 to 30, 15 to 35 or 15 to 40 nucleotides. Alternatively, the additional sequence may be a sequence of 20 to 25, 20 to 30, 20 to 35, 20 to 40, 25 to 30, 25 to 35 or 25 to 40 nucleotides. Alternatively, the additional sequence may be a sequence of 30 to 35, 30 to 40 or 35 to 40 nucleotides.

In another embodiment, the additional sequence may be a sequence of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39 or 40 nucleotides.

The additional sequence may be an arbitrary nucleotide sequence or an arbitrarily arranged nucleotide sequence.

The additional sequence may be a known nucleotide sequence in the art. For example, the additional sequence may be a hammerhead ribozyme nucleotide sequence. Here, the hammerhead ribozyme nucleotide sequence may be SEQ ID NO: 261 or SEQ ID NO: 262. The examples are for an illustrative purpose only, and the scope of the present disclosure is not limited thereto.

### 2-6) Chemical modification

The wildtype crRNA or the engineered crRNA described above may optionally comprise a chemical modification of at least one nucleotide. Here, the chemical modification may be a modification of any of various covalent bonds that may occur in a nucleotide base and/or sugar. For example, the chemical modification may include methylation, halogenation, acetylation, phosphorylation, phosphorothioate linkage, locked nucleic acid (LNA), 2'-O-methyl 3'phosphorothioate(MS), or 2'-O-methyl 3'thioPACE(MSP), or may include all modifications of a nucleic acid described in WO 2019/089820 A1, but is not limited thereto.

### 2-7) Examples of crRNA (examples of engineered crRNA)

Based on the previous description, we describe examples of a wildtype crRNA and engineered crRNAs. Specific descriptions of the components included in the examples below are the same as those described above for the corresponding components. The examples are for an illustrative purpose only, and the scope of the present disclosure is not limited thereto.

In an embodiment, the wildtype crRNA may comprise a wildtype repeat sequence and a guide sequence. Here, the wildtype repeat sequence may be 5'-GUUGCAGAACCCGAAUAGACGAAUGAAGGAAUGCAAC-3' (SEQ ID NO: 312). Here, the guide sequence may be a sequence of 15 to 40 nucleotides. The wildtype repeat sequence and the guide sequence may be sequentially located in the wildtype crRNA in a 5' to 3' direction (5'-[wildtype repeat sequence]-[guide sequence]-3'). As an example, the wildtype crRNA may comprise 5'-GUUGCAGAACCCGAAUAGACGAAUGAAGGAAUGCAACNNNNNNNNNNNN NNNNNNNN-3' (SEQ ID NO: 405). Here, N may each be A, C, G or U.

In an embodiment, the engineered crRNA may be a crRNA in which a U-rich tail sequence is added to the 3' end of the wildtype crRNA.

In an embodiment, the engineered crRNA may comprise a wildtype repeat sequence, a guide sequence and a U-rich tail sequence. Here, the wildtype repeat sequence may be 5'-GUUGCAGAACCCGAAUAGACGAAUGAAGGAAUGCAAC-3' (SEQ ID NO: 312). Here, the guide sequence may be a sequence of 15 to 40 nucleotides. Here, the U-rich tail sequence may be 5'-(UₐN)_{d}Uₑ-3', 5'-UₐVUₐVUₑ-3', or 5'-UₐVUₐVUₐVUₑ-3'. Here, N may be A, C, G or U, and each V may be independently A, C or G. a may be an integer of 0 to 4, d may be an integer of 0 to 3, and e may be an integer of 0 to 10. The wildtype repeat sequence, guide sequence, and U-rich tail sequence may be sequentially located in the wildtype crRNA in a 5' to 3' direction (5'-[wildtype repeat sequence]-[guide sequence]- [U-rich tail sequence]-3'). The engineered crRNA may optionally further comprise an additional sequence. Here, the additional sequence may be a sequence of 1 to 40 nucleotides (a sequence of 1 to 40 nts). The additional sequence may be located at the 5' end of the engineered crRNA (5'-[additional sequence]-[wildtype repeat sequence]-[guide sequence]-[U-rich tail sequence]-3').

For example, the engineered crRNA may comprise SEQ ID NO: 312, a guide sequence and a U-rich tail sequence. Here, the guide sequence may be a sequence of 15 to 40 nucleotides. Here, the U-rich tail sequence may be 5'-UUUU-3', 5'-UUUUAUU-3', or 5'-UUUUAUUUU-3'. The engineered crRNA may comprise SEQ ID NO: 312, the guide sequence and the U-rich tail sequence sequentially in a 5' to 3' direction (5'-[SEQ ID NO: 312]-[guide sequence]-[U-rich tail sequence]-3'). As an example, the engineered crRNA may comprise 5'-GUUGCAGAACCCGAAUAGACGAAUGAAGGAAUGCAACNNNNNNNNNNNN NNNNNNNNUUUUAUUUU-3' (SEQ ID NO: 406). Here, N may each be A, C, G or U. As another example, the engineered crRNA may comprise 5'-GUUGCAGAACCCGAAUAGACGAAUGAAGGAAUGCAACNNNNNNNNNNNN NNNNNNNNUUUU-3' (SEQ ID NO: 407). Here, N may each be A, C, G or U.

In another embodiment, the engineered crRNA may be a wildtype crRNA of which a part of a nucleotide sequence is artificially modified.

In an embodiment, the engineered crRNA may comprise a modified form of a wildtype repeat sequence of which at least one nucleotide is substituted with another nucleotide. The engineered crRNA may comprise a sixth sequence, a seventh sequence and a guide sequence. Here, the sixth sequence, the seventh sequence, and the guide sequence may be sequentially located in the engineered crRNA in a 5' to 3' direction (5'-[6th sequence]-[7th sequence]- [guide sequence]-3'). Here, the sixth sequence may be at least one sequence selected from the following sequences: 5'-GUUGCAGAACCCGAAUAG(N)ₐUGAAGGA-3' (a sequence of ID NO: 313); '5'-GUUGCAGAACCCGAAUAG(B)ₐNNNNBUGAAGG'-3' (SEQ ID NO: 314); '5'-GUUGCAGAACCCGAAUAG(N)_{b}NNNBNUGAAGG'-3' (SEQ ID NO: 315); '5'-GUUGCAGAACCCGAAUAG(N)_{c}NNBNNUGAAGG'-3' (SEQ ID NO: 316); '5'-GUUGCAGAACCCGAAUAG(N)_{d}NBNNNUGAAGG'-3' (SEQ ID NO: 317); and '5'-GUUGCAGAACCCGAAUAG(N)ₐBNNNNUGAAGG'-3' (SEQ ID NO: 318). Here, N may each be independently A, C, G or U, and each B may be independently U, C or G. a may be an integer from 0 to 4, b may be an integer from 0 to 1, c may be an integer from 0 to 2, and d may be an integer from 0 to 3. In (N)ₐ, (N)_{c} or (N)_{d}, when a, c and d are integers other than 0 or 1, N in (N)ₐ, (N)_{c} or (N)_{d} may each independently be A, C, G or U. Here, for (B)ₐ, when a is an integer other than 0 or 1, each B in (B)ₐ may be each independently U, C, or G. Here, the seventh sequence may be a sequence having sequence identity of at least 70% or more to '5'-AUGCAA'-3' ora sequence of'5'-AUGC'AC-3'. Here, the guide sequence may be a sequence of 15 to 40 nucleotides. The engineered crRNA may optionally further comprise an additional sequence. Here, the additional sequence may be a sequence of 1 to 40 nucleotides (a sequence of 1 to 40 nts). The additional sequence may be located at the 5' end of the engineered crRNA (5'-[additional sequence]-[6th sequence]-[7th sequence]-[guide sequence]-3').

For example, the engineered crRNA may comprise a sixth sequence, a seventh sequence and a guide sequence. Here, the sixth sequence may be 5'-GUUGCAGAACCCGAAUAGNGNNNUGAAGGA-3' (SEQ ID NO: 408) or a sequence having sequence identity of 70% or more to SEQ ID NO: 408. Here, the sequence having sequence identity of at least 70% or more to SEQ ID NO: 408 may be a sequence having sequence identity of at least 70% or more to the remaining sequence thereof except for 5'-GNGNNNUG-3'. Here, N may each independently be A, C, G or U. Here, the seventh sequence may be 5'-AUGCAAC-3' or a sequence having sequence identity of at least 70% or more to 5'-AUGCAAC-3'. Here, the guide sequence may be a sequence of 15 to 40 nucleotides. The engineered crRNA may comprise the sixth sequence, the seventh sequence and the guide sequence sequentially in a 5' to 3' direction (5'-[SEQ ID NO: 408 or sequence having sequence identity of 70% or more thereto]-[5'-AUGCAAC-3' or sequence having sequence identity of 70% or more thereto]-[guide sequence]-3'). As an example, the engineered crRNA may comprise 5'-GUUGCAGAACCCGAAUAGNGNNNUGAAGGAAUGCAACNNNNNNNNNNNN NNNNNNNN-3' (SEQ ID NO: 409). Here, N may each be A, C, G or U. As another example, the engineered crRNA may comprise 5'-GUUGCAGAACCCGAAUAGAGCAAUGAAGGAAUGCAACNNNNNNNNNNNN NNNNNNNN-3' (SEQ ID NO: 410).

In another embodiment, the engineered crRNA may be an engineered form of a wildtype crRNA of which a part of a nucleotide sequence is artificially modified are/or which is modified to have a shorter length.

In an embodiment, the engineered crRNA may comprise a modified form of a wildtype repeat sequence of which a part is deleted. The engineered crRNA may comprise a part of the wildtype repeat sequence and a guide sequence. Here, the part of the wildtype repeat sequence and the guide sequence may be sequentially located in the engineered crRNA in a 5' to 3' direction (5'-[part of wildtype repeat sequence]-[guide sequence]-3'). Here, the part of the wildtype repeat sequence may be a part of SEQ ID NO: 312, and may not comprise a partial sequence at the 5' end of SEQ ID NO: 312. Here, the guide sequence may be a sequence of 15 to 40 nucleotides. The engineered crRNA may optionally further comprise an additional sequence. Here, the additional sequence may be a sequence of 1 to 40 nucleotides (a sequence of 1 to 40 nts). The additional sequence may be located at the 5' end of the engineered crRNA (5'-[additional sequence]-[partial sequence of wildtype repeat sequence]-[guide sequence]-3'). For example, the engineered crRNA may comprise 5'-GAAUAGACGAAUGAAGGAAUGCAACNNNNNNNNNNNNNNNNNNNN-3' (SEQ ID NO: 411). Here, N may each be A, C, G or U.

In another embodiment, the engineered crRNA may comprise a modified form of a wildtype repeat sequence of which at least one nucleotide is substituted with another sequence and of which a part of a nucleotide sequence is deleted. The engineered crRNA may comprise a sixth sequence, a seventh sequence and a guide sequence. Here, the sixth sequence, the seventh sequence, and the guide sequence may be sequentially located in the engineered crRNA in a 5' to 3' direction (5'-[6th sequence]-[7th sequence]- [guide sequence]-3'). Here, the sixth sequence may be at least one sequence selected from the following sequences: 5'-GUUGCAGAACCCGAAUAG(N)ₐUGAAGGA-3' (SEQ ID NO: 313); 5'-GUUGCAGAACCCGAAUAG(B)ₐNNNNBUGAAGGA-3' (SEQ ID NO: 314); 5'-GUUGCAGAACCCGAAUAG(N)_{b}NNNBNUGAAGGA-3' (SEQ ID NO: 315); 5'-GUUGCAGAACCCGAAUAG(N)_{c}NNBNNUGAAGGA-3' (SEQ ID NO: 316); 5'-GUUGCAGAACCCGAAUAG(N)_{d}NBNNNUGAAGGA-3' (SEQ ID NO: 317); 5'-GUUGCAGAACCCGAAUAG(N)ₐBNNNNUGAAGGA-3' (SEQ ID NO: 318); and a part of a sequence selected from SEQ ID NOS: 313 to 318. Here, N may be each independently A, C, G or U, and each B may be independently U, C or G. a may be an integer from 0 to 4, b may be an integer from 0 to 1, c may be an integer from 0 to 2, and d may be an integer from 0 to 3. For (N)ₐ, (N)_{c} or (N)_{d}, when a, c and d are integers other than 0 or 1, N in (N)ₐ, (N)_{c} or (N)_{d} may each independently be A, C, G or U. Here, for (B)ₐ, when a is an integer other than 0 or 1, each B in (B)ₐ may be independently U, C, or G. Here, the seventh sequence may be a sequence having sequence identity of at least 70% or more to 5'-AUGCAAC-3' or 5'-AUGCAAC-3'. Here, the guide sequence may be a sequence of 15 to 40 nucleotides. The engineered crRNA may optionally further comprise an additional sequence. Here, the additional sequence may be a sequence of 1 to 40 nucleotides (a sequence of 1 to 40 nts). The additional sequence may be located at the 5' end of the engineered crRNA (5'-[additional sequence]-[6th sequence]-[7th sequence]-[guide sequence]-3').

For example, the engineered crRNA may comprise a sixth sequence, a seventh sequence and a guide sequence. Here, the sixth sequence may be SEQ ID NO: 408, or a part of SEQ ID NO: 408. Here, the part of SEQ ID NO: 408 may comprise 5'-NGNNNUGAAGGA-3' (SEQ ID NO: 412) while not comprising a partial sequence at the 5' end of SEQ ID NO: 408. Here, the part of SEQ ID NO: 408 may be 5'-UUGCAGAACCCGAAUAGNGNNNUGAAGGA-3' (SEQ ID NO: 413), 5'-UGCAGAACCCGAAUAGNGNNNUGAAGGA-3' (SEQ ID NO: 414), 5'-GCAGAACCCGAAUAGNGNNNUGAAGGA-3' (SEQ ID NO: 415), 5'-CAGAACCCGAAUAGNGNNNUGAAGGA-3' (SEQ ID NO: 416), 5'-AGAACCCGAAUAGNGNNNUGAAGGA-3' (SEQ ID NO: 417), 5'-GAACCCGAAUAGNGNNNUGAAGGA-3' (SEQ ID NO: 418), 5'-AACCCGAAUAGNGNNNUGAAGGA-3' (SEQ ID NO: 419), 5'-ACCCGAAUAGNGNNNUGAAGGA-3' (SEQ ID NO: 420), 5'-CCCGAAUAGNGNNNUGAAGGA-3' (SEQ ID NO: 421), 5'-CCGAAUAGNGNNNUGAAGGA-3' (SEQ ID NO: 422), 5'-CGAAUAGNGNNNUGAAGGA-3' (SEQ ID NO: 423), 5'-GAAUAGNGNNNUGAAGGA-3' (SEQ ID NO: 424), 5'-AAUAGNGNNNUGAAGGA-3' (SEQ ID NO: 425), 5'-AUAGNGNNNUGAAGGA-3' (SEQ ID NO: 426), 5'-UAGNGNNNUGAAGGA-3' (SEQ ID NO: 427), 5'-AGNGNNNUGAAGGA-3' (SEQ ID NO: 428), or 5'-NGNNNUGAAGGA-3' (SEQ ID NO: 412). Here, N may each independently be A, C, G or U. Here, the seventh sequence may be 5'-AUGCAAC-3' or a sequence having sequence identity of at least 70% or more to 5'-AUGCAAC-3'. Here, the guide sequence may be a sequence of 15 to 40 nucleotides. The engineered crRNA may comprise the sixth sequence, the seventh sequence and the guide sequence sequentially in a 5' to 3' direction (5'-[SEQ ID NO: 408 or part thereof]-[5'-AUGCAAC-3' or sequence having sequence identity of at least 70% or more thereto]-[guide sequence]-3'). As an example, the engineered crRNA may comprise 5'-GAAUAGNGNNNUGAAGGAAUGCAACNNNNNNNNNNNNNNNNNNNN-3' (SEQ ID NO: 429). Here, N may each be A, C, G or U. As another example, the engineered crRNA may comprise 5'-GAAUAGAGCAAUGAAGGAAUGCAACNNNNNNNNNNNNNNNNNNNN-3' (SEQ ID NO: 430).

In another embodiment, the engineered crRNA may be an engineered form of a wildtype crRNA, of which a part of a nucleotide sequence is artificially modified, which is modified to have a shorter length, and/or to which a U-rich tail sequence is added to the 3' end.

In an embodiment, the engineered crRNA may comprise a modified form of a wildtype repeat sequence, of which a part of a nucleotide sequence is deleted, and have a U-rich tail sequence added to its 3' end. The engineered crRNA may comprise a part of the wildtype repeat sequence, a guide sequence, and a U-rich tail sequence. Here, the part of the wildtype repeat sequence, the guide sequence, and the u-rich tail sequence may be located in the engineered crRNA in a 5' to 3' direction (5'-[part of wildtype repeat sequence]-[guide sequence]-[U-rich tail sequence]-3'). Here, the part of the wildtype repeat sequence may be a part of SEQ ID NO: 312, and may not comprise a partial sequence at the 5' end of SEQ ID NO: 312. Here, the guide sequence may be a sequence of 15 to 40 nucleotides. Here, the U-rich tail sequence may be 5'-(UₐN)_{d}Uₑ-3', 5'-UₐVUₐVUₑ-3', or 5'-UₐVUₐVUₐVUₑ-3'. Here, N may be A, C, G or U, and each V may be independently A, C or G. a may be an integer of 0 to 4, d may be an integer of 0 to 3, and e may be an integer of 0 to 10. The engineered crRNA may optionally further comprise an additional sequence. Here, the additional sequence may be a sequence of 1 to 40 nucleotides (a sequence of 1 to 40 nts). The additional sequence may be located at the 5' end of the engineered crRNA (5'-[additional sequence]-[part of wildtype repeat sequence]-[guide sequence]-[U-rich tail sequence]-3'). As an example, the engineered crRNA may comprise 5'-GAAUAGACGAAUGAAGGAAUGCAACNNNNNNNNNNNNNNNNNNNNUUUU -3' (SEQ ID NO: 431). Here, N may each be A, C, G or U.

In another embodiment, the engineered crRNA may comprise a modified form of a wildtype repeat sequence, of which at least one nucleotide is substituted with another nucleotide, and have a U-rich tail sequence added to its 3' end. The engineered crRNA may comprise a sixth sequence, a seventh sequence, a guide sequence and a U-rich tail sequence. Here, the sixth sequence, the seventh sequence, the guide sequence, and the U-rich tail sequence may be sequentially located in the engineered crRNA in a 5' to 3' direction (5'-[6th sequence]- [7th sequence]-[guide sequence]-[U-rich tail sequence]-3'). Here, the sixth sequence may be at least one sequence selected from the following sequences:5'-GUUGCAGAACCCGAAUAG(N)ₐUGAAGGA-3' (SEQ ID NO: 313); 5'-GUUGCAGAACCCGAAUAG(B)ₐNNNNBUGAAGGA-3' (SEQ ID NO: 314); 5'-GUUGCAGAACCCGAAUAG(N)_{b}NNNBNUGAAGGA-3' (SEQ ID NO: 315); 5'-GUUGCAGAACCCGAAUAG(N)_{c}NNBNNUGAAGGA-3' (SEQ ID NO: 316); 5'-GUUGCAGAACCCGAAUAG(N)_{d}NBNNNUGAAGGA-3' (SEQ ID NO: 317); and 5'-GUUGCAGAACCCGAAUAG(N)ₐBNNNNUGAAGGA-3' (SEQ ID NO: 318). Here, N may each be independently A, C, G or U, and each B may be independently U, C or G. a may be an integer from 0 to 4, b may be an integer from 0 to 1, c may be an integer from 0 to 2, and d may be an integer from 0 to 3. For (N)ₐ, (N)_{c} or (N)_{d}, when a, c and d are integers other than 0 or 1, N in (N)ₐ, (N)_{c} or (N)_{d} may each independently be A, C, G or U. Here, for (B)ₐ, when a is an integer other than 0 or 1, each B in (B)ₐ may be independently U, C, or G. Here, the seventh sequence may be 5'-AUGCAAC-3' or a sequence having sequence identity of at least 70% or more to 5'-AUGCAAC-3'. Here, the guide sequence may be a sequence of 15 to 40 nucleotides. Here, the U-rich tail sequence may be 5'-(UₐN)_{d}Uₑ-3', 5'-UₐVUₐVUₑ-3', or 5'-UₐVUₐVUₐVUₑ-3'. Here, N may be A, C, G or U, and each V may be independently A, C or G. a may be an integer of 0 to 4, d may be an integer of 0 to 3, and e may be an integer of 0 to 10. The engineered crRNA may optionally further comprise an additional sequence. Here, the additional sequence may be a sequence of 1 to 40 nucleotides (a sequence of 1 to 40 nts). The additional sequence may be located at the 5' end of the engineered crRNA (5'-[additional sequence]-[6th sequence]-[7th sequence]-[guide sequence]-[U-rich tail sequence]-3').

For example, the engineered crRNA may comprise a sixth sequence, a seventh sequence and a guide sequence. Here, the sixth sequence may be SEQ ID NO: 408 or a sequence having sequence identity of at least 70% or more to SEQ ID NO: 408. Here, the sequence having sequence identity of at least 70% or more to SEQ ID NO: 408 may be a sequence having sequence identity of at least 70% or more to the remaining sequence thereof except for 5'-GNGNNNUG-3'. Here, N may each independently be A, C, G or U. Here, the seventh sequence may be 5'-AUGCAAC-3' or a sequence having sequence identity of at least 70% or more to 5'-AUGCAAC-3'. Here, the guide sequence may be a sequence of 15 to 40 nucleotides. Here, the U-rich tail sequence may be 5'-UUUU-3', 5'-UUUUAUU-3', or 5'-UUUUAUUUU-3'. The engineered crRNA may comprise the sixth sequence, the seventh sequence, the guide sequence and the U-rich tail sequence sequentially in a 5' to 3' direction (5'-[SEQ ID NO: 408 or sequence having sequence identity of at least 70% or more thereto]-[5'-AUGCAAC-3' or sequence having sequence identity of at least 70% or more thereto]-[guide sequence]-[U-rich tail sequence]-3'). As an example, the engineered crRNA may comprise 5'-GUUGCAGAACCCGAAUAGNGNNNUGAAGGAAUGCAACNNNNNNNNNNNN NNNNNNNNUUUU-3' (SEQ ID NO: 432). Here, N may each be A, C, G or U. As another example, the engineered crRNA may comprise 5'-GUUGCAGAACCCGAAUAGAGCAAUGAAGGAAUGCAACNNNNNNNNNNNN NNNNNNNNUUUU-3' (SEQ ID NO: 433). As yet another example, the engineered crRNA may comprise 5'-GUUGCAGAACCCGAAUAGAGCAAUGAAGGAAUGCAACNNNNNNNNNNNN NNNNNNNNUUUUAUUUU-3' (SEQ ID NO: 434).

As an example, the engineered crRNA may comprise a modified form of a wildtype repeat sequence, of which at least one nucleotide is substituted with another nucleotide and/or of which a part of a nucleotide sequence is deleted, and/or have a U-rich tail sequence added to its 3' end. The engineered crRNA may comprise a sixth sequence, a seventh sequence, a guide sequence and a U-rich tail sequence. Here, the sixth sequence, the seventh sequence, the guide sequence, and the U-rich tail sequence may be sequentially located in the engineered crRNA in a 5' to 3' direction (5'-[6th sequence]- [7th sequence]-[guide sequence]-[U-rich tail sequence]-3'). Here, the sixth sequence may be at least one sequence selected from the following sequences: 5'-GUUGCAGAACCCGAAUAG(N)ₐUGAAGGA-3' (SEQ ID NO: 313); 5'-GUUGCAGAACCCGAAUAG(B)ₐNNNNBUGAAGGA-3' (SEQ ID NO: 314); 5'-GUUGCAGAACCCGAAUAG(N)_{b}NNNBNUGAAGGA-3' (SEQ ID NO: 315); 5'-GUUGCAGAACCCGAAUAG(N)_{c}NNBNNUGAAGGA-3' (SEQ ID NO: 316); 5'-GUUGCAGAACCCGAAUAG(N)_{d}NBNNNUGAAGGA-3' (SEQ ID NO: 317); 5'-GUUGCAGAACCCGAAUAG(N)ₐBNNNNUGAAGGA-3' (SEQ ID NO: 318); and a part of a sequence selected from SEQ ID NOS: 313 to 318. Here, N may each be independently A, C, G or U, and each B may be independently U, C or G. a may be an integer from 0 to 4, b may be an integer from 0 to 1, c may be an integer from 0 to 2, and d may be an integer from 0 to 3. For (N)ₐ, (N)_{c} or (N)_{d}, when a, c and d are integers other than 0 or 1, N in (N)ₐ, (N)_{c} or (N)_{d} may each independently be A, C, G or U. Here, for (B)ₐ, when a is an integer other than 0 or 1, each B in (B)ₐ may be each independently U, C, or G. Here, the seventh sequence may be 5'-AUGCAAC-3' or a sequence having sequence identity of at least 70% or more to 5'-AUGCAAC-3'. Here, the guide sequence may be a sequence of 15 to 40 nucleotides. Here, the U-rich tail sequence may be 5'-(UₐN)_{d}Uₑ-3', 5'-UₐVUₐVUₑ-3', or 5'-UₐVUₐVUₐVUₑ-3'. Here, N may be A, C, G or U, and each V may be independently A, C or G. a may be an integer of 0 to 4, d may be an integer of 0 to 3, and e may be an integer of 0 to 10. The engineered crRNA may optionally further comprise an additional sequence. Here, the additional sequence may be a sequence of 1 to 40 nucleotides (a sequence of 1 to 40 nts). The additional sequence may be located at the 5' end of the engineered crRNA (5'-[additional sequence]-[6th sequence]-[7th sequence]-[guide sequence]-[U-rich tail sequence]-3').

As an example, the engineered crRNA may comprise a sixth sequence, a seventh sequence and a guide sequence. Here, the sixth sequence may be SEQ ID NO: 408, or a part of SEQ ID NO: 408. Here, the part of SEQ ID NO: 408 may comprise 5'-NGNNNUGAAGGA-3' (SEQ ID NO: 412) while not comprising a partial sequence at the 5' end of SEQ ID NO: 408. Here, the part of SEQ ID NO: 408 may be a sequence selected from SEQ ID NOS: 412 to 428. Here, N may each independently be A, C, G or U. Here, the seventh sequence may be 5'-AUGCAAC-3' or a sequence having sequence identity of at least 70% or more to 5'-AUGCAAC-3'. Here, the guide sequence may be a sequence of 15 to 40 nucleotides. Here, the U-rich tail sequence may be 5'-UUUU-3', 5'-UUUUAUU-3', or 5'-UUUUAUUUU-3'. The engineered crRNA may comprise the sixth sequence, the seventh sequence, the guide sequence and the U-rich tail sequence sequentially in a 5' to 3' direction (5'-[part of SEQ ID NO: 408 ]-[5'-AUGCAAC-3' or sequence having sequence identity of at least 70% or more thereto]-[guide sequence]-[U-rich tail sequence]-3'). As an example, the engineered crRNA may comprise 5'-GAAUAGNGNNNUGAAGGAAUGCAACNNNNNNNNNNNNNNNNNNNNUUUU -3' (SEQ ID NO: 435). Here, N may each be A, C, G or U. As another example, the engineered crRNA may comprise 5'-GAAUAGAGCAAUGAAGGAAUGCAACNNNNNNNNNNNNNNNNNNNNUUUU -3' (SEQ ID NO: 436). As yet another example, the engineered crRNA may comprise 5'-GAAUAGAGCAAUGAAGGAAUGCAACNNNNNNNNNNNNNNNNNNNNUUUU AUUUU-3' (SEQ ID NO: 545).

### 3. Engineered guide RNA

An engineered guide RNAs comprise an engineered trans-activating CRISPR RNA (tracrRNA) and a CRISPR RNA (crRNA). Here, the crRNA may be a wildtype crRNA or an engineered crRNA. Here, the engineered tracrRNA, the wildtype crRNA, and the engineered crRNA are as described above.

The engineered guide RNA may be an engineered dual guide RNA or an engineered single guide RNA.

Hereinafter, the engineered dual guide RNA and the engineered single guide RNA are described in detail.

### 3-1) Dual guide RNA

The engineered dual guide RNA is a guide RNA composed of two RNA molecules in which an engineered tracrRNA and a crRNA are included as separate RNA molecules. That is, in the engineered dual guide RNA, an engineered tracrRNA and a crRNA exist each independently.

Here, the crRNA may be a wildtype crRNA or an engineered crRNA.

Here, the engineered tracrRNA, the wildtype crRNA, and the engineered crRNA are as described above.

### 3-2) Single guide RNA

An engineered single guide RNA is a single RNA molecule in which an engineered tracrRNA, a linker and a crRNA are included. Here, the engineered single guide RNA is a single RNA molecule in which the engineered tracrRNA, the linker and the crRNA are linked to each other.

The engineered single guide RNA may be 5'-[engineered tracrRNA]-[linker]-[crRNA]-3'.

Here, the crRNA may be a wildtype crRNA or an engineered crRNA.

Here, the engineered tracrRNA, the wildtype crRNA, and the engineered crRNA are as described above.

Here, the linker serves to link the engineered tracrRNA to the crRNA. That is, the engineered single guide RNA is a single RNA molecule in which the engineered tracrRNA and crRNA are linked by the linker.

The linker may be a sequence that does not affect functions of the engineered tracrRNA and/or the crRNA. Alternatively, the linker may be a sequence that does not form an RNA duplex with the engineered tracrRNA and/or the crRNA.

The linker may be a sequence of 1 to 30 nucleotides.

In an embodiment, the linker may be a sequence of 1 to 5, 5 to 10, 10 to 15, 15 to 20, 20 to 25, or 25 to 30 nucleotides.

In another embodiment, the linker may be a sequence of 1 to 30, 5 to 30, 10 to 30, 15 to 30, 20 to 30, or 25 to 30 nucleotides.

In another embodiment, the linker is as sequence of 1, 2, 3, 4, 5, 6, 7, 8, 9, 10, 11, 12, 13, 14, 15, 16 17, 18, 19, 20, 21, 22, 23, 24, 25, 26, 27, 28, 29 or 30 nucleotides. For example, the linker may be a sequence of 5'-GAAA-3', but is not limited thereto.

### 3-3) Examples of engineered guide RNA

Based on the previous description, examples of the engineered guide RNA are described. Specific descriptions of components included in the examples below are the same as those described above for the corresponding components. The examples are for an illustrative purpose only, and the scope of the present disclosure is not limited thereto.

In the present disclosure, the engineered guide RNA may comprise an engineered tracrRNA and a crRNA. Here, the crRNA may be a wildtype crRNA or an engineered crRNA. The engineered tracrRNA may be one of the examples described in the section "1-8) Examples of engineered tracrRNA" described above. The crRNA may be one of the examples described in the section "2-7) Examples of crRNA". The engineered guide RNA may be an engineered dual guide RNA. Here, the engineered dual guide RNA may comprise the engineered tracrRNA and the crRNA as separate RNA molecules. Alternatively, the engineered guide RNA may be an engineered single guide RNA. Here, the engineered single guide RNA may further comprise a linker wherein the engineered tracrRNA and the crRNA may be linked by the linker.

In another embodiment, the engineered guide RNA may comprise an engineered tracrRNA and a wildtype crRNA.

The engineered tracrRNA may be a tracrRNA modified not to comprise a sequence of five or more consecutive uridines. Here, the engineered tracrRNA may comprise four or less consecutive uridines. The engineered tracrRNA may optionally be further modified to have a length shorter than the wildtype tracrRNA. Here, the engineered tracrRNA may not comprise a part of the wildtype tracrRNA.

In an embodiment, the engineered guide RNA may comprise:
(i) an engineered tracrRNA that does not comprise a sequence of five or more consecutive uridines and comprises a sequence of four or less consecutive uridines and (ii) a wildtype crRNA.

Here, the engineered tracrRNA may comprise a first sequence, a second sequence, a third sequence, a fourth sequence, and a fifth sequence. Here, the first sequence, the second sequence, the third sequence, the fourth sequence, and the fifth sequence may be sequentially located in the engineered tracrRNA in a 3' to 5' direction (5'-[5th sequence]-[4th sequence]-[3rd sequence]-[2nd sequence]-[1st sequence]-3'). Here, the first sequence may be at least one sequence selected from the following sequences: 5'-CAAAUUCA(N)aCCUCUCCAAUUCUGCACAA-3' (SEQ ID NO: 2); 5'-CAAAUUCAVNNNN(V)aCCUCUCCAAUUCUGCACAA-3' (SEQ ID NO: 3); 5'-CAAAUUCANVNNN(N)bCCUCUCCAAUUCUGCACAA-3' (SEQ ID NO: 4); 5'-CAAAUUCANNVNN(N)cCCUCUCCAAUUCUGCACAA-3' (SEQ ID NO: 5); 5'-CAAAUUCANNNVN(N)dCCUCUCCAAUUCUGCACAA-3' (SEQ ID NO: 6); and 5'-CAAAUUCANNNNV(N)aCCUCUCCAAUUCUGCACAA-3' (SEQ ID NO: 7). Here, N may be each independently A, C, G or U, and each V may be independently A, C or G. Here, a may be an integer from 0 to 4, b may be an integer from 0 to 1, c may be an integer from 0 to 2, and d may be an integer from 0 to 3. For (N)ₐ, (N)_{c} or (N)_{d}, when a, c and d are integers other than 0 or 1, N in (N)ₐ, (N)_{c} or (N)_{d} may each independently be A, C, G or U. For (V)ₐ, when a is an integer other than 0 or 1, each V in (V)ₐ may be independently A, C or G. Here, the second sequence may be SEQ ID NO: 211 or a sequence having sequence identity of at least 70% or more to SEQ ID NO: 211. Here, the third sequence may be SEQ ID NO: 212 or a sequence having sequence identity of at least 70% or more to SEQ ID NO: 212. Here, the fourth sequence may be SEQ ID NO: 213 or a sequence having sequence identity of at least 70% or more to SEQ ID NO: 213. Here, the fifth sequence may be SEQ ID NO: 248 or a sequence having sequence identity of at least 70% or more to SEQ ID NO: 248. The wildtype crRNA may comprise a wildtype repeat sequence and a guide sequence. Here, the wildtype repeat sequence may be SEQ ID NO: 312. Here, the guide sequence may be a sequence of 15 to 40 nucleotides. The wildtype repeat sequence and the guide sequence may be sequentially located in the wildtype crRNA in a 5' to 3' direction (5'-[wildtype repeat sequence]-[guide sequence]-3'). The engineered guide RNA may be an engineered dual guide RNA. Here, the engineered dual guide RNA may comprise the engineered tracrRNA and the wildtype crRNA as separate RNA molecules. Alternatively, the engineered guide RNA may be an engineered single guide RNA. Here, the engineered single guide RNA may further comprise a linker wherein the engineered tracrRNA and wildtype crRNA may be linked by the linker.

For example, the engineered guide RNA may comprise an engineered tracrRNA and a wildtype crRNA which are described below. The engineered tracrRNA may comprise a first sequence, a second sequence, a third sequence, a fourth sequence and a fifth sequence which are described below. Here, the first sequence may be SEQ ID NO: 111 or a sequence having sequence identity of 70% or more to SEQ ID NO: 111. Here, the sequence having sequence identity of at least 70% or more to SEQ ID NO: 111 may be a sequence having sequence identity of at least 70% or more to the remaining sequence thereof except for 5'-CANNNCNC-3'. Here, N may each independently be A, C, G or U. Here, the second sequence may be SEQ ID NO: 211 or a sequence having sequence identity of 70% or more to SEQ ID NO: 211. Here, the third sequence may be SEQ ID NO: 212 or a sequence having sequence identity of at least 70% or more to SEQ ID NO: 212. Here, the fourth sequence may be SEQ ID NO: 213 or a sequence having sequence identity of at least 70% or more to SEQ ID NO: 213. Here, the fifth sequence may be SEQ ID NO: 248 or a sequence having sequence identity of at least 70% or more to SEQ ID NO: 248. The engineered tracrRNA may comprise the first sequence, the second sequence, the third sequence, the fourth sequence, and the fifth sequence sequentially in a 3' to 5' direction. The wildtype crRNA may comprise a wildtype repeat sequence and a guide sequence. Here, the wildtype repeat sequence may be SEQ ID NO: 312. Here, the guide sequence may be a sequence of 15 to 40 nucleotides. In the wildtype crRNA, the wildtype repeat sequence and the guide sequence may be sequentially located in a 5' to 3' direction. The engineered guide RNA may be an engineered dual guide RNA or an engineered single guide RNA. Here, the engineered single guide RNA may further comprise a linker wherein the engineered tracrRNA and the wildtype crRNA may be linked by the linker. As an example, the engineered guide RNA may comprise 5'-CUUCACUGAUAAAGUGGAGAACCGCUUCACCAAAAGCUGUCCCUUAGGG GAUUAGAACUUGAGUGAAGGUGGGCUGCUUGCAUCAGCCUAAUGUCGA GAAGUGCUUUCUUCGGAAAGUAACCCUCGAAACAAAUUCANNNCNCCUC UCCAAUUCUGCACAA-3' (SEQ ID NO: 269), which is an engineered tracrRNA, and 5'-GUUGCAGAACCCGAAUAGACGAAUGAAGGAAUGCAACNNNNNNNNNNNN NNNNNNNN-3' (SEQ ID NO: 405) which is a wildtype crRNA. As another example, the engineered guide RNA may comprise 5'-CUUCACUGAUAAAGUGGAGAACCGCUUCACCAAAAGCUGUCCCUUAGGG GAUUAGAACUUGAGUGAAGGUGGGCUGCUUGCAUCAGCCUAAUGUCGA GAAGUGCUUUCUUCGGAAAGUAACCCUCGAAACAAAUUCANNNCNCCUC UCCAAUUCUGCACAAGAAAGUUGCAGAACCCGAAUAGACGAAUGAAGGA AUGCAACNNNNNNNNNNNNNNNNNNNN-3' (SEQ ID NO: 437). As yet another example, the engineered guide RNA may comprise 5'-CUUCACUGAUAAAGUGGAGAACCGCUUCACCAAAAGCUGUCCCUUAGGG GAUUAGAACUUGAGUGAAGGUGGGCUGCUUGCAUCAGCCUAAUGUCGA GAAGUGCUUUCUUCGGAAAGUAACCCUCGAAACAAAUUCAGUGCUCCUC UCCAAUUCUGCACAA-3' (SEQ ID NO: 270), which is an engineered tracrRNA, and SEQ ID NO: 405 which is a wildtype crRNA. As still yet another example the engineered guide RNA may comprise 5'-CUUCACUGAUAAAGUGGAGAACCGCUUCACCAAAAGCUGUCCCUUAGGG GAUUAGAACUUGAGUGAAGGUGGGCUGCUUGCAUCAGCCUAAUGUCGA GAAGUGCUUUCUUCGGAAAGUAACCCUCGAAACAAAUUCAGUGCUCCUC UCCAAUUCUGCACAAGAAAGUUGCAGAACCCGAAUAGACGAAUGAAGGA AUGCAACNNNNNNNNNNNNNNNNNNNN-3' (SEQ ID NO: 438). Here, N may each be A, C, G or U.

In another embodiment, the engineered guide RNA may comprise:
(i) an engineered tracrRNA that does not comprise a sequence of five or more consecutive uridines and comprises four or less consecutive uridines, and optionally does not comprise a part of a wildtype tracrRNA; and (ii) a wildtype crRNA.

Here, the engineered tracrRNA may comprise a first sequence, a second sequence, a third sequence, a fourth sequence, and a fifth sequence. Here, the first sequence, the second sequence, the third sequence, the fourth sequence, and the fifth sequence may be sequentially located in the engineered tracrRNA in a 3' to 5' direction (5'-[5th sequence]-[4th sequence]-[3rd sequence]-[2nd sequence]-[1st sequence]-3'). Here, the first sequence may be at least one sequence selected from the following sequences: 5'-CAAAUUCA(N)aCCUCUCCAAUUCUGCACAA-3' (SEQ ID NO: 2); 5'-CAAAUUCAVNNNN(V)aCCUCUCCAAUUCUGCACAA-3' (SEQ ID NO: 3); 5'-CAAAUUCANVNNN(N)bCCUCUCCAAUUCUGCACAA-3' (SEQ ID NO: 4); 5'-CAAAUUCANNVNN(N)cCCUCUCCAAUUCUGCACAA-3' (SEQ ID NO: 5); 5'-CAAAUUCANNNVN(N)dCCUCUCCAAUUCUGCACAA-3' (SEQ ID NO: 6); 5'-CAAAUUCANNNNV(N)aCCUCUCCAAUUCUGCACAA-3' (SEQ ID NO: 7); and a part of a sequence selected from SEQ ID NOS: 2 to 7. Here, N may be each independently A, C, G or U, and each V may be independently A, C or G. Here, a may be an integer from 0 to 4, b may be an integer from 0 to 1, c may be an integer from 0 to 2, and d may be an integer from 0 to 3. For (N)ₐ, (N)_{c} or (N)_{d}, when a, c and d are integers other than 0 or 1, N in (N)ₐ, (N)_{c} or (N)_{d} may each independently be A, C, G or U. For (V)ₐ, when a is an integer other than 0 or 1, each V in (V)ₐ may be independently A, C or G. Here, the second sequence may be SEQ ID NO: 211 or a sequence having sequence identity of 70% or more to SEQ ID NO: 211. Here, the third sequence may be SEQ ID NO: 212 or a sequence having sequence identity of at least 70% or more to SEQ ID NO: 212. Here, the fourth sequence may be SEQ ID NO: 213, a sequence having sequence identity of at least 70% or more to SEQ ID NO: 213, or a part of SEQ ID NO: 213. Here, the fifth sequence may be SEQ ID NO: 248, a sequence having sequence identity of at least 70% or more to SEQ ID NO: 248, or a part of SEQ ID NO: 248. The wildtype crRNA may comprise a wildtype repeat sequence and a guide sequence. Here, the wildtype repeat sequence may be SEQ ID NO: 312. Here, the guide sequence may be a sequence of 15 to 40 nucleotides. The wildtype repeat sequence and the guide sequence may be sequentially located in the wildtype crRNA in a 5' to 3' direction (5'-[wildtype repeat sequence]-[guide sequence]-3'). The engineered guide RNA may be an engineered dual guide RNA. Here, the engineered dual guide RNA may comprise the engineered tracrRNA and the wildtype crRNA as separate RNA molecules. Alternatively, the engineered guide RNA may be an engineered single guide RNA. Here, the engineered single guide RNA may further comprise a linker wherein the engineered tracrRNA and the wildtype crRNA may be linked by the linker.

For example, the engineered guide RNA may comprise an engineered tracrRNA and a wildtype crRNA which are described below. The engineered tracrRNA may comprise a first sequence, a second sequence, a third sequence, a fourth sequence and a fifth sequence which are described below. Here, the first sequence may be SEQ ID NO: 111 or a part of SEQ ID NO: 111. Here, the part of SEQ ID NO: 111 may comprise SEQ ID NO: 272 in SEQ ID NO: 111 while not comprising a partial sequence at the 3' end of SEQ ID NO: 111. Here, the part of SEQ ID NO: 111 may be a sequence selected from SEQ ID NOS: 272 to 290. Here, N may each independently be A, C, G or U. Here, the second sequence may be SEQ ID NO: 211 or a sequence having sequence identity of 70% or more to SEQ ID NO: 211. Here, the third sequence may be SEQ ID NO: 212 or a sequence having sequence identity of at least 70% or more to SEQ ID NO: 212. Here, the fourth sequence may be SEQ ID NO: 213, a sequence having sequence identity of at least 70% or more to SEQ ID NO: 213, or a part of SEQ ID NO: 213. Here, the part of SEQ ID NO: 213 may be a sequence obtained by deleting at least one pair of nucleotides forming a complementary base pair and/or at least one nucleotide not involved in forming a complementary base pair from SEQ ID NO: 213. Here, the part of SEQ ID NO: 213 may be a sequence selected from SEQ ID NOS: 214 to 217, SEQ ID NO: 231 to 247, 5'-CCUUAGGUG-3', 5'-CUUAGUG-3', 5'-CUUAGG-3', and 5'-UUAG-3' wherein 5'-UUAG-3' included in the selected sequence may be substituted with 5'-GAAA-3'. Here, the fifth sequence may be SEQ ID NO: 248, a sequence having sequence identity of at least 70% or more to SEQ ID NO: 248, or a part of SEQ ID NO: 248. Here, the part of the SEQ ID NO: 248 may be a sequential partial sequence at the 3' end of SEQ ID NO: 248, and may be selected from 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', and SEQ ID NOS: 249 to 259. In the engineered tracrRNA,the first sequence, the second sequence, the third sequence, the fourth sequence and the fifth sequence may be sequentially located in a 3' to 5' direction. The wildtype crRNA may comprise a wildtype repeat sequence and a guide sequence. Here, the wildtype repeat sequence may be SEQ ID NO: 312. Here, the guide sequence may be a sequence of 15 to 40 nucleotides. In the wildtype crRNA, the wildtype repeat sequence and the guide sequence may be sequentially located in a 5' to 3' direction. The engineered guide RNA may be an engineered dual guide RNA or an engineered single guide RNA. Here, the engineered single guide RNA may further comprise a linker wherein the engineered tracrRNA and the wildtype crRNA may be linked by the linker. As an example, the engineered guide RNA may comprise 5'-ACCGCUUCACCAAUUAGUUGAGUGAAGGUGGGCUGCUUGCAUCAGCCU AAUGUCGAGAAGUGCUUUCUUCGGAAAGUAACCCUCGAAACAAAUUCAN NNCNCCUCUCCAAUUCUGCACAA-3' (SEQ ID NO: 301), which is an engineered tracrRNA, and SEQ ID NO: 405 which is a wildtype crRNA. As another example, the engineered guide RNA may comprise 5'-ACCGCUUCACCAAUUAGUUGAGUGAAGGUGGGCUGCUUGCAUCAGCCU AAUGUCGAGAAGUGCUUUCUUCGGAAAGUAACCCUCGAAACAAAUUCAN NNCNCCUCUCCAAUUCUGCACAAGAAAGUUGCAGAACCCGAAUAGACGA AUGAAGGAAUGCAACNNNNNNNNNNNNNNNNNNNN-3' (SEQ ID NO: 439). As yet another example, the engineered guide RNA may comprise 5'-ACCGCUUCACCAAAAGCUGUCCCUUAGGGGAUUAGAACUUGAGUGAAGG UGGGCUGCUUGCAUCAGCCUAAUGUCGAGAAGUGCUUUCUUCGGAAAG UAACCCUCGAAACAAAUUCANNNCNCCUCUCCAAUUCUGCACAA-3' (SEQ ID NO: 304), which is an engineered tracrRNA, and SEQ ID NO: 405 which is a wildtype crRNA. As still yet another example, the engineered guide RNA may comprise 5'-ACCGCUUCACCAAAAGCUGUCCCUUAGGGGAUUAGAACUUGAGUGAAGG UGGGCUGCUUGCAUCAGCCUAAUGUCGAGAAGUGCUUUCUUCGGAAAG UAACCCUCGAAACAAAUUCANNNCNCCUCUCCAAUUCUGCACAAGAAAGU UGCAGAACCCGAAUAGACGAAUGAAGGAAUGCAACNNNNNNNNNNNNNN NNNNNN-3' (SEQ ID NO: 440). As still yet another example, the engineered guide RNA may comprise 5'-ACCGCUUCACCAAUUAGUUGAGUGAAGGUGGGCUGCUUGCAUCAGCCU AAUGUCGAGAAGUGCUUUCUUCGGAAAGUAACCCUCGAAACAAAUUCAG UGCUCCUCUCCAAUUCUGCACAA-3' (SEQ ID NO: 306), which is an engineered tracrRNA, and SEQ ID NO: 405 which is a wildtype crRNA. As still yet another example, the engineered guide RNA may comprise 5'-ACCGCUUCACCAAUUAGUUGAGUGAAGGUGGGCUGCUUGCAUCAGCCU AAUGUCGAGAAGUGCUUUCUUCGGAAAGUAACCCUCGAAACAAAUUCAG UGCUCCUCUCCAAUUCUGCACAAGAAAGUUGCAGAACCCGAAUAGACGA AUGAAGGAAUGCAACNNNNNNNNNNNNNNNNNNNN-3' (SEQ ID NO: 441). As still yet another example, the engineered guide RNA may comprise 5'-ACCGCUUCACCAAAAGCUGUCCCUUAGGGGAUUAGAACUUGAGUGAAGG UGGGCUGCUUGCAUCAGCCUAAUGUCGAGAAGUGCUUUCUUCGGAAAG UAACCCUCGAAACAAAUUCAGUGCUCCUCUCCAAUUCUGCACAA-3' (SEQ ID NO: 308), which is an engineered tracrRNA, and SEQ ID NO: 405 which is a wildtype crRNA. As still yet another example, the engineered guide RNA may comprise 5'-ACCGCUUCACCAAAAGCUGUCCCUUAGGGGAUUAGAACUUGAGUGAAGG UGGGCUGCUUGCAUCAGCCUAAUGUCGAGAAGUGCUUUCUUCGGAAAG UAACCCUCGAAACAAAUUCAGUGCUCCUCUCCAAUUCUGCACAAGAAAG UUGCAGAACCCGAAUAGACGAAUGAAGGAAUGCAACNNNNNNNNNNNNN NNNNNNN-3' (SEQ ID NO: 442). Here, N may each be A, C, G or U.

In another embodiment, the engineered guide RNA may comprise an engineered tracrRNA and an engineered crRNA.

The engineered tracrRNA may be a tracrRNA modified not to comprise a sequence of five or more consecutive uridines. Here, the engineered tracrRNA may comprise a sequence of four or less consecutive uridines. The engineered tracrRNA may be optionally further modified to have a shorter length than a wildtype tracrRNA. For example, the engineered tracrRNA may not comprise a part of the wildtype tracrRNA. The engineered crRNA may be an engineered form of a wildtype tracrRNA which is modified to have a shorter length and/or to the 3' end of which a U-rich tail sequence is added.

In an embodiment, the engineered guide RNA comprises:
(i) an engineered tracrRNA that does not comprise a sequence of five or more consecutive uridines, comprises a sequence of four or less consecutive uridines, and does not comprise a part of a wildtype tracrRNA; and (ii) an engineered crRNA comprising a wildtype repeat sequence and a U-rich tail sequence.

Here, the engineered tracrRNA may comprise a first sequence, a second sequence, a third sequence, a fourth sequence, and a fifth sequence. Here, the first sequence, the second sequence, the third sequence, the fourth sequence, and the fifth sequence may be sequentially located in the engineered tracrRNA in a 3' to 5' direction (5'-[5th sequence]-[4th sequence]-[3rd sequence]-[2nd sequence]-[1st sequence]-3'). Here, the first sequence may be at least one sequence selected from the following sequences: 5'-CAAAUUCA(N)aCCUCUCCAAUUCUGCACAA-3' (SEQ ID NO: 2); 5'-CAAAUUCAVNNNN(V)aCCUCUCCAAUUCUGCACAA-3' (SEQ ID NO: 3); 5'-CAAAUUCANVNNN(N)bCCUCUCCAAUUCUGCACAA-3' (SEQ ID NO: 4); 5'-CAAAUUCANNVNN(N)cCCUCUCCAAUUCUGCACAA-3' (SEQ ID NO: 5); 5'-CAAAUUCANNNVN(N)dCCUCUCCAAUUCUGCACAA-3' (SEQ ID NO: 6); 5'-CAAAUUCANNNNV(N)aCCUCUCCAAUUCUGCACAA-3' (SEQ ID NO: 7); and a part of a sequence selected from SEQ ID NOS: 2 to 7. Here, N may be each independently A, C, G or U, and each V may be independently A, C or G. Here, a may be an integer from 0 to 4, b may be an integer from 0 to 1, c may be an integer from 0 to 2, and d may be an integer from 0 to 3. For (N)ₐ, (N)_{c} or (N)_{d}, when a, c and d are integers other than 0 or 1, N in (N)ₐ, (N)_{c} or (N)_{d} may each independently be A, C, G or U. For (V)ₐ, when a is an integer other than 0 or 1, each V in (V)ₐ may be independently A, C or G. Here, the second sequence may be SEQ ID NO: 211 or a sequence having sequence identity of 70% or more to SEQ ID NO: 211. Here, the third sequence may be SEQ ID NO: 212 or a sequence having sequence identity of at least 70% or more to SEQ ID NO: 212. Here, the fourth sequence may be SEQ ID NO: 213, a sequence having sequence identity of at least 70% or more to SEQ ID NO: 213, or a part of SEQ ID NO: 213. Here, the fifth sequence may be SEQ ID NO: 248, a sequence having sequence identity of at least 70% or more to SEQ ID NO: 248, or a part of SEQ ID NO: 248.

The engineered crRNA may comprise a wildtype repeat sequence, a guide sequence, and a U-rich tail sequence. Here, the wildtype repeat sequence may be SEQ ID NO: 312. Here, the guide sequence may be a sequence of 15 to 40 nucleotides. Here, the U-rich tail sequence may be 5'-(UₐN)_{d}Uₑ-3', 5'-UₐVUₐVUₑ-3', or 5'-UₐVUₐVUₐVUₑ-3'. Here, N may be A, C, G or U, and each V may be independently A, C or G. a may be an integer of 0 to 4, d may be an integer of 0 to 3, and e may be an integer of 0 to 10. The wildtype repeat sequence, the guide sequence, and the U-rich tail sequence may be sequentially located in the wildtype crRNA in a 5' to 3' direction (5'-[wildtype repeat sequence]-[guide sequence]- [U-rich tail sequence]-3'). The engineered guide RNA may be an engineered dual guide RNA. Here, the engineered dual guide RNA may comprise the engineered tracrRNA and the wildtype crRNA as separate RNA molecules. Alternatively, the engineered guide RNA may be an engineered single guide RNA. Here, the engineered single guide RNA may further comprise a linker wherein the engineered tracrRNA and the engineered crRNA may be linked by the linker.

For example, the engineered guide RNA may comprise an engineered tracrRNA and an engineered crRNA which are described below. The engineered tracrRNA may comprise a first sequence, a second sequence, a third sequence, a fourth sequence and a fifth sequence which are described below. Here, the first sequence may be SEQ ID NO: 111 or a part of SEQ ID NO: 111. Here, the part of SEQ ID NO: 111 may comprise SEQ ID NO: 272 while not comprising a partial sequence at the 3' end of SEQ ID NO: 111. Here, the part of SEQ ID NO: 111 may be a sequence selected from SEQ ID NOS: 272 to 290. Here, N may each independently be A, C, G or U. Here, the second sequence may be SEQ ID NO: 211 or a sequence having sequence identity of 70% or more to SEQ ID NO: 211. Here, the third sequence may be SEQ ID NO: 212 or a sequence having sequence identity of at least 70% or more to SEQ ID NO: 212. Here, the fourth sequence may be SEQ ID NO: 213, a sequence having sequence identity of at least 70% or more to SEQ ID NO: 213, or a part of SEQ ID NO: 213. Here, the part of SEQ ID NO: 213 may be a sequence obtained by deleting at least one pair of nucleotides forming a complementary base pair and/or at least one nucleotide not involved in forming a complementary base pair from SEQ ID NO: 213. Here, the part of SEQ ID NO: 213 may be a sequence selected from SEQ ID NOS: 214 to 217, SEQ ID NO: 231 to 247, 5'-CCUUAGGUG-3', 5'-CUUAGUG-3', 5'-CUUAGG-3', and 5'-UUAG-3' wherein 5'-UUAG-3' included in the selected sequence may be substituted with 5'-GAAA-3'. Here, the fifth sequence may be SEQ ID NO: 248, a sequence having sequence identity of at least 70% or more to SEQ ID NO: 248, or a part of SEQ ID NO: 248. Here, the part of the SEQ ID NO: 248 may be a sequential partial sequence at the 3' end of SEQ ID NO: 248, and may be selected from 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', and SEQ ID NOS: 249 to 259. In the engineered tracrRNA, the first sequence, the second sequence, the third sequence, the fourth sequence and the fifth sequence may be sequentially located in a 3' to 5' direction. The engineered crRNA may comprise SEQ ID NO: 312, a guide sequence and a U-rich tail sequence. Here, the guide sequence may be a sequence of 15 to 40 nucleotides. Here, the U-rich tail sequence may be 5'-UUUU-3', 5'-UUUUUAUU-3', or 5'-UUUUAUUUUU-3'. The engineered crRNA may comprise SEQ ID NO: 312, the guide sequence, and the U-rich tail sequence sequentially in a 5' to 3' direction. The engineered guide RNA may be an engineered dual guide RNA or an engineered single guide RNA. Here, the engineered single guide RNA may further comprise a linker wherein the engineered tracrRNA and the engineered crRNA may be linked by the linker. As an example, the engineered guide RNA may comprise SEQ ID NO: 301, which is an engineered tracrRNA, and 5'-GUUGCAGAACCCGAAUAGACGAAUGAAGGAAUGCAACNNNNNNNNNNNN NNNNNNNNUUUU-3' (SEQ ID NO: 407) which is an engineered crRNA. As another example, the engineered guide RNA may comprise 5'-ACCGCUUCACCAAUUAGUUGAGUGAAGGUGGGCUGCUUGCAUCAGCCU AAUGUCGAGAAGUGCUUUCUUCGGAAAGUAACCCUCGAAACAAAUUCAN NNCNCCUCUCCAAUUCUGCACAAGAAAGUUGCAGAACCCGAAUAGACGA AUGAAGGAAUGCAACNNNNNNNNNNNNNNNNNNNNUUUU-3' (SEQ ID NO: 443). As yet another example, the engineered guide RNA may comprise SEQ ID NO: 304, which is an engineered tracrRNA, and SEQ ID NO: 407 which is an engineered crRNA. As still yet another example, the engineered guide RNA may comprise 5'-ACCGCUUCACCAAAAGCUGUCCCUUAGGGGAUUAGAACUUGAGUGAAGG UGGGCUGCUUGCAUCAGCCUAAUGUCGAGAAGUGCUUUCUUCGGAAAG UAACCCUCGAAACAAAUUCANNNCNCCUCUCCAAUUCUGCACAAGAAAGU UGCAGAACCCGAAUAGACGAAUGAAGGAAUGCAACNNNNNNNNNNNNNN NNNNNNUUUU-3' (SEQ ID NO: 444). As still yet another example, the engineered guide RNA may comprise SEQ ID NO: 306, which is an engineered tracrRNA, and SEQ ID NO: 407 which is an engineered crRNA. As still yet another example, the engineered guide RNA may comprise 5'-ACCGCUUCACCAAUUAGUUGAGUGAAGGUGGGCUGCUUGCAUCAGCCU AAUGUCGAGAAGUGCUUUCUUCGGAAAGUAACCCUCGAAACAAAUUCAG UGCUCCUCUCCAAUUCUGCACAAGAAAGUUGCAGAACCCGAAUAGACGA AUGAAGGAAUGCAACNNNNNNNNNNNNNNNNNNNNUUUU-3' (SEQ ID NO: 445). As still yet another example, the engineered guide RNA may comprise SEQ ID NO: 308, which is an engineered tracrRNA, and SEQ ID NO: 407 which is an engineered crRNA. As still yet another example, the engineered guide RNA may comprise 5'-ACCGCUUCACCAAAAGCUGUCCCUUAGGGGAUUAGAACUUGAGUGAAGG UGGGCUGCUUGCAUCAGCCUAAUGUCGAGAAGUGCUUUCUUCGGAAAG UAACCCUCGAAACAAAUUCAGUGCUCCUCUCCAAUUCUGCACAAGAAAG UUGCAGAACCCGAAUAGACGAAUGAAGGAAUGCAACNNNNNNNNNNNNN NNNNNNNUUUU-3' (SEQ ID NO: 446). As still yet another example, the engineered guide RNA may comprise SEQ ID NO: 269, which is an engineered tracrRNA, and SEQ ID NO: 407 which is an engineered crRNA. In still yet another example, the engineered guide RNA may comprise 5'-CUUCACUGAUAAAGUGGAGAACCGCUUCACCAAAAGCUGUCCCUUAGGG GAUUAGAACUUGAGUGAAGGUGGGCUGCUUGCAUCAGCCUAAUGUCGA GAAGUGCUUUCUUCGGAAAGUAACCCUCGAAACAAAUUCANNNCNCCUC UCCAAUUCUGCACAAGAAAGUUGCAGAACCCGAAUAGACGAAUGAAGGA AUGCAACNNNNNNNNNNNNNNNNNNNNUUUU-3' (SEQ ID NO: 447). As still yet another example, the engineered guide RNA may comprise SEQ ID NO: 270, which is an engineered tracrRNA, and SEQ ID NO: 407 which is an engineered crRNA. As still yet another example, the engineered guide RNA may comprise 5'-CUUCACUGAUAAAGUGGAGAACCGCUUCACCAAAAGCUGUCCCUUAGGG GAUUAGAACUUGAGUGAAGGUGGGCUGCUUGCAUCAGCCUAAUGUCGA GAAGUGCUUUCUUCGGAAAGUAACCCUCGAAACAAAUUCAGUGCUCCUC UCCAAUUCUGCACAAGAAAGUUGCAGAACCCGAAUAGACGAAUGAAGGA AUGCAACNNNNNNNNNNNNNNNNNNNNUUUU-3' (SEQ ID NO: 448). Here, N may each be A, C, G or U.

In another embodiment, the engineered guide RNA may comprise:
(i) an engineered tracrRNA that does not comprise a sequence of five or more consecutive uridines and comprises a sequence of four or less consecutive uridines, and optionally does not comprise a part of a wildtype tracrRNA; and (ii) an engineered crRNA that is a modified form of a wildtype repeat sequence of which a part of a nucleotide sequence is deleted. Here, the engineered tracrRNA may comprise a first sequence, a second sequence, a third sequence, a fourth sequence, and a fifth sequence. Here, the first sequence, the second sequence, the third sequence, the fourth sequence, and the fifth sequence may be sequentially located in a 3' to 5' direction (5'-[5th sequence). sequence]-[4th sequence]-[3rd sequence]-[2nd sequence]-[1st sequence]-3'). Here, the first sequence may be at least one sequence selected from the following sequences: 5'-CAAAUUCA(N)aCCUCUCCAAUUCUGCACAA-3' (SEQ ID NO: 2); 5'-CAAAUUCAVNNNN(V)aCCUCUCCAAUUCUGCACAA-3' (SEQ ID NO: 3); 5'-CAAAUUCANVNNN(N)bCCUCUCCAAUUCUGCACAA-3' (SEQ ID NO: 4); 5'-CAAAUUCANNVNN(N)cCCUCUCCAAUUCUGCACAA-3' (SEQ ID NO: 5); 5'-CAAAUUCANNNVN(N)dCCUCUCCAAUUCUGCACAA-3' (SEQ ID NO: 6); 5'-CAAAUUCANNNNV(N)aCCUCUCCAAUUCUGCACAA-3' (SEQ ID NO: 7); and a part of a sequence selected from SEQ ID NOS: 2 to 7. Here, N may be each independently A, C, G or U, and each V may be independently A, C or G. Here, a may be an integer from 0 to 4, b may be an integer from 0 to 1, c may be an integer from 0 to 2, and d may be an integer from 0 to 3. For (N)ₐ, (N)_{c} or (N)a, when a, c and d are integers other than 0 or 1, N in (N)ₐ, (N)_{c} or (N)_{d} may each independently be A, C, G or U. For (V)ₐ, when a is an integer other than 0 or 1, each V in (V)ₐ may be independently A, C or G. Here, the second sequence may be SEQ ID NO: 211 or a sequence having sequence identity of 70% or more to SEQ ID NO: 211. Here, the third sequence may be SEQ ID NO: 212 or a sequence having sequence identity of at least 70% or more to SEQ ID NO: 212. Here, the fourth sequence may be SEQ ID NO: 213, a sequence having sequence identity of at least 70% or more to SEQ ID NO: 213, or a part of SEQ ID NO: 213. Here, the fifth sequence may be SEQ ID NO: 248, a sequence having sequence identity of at least 70% or more to SEQ ID NO: 248, or a part of SEQ ID NO: 248.

The engineered crRNA may be a wildtype repeat sequence of which a part of a nucleotide sequence is deleted. The engineered crRNA may comprise a part of the wildtype repeat sequence and a guide sequence. The engineered crRNA may optionally further comprise a U-rich tail sequence. Here, the part of the wildtype repeat sequence may be a part of SEQ ID NO: 312, and may not comprise a partial sequence at the 5' end of SEQ ID NO: 312. Here, the guide sequence may be a sequence of 15 to 40 nucleotides. Here, the U-rich tail sequence may be 5'-(UₐN)_{d}Uₑ-3', 5'-UₐVUₐVUₑ-3', or 5'-UₐVUₐVUₐVUₑ-3'. Here, N may be A, C, G or U, and each V may be independently A, C or G. a may be an integer of 0 to 4, d may be an integer of 0 to 3, and e may be an integer of 0 to 10. The part of the wildtype repeat sequence and the guide sequence may be sequentially located in the engineered crRNA in a 5' to 3' direction (5'-[part of wildtype repeat sequence]-[guide sequence]-3'). Here, when a U-rich tail sequence is optionally further included, the U-rich tail sequence may be located at the 3' end of the engineered crRNA (5'-[part of wildtype repeat sequence]-[guide sequence]-[U-rich tail]-3'). The engineered guide RNA may be an engineered dual guide RNA. Here, the engineered dual guide RNA may comprise the engineered tracrRNA and the engineered crRNA as separate RNA molecules. Alternatively, the engineered guide RNA may be an engineered single guide RNA. Here, the engineered single guide RNA may further comprise a linker wherein the engineered tracrRNA and the engineered crRNA may be linked by the linker.

For example, the engineered guide RNA may comprise an engineered tracrRNA and an engineered crRNA which are described below. The engineered tracrRNA may comprise a first sequence, a second sequence, a third sequence, a fourth sequence and a fifth sequence which are described below. Here, the first sequence may be SEQ ID NO: 111 or a part of SEQ ID NO: 111. Here, the part of SEQ ID NO: 111 may comprise SEQ ID NO: 272 while not comprising a partial sequence at the 3' end of SEQ ID NO: 111. Here, the part of SEQ ID NO: 111 may be a sequence selected from SEQ ID NOS: 272 to 290. Here, N may each independently be A, C, G or U. Here, the second sequence may be SEQ ID NO: 211 or a sequence having sequence identity of 70% or more to SEQ ID NO: 211. Here, the third sequence may be SEQ ID NO: 212 or a sequence having sequence identity of at least 70% or more to SEQ ID NO: 212. Here, the fourth sequence may be SEQ ID NO: 213, a sequence having sequence identity of at least 70% or more to SEQ ID NO: 213, or a part of SEQ ID NO: 213. Here, the part of SEQ ID NO: 213 may be a sequence obtained by deleting at least one pair of nucleotides forming a complementary base pair and/or at least one nucleotide not involved in forming a complementary base pair from SEQ ID NO: 213. Here, the part of SEQ ID NO: 213 may be a sequence selected from SEQ ID NOS: 214 to 217, SEQ ID NO: 231 to 247, 5'-CCUUAGGUG-3', 5'-CUUAGUG-3', 5'-CUUAGG-3', and 5'-UUAG-3' wherein 5'-UUAG-3' included in the selected sequence may be substituted with 5'-GAAA-3'. Here, the fifth sequence may be SEQ ID NO: 248, a sequence having sequence identity of at least 70% or more to SEQ ID NO: 248, or a part of SEQ ID NO: 248. Here, the part of SEQ ID NO: 248 may be a sequential partial sequence at the 3' end of SEQ ID NO: 248, and may be selected from 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', and SEQ ID NOS: 249 to 259. In the engineered tracrRNA, the first sequence, the second sequence, the third sequence, the fourth sequence, and the fifth sequence may be sequentially located in a 3' to 5' direction. The engineered crRNA may comprise a part of SEQ ID NO: 312 and a guide sequence. The engineered crRNA may optionally further comprise a U-rich tail sequence. Here, the part of SEQ ID NO: 312 may not comprise a partial sequence at the 5' end of SEQ ID NO: 312. Here, the guide sequence may be a sequence of 15 to 40 nucleotides. Here, the U-rich tail sequence may be 5'-UUUU-3', 5'-UUUUUAUU-3', or 5'-UUUUAUUUUU-3'. The engineered crRNA may comprise the part of SEQ ID NO: 312 and the guide sequence in a 5' to 3' direction, wherein when a U-rich tail sequence is further included, the U-rich tail sequence may be included at the 3' end. The engineered guide RNA may be an engineered dual guide RNA or an engineered single guide RNA. Here, the engineered single guide RNA may further comprise a linker wherein the engineered tracrRNA and the engineered crRNA may be linked by the linker. As an example, the engineered guide RNA may comprise 5'-ACCGCUUCACCAAAAGCUGUCCCUUAGGGGAUUAGAACUUGAGUGAAGG UGGGCUGCUUGCAUCAGCCUAAUGUCGAGAAGUGCUUUCUUCGGAAAG UAACCCUCGAAACAAAUUCANNNCNCCUCUC-3' (SEQ ID NO: 305), which is an engineered tracrRNA, and 5'-GAAUAGACGAAUGAAGGAAUGCAACNNNNNNNNNNNNNNNNNNNN-3' (SEQ ID NO: 411) which is an engineered crRNA. As another example, the engineered guide RNA may comprise 5'-ACCGCUUCACCAAAAGCUGUCCCUUAGGGGAUUAGAACUUGAGUGAAGG UGGGCUGCUUGCAUCAGCCUAAUGUCGAGAAGUGCUUUCUUCGGAAAG UAACCCUCGAAACAAAUUCANNNCNCCUCUCGAAAGAAUAGACGAAUGAA GGAAUGCAACNNNNNNNNNNNNNNNNNNNN-3' (SEQ ID NO: 449). As yet another example, the engineered guide RNA may comprise SEQ ID NO: 305, which is an engineered tracrRNA, and 5'-GAAUAGACGAAUGAAGGAAUGCAACNNNNNNNNNNNNNNNNNNNNUUUU -3' (SEQ ID NO: 431) which is an engineered crRNA. As still yet another example, the engineered guide RNA may comprise 5'-ACCGCUUCACCAAAAGCUGUCCCUUAGGGGAUUAGAACUUGAGUGAAGG UGGGCUGCUUGCAUCAGCCUAAUGUCGAGAAGUGCUUUCUUCGGAAAG UAACCCUCGAAACAAAUUCANNNCNCCUCUCGAAAGAAUAGACGAAUGAA GGAAUGCAACNNNNNNNNNNNNNNNNNNNNUUUU-3' (SEQ ID NO: 450). As still yet another example, the engineered guide RNA may comprise ACCGCUUCACCAAUUAGUUGAGUGAAGGUGGGCUGCUUGCAUCAGCCU AAUGUCGAGAAGUGCUUUCUUCGGAAAGUAACCCUCGAAACAAAUUCAG UGCUCCUCUC-3' (SEQ ID NO: 307), which is an engineered tracrRNA, and SEQ ID NO: 431 which is an engineered crRNA. As still yet another example, the engineered guide RNA may comprise 5'-ACCGCUUCACCUUAGGAGUGAAGGUGGGCUGCUUGCAUCAGCCUAAUG UCGAGAAGUGCUUUCUUCGGAAAGUAACCCUCGAAACAAAUUCAGUGCU CCUCUCGAAAGAAUAGACGAAUGAAGGAAUGCAACNNNNNNNNNNNNNN NNNNNNUUUU-3' (SEQ ID NO: 451). As still yet another example, the engineered guide RNA may comprise 5'-ACCGCUUCACCAAAAGCUGUCCCUUAGGGGAUUAGAACUUGAGUGAAGG UGGGCUGCUUGCAUCAGCCUAAUGUCGAGAAGUGCUUUCUUCGGAAAG UAACCCUCGAAACAAAUUCAGUGCUCCUCUC-3' (SEQ ID NO: 309), which is an engineered tracrRNA, and SEQ ID NO: 431 which is an engineered crRNA. As still yet another example, the engineered guide RNA may comprise 5'-ACCGCUUCACCAAAAGCUGUCCCUUAGGGGAUUAGAACUUGAGUGAAGG UGGGCUGCUUGCAUCAGCCUAAUGUCGAGAAGUGCUUUCUUCGGAAAG UAACCCUCGAAACAAAUUCAGUGCUCCUCUCGAAAGAAUAGACGAAUGA AGGAAUGCAACNNNNNNNNNNNNNNNNNNNNUUUU-3' (SEQ ID NO: 452). Here, N may each be A, C, G or U.

In another embodiment, the engineered guide RNA may comprise an engineered tracrRNA and an engineered crRNA.

The engineered tracrRNA may be a tracrRNA modified not to comprise a sequence of five or more consecutive uridines. Here, the engineered tracrRNA may comprise a sequence of four or less consecutive uridines. The engineered tracrRNA may optionally be further modified to have a shorter length than a wildtype tracrRNA. Here, the engineered tracrRNA may not comprise a part of the wildtype tracrRNA.

In this embodiment, the engineered crRNA may be an engineered form of a wildtype crRNA, of which a part of a nucleotide sequence is artificially modified, which is modified to have a shorter length, and/or to which a U-rich tail sequence added to the 3' end.

In an embodiment, the engineered guide RNA comprises:
(i) an engineered tracrRNA that does not comprise a sequence of five or more consecutive uridines and comprises a sequence of four or less consecutive uridines, and optionally does not comprise a part of a wildtype tracrRNA; and (ii) an engineered crRNA that is a wildtype repeat sequence in which at least one nucleotide is substituted with another nucleotide.

The engineered tracrRNA may comprise a first sequence, a second sequence, a third sequence, a fourth sequence, and a fifth sequence. Here, the first sequence, the second sequence, the third sequence, the fourth sequence, and the fifth sequence may be sequentially located in a 3' to 5' direction (5'-[5th sequence]-[4th sequence]-[3rd sequence]-[2nd sequence]-[1st sequence]-3'). Here, the first sequence may be at least one sequence selected from the following sequences: 5'-CAAAUUCA(N)aCCUCUCCAAUUCUGCACAA-3' (SEQ ID NO: 2); 5'-CAAAUUCAVNNNN(V)aCCUCUCCAAUUCUGCACAA-3' (SEQ ID NO: 3); 5'-CAAAUUCANVNNN(N)bCCUCUCCAAUUCUGCACAA-3' (SEQ ID NO: 4); 5'-CAAAUUCANNVNN(N)cCCUCUCCAAUUCUGCACAA-3' (SEQ ID NO: 5); 5'-CAAAUUCANNNVN(N)dCCUCUCCAAUUCUGCACAA-3' (SEQ ID NO: 6); 5'-CAAAUUCANNNNV(N)aCCUCUCCAAUUCUGCACAA-3' (SEQ ID NO: 7); and a part of a sequence selected from SEQ ID NOS: 2 to 7. Here, N may be each independently A, C, G or U, and each V may be independently A, C or G. Here, a may be an integer from 0 to 4, b may be an integer from 0 to 1, c may be an integer from 0 to 2, and d may be an integer from 0 to 3. For (N)ₐ, (N)_{c} or (N)_{d}, when a, c and d are integers other than 0 or 1, N in (N)ₐ, (N)_{c} or (N)_{d} may each independently be A, C, G or U. For (V)ₐ, when a is an integer other than 0 or 1, each V in (V)ₐ may be independently A, C or G. Here, the second sequence may be SEQ ID NO: 211 or a sequence having sequence identity of 70% or more to SEQ ID NO: 211. Here, the third sequence may be SEQ ID NO: 212 or a sequence having sequence identity of at least 70% or more to SEQ ID NO: 212. Here, the fourth sequence may be SEQ ID NO: 213, a sequence having sequence identity of at least 70% or more to SEQ ID NO: 213, or a part of SEQ ID NO: 213. Here, the fifth sequence may be SEQ ID NO: 248, a sequence having sequence identity of at least 70% or more to SEQ ID NO: 248, or a part of SEQ ID NO: 248. The engineered crRNA may comprise a modified form of a wildtype repeat sequence of which at least one nucleotide is substituted with another nucleotide. The engineered crRNA may optionally a modified form of a wildtype repeat sequence of which a part of a nucleotide sequence is deleted.

The engineered crRNA may comprise a sixth sequence, a seventh sequence and a guide sequence. Here, the sixth sequence, the seventh sequence, and the guide sequence may be sequentially located in the engineered crRNA in a 5' to 3' direction (5'-[6th sequence]-[7th sequence]- [guide sequence]-3'). Here, the sixth sequence may be at least one sequence selected from the following sequences: 5'-GUUGCAGAACCCGAAUAG(N)aUGAAGGA-3' (SEQ ID NO: 313); 5'-GUUGCAGAACCCGAAUAG(B)aNNNNBUGAAGGA-3' (SEQ ID NO: 314); 5'-GUUGCAGAACCCGAAUAG(N)bNNNBNUGAAGGA-3' (SEQ ID NO: 315); 5'-GUUGCAGAACCCGAAUAG(N)cNNBNNUGAAGGA-3' (SEQ ID NO: 316); 5'-GUUGCAGAACCCGAAUAG(N)dNBNNNUGAAGGA-3' (SEQ ID NO: 317); 5'-GUUGCAGAACCCGAAUAG(N)aBNNNNUGAAGGA-3' (SEQ ID NO: 318); and a part of a sequence selected from SEQ ID NOS: 313 to 318. Here, N may each be independently A, C, G or U, and each B may be independently U, C or G. Here, a may be an integer from 0 to 4, b may be an integer from 0 to 1, c may be an integer from 0 to 2, and d may be an integer from 0 to 3. For (N)ₐ, (N)_{c} or (N)_{d}, when a, c and d are integers other than 0 or 1, N in (N)ₐ, (N)_{c} or (N)_{d} may each independently be A, C, G or U. Here, for (B)ₐ, when a is an integer other than 0 or 1, each B in (B)ₐ may be each independently U, C, or G. Here, the seventh sequence may be 5'-AUGCAAC-3' or a sequence having sequence identity of at least 70% or more to 5'-AUGCAAC-3'. Here, the guide sequence may be a sequence of 15 to 40 nucleotides. The engineered guide RNA may be an engineered dual guide RNA. Here, the engineered dual guide RNA may comprise the engineered tracrRNA and the engineered crRNA as separate RNA molecules. Alternatively, the engineered guide RNA may be an engineered single guide RNA. Here, the engineered single guide RNA may further comprise a linker wherein the engineered tracrRNA and the engineered crRNA may be linked by the linker.

For example, the engineered guide RNA may comprise an engineered tracrRNA and an engineered crRNA which are described below. The engineered tracrRNA may comprise a first sequence, a second sequence, a third sequence, a fourth sequence and a fifth sequence which are described below. Here, the first sequence may be SEQ ID NO: 111 or a part of SEQ ID NO: 111. Here, the part of the SEQ ID NO: 111 may comprise SEQ ID NO: 272 while not comprising a partial sequence at the 3' end of SEQ ID NO: 111. Here, the part of the SEQ ID NO: 111 may be selected from SEQ ID NOS: 272 to 290. Here, N may each independently be A, C, G or U. Here, the second sequence may be SEQ ID NO: 211 or a sequence having sequence identity of 70% or more to SEQ ID NO: 211. Here, the third sequence may be SEQ ID NO: 212 or a sequence having sequence identity of at least 70% or more to SEQ ID NO: 212. Here, the fourth sequence may be SEQ ID NO: 213, a sequence having sequence identity of at least 70% or more to SEQ ID NO: 213, or a part of SEQ ID NO: 213. Here, the part of SEQ ID NO: 213 may be a sequence obtained by deleting at least one pair of nucleotides forming a complementary base pair and/or at least one nucleotide not involved in forming a complementary base pair from SEQ ID NO: 213. Here, the part of SEQ ID NO: 213 may be a sequence selected from SEQ ID NOS: 214 to 217, SEQ ID NO: 231 to 247, 5'-CCUUAGGUG-3', 5'-CUUAGUG-3', 5'-CUUAGG-3', and 5'-UUAG-3' wherein 5'-UUAG-3' included in the selected sequence may be substituted with 5'-GAAA-3'. Here, the fifth sequence may be SEQ ID NO: 248, a sequence having sequence identity of at least 70% or more to SEQ ID NO: 248, or a part of SEQ ID NO: 248. Here, the part of SEQ ID NO: 248 may be a sequential partial sequence at the 3' end of SEQ ID NO: 248, and may be a sequence selected from the group consisting of 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', and SEQ ID NOS: 249 to 259. In the engineered tracrRNA, the first sequence, the second sequence, the third sequence, the fourth sequence, and the fifth sequence may be sequentially located in a 3' to 5' direction. The engineered crRNA may comprise a sixth sequence, a seventh sequence, and a guide sequence. Here, the sixth sequence may be SEQ ID NO: 408, or a part of SEQ ID NO: 408. Here, the part of SEQ ID NO: 408 may comprise 5'-NGNNNUGAAGGA-3' (SEQ ID NO: 412) while not comprising a partial sequence at the 5' end of SEQ ID NO: 408. Here, the part of SEQ ID NO: 408 may be a sequence selected from SEQ ID NOS: 412 to 428. Here, N may each independently be A, C, G or U. Here, the seventh sequence may be 5'-AUGCAAC-3' or a sequence having sequence identity of at least 70% or more to 5'-AUGCAAC-3'. Here, the guide sequence may be a sequence of 15 to 40 nucleotides. The engineered crRNA may comprise the sixth sequence, the seventh sequence, and the guide sequence sequentially in a 5' to 3' direction. The engineered guide RNA may be an engineered dual guide RNA or an engineered single guide RNA. Here, the engineered single guide RNA may further comprise a linker wherein the engineered tracrRNA and the engineered crRNA may be linked by the linker. As an example, the engineered guide RNA may comprise SEQ ID NO: 301, which is an engineered tracrRNA, and 5'-GUUGCAGAACCCGAAUAGNGNNNUGAAGGAAUGCAACNNNNNNNNNNNN NNNNNNNN-3' (SEQ ID NO: 409) which is an engineered crRNA. As yet another example, the engineered guide RNA may comprise 5'-ACCGCUUCACCAAUUAGUUGAGUGAAGGUGGGCUGCUUGCAUCAGCCU AAUGUCGAGAAGUGCUUUCUUCGGAAAGUAACCCUCGAAACAAAUUCAN NNCNCCUCUCCAAUUCUGCACAAGAAAGUUGCAGAACCCGAAUAGNGNN NUGAAGGAAUGCAACNNNNNNNNNNNNNNNNNNNN-3' (SEQ ID NO: 453). As still yet another example, the engineered guide RNA may comprise SEQ ID NO: 304, which is an engineered tracrRNA, and SEQ ID NO: 409 which is an engineered crRNA. As still yet another example, the engineered guide RNA may comprise 5'-ACCGCUUCACCAAAAGCUGUCCCUUAGGGGAUUAGAACUUGAGUGAAGG UGGGCUGCUUGCAUCAGCCUAAUGUCGAGAAGUGCUUUCUUCGGAAAG UAACCCUCGAAACAAAUUCANNNCNCCUCUCCAAUUCUGCACAAGAAAGU UGCAGAACCCGAAUAGNGNNNUGAAGGAAUGCAACNNNNNNNNNNNNNN NNNNNN-3' (SEQ ID NO: 454). As still yet another example, the engineered guide RNA may comprise SEQ ID NO: 306, which is an engineered tracrRNA, and 5'-GUUGCAGAACCCGAAUAGAGCAAUGAAGGAAUGCAACNNNNNNNNNNNN NNNNNNNN-3' (SEQ ID NO: 410) which is an engineered crRNA. As still yet another example, the engineered guide RNA may comprise 5'-ACCGCUUCACCAAUUAGUUGAGUGAAGGUGGGCUGCUUGCAUCAGCCU AAUGUCGAGAAGUGCUUUCUUCGGAAAGUAACCCUCGAAACAAAUUCAG UGCUCCUCUCCAAUUCUGCACAAGAAAGUUGCAGAACCCGAAUAGAGCA AUGAAGGAAUGCAACNNNNNNNNNNNNNNNNNNNN-3' (SEQ ID NO: 455). As another example, the engineered guide RNA may comprise SEQ ID NO: 308, which is an engineered tracrRNA, and SEQ ID NO: 410 which is an engineered crRNA. As still yet another example, the engineered guide RNA may comprise 5'-ACCGCUUCACCAAAAGCUGUCCCUUAGGGGAUUAGAACUUGAGUGAAGG UGGGCUGCUUGCAUCAGCCUAAUGUCGAGAAGUGCUUUCUUCGGAAAG UAACCCUCGAAACAAAUUCAGUGCUCCUCUCCAAUUCUGCACAAGAAAG UUGCAGAACCCGAAUAGAGCAAUGAAGGAAUGCAACNNNNNNNNNNNNN NNNNNNN-3' (SEQ ID NO: 456). As still yet another example, the engineered guide RNA may comprise SEQ ID NO: 269, which is an engineered tracrRNA, and SEQ ID NO: 409 which is an engineered crRNA. As still yet another example, the engineered guide RNA may comprise 5'-CUUCACUGAUAAAGUGGAGAACCGCUUCACCAAAAGCUGUCCCUUAGGG GAUUAGAACUUGAGUGAAGGUGGGCUGCUUGCAUCAGCCUAAUGUCGA GAAGUGCUUUCUUCGGAAAGUAACCCUCGAAACAAAUUCANNNCNCCUC UCCAAUUCUGCACAAGAAAGUUGCAGAACCCGAAUAGNGNNNUGAAGGA AUGCAACNNNNNNNNNNNNNNNNNNNN-3' (SEQ ID NO: 457). As still yet another example, the engineered guide RNA may comprise SEQ ID NO: 270, which is an engineered tracrRNA, and SEQ ID NO: 410 which is an engineered crRNA. As still yet another example, the engineered guide RNA may comprise 5'-CUUCACUGAUAAAGUGGAGAACCGCUUCACCAAAAGCUGUCCCUUAGGG GAUUAGAACUUGAGUGAAGGUGGGCUGCUUGCAUCAGCCUAAUGUCGA GAAGUGCUUUCUUCGGAAAGUAACCCUCGAAACAAAUUCAGUGCUCCUC UCCAAUUCUGCACAAGAAAGUUGCAGAACCCGAAUAGAGCAAUGAAGGA AUGCAACNNNNNNNNNNNNNNNNNNNN-3' (SEQ ID NO: 458). Here, N may each be A, C, G or U.

In another embodiment, the engineered guide RNA may comprise:
(i) an engineered tracrRNA that does not comprise a sequence of five or more consecutive uridines and comprises four or less consecutive uridines, and optionally does not comprise a part of a wildtype tracrRNA; and (ii) an engineered crRNA that is a modified form of a wildtype repeat sequence of which at least one nucleotide is substituted with another nucleotide.

The engineered tracrRNA may comprise a first sequence, a second sequence, a third sequence, a fourth sequence, and a fifth sequence. Here, the first sequence, the second sequence, the third sequence, the fourth sequence, and the fifth sequence may be sequentially located in the engineered tracrRNA in a 3' to 5' direction (5'-[5th sequence]-[4th sequence]-[3rd sequence]-[2nd sequence]-[1st sequence]-3'). Here, the first sequence may be at least one sequence selected from the following sequences: 5'-CAAAUUCA(N)aCCUCUCCAAUUCUGCACAA-3' (SEQ ID NO: 2); 5'-CAAAUUCAVNNNN(V)aCCUCUCCAAUUCUGCACAA-3' (SEQ ID NO: 3); 5'-CAAAUUCANVNNN(N)bCCUCUCCAAUUCUGCACAA-3' (SEQ ID NO: 4); 5'-CAAAUUCANNVNN(N)cCCUCUCCAAUUCUGCACAA-3' (SEQ ID NO: 5); 5'-CAAAUUCANNNVN(N)dCCUCUCCAAUUCUGCACAA-3' (SEQ ID NO: 6); 5'-CAAAUUCANNNNV(N)aCCUCUCCAAUUCUGCACAA-3' (SEQ ID NO: 7); and a part of a sequence selected from SEQ ID NOS: 2 to 7. Here, N may be each independently A, C, G or U, and each V may be independently A, C or G. Here, a may be an integer from 0 to 4, b may be an integer from 0 to 1, c may be an integer from 0 to 2, and d may be an integer from 0 to 3. For (N)ₐ, (N)_{c} or (N)_{d}, when a, c and d are integers other than 0 or 1, N in (N)ₐ, (N)_{c} or (N)_{d} may each independently be A, C, G or U. For (V)ₐ, when a is an integer other than 0 or 1, each V in (V)ₐ may be independently A, C or G. Here, the second sequence may be SEQ ID NO: 211 or a sequence having sequence identity of 70% or more to SEQ ID NO: 211. Here, the third sequence may be SEQ ID NO: 212 or a sequence having sequence identity of at least 70% or more to SEQ ID NO: 212. Here, the fourth sequence may be SEQ ID NO: 213, a sequence having sequence identity of at least 70% or more to SEQ ID NO: 213, or a part of SEQ ID NO: 213. Here, the fifth sequence may be SEQ ID NO: 248, a sequence having sequence identity of at least 70% or more to SEQ ID NO: 248, or a part of SEQ ID NO: 248.

The engineered crRNA may comprise a wildtype repeat sequence of which at least one nucleotide is substituted with another nucleotide and have a U-rich tail sequence added to its 3' end. The engineered crRNA may optionally comprise a modified form of a wildtype repeat sequence of which a part of a nucleotide sequence is deleted. The engineered crRNA may comprise a sixth sequence, a seventh sequence, a guide sequence, and a U-rich tail sequence. Here, the sixth sequence, the seventh sequence, the guide sequence, and the U-rich tail sequence may be sequentially located in the engineered crRNA in a 5' to 3' direction (5'-[6th sequence]- [7th sequence]-[guide sequence]-[U-rich tail sequence]-3'). Here, the sixth sequence may be at least one sequence selected from the following sequences: 5'-GUUGCAGAACCCGAAUAG(N)aUGAAGGA-3' (SEQ ID NO: 313); 5'-GUUGCAGAACCCGAAUAG(B)aNNNNBUGAAGGA-3' (SEQ ID NO: 314); 5'-GUUGCAGAACCCGAAUAG(N)bNNNBNUGAAGGA-3' (SEQ ID NO: 315); 5'-GUUGCAGAACCCGAAUAG(N)cNNBNNUGAAGGA-3' (SEQ ID NO: 316); 5'-GUUGCAGAACCCGAAUAG(N)dNBNNNUGAAGGA-3' (SEQ ID NO: 317); 5'-GUUGCAGAACCCGAAUAG(N)aBNNNNUGAAGGA-3' (SEQ ID NO: 318); and a part of one sequence selected from SEQ ID NOS: 313 to 318. Here, N may each be independently A, C, G or U, and each B may be independently U, C or G. Here, a may be an integer from 0 to 4, b may be an integer from 0 to 1, c may be an integer from 0 to 2, and d may be an integer from 0 to 3. For (N)ₐ, (N)_{c} or (N)_{d}, when a, c and d are integers other than 0 or 1, N in (N)ₐ, (N)_{c} or (N)_{d} may each independently be A, C, G or U. Here, for (B)ₐ, when a is an integer other than 0 or 1, each B in (B)ₐ may be each independently U, C, or G. Here, the seventh sequence may be 5'-AUGCAAC-3' or a sequence having sequence identity of at least 70% or more to 5'-AUGCAAC-3'. Here, the guide sequence may be a sequence of 15 to 40 nucleotides. Here, the U-rich tail sequence may be 5'-(UₐN)_{d}Uₑ-3', 5'-UₐVUₐVUₑ-3', or 5'-UₐVUₐVUₐVUₑ-3'. Here, N may be A, C, G or U, and each V may be independently A, C or G. a may be an integer of 0 to 4, d may be an integer of 0 to 3, and e may be an integer of 0 to 10. The engineered guide RNA may be an engineered dual guide RNA. Here, the engineered dual guide RNA may comprise the engineered tracrRNA and the engineered crRNA as separate RNA molecules. Alternatively, the engineered guide RNA may be an engineered single guide RNA. Here, the engineered single guide RNA may further comprise a linker wherein the engineered tracrRNA and the engineered crRNA may be linked by the linker.

For example, the engineered guide RNA may comprise an engineered tracrRNA and an engineered crRNA which are described below. The engineered tracrRNA may comprise a first sequence, a second sequence, a third sequence, a fourth sequence and a fifth sequence which are described below. Here, the first sequence may be SEQ ID NO: 111 or a part of SEQ ID NO: 111. Here, the part of the SEQ ID NO: 111 may comprise SEQ ID NO: 272 while not comprising a partial sequence at the 3' end of SEQ ID NO: 111. Here, the part of the SEQ ID NO: 111 may be a sequence selected from SEQ ID NOS: 272 to 290. Here, N may each independently be A, C, G or U. Here, the second sequence may be SEQ ID NO: 211 or a sequence having sequence identity of 70% or more to SEQ ID NO: 211. Here, the third sequence may be SEQ ID NO: 212 or a sequence having sequence identity of at least 70% or more to SEQ ID NO: 212. Here, the fourth sequence may be SEQ ID NO: 213, a sequence having sequence identity of at least 70% or more to SEQ ID NO: 213, or a part of SEQ ID NO: 213. Here, the part of SEQ ID NO: 213 may be a sequence obtained by deleting at least one pair of nucleotides forming a complementary base pair and/or at least one nucleotide not involved in forming a complementary base pair from SEQ ID NO: 213. Here, the part of SEQ ID NO: 213 may be a sequence selected from the group consisting of SEQ ID NOS: 214 to 217, SEQ ID NO: 231 to 247, 5'-CCUUAGGUG-3', 5'-CUUAGUG-3', 5'-CUUAGG-3', and 5'-UUAG-3' wherein 5'-UUAG-3' included in the selected sequence may be substituted with 5'-GAAA-3'. Here, the fifth sequence may be SEQ ID NO: 248, a sequence having sequence identity of at least 70% or more to SEQ ID NO: 248, or a part of SEQ ID NO: 248. Here, the part of SEQ ID NO: 248 may be a sequential partial sequence at the 3' end of SEQ ID NO: 248, and may be selected from the group consisting of 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', and SEQ ID NOS: 249 to 259. In the engineered tracrRNA, the first sequence, the second sequence, the third sequence, the fourth sequence, and the fifth sequence may be sequentially located in "a 3' to 5' direction. The engineered crRNA may comprise a sixth sequence, a seventh sequence, a guide sequence, and a U-rich tail sequence. Here, the sixth sequence may be SEQ ID NO: 408, or a part of SEQ ID NO: 408. Here, the part of SEQ ID NO: 408 may comprise 5'-NGNNNUGAAGGA-3' (SEQ ID NO: 412) while not comprising a partial sequence at the 5' end of SEQ ID NO: 408. Here, the part of SEQ ID NO: 408 may be a sequence selected from SEQ ID NOS: 412 to 428. Here, N may each independently be A, C, G or U. Here, the seventh sequence may be 5'-AUGCAAC-3' or a sequence having sequence identity of at least 70% or more to 5'-AUGCAAC-3'. Here, the guide sequence may be a sequence of 15 to 40 nucleotides. Here, the U-rich tail sequence may be 5'-UUUU-3', 5'-UUUUUAUU-3', or 5'-UUUUAUUUUU-3'. The engineered crRNA may comprise the sixth sequence, the seventh sequence, the guide sequence, and the U-rich tail sequence sequentially in a 5' to 3' direction. The engineered guide RNA may be an engineered dual guide RNA or an engineered single guide RNA. Here, the engineered single guide RNA may further comprise a linker wherein the engineered tracrRNA and the engineered crRNA may be linked by the linker. As an example, the engineered guide RNA may comprise SEQ ID NO: 301, which is an engineered tracrRNA, and 5'-GUUGCAGAACCCGAAUAGNGNNNUGAAGGAAUGCAACNNNNNNNNNNNN NNNNNNNNUUUU-3' (SEQ ID NO: 432) which is an engineered crRNA. As another example, the engineered guide RNA may comprise 5'-ACCGCUUCACCAAUUAGUUGAGUGAAGGUGGGCUGCUUGCAUCAGCCU AAUGUCGAGAAGUGCUUUCUUCGGAAAGUAACCCUCGAAACAAAUUCAN NNCNCCUCUCCAAUUCUGCACAAGAAAGUUGCAGAACCCGAAUAGNGNN NUGAAGGAAUGCAACNNNNNNNNNNNNNNNNNNNNUUUU-3' (SEQ ID NO: 459). As yet another example, the engineered guide RNA may comprise SEQ ID NO: 304, which is an engineered tracrRNA, and SEQ ID NO: 432 which is an engineered crRNA. As still yet another example, the engineered guide RNA may comprise 5'-ACCGCUUCACCAAAAGCUGUCCCUUAGGGGAUUAGAACUUGAGUGAAGG UGGGCUGCUUGCAUCAGCCUAAUGUCGAGAAGUGCUUUCUUCGGAAAG UAACCCUCGAAACAAAUUCANNNCNCCUCUCCAAUUCUGCACAAGAAAGU UGCAGAACCCGAAUAGNGNNNUGAAGGAAUGCAACNNNNNNNNNNNNNN NNNNNNUUUU-3' (SEQ ID NO: 460). As still yet another example, the engineered guide RNA may comprise SEQ ID NO: 306, which is an engineered tracrRNA, and 5'-GUUGCAGAACCCGAAUAGAGCAAUGAAGGAAUGCAACNNNNNNNNNNNN NNNNNNNNUUUU-3' (SEQ ID NO: 433) which is an engineered crRNA. As still yet another example, the engineered guide RNA may comprise 5'-ACCGCUUCACCAAUUAGUUGAGUGAAGGUGGGCUGCUUGCAUCAGCCU AAUGUCGAGAAGUGCUUUCUUCGGAAAGUAACCCUCGAAACAAAUUCAG UGCUCCUCUCCAAUUCUGCACAAGAAAGUUGCAGAACCCGAAUAGAGCA AUGAAGGAAUGCAACNNNNNNNNNNNNNNNNNNNNUUUU-3' (SEQ ID NO: 461). As still yet another example, the engineered guide RNA may comprise SEQ ID NO: 308, which is an engineered tracrRNA, and SEQ ID NO: 433 which is an engineered crRNA. As still yet another example, the engineered guide RNA may comprise 5'-ACCGCUUCACCAAAAGCUGUCCCUUAGGGGAUUAGAACUUGAGUGAAGG UGGGCUGCUUGCAUCAGCCUAAUGUCGAGAAGUGCUUUCUUCGGAAAG UAACCCUCGAAACAAAUUCAGUGCUCCUCUCCAAUUCUGCACAAGAAAG UUGCAGAACCCGAAUAGAGCAAUGAAGGAAUGCAACNNNNNNNNNNNNN NNNNNNNUUUU-3' (SEQ ID NO: 462). As still yet another example, the engineered guide RNA may comprise SEQ ID NO: 269, which is an engineered tracrRNA, and SEQ ID NO: 432 which is an engineered crRNA. As still yet another example, the engineered guide RNA may comprise 5'-CUUCACUGAUAAAGUGGAGAACCGCUUCACCAAAAGCUGUCCCUUAGGG GAUUAGAACUUGAGUGAAGGUGGGCUGCUUGCAUCAGCCUAAUGUCGA GAAGUGCUUUCUUCGGAAAGUAACCCUCGAAACAAAUUCANNNCNCCUC UCCAAUUCUGCACAAGAAAGUUGCAGAACCCGAAUAGNGNNNUGAAGGA AUGCAACNNNNNNNNNNNNNNNNNNNNUUUU-3' (SEQ ID NO: 463). As still yet another example, the engineered guide RNA may comprise SEQ ID NO: 270, which is an engineered tracrRNA, and SEQ ID NO: 433 which is an engineered crRNA. As still yet another example, the engineered guide RNA may comprise 5'-CUUCACUGAUAAAGUGGAGAACCGCUUCACCAAAAGCUGUCCCUUAGGG GAUUAGAACUUGAGUGAAGGUGGGCUGCUUGCAUCAGCCUAAUGUCGA GAAGUGCUUUCUUCGGAAAGUAACCCUCGAAACAAAUUCAGUGCUCCUC UCCAAUUCUGCACAAGAAAGUUGCAGAACCCGAAUAGAGCAAUGAAGGA AUGCAACNNNNNNNNNNNNNNNNNNNNUUUU-3' (SEQ ID NO: 464). Here, N may each be A, C, G or U.

### 4. Uses of engineered guide RNA

The engineered guide RNA may constitute an engineered CRISPR/Cas12f1 (Cas14a1) system along with Cas12f1 (Cas14a1).

The engineered guide RNA may bind to a Cas12f1 (Cas14a1) protein to form an engineered CRISPR/Cas12f1 complex (or CRISPR/Cas14a1 complex).

The engineered guide RNA may be used for cleaving, editing, or modifying a target nucleic acid or target gene by an engineered CRISPR/Cas12f1 (Cas14a1) system and can allow this action to be performed more effectively.

### <Engineered CRISPR/Cas12f1(Cas14a1) system>

Another aspect disclosed by the present specification relates to an engineered CRISPR/Cas12f1 (Cas14a1) system. The engineered CRISPR/Cas12f1 (Cas14a1) system includes all forms in which the engineered guide RNA and the Cas12f1 (Cas14a1) protein are normally expressed and/or normally function. The engineered CRISPR/Cas12f1 (Cas14a1) system allows cleavage, editing or modification of a target nucleic acid or target gene to be performed more effectively. In particular, the engineered CRISPR/Cas12f1 (Cas14a1) system may bring about the following effects:
increased stability of a guide RNA-Cas12f1 protein complex;
increased cleavage efficiency for a target nucleic acid by the CRISPR/Cas12f1 (Cas14a1) system; and
increased editing or modifying efficiency for a target nucleic acid by the CRISPR/Cas12f1 (Cas14a1) system.

More specifically, the engineered CRISPR/Cas12f1 (Cas14a1) system comprises:
an engineered guide RNA or a nucleic acid encoding the same; and
a Cas12f1 (Cas14a1) protein or a nucleic acid encoding the same.

Each configuration will be described in detail below.

### 1. Engineered guide RNA

The engineered CRISPR/Cas12f1 (Cas14a1) system disclosed herein comprises an engineered guide RNA that recognizes a target sequence present in a target nucleic acid or target gene. In general, one engineered guide RNA can recognize one target sequence. The engineered guide RNA is as described above in the section "<Engineered guide RNA>" and has the same characteristics and structure as each configuration described in the section.

In addition, a nucleic acid encoding the engineered guide RNA may be DNA encoding the engineered guide RNA for transcribing the engineered guide RNA.

### 2. Cas12f1 (Cas14a1) protein

The Cas12f1 protein, which is a major protein component of a CRISPR/Cas12f1 (Cas14a1) system, is one of the effector proteins named Cas14 in a previous study (Harrington et al., Science, 362, 839-842 (2018)) and is also called a Cas14a1 protein. In the present specification, the term "Cas12f1 protein" is used interchangeably with "Cas14a1 protein" or "Cas12f1 (Cas14a1) protein". The Cas12f1 protein disclosed herein may be a wildtype Cas12f1 protein (wildtype Cas14a1 protein) existing in nature. Alternatively, the Cas12f1 protein may be a variant of the wildtype Cas12f1 protein, wherein the variant is referred to as "Cas12f1 variant" or "Cas14a1 variant". The Cas12f1 variant may be a variant having the same function as the wildtype Cas12f1 protein, a variant of which some or all functions are modified as compared with the wildtype Cas12f1 protein, and/or a variant to which an additional function is added as compared with the wildtype Cas12f1 protein. The meaning of "Cas12f1 protein" may be appropriately interpreted according to the context, and is interpreted in the broadest sense except for a particular case. Hereinafter, the Cas12f1 protein will be described in detail.

### 2-1) Wildtype Cas12f1 (Cas14a1) protein

The Cas12f1 protein may be a wildtype Cas12f1 protein. Here, the Cas12f1 protein is capable of cleaving a double-strand or a single-strand of a target nucleic acid or target gene. The Cas12f1 protein is capable of recognizing a protospacer adjacent motif (PAM) sequence present in a target nucleic acid or target gene. Here, the PAM sequence is a unique sequence determined according to the Cas14a1 protein. The PAM sequence for the Cas12f1 protein may be a T-rich sequence. The PAM sequence for the Cas12f1 protein may be 5'-TTTR-3'. Here, R may be A or G. For example, the PAM sequence may be 5'-TTTA-3' or 5'-TTTG-3'.

In an embodiment, the Cas12f1 protein may be derived from the Cas14 family (Harrington et al., Science 362, 839-842 (2018); US 2020/0172886 A1).

In another embodiment, the Cas12f1 protein may be a Cas14a1 protein derived from an uncultured archaeon (Harrington et al., Science 362, 839-842 (2018); US 2020/0172886 A1). For example, the Cas14a1 protein may be an amino acid sequence of SEQ ID NO: 465.

**[Table 1]**

| Cas14a1 (Cas12f1) protein | |
|---|---|
| Name | Amino acid sequence |
| Cas12f1 protein | mAKNTITKTLKLRIVRPYNSAEVEKIVADEKNNREKIALEKNKDKVK EACSKHLKVAAYCTTQVERNACLFCKARKLDDKFYQKLRGQFPDA VFWQEISEIFRQLQKQAAEIYNQSLIELYYEIFIKGKGIANASSVEHY |
| | LSDVCYTRAAELFKNAAIASGLRSKIKSNFRLKELKNMKSGLPTTK SDNFPIPLVKQKGGQYTGFEISNHNSDFIIKIPFGRWQVKKEIDKYR PWEKFDFEQVQKSPKPISLLLSTQRRKRNKGWSKDEGTEAEIKKV MNGDYQTSYIEVKRGSKIGEKSAWMLNLSIDVPKIDKGVDPSIIGGI DVGVKSPLVCAINNAFSRYSISDNDLFHFNKKMFARRRILLKKNRH KRAGHGAKNKLKPITILTEKSERFRKKLIERWACEIADFFIKNKVGT VQMENLESMKRKEDSYFNIRLRGFWPYAEMQNKIEFKLKQYGIEIR KVAPNNTSKTCSKCGHLNNYFNFEYRKKNKFPHFKCEKCNFKEN ADYNAALNISNPKLKSTKEEP (SEQ ID NO: 465) |

### 2-2) Cas12f1 (Cas14a1) variant - Cas12f1 (Cas14a1) mutant

The Cas12f1 protein may be a Cas12f1 variant. The Cas12f1 variant may be a variant of a wildtype Cas12f1 protein of which at least one amino acid in an amino acid sequence is modified. Here, the modification may be deletion and/or substitution. Here, the Cas12f1 variant is referred to as "Cas12f1 mutant" or "Cas14a1 mutant".

In an embodiment, the Cas12f1 mutant may be obtained by deleting at least one amino acid in an amino acid sequence of a wildtype Cas12f1 protein. For example, the Cas12f1 mutant may be obtained by deleting at least one amino acid in a RuvC domain included in a wildtype Cas12f1 protein. Alternatively, the Cas12f1 mutant may be obtained by deleting at least one amino acid in a domain that recognizes a PAM included in a wildtype Cas12f1 protein. Alternatively, the Cas12f1 mutant may be obtained by deleting at least one amino acid in an amino acid sequence of SEQ ID NO: 465.

In another embodiment, the Cas12f1 mutant may be obtained by substituting at least one amino acid in an amino acid sequence of a wildtype Cas12f1 protein with other amino acid(s). Here, the substitution may be such that one amino acid is substituted with one other amino acid. Alternatively, the substitution may be such that one amino acid is substituted with a plurality of other amino acids. Alternatively, the substitution may be such that a plurality of amino acids are substituted with one other amino acid. Alternatively, the substitution may be such that a plurality of amino acids are substituted with a plurality of other amino acids wherein the number of amino acids to be substituted and the number of substituting amino acids may be the same or different from each other. For example, the Cas12f1 mutant may be obtained by substituting at least one amino acid in a RuvC domain included in a wildtype Cas12f1 protein with other amino acid(s). Alternatively, the Cas12f1 mutant may be obtained by substituting at least one amino acid in a domain, which recognizes a PAM included in a wildtype Cas12f1 protein, with other amino acid(s). Alternatively, the Cas12f1 mutant may be obtained by substituting at least one amino acid in an amino acid sequence of SEQ ID NO: 465 with other amino acid(s).

The Cas12f1 mutant may be a variant having the same function as a wildtype Cas12f1 protein or a variant of which some or all functions are modified as compared with a wildtype Cas12f1 protein. For example, the Cas12f1 mutant may be a variant in which modification is made to cleave only one strand in a double-strand of a target nucleic acid. Alternatively, the Cas12f1 mutation may be a variant in which modification is made to recognize a PAM sequence other than 5'-TTTA-3' or 5'-TTTG-3'.

### 2-3) Cas12f1 (Cas14a1) variant - Cas12f1 (Cas14a1) fusion protein

The Cas12f1 protein may be a Cas12f1 variant. The Cas12f1 variant may be a variant obtained by adding a domain, peptide or protein having an additional function to a wildtype Cas12f1 protein or a Cas12f1 mutant. Here, the variant, to which the domain, peptide or protein having an additional function is added, is referred to as "Cas12f1 fusion protein" or "Cas14a1 fusion protein". The domain, peptide or protein having an additional function may be added to the N-terminus and/or C-terminus, and/or in an amino acid sequence, of a wildtype Cas12f1 protein or a Cas12f1 mutant. The domain, peptide or protein having an additional function may be a domain, peptide or protein having the same or different function as a wildtype Cas12f1 protein. For example, the domain, peptide or protein having an additional function may be a domain, peptide or protein having methylase activity, demethylase activity, transcription activation activity, transcription repression activity, transcription release factor activity, histone modification activity, RNA cleavage activity or nucleic acid binding activity, or may be a tag or reporter protein used for isolation and purification of a protein (including a peptide), but is not limited thereto. Alternatively, the domain, peptide or protein having an additional function may be reverse transcriptase or deaminase.

In an embodiment, the Cas12f1 fusion protein may comprise a wildtype Cas12f1 protein and deaminase. Here, the deaminase may be cytosine deaminase, cytidine deaminase, or adenine deaminase. Here, the Cas12f1 fusion protein may optionally further comprise a domain, peptide or protein having an additional function. In an embodiment, the Cas12f1 fusion protein may be an amino acid sequence of SEQ ID NO: 466 or an amino acid sequence of SEQ ID NO: 467. In another embodiment, the Cas12f1 fusion protein may be an amino acid sequence of SEQ ID NO: 468 or an amino acid sequence of SEQ ID NO: 469.

In another embodiment, the Cas12f1 fusion protein may comprise a wildtype Cas12f1 protein and reverse transcriptase. Here, the reverse transcriptase may be Moloney murine leukemia virus (M-MLV) reverse transcriptase or a variant thereof. Here, the Cas12f1 fusion protein may optionally further comprise a domain, peptide or protein having an additional function. In an embodiment, the Cas12f1 fusion protein may be an amino acid sequence of SEQ ID NO: 470.

The Cas12f1 fusion protein may be a variant having the same function as a wildtype Cas12f1 protein and an additional function. Alternatively, the Cas12f1 fusion protein may be a variant having modification of some or all functions of a wildtype Cas12f1 protein and an additional function. For example, the Cas12f1 fusion protein may be such that modification is made to be able to cut only one strand in a double strand of a target nucleic acid and to perform base editing or prime editing on a uncleaved strand. Here, the Cas12f1 fusion protein may comprise a Cas12f1 mutant and deaminase wherein the base editing may be performed by the deaminase. Alternatively, the Cas12f1 fusion protein may comprise a Cas12f1 mutant and reverse transcriptase wherein the prime editing may be performed by the reverse transcriptase.

### 2-4) Other additional elements included in Cas12f1 (Cas14a1) variant

The Cas12f1 protein may be a Cas12f1 variant. The Cas12f1 variant may be a wildtype Cas12f1 protein, a Cas12f1 mutant, or a Cas12f1 fusion protein, in which a nuclear localization sequence (NLS) or a nuclear export sequence (NES) is optionally further included. The NLS and NES are signal peptides for positioning the Cas12f1 protein in or outside a cell. The terms "NLS" and "NES" include all meanings recognized by those skilled in the art, and may be appropriately interpreted according to the context. For example, the NLS may be, but is not limited to, an NLS sequence derived from the NLS of an SV40 virus large T-antigen, having the amino acid sequence PKKKRKV (SEQ ID NO: 471); the NLS from a nucleoplasmin (for example, the nucleoplasmin bipartite NLS having the sequenceKRPAATKKAGQAKKKK (SEQ ID NO: 472)); the c-myc NLS having the amino acid sequence PAAKRVKLD (SEQ ID NO: 473) or RQRRNELKRSP (SEQ ID NO: 474); the hRNPA1 M9 NLS having thea sequenceNQSSNFGPMKGGNFGGRSSGPYGGGGQYFAKPRNQGGY (SEQ ID NO: 475); the sequence RMRIZFKNKGKDTAELRRRRVEVSVELRKAKKDEQILKRRNV (SEQ ID NO: 476) of an IBB domain from importin alpha; the sequences VSRKRPRP (SEQ ID NO: 477) and PPKKARED (SEQ ID NO: 478) of myoma T protein; the sequence PQPKKKPL (SEQ ID NO: 479) of human p53; the sequence SALIKKKKKMAP (SEQ ID NO: 480) of mouse c-abl IV; the sequences DRLRR (SEQ ID NO: 481) and PKQKKRK (SEQ ID NO: 482) of influenza virus NS1; the sequence RKLKKKIKKL (SEQ ID NO: 483) of hepatitis virus delta antigen; the sequence REKKKFLKRR (SEQ ID NO: 484) of mouse Mx1 protein; the sequence KRKGDEVDGVDEVAKKKSKK (SEQ ID NO: 485) of human poly (ADP-ribose) polymerase; or the sequence RKCLQAGMNLEARKTKK (SEQ ID NO: 486) of steroid hormone receptor (human) glucocorticoid.

For example, the Cas12f1 variant may be a wildtype Cas12f1 protein to which an NLS is added. In an embodiment, the Cas12f1 variant may be an amino acid sequence of SEQ ID NO: 738(PKKKRKVGIHGVPAAMAKNTITKTLKLRIVRPYNSAEVEKIVADEKNNREKI ALEKNKDKVKEACSKHLKVAAYCTTQVERNACLFCKARKLDDKFYQKLRGQF PDAVFWQEISEIFRQLQKQAAEIYNQSLIELYYEIFIKGKGIANASSVEHYLSDV CYTRAAELFKNAAIASGLRSKIKSNFRLKELKNMKSGLPTTKSDNFPIPLVKQK GGQYTGFEISNHNSDFIIKIPFGRWQVKKEIDKYRPWEKFDFEQVQKSPKPISL LLSTQRRKRNKGWSKDEGTEAEIKKVMNGDYQTSYIEVKRGSKIGEKSAWML NLSIDVPKIDKGVDPSIIGGIDVGVKSPLVCAINNAFSRYSISDNDLFHFNKKMF ARRRILLKKNRHKRAGHGAKNKLKPITILTEKSERFRKKLIERWACEIADFFIKN KVGTVQMENLESMKRKEDSYFNIRLRGFWPYAEMQNKIEFKLKQYGIEIRKVA PNNTSKTCSKCGHLNNYFNFEYRKKNKFPHFKCEKCNFKENADYNAALNISN PKLKSTKEEPKRPAATKKAGQAKKKK (SEQ ID NO: 738)).

In addition, the Cas12f1 variant may be a wildtype Cas12f1 protein, a Cas12f1 mutant, or a Cas12f1 fusion protein, in which a tag is optionally further included. The tag may be a functional domain, peptide or protein for isolation and purification and/or tracking of a Cas12f1 protein, and may be one of the tags exemplified in the section "Tag"in definition of terms as described above, but is not limited thereto.

### 2-5) Nucleic acid encoding Cas12f1 (Cas14a1) protein

A nucleic acid encoding a Cas12f1 protein may be a nucleic acid encoding a wildtype Cas12f1 protein or a nucleic acid encoding a Cas12f1 variant. The nucleic acid encoding a Cas12f1 protein may be codon-optimized according to the target into which the Cas12f1 protein is to be introduced.

"Codon optimization" refers to a process of modifying a native nucleic acid sequence for enhanced expression in a cell of interest by replacing at least one codon in the native sequence with a codon, which is used more frequently or most frequently in a gene of a target cell, while maintaining its native amino acid sequence. Different species have specific biases for specific codons of specific amino acids, and codon bias (differences in codon usage between organisms) is often correlated with translation efficiency of an mRNA, which is considered to be dependent on the nature of codons being translated and availability of specific tRNA molecules. Predominance of tRNA selected in a cell generally reflects the most frequently used codon in peptide synthesis. Thus, genes may be tailored for optimal gene expression in a given organism based on codon optimization.

In an embodiment, the nucleic acid encoding a Cas12f1 protein may be a nucleic acid encoding a human codon-optimized Cas12f1 protein. For example, the nucleic acid encoding a human codon-optimized Cas12f1 protein may be SEQ ID NO: 487.

**[Table 2]**

| Name | Sequence |
|---|---|
| Nucleic acid encoding human codon-optimized Cas12f1 protein | ATGGCCAAGAACACAATTACAAAGACACTGAAGCTGAGGATCG TGAGACCATACAACAGCGCTGAGGTCGAGAAGATTGTGGCTGA TGAAAAGAACAACAGGGAAAAGATCGCCCTCGAGAAGAACAAG GATAAGGTGAAGGAGGCCTGCTCTAAGCACCTGAAAGTGGCC GCCTACTGCACCACACAGGTGGAGAGGAACGCCTGTCTGTTTT GTAAAGCTCGGAAGCTGGATGATAAGTTTTACCAGAAGCTGCG GGGCCAGTTCCCCGATGCCGTCTTTTGGCAGGAGATTAGCGAG ATCTTCAGACAGCTGCAGAAGCAGGCCGCCGAGATCTACAACC AGAGCCTGATCGAGCTCTACTACGAGATCTTCATCAAGGGCAA GGGCATTGCCAACGCCTCCTCCGTGGAGCACTACCTGAGCGA CGTGTGCTACACAAGAGCCGCCGAGCTCTTTAAGAACGCCGCT ATCGCTTCCGGGCTGAGGAGCAAGATTAAGAGTAACTTCCGGC TCAAGGAGCTGAAGAACATGAAGAGCGGCCTGCCCACTACAAA GAGCGACAACTTCCCAATTCCACTGGTGAAGCAGAAGGGGGG CCAGTACACAGGGTTCGAGATTTCCAACCACAACAGCGACTTT ATTATTAAGATCCCCTTTGGCAGGTGGCAGGTCAAGAAGGAGA TTGACAAGTACAGGCCCTGGGAGAAGTTTGATTTCGAGCAGGT GCAGAAGAGCCCCAAGCCTATTTCCCTGCTGCTGTCCACACAG CGGCGGAAGAGGAACAAGGGGTGGTCTAAGGATGAGGGGACC GAGGCCGAGATTAAGAAAGTGATGAACGGCGACTACCAGACAA GCTACATCGAGGTCAAGCGGGGCAGTAAGATTGGCGAGAAGA GCGCCTGGATGCTGAACCTGAGCATTGACGTGCCAAAGATTGA |
| | TAAGGGCGTGGATCCCAGCATCATCGGAGGGATCGATGTGGG GGTCAAGAGCCCCCTCGTGTGCGCCATCAACAACGCCTTCAGC AGGTACAGCATCTCCGATAACGACCTGTTCCACTTTAACAAGAA GATGTTCGCCCGGCGGAGGATTTTGCTCAAGAAGAACCGGCAC AAGCGGGCCGGACACGGGGCCAAGAACAAGCTCAAGCCCATC ACTATCCTGACCGAGAAGAGCGAGAGGTTCAGGAAGAAGCTCA TCGAGAGATGGGCCTGCGAGATCGCCGATTTCTTTATTAAGAA CAAGGTCGGAACAGTGCAGATGGAGAACCTCGAGAGCATGAA GAGGAAGGAGGATTCCTACTTCAACATTCGGCTGAGGGGGTTC TGGCCCTACGCTGAGATGCAGAACAAGATTGAGTTTAAGCTGA AGCAGTACGGGATTGAGATCCGGAAGGTGGCCCCCAACAACA CCAGCAAGACCTGCAGCAAGTGCGGGCACCTCAACAACTACTT CAACTTCGAGTACCGGAAGAAGAACAAGTTCCCACACTTCAAG TGCGAGAAGTGCAACTTTAAGGAGAACGCCGATTACAACGCCG CCCTGAACATCAGCAACCCTAAGCTGAAGAGCACTAAGGAGGA GCCC (SEQ ID NO: 487) |

### 3. Engineered guide RNA-Cas12f1 (Cas14a1) protein(s) complex (CRISPR complex)

The engineered CRISPR/Cas12f1 (Cas14a1) system disclosed by the present specification may be provided in a form of a CRISPR complex. The CRISPR complex comprises an engineered guide RNA and a Cas12f1 protein. Here, the CRISPR complex comprises an engineered guide RNA and two Cas12f1 proteins (Satoru N. Takeda et al., Molecular Cell, 81, 1-13, (2021)). The CRISPR complex may be a ribonucleoprotein (RNP) formed by interaction of an engineered guide RNA with a Cas12f1 protein. Here, the CRISPR complex may be a ribonucleoprotein (RNP) formed by interaction of an engineered guide RNA with one Cas12f1 protein or an RNP formed by interaction of an engineered guide RNA with two Cas12f1 proteins (Satoru N (Takeda et al., Molecular Cell, 81, 1-13, (2021)). The CRISPR complex is referred to as "engineered guide RNA-Cas12f1 (Cas14a1) protein complex", "engineered CRISPR/Cas12f1 complex" or "engineered CRISPR/Cas14a1 complex", and the terms are used interchangeably herein. Specific descriptions of the components included in the examples below are the same as those described above for the corresponding components. The examples are for an illustrative purpose only, and the scope of the present disclosure is not limited thereto.

In an embodiment, the CRISPR complex may be an RNP formed by combination of a wildtype Cas12f1 protein and an engineered guide RNA. Here, the CRISPR complex may comprise two wildtype Cas12f1 proteins and an engineered guide RNA. Here, the wildtype Cas12f1 protein may be an amino acid sequence of SEQ ID NO: 465. Here, the engineered guide RNA comprises an engineered tracrRNA and a crRNA, wherein the engineered tracrRNA is modified not to comprise a sequence of five or more consecutive uridines or modified not to comprise a sequence of five or more consecutive uridines and to have a shorter length than a wildtype tracrRNA. Here, the engineered tracrRNA may be a sequence that does not comprise a sequence of five or more consecutive uridines. Alternatively, the engineered tracrRNA may be a sequence that does not comprise a sequence of five or more consecutive uridines, and does not comprise a sequence of 1 to 24 nucleotides at the 5' end of a wildtype tracrRNA and/or a sequence of 1 to 28 nucleotides at the 3' end of a wildtype tracrRNA. Here, the crRNA may be a wildtype crRNA or an engineered crRNA.

In an embodiment, the CRISPR complex may be an RNP formed by combination of a wildtype Cas12f1 protein and an engineered guide RNA. Here, the CRISPR complex may comprise two wildtype Cas12f1 proteins and an engineered guide RNA. Here, the wildtype Cas12f1 protein may be an amino acid sequence of SEQ ID NO: 465. Here, the engineered guide RNA may be one of the examples described above in the section "3-3) Examples of engineered guide RNA".

In another embodiment, the CRISPR complex may be an RNP formed by combination of a Cas12f1 variant and an engineered guide RNA. Here, the CRISPR complex may comprise two Cas12f1 variants and an engineered guide RNA. Here, the Cas12f1 variant may be a Cas12f1 mutant or a Cas12f1 fusion protein. Here, the engineered guide RNA comprises an engineered tracrRNA and a crRNA, wherein the engineered tracrRNA is modified not to comprise a sequence of five or more consecutive uridines or modified not to comprise a sequence of five or more consecutive uridines and to have a shorter length than a wildtype tracrRNA. Here, the engineered tracrRNA may be a sequence that does not comprise a sequence of five or more consecutive uridines. Alternatively, the engineered tracrRNA may be a sequence that does not comprise a sequence of five or more consecutive uridines, and does not comprise a sequence of 1 to 24 nucleotides at the 5' end of a wildtype tracrRNA and/or a sequence of 1 to 28 nucleotides at the 3' end of a wildtype tracrRNA. Here, the crRNA may be a wildtype crRNA or an engineered crRNA.

In an embodiment, the CRISPR complex may be an RNP formed by combination of a Cas12f1 variant and an engineered guide RNA. Here, the CRISPR complex may comprise two Cas12f1 variants and an engineered guide RNA. Here, the Cas12f1 variant may be a Cas14a1 mutant obtained by substituting at least one amino acid in an amino acid sequence of SEQ ID NO: 465 with other amino acid(s). Here, the two Cas12f1 variants may be the same or different. Here, the engineered guide RNA may be one of the examples described above in the section "3-3) Examples of engineered guide RNA".

In another embodiment, the CRISPR complex may be an RNP formed by combination of a Cas12f1 variant and an engineered guide RNA. Here, the CRISPR complex may comprise two Cas12f1 variants and an engineered guide RNA. Here, the Cas12f1 variant may be a Cas12f1 fusion protein obtained by adding a domain, peptide or protein having an additional function to a wildtype Cas12f1 protein or a Cas12f1 mutant. The Cas12f1 fusion protein may be a protein having an amino acid sequence of SEQ ID NO: 466, an amino acid sequence of SEQ ID NO: 467, an amino acid sequence of SEQ ID NO: 468, an amino acid sequence of SEQ ID NO: 469, or an amino acid sequence of SEQ ID NO: 470. Here, the two Cas12f1 variants may be the same or different. Here, the engineered guide RNA may be one of the examples described above in the section "3-3) Examples of engineered guide RNA".

In another embodiment, the CRISPR complex may be an RNP formed by combination of a wildtype Cas12f1 protein, a Cas12f1 variant, and an engineered guide RNA. Here, the wildtype Cas12f1 protein may be an amino acid sequence of SEQ ID NO: 465. Here, the Cas12f1 variant may be a Cas12f1 mutant or a Cas12f1 fusion protein. Here, the engineered guide RNA comprises an engineered tracrRNA and a crRNA, wherein the engineered tracrRNA is modified not to comprise a sequence of five or more consecutive uridines or modified not to comprise a sequence of five or more consecutive uridines and to have a shorter length than a wildtype tracrRNA. Here, the engineered tracrRNA may be a sequence that does not comprise a sequence of five or more consecutive uridines. Alternatively, the engineered tracrRNA may be a sequence that does not comprise a sequence of five or more consecutive uridines, and does not comprise a sequence of 1 to 24 nucleotides at the 5' end of a wildtype tracrRNA and/or a sequence of 1 to 28 nucleotides at the 3' end of a wildtype tracrRNA. Here, the crRNA may be a wildtype crRNA or an engineered crRNA.

In an embodiment, the CRISPR complex may be an RNP formed by combination of a wildtype Cas12f1 protein, a Cas12f1 variant, and an engineered guide RNA. Here, the wildtype Cas12f1 protein may be an amino acid sequence of SEQ ID NO: 465. Here, the Cas12f1 variant may be a Cas12f1 mutant obtained by substituting at least one amino acid in an amino acid sequence of SEQ ID NO: 465 with other amino acid(s). Here, the engineered guide RNA may be one of the examples described above in the section "3-3) Examples of engineered guide RNA".

In another embodiment, the CRISPR complex may be an RNP formed by combination of a wildtype Cas12f1 protein, a Cas12f1 variant, and an engineered guide RNA. Here, the wildtype Cas12f1 protein may be an amino acid sequence of SEQ ID NO: 465. Here, the Cas12f1 variant may be a Cas12f1 fusion protein obtained by adding a domain, peptide or protein having an additional function to a wildtype Cas12f1 protein or a Cas12f1 mutant. The Cas12f1 fusion protein may be a protein having an amino acid sequence of SEQ ID NO: 466, an amino acid sequence of SEQ ID NO: 467, an amino acid sequence of SEQ ID NO: 468, an amino acid sequence of SEQ ID NO: 469, or an amino acid sequence of SEQ ID NO: 470. Here, the engineered guide RNA may be one of the examples described above in the section "3-3) Examples of engineered guide RNA".

### 4. Uses of engineered CRISPR/Cas12f1 (Cas14a1) system

The engineered CRISPR/Cas12f1 (Cas14a1) system may be used for cleaving, editing, or modifying a target nucleic acid or target gene, and can allow this action to be performed more effectively.

In an embodiment, the use may comprise bringing an engineered CRISPR/Cas14a1 complex in contact with a target nucleic acid or target gene in a target cell.

In another embodiment, the use may comprise inducing an engineered CRISPR/Cas14a1 complex to come in contact with a target nucleic acid or target gene in a target cell. Here, the induction method is not particularly limited as long as it allows the engineered CRISPR/Cas14a1 complex to come in contact with the target nucleic acid in the cell. For example, the induction may be achieved by delivering, into a cell, an engineered guide RNA or a nucleic acid encoding the same, and a Cas14a1 protein or a nucleic acid encoding the same.

### <Modification of target using engineered CRISPR/Cas12f1 (Cas14a1) system>

Another aspect disclosed by the present specification relates to modifying a target using an engineered CRISPR/Cas12f1 (Cas14a1) system. More specifically, the aspect relates to a composition comprising an engineered CRISPR/Cas12f1 (Cas14a1) system and to modifying a target nucleic acid or target gene by a method using the composition. The composition comprising the engineered CRISPR/Cas12f1 (Cas14a1) system and the method using the same allow cleavage, editing or modifying of a target nucleic acid or target gene to be performed more effectively. In particular, use of the composition comprising the engineered CRISPR/Cas12f1 (Cas14a1) system may bring about the following effects:
increased stability of a guide RNA-Cas12f1 protein complex;
increased cleavage efficiency of a target nucleic acid by the CRISPR/Cas12f1 (Cas14a1) system; and
increased diting or modifying efficiency of a target nucleic acid by the CRISPR/Cas12f1 (Cas14a1) system.

Hereinafter, the composition and the method using the same will be described in detail.

### 1. Composition

The composition disclosed herein may comprise an engineered CRISPR/Cas12f1 (Cas14a1) system. The engineered CRISPR/Cas12f1 (Cas14a1) system is as described above in the section "<Engineered CRISPR/Cas12f1 (Cas14a1) system>" and has the same characteristics and structure as each configuration described in the section.

More specifically, the composition may comprise:
an engineered guide RNA or a nucleic acid encoding the same; and
a Cas12f1 (Cas14a1) protein or a nucleic acid encoding the same.

Here, the composition may optionally further comprise one or more engineered guide RNAs or nucleic acids encoding the same. Here, the composition may comprise a plurality of engineered guide RNAs, and the plurality of engineered guide RNAs may each recognize a different target sequence.

Here, the composition may optionally further comprise one or more Cas12f1 (Cas14a1) proteins or nucleic acids encoding the same. Here, the composition may comprise two or more Cas12f1 (Cas14a1) proteins having different amino acid sequences. Here, the two or more Cas12f1 (Cas14a1) proteins having different amino acid sequences may be a wildtype Cas12f1 (Cas14a1) protein and a Cas14a1 variant.

The engineered guide RNA is as described above in the section "<Engineered guide RNA>" and has the same characteristics and structure as each configuration described in the section. In addition, the Cas12f1 (Cas14a1) protein is as described above in the section "2. Cas12f1 (Cas14a1) protein" and has the same characteristics and structure as each configuration described in the section.

The composition may be in a form of a nucleic acid. Alternatively, the composition may be in a form in which a nucleic acid and a protein are mixed. Hereinafter, the form of the composition will be described in detail.

### 1-1) Form of nucleic acid

The composition may be in a form of a nucleic acid. The nucleic acid may be in a form of DNA or RNA, or in a mixed form thereof. The nucleic acid may be in a form of a vector. Here, the vector may be a viral vector or a non-viral vector.

In addition, a shape of the nucleic acid may be circular or linear.

In addition, a shape of the nucleic acid may be double-stranded or single-stranded.

### a) Viral vector

The composition may be in a form of a viral vector. For example, the viral vector may be one or more viral vectors selected from the group consisting of : a retroviral (retrovirus) vector, a lentiviral (lentivirus) vector, an adenoviral (adenovirus) vector, or an adeno-associated viral (adeno-associated virus; AAV) vector, a vaccinia viral (vaccinia virus) vector, a poxviral (poxvirus) vector, and a herpes simplex viral (herpes simplex virus) vector.

In an embodiment, the composition may be in a form of a viral vector. That is, the composition may comprise a nucleic acid encoding an engineered guide RNA and a nucleic acid encoding a Cas12f1 (Cas14a1) protein in a form of a viral vector. Here, the composition may comprise a first viral vector comprising a nucleic acid encoding an engineered guide RNA and a second viral vector comprising a nucleic acid encoding a Cas12f1 protein. Alternatively, the composition may comprise a viral vector comprising both a nucleic acid encoding an engineered guide RNA and a nucleic acid encoding a Cas12f1 protein. In an embodiment, the composition may comprise an adeno-associated viral vector comprising both a nucleic acid encoding an engineered guide RNA and a nucleic acid encoding a Cas12f1 protein.

In another embodiment, when the composition comprises nucleic acids encoding a plurality of engineered guide RNAs and a nucleic acid encoding a Cas12f1 protein, the composition may comprise a first viral vector comprising all of the nucleic acids encoding a plurality of engineered guide RNAs and a second viral vector comprising the nucleic acid encoding a Cas12f1 protein. Alternatively, the composition may be in a form of a single viral vector comprising all of the nucleic acids encoding a plurality of engineered guide RNAs and the nucleic acid encoding a Cas12f1 protein. Alternatively, the composition may comprise a plurality of (as much as the number of engineered guide RNAs) viral vectors, each of which comprises a nucleic acid encoding one engineered guide RNA and a nucleic acid encoding a Cas12f1 protein.

In another embodiment, when the composition comprises a nucleic acid encoding an engineered guide RNA and nucleic acids encoding two or more Cas12f1 proteins (for example, a first Cas12f1 protein and a second Cas12f1 protein), the composition may comprise a first viral vector comprising a nucleic acid encoding an engineered guide RNA, a second viral vector comprising a nucleic acid encoding a first Cas12f1 protein, and a third viral vector comprising a nucleic acid encoding a second Cas12f1 protein. Alternatively, the composition may be in a form of a single viral vector comprising both a nucleic acid encoding an engineered guide RNA and nucleic acids encoding two or more Cas12f1 proteins (for example, a first Cas12f1 protein and a second Cas12f1 protein). Alternatively, the composition may comprise a first viral vector comprising a nucleic acid encoding an engineered guide RNA and a nucleic acid encoding a first Cas12f1 protein, and a second viral vector comprising a nucleic acid encoding a second Cas12f1 protein.

### b) Non-viral vector

The composition may be in a form of a non-viral vector. Here, the engineered guide RNA in the composition may be in a form of RNA or a nucleic acid encoding the same.

The non-viral vector may be a plasmid, phage, naked DNA, a DNA complex, mRNA (transcript) or a PCR amplicon, but is not limited thereto. For example, the plasmid may be selected from the group consisting of: pcDNA series, pSC101, pGV1106, pACYC177, ColE1, pKT230, pME290, pBR322, pUC8/9, pUC6, pBD9, pHC79, pIJ61, pLAFR1, pHV14, pGEX series, pET series, and pUC19. For example, the phage may be selected from: λgt4λB, λ-Charon, λΔz1, and M13.

In an embodiment, the composition may be in a form of a non-viral vector. That is, the composition may comprise a nucleic acid encoding an engineered guide RNA and a nucleic acid encoding a Cas12f1 protein in a form of a non-viral vector. Here, the composition may comprise a first non-viral vector comprising a nucleic acid encoding an engineered guide RNA and a second non-viral vector comprising a nucleic acid encoding a Cas12f1 protein. Alternatively, the composition may comprise a non-viral vector comprising both a nucleic acid encoding an engineered guide RNA and a nucleic acid encoding a Cas12f1 protein. In an embodiment, the composition may comprise a plasmid comprising both a nucleic acid encoding an engineered guide RNA and a nucleic acid encoding a Cas12f1 protein. In another embodiment, the composition may comprise a PCR amplicon comprising a nucleic acid encoding an engineered guide RNA and an mRNA encoding a Cas12f1 protein. In another embodiment, the composition may comprise a PCR amplicon comprising a nucleic acid encoding an engineered guide RNA and naked DNA comprising a nucleic acid encoding a Cas12f1 protein.

In another embodiment, the composition may be in a form of a non-viral vector. That is, the composition may comprise nucleic acids encoding an engineered guide RNA and a Cas12f1 protein in a form of a non-viral vector. In an embodiment, the composition may comprise an engineered guide RNA, and an mRNA encoding a Cas12f1 protein. In another embodiment, the composition may comprise an engineered guide RNA, and a plasmid comprising a nucleic acid encoding a Cas12f1 protein.

### c) Mixing of viral and non-viral vectors

The composition may be in a form in which a viral vector and a non-viral vector are mixed. The description of the viral vector and the non-viral vector is as described above. Here, the engineered guide RNA in the composition may be in a form of RNA or a nucleic acid encoding the same.

In an embodiment, the composition may comprise a viral vector comprising a nucleic acid encoding an engineered guide RNA and a non-viral vector comprising a nucleic acid encoding a Cas12f1 protein. In an embodiment, the composition may comprise an adeno-associated viral vector comprising a nucleic acid encoding an engineered guide RNA and an mRNA encoding a Cas12f1 protein. In another embodiment, the composition may comprise a lentiviral vector comprising a nucleic acid encoding an engineered guide RNA and a plasmid comprising a nucleic acid encoding a Cas12f1 protein.

In another embodiment, the composition may comprise a non-viral vector comprising a nucleic acid encoding an engineered guide RNA and a viral vector comprising a nucleic acid encoding a Cas12f1 protein. In an embodiment, the composition may comprise a PCR amplicon comprising a nucleic acid encoding an engineered guide RNA and an adeno-associated viral vector comprising a nucleic acid encoding a Cas12f1 protein. In another embodiment, the composition may comprise a plasmid comprising a nucleic acid encoding an engineered guide RNA and a lentiviral vector comprising a nucleic acid encoding a Cas12f1 protein.

In another embodiment, the composition may comprise an engineered guide RNA and a viral vector comprising a nucleic acid encoding a Cas12f1 protein. In an embodiment, the composition may comprise an engineered guide RNA and an adeno-associated viral vector comprising a nucleic acid encoding a Cas12f1 protein.

In another embodiment, the composition may comprise an engineered guide RNA and a non-viral vector comprising a nucleic acid encoding a Cas12f1 protein. In an embodiment, the composition may comprise an engineered guide RNA and an mRNA encoding a Cas12f1 protein. In another embodiment, the composition may comprise an engineered guide RNA and a plasmid comprising a nucleic acid encoding a Cas12f1 protein.

### d) Others - additional component of vector

The vector described above may optionally further comprise a regulatory/control element, a promoter and/or an additionally expressed element.

### Regulatory/control element

The vector may optionally comprise a regulatory/control element. Here, the regulatory/control element may be operably linked to a sequence encoding each component included in the vector (i.e., a nucleic acid encoding an engineered guide RNA and/or a nucleic acid encoding a Cas12f1 protein). The regulatory/control element may be, but is not limited to, an enhancer, an intron, a termination signal, a polyadenylation signal, a Kozak consensus sequence, an internal ribosome entry site (IRES), a splice acceptor, a 2A sequence and/or a replication origin. Here, the replication origin may be, but is not limited to, an f1 origin of replication, an SV40 origin of replication, a pMB1 origin of replication, an adeno origin of replication, an AAV origin of replication, and/or a BBV origin of replication.

### Promoter

The vector may optionally comprise a promoter. Here, the promoter may be operably linked to a sequence encoding each component included in the vector (i.e., a nucleic acid encoding an engineered guide RNA and/or a nucleic acid encoding a Cas12f1 protein). The promoter is not limited as long as it is capable of properly expressing a sequence encoding each component included in the vector (i.e., a nucleic acid encoding an engineered guide RNA and/or a nucleic acid encoding a Cas12f1 protein). In an embodiment, the promoter sequence may be a promoter that promotes transcription of an RNA polymerase (for example, Pol I, Pol II, or Pol III). For example, the promoter may be, but is not limited to, one selected from: an SV40 early promoter, a mouse mammary tumor virus long terminal repeat (LTR) promoter, an adenovirus major late promoter (Ad MLP), a herpes simplex virus (HSV) promoter, a cytomegalovirus (CMV) promoter such as CMV immediate early promoter region (CMVIE), a rous sarcoma virus (RSV) promoter, a CBA promoter, a human U6 small nuclear promoter (U6) (Miyagishi et al., Nature Biotechnology 20, 497 - 500 (2002)), an enhanced U6 promoter (e.g., Xia et al., Nucleic Acids Res.2003 Sep 1:31(17)), a 7SK promoter, and a human H1 promoter (H1).

### Additionally expressed elements

The vector may optionally comprise an additionally expressed element. The vector may comprise a nucleic acid sequence encoding an additionally expressed element to be expressed as necessary by those skilled in the art in addition to a nucleic acid encoding an engineered guide RNA and/or a nucleic acid encoding a Cas12f1 protein. For example, the additionally expressed element may be one of the tags exemplified in the section "Tag" in definition of terms as described above, but is not limited thereto. For example, the additionally expressed element may be a herbicide resistance gene such as glyphosate, glufosinate ammonium or phosphinothricin; or an antibiotic resistance gene such as ampicillin, kanamycin, G418, bleomycin, hygromycin or chloramphenicol, but is not limited thereto.

### 1-2) Mixed form of nucleic acid and protein

The composition may be in a form in which a nucleic acid and a protein are mixed. Here, the nucleic acid is as described above in the section "1-1) Form of nucleic acid". Here, the composition comprises a Cas12f1 protein. Here, the composition may comprise two Cas12f1 proteins, and the two Cas12f1 proteins may be Cas12f1 proteins having the same amino acid sequence or Cas12f1 proteins having different amino acid sequences.

In an embodiment, the composition may comprise a viral vector comprising a nucleic acid encoding an engineered guide RNA and a Cas12f1 protein. In an embodiment, the composition may comprise an adeno-associated viral vector comprising a nucleic acid encoding an engineered guide RNA and a Cas12f1 protein.

In another embodiment, the composition may comprise a non-viral vector comprising a nucleic acid encoding an engineered guide RNA and a Cas12f1 protein. In an embodiment, the composition may comprise a plasmid comprising a nucleic acid encoding an engineered guide RNA and a Cas12f1 protein. In another embodiment, the composition may comprise a PCR amplicon comprising a nucleic acid encoding an engineered guide RNA and a Cas12f1 protein.

In another embodiment, the composition may comprise an engineered guide RNA and a Cas12f1 protein. Here, the composition may be in a form of an RNP which is in a form of an engineered CRISPR/Cas12f1 complex. In a case where a composition comprises a plurality of engineered guide RNAs and Cas12f1 proteins, the composition may comprise a plurality of engineered CRISPR/Cas12f1 complexes, and the plurality of engineered CRISPR/Cas12f1 complexes each may recognize a different target sequence.

### 1-3) Use of composition

The composition may be used for cleaving, editing, or modifying a target nucleic acid or target gene using an engineered CRISPR/Cas12f1 (Cas14a1) system, and may allow this action to be performed more effectively.

### 2. Method of modifying target using engineered CRISPR/Cas12f1 (Cas14a1) system

The method disclosed herein relates to a method using an engineered CRISPR/Cas12f1 (Cas14a1) system. More specifically, the method relates to a method of introducing (or delivering) a composition comprising an engineered CRISPR/Cas12f1 (Cas14a1) system into a subject and/or a target cell so that a target nucleic acid or target gene present in the subject and/or the target cell is modified. Alternatively, the method relates to a method of introducing (or delivering) an engineered guide RNA into a subject and/or a target cell so that a target nucleic acid or target gene present in the subject and/or the target cell is targeted. Here, the subject may be a plant or an animal, or some tissue thereof, wherein the animal may be a human or a non-human animal. Here, the target cell may be a prokaryotic cell or a eukaryotic cell. Here, the eukaryotic cell may be yeast, a plant cell, an animal cell, and/or a human cell, but is not limited thereto. The method may be performed in vitro, ex vivo or in vivo. Here, the in vivo may be in a human body or in a non-human animal body.

The method may be a method that comprises introducing (or delivering, treating) an engineered guide RNA or an engineered CRISPR/Cas12f1 (Cas14a1) system into a subject and/or a target cell. The engineered guide RNA is as described above in the section "<Engineered guide RNA>" and has the same characteristics and structure as each configuration described in the section. In addition, the engineered CRISPR/Cas12f1 (Cas14a1) system is as described above in the section "<Engineered CRISPR/Cas12f1 (Cas14a1) system>" and has the same characteristics and structure as each configuration described in the section.

Hereinafter, a detailed description will be given using embodiments of the method.

### 2-1) Method example (1)

In an embodiment, the method may be a method of modifying a target nucleic acid or target gene, comprising treating a eukaryotic cell with a composition.

Here, the composition may comprise:
an engineered guide RNA or a nucleic acid encoding the same; and
a Cas12f1 protein or a nucleic acid encoding the same.

The composition is as described above in the section "1. Composition" and has the same characteristics and structure as each configuration described in the section. In addition, the engineered guide RNA is as described above in the section "<Engineered guide RNA>" and has the same characteristics and structure as each configuration described in the section. In addition, the Cas12f1 protein is as described above in the section "2. Cas12f1 (Cas14a1) protein" in <Engineered CRISPR/Cas14a1 system> and has the same characteristics and structure as each configuration described in the section.

The composition may be a viral vector or a non-viral vector, or a mixed form thereof. Alternatively, the composition may be in a form in which a nucleic acid and a protein are mixed. The viral vector or non-viral vector, or a mixed form thereof is as described above in the section "1-1) Form of nucleic acid" and has the same characteristics and structure as each configuration described in the section. In addition, the mixed form of a nucleic acid and a protein is as described in the section "1-2) Mixed form of nucleic acid and protein" and has the same characteristics and structure as each configuration described in the section.

In an embodiment, the composition may be in a form of a viral vector comprising a nucleic acid encoding an engineered guide RNA and a nucleic acid encoding a Cas12f1 protein. Here, the viral vector may be at least one selected from: a retroviral vector, a lentiviral vector, an adenoviral vector, an adeno-associated viral vector, a vaccinia virus vector, a poxvirus vector, and a herpes simplex virus vector. Here, the composition may comprise one viral vector comprising both a nucleic acid encoding an engineered guide RNA and a nucleic acid encoding a Cas12f1 protein. Alternatively, the composition may comprise two viral vectors which respectively comprise a nucleic acid encoding an engineered guide RNA and a nucleic acid encoding a Cas12f1 protein.

In another embodiment, the composition may be in a form of a non-viral vector comprising a nucleic acid encoding an engineered guide RNA and a nucleic acid encoding a Cas12f1 protein. Here, the non-viral vector may be a plasmid. Here, the composition may comprise a single plasmid comprising both a nucleic acid encoding an engineered guide RNA and a nucleic acid encoding a Cas12f1 protein. Alternatively, the composition may comprise two plasmids which respectively comprise a nucleic acid encoding an engineered guide RNA and a nucleic acid encoding a Cas12f1 protein.

In another embodiment, the composition may comprise a PCR amplicon comprising a nucleic acid encoding an engineered guide RNA and an mRNA encoding a Cas12f1 protein.

In another embodiment, the composition may comprise a PCR amplicon comprising a nucleic acid encoding an engineered guide RNA, and a Cas12f1 protein. Alternatively, the composition may comprise an engineered CRISPR/Cas12f1 (Cas14a1) complex in which an engineered guide RNA and a Cas12f1 protein are combined.

The eukaryotic cell may contain a target nucleic acid or target gene. The eukaryotic cell may be yeast, a plant cell, an animal cell, and/or a human cell, but is not limited thereto.

Treating the eukaryotic cell with the composition may be performed to introduce an engineered CRISPR/Cas12f1 (Cas14a1) system into the eukaryotic cell. Alternatively, treating the eukaryotic cell with the composition may be performed to bring an engineered CRISPR/Cas12f1 (Cas14a1) system in contact with a target nucleic acid or target gene present in the eukaryotic cell.

Treating the eukaryotic cell with the composition may be performed using electroporation, gene gun, sonoporation, magnetofection, nanoparticles, and/or transient cell compression or squeezing. Alternatively, treating the eukaryotic cell with the composition may be performed using cationic liposome, lithium acetate-dimethyl sulfoxide (DMSO), lipid-mediated transfection, calcium phosphate precipitation, lipofection, polyethyleneimine (PEI)-mediated transfection, diethylaminoethyl (DEAE)-dextran-mediated transfection, and/or nanoparticle-mediated nucleic acid delivery (Panyam et al., Adv Drug Deliv Rev.2012 Sep 13.doi: 10.1016/j.addr.2012.09.023), but the treatment method is not limited thereto.

The method may optionally further comprise culturing the eukaryotic cell. Here, the culturing may be culturing the eukaryotic cell after being treated with the composition.

### 2-2. Method example (2)

In another embodiment, the method may be a method of modifying a target nucleic acid or target gene, comprising treating a eukaryotic cell with an engineered CRISPR/Cas12f1 (Cas14a1) system.

Here, the engineered CRISPR/Cas12f1 (Cas14a1) system may comprise:
an engineered guide RNA or a nucleic acid encoding the same; and
a Cas12f1 protein or a nucleic acid encoding the same.

The engineered CRISPR/Cas12f1 (Cas14a1) system is as described above in the section "<Engineered CRISPR/Cas12f1 (Cas14a1) system>" and has the same characteristics and structure as each configuration described in the section.

The engineered CRISPR/Cas12f1 (Cas14a1) system may be a viral vector, a non-viral vector, or a mixed form thereof. Alternatively, the engineered CRISPR/Cas12f1 (Cas14a1) system may be in a form in which a nucleic acid and a protein are mixed. The viral vector or non-viral vector, or a mixed form thereof is as described above in the section "1-1) Form of nucleic acid" and has the same characteristics and structure as each configuration described in the section. In addition, the form in which a nucleic acid and a protein are mixed is as described above in the section "1-2) Mixed form of nucleic acid and protein" and has the same characteristics and structure as each configuration described in the section.

In an embodiment, the engineered CRISPR/Cas12f1 (Cas14a1) system may comprise a PCR amplicon comprising a nucleic acid encoding an engineered guide RNA and an mRNA encoding a Cas12f1 protein.

In another embodiment, the engineered CRISPR/Cas12f1 (Cas14a1) system may comprise a PCR amplicon comprising a nucleic acid encoding an engineered guide RNA and a Cas12f1 protein.

In another embodiment, the engineered CRISPR/Cas12f1 (Cas14a1) system may comprise an engineered CRISPR/Cas12f1 (Cas14a1) complex in which an engineered guide RNA and a Cas12f1 protein are combined.

The eukaryotic cell may comprise a target nucleic acid or target gene. The eukaryotic cell may be yeast, a plant cell, an animal cell, and/or a human cell, but is not limited thereto.

Treating the eukaryotic cell with the engineered CRISPR/Cas12f1 (Cas14a1) system may be performed to form an engineered CRISPR/Cas12f1 (Cas14a1) complex in the eukaryotic cell. Alternatively, treating the eukaryotic cell with the engineered CRISPR/Cas12f1 (Cas14a1) system may be performed to bring the engineered CRISPR/Cas12f1 (Cas14a1) complex in contact with a target nucleic acid or target gene present in the eukaryotic cell.

Treating the eukaryotic cell with the engineered CRISPR/Cas12f1 (Cas14a1) system may be performed using electroporation, gene gun, sonoporation, magnetofection, nanoparticles, and/or transient cell compression or squeezing. Alternatively, treating the eukaryotic cell with the composition may be performed using cationic liposome, lithium acetate-dimethyl sulfoxide (DMSO), lipid-mediated transfection, calcium phosphate precipitation, lipofection, polyethyleneimine (PEI)-mediated transfection, diethylaminoethyl (DEAE)-dextran-mediated transfection, and/or nanoparticle-mediated nucleic acid delivery (Panyam et al., Adv Drug Deliv Rev.2012 Sep 13.doi: 10.1016/j.addr.2012.09.023), but the treatment method is not limited thereto.

Treating the eukaryotic cell with the engineered CRISPR/Cas12f1 (Cas14a1) system may be performed by simultaneous treatment with an engineered guide RNA or a nucleic acid encoding the same, and a Cas12f1 protein or a nucleic acid encoding the same. Alternatively, treating the eukaryotic cell with the engineered CRISPR/Cas12f1 (Cas14a1) system may be performed by sequential treatment with an engineered guide RNA or a nucleic acid encoding the same, and a Cas12f1 protein or a nucleic acid encoding the same.

The method may optionally further comprise culturing the eukaryotic cell. Here, the culturing may be culturing the eukaryotic cell after being treated with the composition.

### 2-3. Method example (3)

In another embodiment, the method may be a method of modifying a target nucleic acid or target gene, comprising treating a eukaryotic cell with an engineered guide RNA or a nucleic acid encoding the same.

Here, the eukaryotic cell may be a cell comprising a Cas12f1 protein or a nucleic acid encoding the same. In addition, the eukaryotic cell may comprise a target nucleic acid or target gene. The eukaryotic cell may be yeast, a plant cell, an animal cell, and/or a human cell, but is not limited thereto.

The engineered guide RNA is as described above in the section "Engineered guide RNA" and has the same characteristics and structure as each configuration described in the section.

The nucleic acid encoding an engineered guide RNA may be in a form of a viral vector or a non-viral vector. The form of a viral vector or a non-viral vector is as described in the section "1-1) Form of nucleic acid" and has the same characteristics and structure as each configuration described in the section.

In an embodiment, the nucleic acid encoding an engineered guide RNA may be in a form of a PCR amplicon comprising a nucleic acid encoding an engineered guide RNA.

In another embodiment, the nucleic acid encoding an engineered guide RNA may be in a form of an adeno-associated viral vector comprising a nucleic acid encoding an engineered guide RNA.

Treating the eukaryotic cell with the engineered guide RNA or a nucleic acid encoding the same may be performed to form an engineered CRISPR/Cas12f1 complex in the eukaryotic cell. Alternatively, treating the eukaryotic cell with the engineered guide RNA or a nucleic acid encoding the same may be performed to bring the engineered CRISPR/Cas12f1 complex in contact with a target nucleic acid or target gene present in the eukaryotic cell.

Treating the eukaryotic cell with the engineered CRISPR/Cas12f1 (Cas14a1) system may be performed using electroporation, gene gun, sonoporation, magnetofection, nanoparticles and/or transient cell compression or squeezing. Alternatively, treating the eukaryotic cell with the engineered CRISPR/Cas12f1 (Cas14a1) system may be performed using cationic liposome, lithium acetate-DMSO, lipid-mediated transfection, calcium phosphate precipitation, lipofection, (PEI)-mediated transfection, (DEAE)-dextran-mediated transfection, and/or nanoparticle-mediated nucleic acid delivery (Panyam et al., Adv Drug Deliv Rev.2012 Sep 13.doi: 10.1016/j.addr.2012.09.023), but the treatment is not limited thereto.

The method may optionally further comprise culturing eukaryotic cells. Here, the culturing may be culturing the eukaryotic cell after being treated with the composition.

### 2-4. Method Example (4)

In an embodiment, the method may be a method of modifying a target nucleic acid or target gene, comprising introducing (injecting) the composition into a subject.

Here, the composition may comprise:
an engineered guide RNA or a nucleic acid encoding the same; and
a Cas12f1 protein or a nucleic acid encoding the same.

The composition is as described above in the section "1. Composition" and has the same characteristics and structure as each configuration described in the section. In addition, the engineered guide RNA is as described above in the section "<Engineered guide RNA>" and has the same characteristics and structure as each configuration described in the section. In addition, the Cas12f1 protein is as described above in the section "2. Cas12f1 (Cas14a1) protein" in <Engineered CRISPR/Cas14a1 system> and has the same characteristics and structure as each configuration described in the section.

The composition may be a viral vector or a non-viral vector, or a mixed form thereof. Alternatively, the composition may be in a form in which a nucleic acid and a protein are mixed. The viral vector or the non-viral vector, or a mixed form thereof is as described above in the section "1-1) Form of nucleic acid" and has the same characteristics and structure as each configuration described in the section. In addition, the form in which a nucleic acid and a protein are mixed is as described above in the section "1-2) Mixed form of nucleic acid and protein" and has the same characteristics and structure as each configuration described in the section.

In an embodiment, the composition may be in a form of a viral vector comprising a nucleic acid encoding an engineered guide RNA and a nucleic acid encoding a Cas12f1 protein. Here, the viral vector may be at least one selected from the group consisting of: a retroviral vector, a lentiviral vector, an adenoviral vector, an adeno-associated viral vector, a vaccinia virus vector, a poxvirus vector, and a herpes simplex virus vector. Here, the composition may comprise one viral vector comprising both a nucleic acid encoding an engineered guide RNA and a nucleic acid encoding a Cas12f1 protein. Alternatively, the composition may comprise two viral vectors which respectively comprise a nucleic acid encoding an engineered guide RNA and a nucleic acid encoding a Cas12f1 protein.

In another embodiment, the composition may be in a form of a non-viral vector comprising a nucleic acid encoding an engineered guide RNA and a nucleic acid encoding a Cas12f1 protein. Here, the non-viral vector may be a plasmid. Here, the composition may comprise a single plasmid comprising both a nucleic acid encoding an engineered guide RNA and a nucleic acid encoding a Cas12f1 protein. Alternatively, the composition may comprise two plasmids which respectively comprise a nucleic acid encoding an engineered guide RNA and a nucleic acid encoding a Cas12f1 protein.

In another embodiment, the composition may comprise a PCR amplicon comprising a nucleic acid encoding an engineered guide RNA, and an mRNA encoding a Cas12f1 protein.

In another embodiment, the composition may comprise a PCR amplicon comprising a nucleic acid encoding an engineered guide RNA, and a Cas12f1 protein. Alternatively, the composition may comprise an engineered CRISPR/Cas12f1 (Cas14a1) complex in which an engineered guide RNA and a Cas12f1 protein are combined.

The subject may have a genome comprising a target nucleic acid or target gene. The subject may be a plant, a non-human animal, a human, or some tissue thereof, but is not limited thereto.

Introducing (injecting) the composition into the subject may be performed to bring an engineered CRISPR/Cas12f1 (Cas14a1) system in contact with a target nucleic acid or target gene present in the subject.

Introducing (injecting) the composition into the subject may be performed using microinjection, electroporation, gene gun, sonoporation, magnetofection, nanoparticles, and/or transient cell compression or squeezing. Alternatively, introducing (injecting) the composition into the subject may be performed using cationic liposome, lithium acetate-dimethyl sulfoxide (DMSO), lipid-mediated transfection, calcium phosphate precipitation, lipofection, polyethyleneimine (PEI)-mediated transfection, diethylaminoethyl (DEAE)-dextran-mediated transfection, and/or nanoparticle-mediated nucleic acid delivery (Panyam et al., Adv Drug Deliv Rev.2012 Sep 13.doi: 10.1016/j.addr.2012.09.023), but the introducing (injecting) method is not limited thereto.

### 3. Modification of target

A target gene or target nucleic acid may be modified using the composition or the method disclosed herein. More specifically, the target gene or target nucleic acid may be modified by an engineered CRISPR/Cas12f1 (Cas14a1) system. Here, the modification may include both i) cleavage or damage of the target gene or target nucleic acid by the engineered CRISPR/Cas12f1 (Cas14a1) system, and ii) repair or recovery of the cleaved (or damaged) target gene or target nucleic acid. Here, the modification may be deletion or substitution of the target gene or target nucleic acid. Alternatively, the modification may be insertion of an additional nucleic acid sequence into the target gene or target nucleic acid. Alternatively, the modification may be insertion and deletion (indel) in which a part of the target gene or target nucleic acid is deleted and an additional sequence is inserted thereinto. Hereinafter, the modification will be described in detail.

### 3-1) Deletion

As a result of performing the method provided herein, all or a part of a target gene or target nucleic acid may be deleted. The deletion means removing a partial nucleotide sequence in the target gene or target nucleic acid. In an embodiment, the method comprises introducing a Cas12f1 protein or a nucleic acid encoding the same, a first engineered guide RNA or a nucleic acid encoding the same, and a second engineered guide RNA or a nucleic acid encoding the same into a cell comprising the target gene or target nucleic acid. This results in deletion of a specific sequence within the target gene or target nucleic acid.

### 3-2) Insertion

As a result of performing the method provided herein, a knock-in may occur in a target gene or target nucleic acid. The knock-in means inserting an additional nucleic acid sequence into a target gene or target nucleic acid. To cause the knock-in, a donor comprising the additional nucleic acid sequence is further required in addition to an engineered CRISPR/Cas12f1 (Cas14a1) system. When the engineered CRISPR/Cas12f1 (Cas14a1) system cleaves a target gene or target nucleic acid in a cell, repair of the cleaved target gene or target nucleic acid occurs. Here, the repair may be performed using homology directed repairing (HDR), wherein the donor participates in the repair process so that the additional nucleic acid sequence can be inserted into the target gene or target nucleic acid. In an embodiment, the method may further comprise delivering a donor into a target cell. Here, the donor comprises an additional nucleic acid of interest, and induces insertion of the additional nucleic acid into the target gene or target nucleic acid. Here, when the donor is delivered into the target cell, one of the above-described treatment methods for the engineered CRISPR/Cas12f1 (Cas14a1) system may be used.

### 3-3) Insertion and deletion (indel)

As a result of performing the method provided herein, insertion and deletion (indel) may occur in a target gene or target nucleic acid. The indel may occur by non-homologous end joining (NHEJ). In general, NHEJ is a method of repairing or recovering a break (for example, cleavage) in a double-strand in DNA in which two compatible ends formed by the break repeatedly contact each other. Repair of a damaged gene or nucleic acid using NHEJ results in partial insertion and/or deletion (indel) of a nucleic acid sequence at the NHEJ repair site. This insertion and/or deletion alters its reading frame and produces a frameshifted transcript mRNA, which, in turn, undergoes nonsense-mediated decay or fails to synthesize a normal protein, thereby losing its original function. Accordingly, when an indel occurs in a target gene or target nucleic acid, the gene or nucleic acid may be inactivated. In an embodiment, as a result of performing the method, deletion and/or addition of one or more bases may occur in the target gene or target nucleic acid.

### 3-4) Substitution or base editing

As a result of performing the method provided herein, base editing may occur in a target gene or target nucleic acid. Base editing means intentionally altering one or more specific nucleotides in a nucleic acid, unlike the indel caused by deletion or addition of any nucleotide in a target gene or target nucleic acid. In other words, the base editing causes an intended point mutation at a specific location in the target gene or target nucleic acid. In an embodiment, as a result of performing the method, substitution of one or more nucleotides by other nucleotides may occur in the target gene or target nucleic acid.

### 3-5) Phenotype by modification of target

Modification of a target nucleic acid or target gene may result in effects of knock-out, knock-down, or knock-in. The "knock-out" means inactivation of a target gene or target nucleic acid, and the "inactivation of a target gene or target nucleic acid" means a state in which transcription and/or translation of the target gene or target nucleic acid does not occur. The knock-out may suppress transcription or expression of a gene, which causes a disease or has an abnormal function, thereby preventing expression of a protein. The "knock-down" means reducing transcription and/or translation of a target gene or target nucleic acid, or reducing expression of a target protein. The knock-down may control expression of a gene or protein to be overexpressed, thereby preventing occurrence of or treating a disease. The "knock-in" means inserting a specific nucleic acid or gene into a target gene or target nucleic acid. Here, the "specific nucleic acid or gene" means a gene or nucleic acid to be inserted or expressed. The knock-in may be used to treat a disease by correcting a mutant gene, which causes a disease, or inserting a normal gene so that expression of the normal gene is induced.

Hereinafter, the present disclosure will be described in detail by way of examples.

These examples are for specifically describing the present disclosure, and it will be clearly understood by those skilled in the art that the scope of the present disclosure is not limited by the examples.

### Examples

### Experimental method

### 1. Plasmid vector construction

By applying a human codon-optimized sequence (SEQ ID NO: 487) of Cas12f1 (Cas14a1), an oligonucleotide (Bionics), which comprises a chicken b-actin promoter, a nuclear-localization signal sequences at both the 5' end and the 3' end, and a T2A-linked eGFP, was synthesized. A template DNA for a guide RNA was synthesized and cloned into a pTwist Amp plasmid vector (Twist Bioscience). When necessary, the cloned vector was used as a template for gRNA-encoding PCR amplicon by using a U6-complementary forward primer and a protospacer-complementary reverse primer. For construction of a dual guide RNA, oligonucleotides encoding a tracrRNA and a crRNA were cloned into a pSilencer 2.0 (ThermoFisher Scientific) using the restriction enzymes BamHI and HindIII (New England Biolabs). The engineered CRISPR/Cas14as1 system_3.0 (geCas14a_3.0) vector consisted of the human codon-optimized oligonucleotide of Cas12f1 (Cas14a1) and the engineered gRNA sequence for Cas14a1. In case of SpCas9, a human codon-optimized oligonucleotide of SpCas9 and a single guide RNA (sgRNA) for SpCas9 were subjected to Gibson assembly using pSpCas9(BB)-2A-EGFP (PX458) V2.0 (Addgene) as a backbone plasmid.

### 2. Guide RNA engineering

Internal modification of gRNA (i. modification of a nucleotide sequence constituting stem 5, the modification i being hereinafter referred to as modification 1 (M1) wherein the modification of a nucleotide sequence of a tracrRNA and/or a crRNA constituting stem 5 is referred to as M1-1, and the modification by deletion in a nucleotide sequence constituting stem 5 is referred to as M1-2; and ii. Modification of a nucleotide sequence constituting stem 2, the modification ii being hereinafter referred to as modification 2 (M2)) was performed by cloning a synthetic oligonucleotide (Macrogen), which carries a modified sequence, into a gRNA encoding vector linearized using the restriction enzymes Apol and BamHI. 5' end modification of a tracrRNA (modification of a nucleotide sequence constituting stem 1, the modification being hereinafter referred to as modification 3 (M3)) was performed by PCR amplification of a canonical or engineered template plasmid vector using a reverse primer targeting a U6 promoter region and a forward primer targeting the 5' end region of the tracrRNA. The PCR amplification was performed using Q5 Hot Start high-fidelity DNA polymerase (NEB), and the PCR products were ligated using KLD Enzyme Mix (NEB). The ligated product was used to transform DH5α E. coli cells. Mutagenesis was confirmed by Sanger sequencing analysis. The modified plasmid vector was purified using NucleoBond ^{®} Xtra Midi EF kit (MN). 1 µg of the purified plasmid was used as a template for mRNA synthesis in which T7 RNA polymerase (NEB) and NTP (Jena Bioscience) are used. The guide RNA was purified using Monarch^{®} RNA cleanup kit (NEB) and aliquoted into cryogenic vials prior to being stored in liquid nitrogen.

3' end modification of a crRNA (modification by addition of U-rich tail, the modification being hereinafter referred to as modification 4 (M4)) was performed using Pfu PCR Master (Biofact) in the presence of a primer whose nucleotide sequence is modified and a canonical gRNA plasmid vector. The PCR amplicon was purified using HiGene^{™} Gel&PCR Purification system (Biofact).

### 3. Human cell culture and transfection

HEK-293T cells (ATCC CRL-11268) were cultured in Dulbecco's modified eagle medium (DMEM) supplemented with 10% heat-inactivated FBS (Corning) and 1% penicillin/streptomycin at 37°C in a 5% CO₂ incubator. Cell transfection was performed by electroporation or lipofection. For the electroporation, 4 X 10⁵ HEK-293T cells were transfected with 2 µg to 5 µg of a plasmid vector comprising a nucleic acid encoding Cas14a1, AsCpf1 or SpCas9 and 2 µg to 5 µg of DNA encoding gRNA using a Neon transfection system (Invitrogen). The electroporation conditions were as follows: 1,300V, 10mA, 3 pulses. For the lipofection, 6 µL to 15 µL of FuGene reagent (Promega) was mixed with 2 µg to 5 µg of a plasmid vector comprising a nucleic acid encoding Cas14a1 and 1.5 µg to 5 µg of a PCR amplicon for 15 minutes. The mixture (300 µL) was added to a 1.5 ml DMEM medium in which 1 X 10 ⁶ cells had been plated 1 day prior to transfection, and the cells were cultured in the presence of the mixture for 72 to 96 hours. After the culture, the cells were harvested, and genomic DNA was prepared using PureHelix^{™} genomic DNA preparation kit (NanoHelix) or Maxwell^{™} RSC nucleic acid isolation workstation (Promega). Target information for transfection is summarized in Table 3 below.

**[Table 3]**

| **Target Name** | **Gene Name** | **Protospacer sequence with PAM sequence (SEQ ID NO)** | **Chromoso me** | **Locatio n^{a}** |
|---|---|---|---|---|
| Target _1 | Intergene | TTTGCACACACACACAGTGGG CTACC (SEQ ID NO: 488) | chr3 | 1202283 53 |
| Target _2 | KRT1 | TTTGCATCCCCAGGACACA CACAC (SEQ ID NO: 489) | chr12 | 5267907 6 |
| Target _3 | Intergene | TTTAAGAACACATACCCCT GGGCC (SEQ ID NO: 490) | chr5 | 1715400 57 |
| Target _a | PGBD2 | TTTGACTCAGCAATCCTATT ACTG (SEQ ID NO: 491) | chr1 | 2489024 70 |
| | | TTTATATTCCTGTGGGTATA TGCC (SEQ ID NO: 492) | | |
| Target _b | LINC02045 | TTTAAGAGGTGATTAGGTC ATGGC (SEQ ID NO: 493) | chr3 | 1482706 85 |
| | | TTTAATCCATTCATGAGGG TGGTG (SEQ ID NO: 494) | | |
| Target _c | Intergene | TTTAGCTCAAATCTGTACTA CTAA (SEQ ID NO: 495) | chr3 | 1758977 10 |
| | | TTTATTATTATAGTGTGTAC TTGA (SEQ ID NO: 496) | | |
| Target _d | LOC105379 078 | TTTAGTCCTCATAGCTTAC CTTC (SEQ ID NO: 497) | chr5 | 9168963 3 |
| | | TTTAATGTATGTTCTCACTC ATA (SEQ ID NO: 498) | | |
| Target _e | LOC100133 077 | TTTGGGTCTGTGGCTGTTG GGCTG (SEQ ID NO: 499) | chr9 | 1378911 28 |
| | | TTTAGAGCCCATCTCAGAT CCCTG (SEQ ID NO: 500) | | |
| Target _f | CNTN5 | TTTACCAGTGAAGATATAA CATTA (SEQ ID NO: 501) | chr11 | 1002074 71 |
| | | TTTAGTGAGGCTTCTTAAG ATGTC (SEQ ID NO: 502) | | |
| Target _g | HEPHL1 | TTTGCTGCTGGTAACAAGG TCATA (SEQ ID NO: 503) | chr11 | 9403329 4 |
| | | TTTAGATCTGAGACCCAGC TGCTC (SEQ ID NO: 504) | | |
| Target _h | CFAP97D2 | TTTGTCCTTGACCACGGCA TCAGC (SEQ ID NO: 505) | chr13 | 1141622 74 |
| | | TTTAAGTGGGAATAATATA GTTCC (SEQ ID NO: 506) | | |
| Target _i | Intergene | TTTGAAGACATCTTGCTGT CAGAC (SEQ ID NO: 507) | chr13 | 1058924 82 |
| | | TTTGTGTATAATTATTCAGA GTAG (SEQ ID NO: 508) | | |
| Target _i | TAOK1 | TTTGGCATCAAGTTAACAT CACAC (SEQ ID NO: 509) | chr17 | 2954438 1 |
| | | TTTAGCACTGAGGCTTGAG ACTTG (SEQ ID NO: 510) | | |

| | | | | |
|---|---|---|---|---|
| ^{a}Listed information is based on the Genome Reference Consortium Human Build 38 patch release 11 (GRCh38.p11). | | | | |

### 4. Measurement of indel efficiency

Genomic DNA was isolated from HEK-293T cells using a PureHelix^{™} genomic DNA preparation kit (NanoHelix). Target-specific primers were synthesized and used to amplify a protospacer-containing region in the presence of KAPA HiFi HotStart DNA polymerase (Roche) according to the manufacturer's instructions. The generated PCR amplicons were labeled with Illumina TruSeq HT dual index. The final PCR product was subjected to 150-bp paired-end sequencing using Illumina iSeq 100. Indel frequency was calculated by MAUND (https://aithub.com/ibs-cae/maund).

### 5. Recombinant Cas14a1

The Cas12f1 gene was cloned into a modified pMAL-c2x plasmid vector (Addgene), and the vector construct was used to transform BL21 (DE3) E. coli cells. E. coli transformant colonies were grown at 37°C in Luria-Bertani (LB) broth until an optical density of 0.7 was reached. The cells were incubated overnight at 30°C in the presence of 0.1 mM of isopropylthio-β-D-galactoside, and then collected by centrifugation at 3,500 g for 30 minutes. The collected cells were resuspended in 20 mM of Tris-HCl (pH 7.6), 500 mM of NaCl, 5 mM of β-mercaptoethanol, and 5% glycerol. The cell lysate was prepared by being subjected to sonication, centrifugated at 15,000 g for 30 minutes, and then filtrated through a 0.45 µm syringe filter (Millipore). The cleared lysate was loaded onto a Ni²⁺- affinity column using a FPLC purification system (ÄKTA Purifier, GE Healthcare). The fraction bound to the Ni²⁺- affinity column was eluted with 20 mM of Tris-HCl (pH 7.5) at 80 mM to 400 mM imidazole gradients. The eluted protein was treated with 1 mg of TEV protease for 6 hours. The cleaved protein was purified in a heparin column with a linear gradient of 0.15 M to 1.6 M NaCl. The recombinant Cas14a1 protein was dialyzed against 20 mM of Tris pH 7.6, 150 mM of NaCl, 5 mM of β-mercaptoethanol, and 5% glycerol. The dialyzed protein was purified again in a monoS column (GE Healthcare) with a linear gradient of 0.5 M to 1.2 M of NaCl. Selected fractions were pooled and dialyzed against 20 mM of Tris pH 7.6, 150 mM of NaCl, 5 mM of β-mercaptoethanol, and 5% glycerol. A concentration of the obtained protein was measured electropherometrically in a coomassie blue-stained SDS-PAGE gel using bovine serum albumin as a standard.

### 6. Off-target analysis using Digenome-seq method

Genome-wide off-target analysis was performed using Digenome-seq method. Briefly, genomic DNA was isolated from HEK-293T cells, and the isolated genomic DNA was treated with a ribonucleoprotein complex formed by pre-incubating 10 µg of recombinant Cas12f1 or AsCas12a protein and 900 nM of engineered guide RNA at room temperature for 2 hours. Digestion of the genomic DNA was performed in a reaction buffer containing 100 mM of NaCl, 10 mM of MgCl₂, 100 µg/ml of BSA, and 50 mM of Tris-HCl (pH 7.9) at 37°C for 8 hours. The digested genomic DNA was treated with RNase A (50 µg/ml) and then purified using a DNeasy Tissue kit (Qiagen). The purified genomic DNA was subjected to whole genome sequencing (WGS) at a sequencing depth of 30x to 40x using Illumina HiSeq X Ten Sequencer. A DNA cleavage score was assigned to each nucleotide position across the entire genome using WGS data, according to the equation. A cut-off value of 2.5 was assigned to screen potential off-targets using the Digenome-seq program (https://github.com/chizksh/digenome-toolkit2) with an additional criterion of six or fewer mismatches with on-target sequence. The screened potential off-targets were validated by targeted deep-sequencing analysis after treatment of HEK293-T cells with Cas14a1 and a guide RNA.

### 7. Droplet digital PCR

A guide RNA or genomic DNA was extracted from HEK-293T cells using a RNeasy Miniprep kit (Qiagen) or a PureHelix^{™} genomic DNA preparation kit (NanoHelix), respectively. For quantification of the guide RNA, a crRNA-specific primer was used for cDNA synthesis, and the synthesized cDNA was used as a template for quantitative real-time PCR. Deletion of exons was analyzed using the QuantiTect SYBR Green RT-PCR kit (Qiagen) in iCycler (Bio-Rad).

### 8. Quantitative analysis of gRNA expression

Canonical or engineered gRNA-encoding PCR amplicons (3 µg) were transfected into HEK293-T cells by using a Neon transfection system (Invitrogen), and the cells were harvested 2 days after the transfection. Total RNA was obtained from the harvested cells using the Maxwell^{®} RSC miRNA Tissue Kit (Promega, AS1460). The obtained RNA was polyadenylated by incubating 5 µg of the RNA preparation with E. coli Poly(A) Polymerase (NEB, M0276) at 37°C for 30 minutes. RNA was purified with a Monarch^{®} RNA Cleanup Kit (NEB, T2050). 500 µg of poly(A)-tailed RNA was reverse transcribed using SuperScript IV Reverse Transcriptase (Invitrogen) in the presence of an RT-specific primer that carries the T6 sequence at its 3' end and an adapter sequence. The guide RNA was PCR amplified using adapter- and gRNA-specific primers. The PCR products were resolved on 2% agarose gels.

### 9. Production of AAV vectors and transduction

The human codon-optimized Cas12f1 gene and sgRNA sequences were cloned into AAV vector plasmids with inverted terminal repeats. The Cas12f1 gene was accompanied by a nuclear localization signal and linked to the enhanced green fluorescent protein (eGFP) gene via a self-cleaving T2A sequence. Transcription of Cas12f1 and sgRNA was induced by a chicken β-actin promoter and a U6 promoter, respectively. In order to produce rAAV2 vectors, HEK-293T cells were transfected with pAAV-ITR-sgRNA-Cas12f1 or pAAV-ITR-sgRNA-SaCas9; pAAVED2/9; and helper plasmids. The transfected HEK-293T cells were cultured in DMEM containing 2% FBS. A recombinant pseudotyped AAV vector stock was generated using polyethylenimine coprecipitated with PEIpro (Polyplus-transfection) and triple-transfection with plasmids at an equal molar ratio in HEK-293T cells. After 72 hours of incubation, the cells were lysed and the particles were purified by iodixanol (Sigma-Aldrich) step-gradient ultracentrifugation. The number of vector genomes was determined by quantitative PCR. HEK-293T cells were infected with rAAV2-Cas12f1-sgRNA or rAAV2-SaCas9-sgRNA at different multiplicities of infection (MOI) of 1, 5, 10, 50 and 100 as determined by quantitative PCR. The transduced cells were maintained in DMEM with 2% FBS for up to 2 weeks. Cells were collected for isolation of genomic DNA at different time points.

### 10. Statistical analysis

Statistical significance tests were performed using Sigma Plot software (ver. 14.0) through a two-tailed Student's t-test. Cases in which a p-value was less than 0.05 were considered statistically significant, and the p-values are shown in each diagram. Data points in box and dot plots represent the full range of values, and each box spans the interquartile range (25th to 75th percentiles). Median and average values are indicated by black and red horizontal lines, respectively. Error bars of all of the dot and bar plot data were plotted using Sigmaplot (v. 14.0), and show the standard deviation value of each data. Sample sizes were not predetermined based on statistical methods. A large-scale validation was performed blinded to information with respect to the Cas type.

### Example 1. Guide RNA engineering

Previous studies (Harrington et al., Science 362, 839-842 (2018), Tautvydas Karvelis et al., Nucleic Acids Research 48, 5016-5023 (2020)) reported that the CRISPR/Cas14a1 system is believed to cleave single-stranded DNA (ssDNA), and postulated that the CRISPR/Cas14a1 system itself may not be suitable for genome editing in eukaryotic cells. Recognizing this problem, in order to apply the CRISPR/Cas12f1 system to eukaryotic cells, the present inventors conducted a study to optimize a guide RNA (gRNA) for the CRISPR/Cas14a1 system.

For gRNA engineering, the following four modification sites (MS) were designated throughout the tracrRNA and crRNA constituting the gRNA of the CRISPR/Cas14a1 system (FIG. 2):
1) MS1 - a region of the tracrRNA constituting stem 5 which comprises a penta-uridinylate (UUUUU) sequence (corresponding to region 1 to be modified in the wildtype tracrRNA) and a region of the crRNA corresponding thereto;
2) MS2 - an internal region of the tracrRNA constituting stem 2 (corresponding to region 4 to be modified in the wildtype tracrRNA);
3) MS3 - 5' end region of the tracrRNA constituting stem 1 (corresponding to region 5 to be modified in the wildtype tracrRNA); and
4) MS4 - 3' end region of the crRNA.
gRNA engineering was performed in MS1, MS2, MS3 and MS4 individually or in various combinations to derive a highly efficient CRISPR-Cas14a1 system. For formation of a stable tracrRNA-crRNA complex, some engineered guide RNAs were used as a single guide RNA (sgRNA) in which the tracrRNA and the crRNA are linked by the linker GAAA.

### Example 2. Engineered guide RNA (MS1)

A canonical gRNA sequence for Cas14a1 reported in a previous study (Harrington et al., Science 362, 839-842 (2018)) was closely investigated. As a result, it was confirmed that the wildtype tracrRNA (SEQ ID NO: 1) comprises an inappropriate sequence that is difficult to be transcribed normally in eukaryotic cells. The wildtype tracrRNA comprises an internal UUUUU(U5) sequence at the 138th to 142nd positions of the trancrRNA in a 5' to 3' direction (FIG. 1). Therefore, in order to transcribe the tracrRNA, the nucleic acid encoding the tracrRNA comprises a sequence of five consecutive thymidines (T5). Here, the sequence of five consecutive thymidines (T5) may act as a termination sequence under a U6 promoter, which prevents the tracrRNA from being transcribed normally, and thus there is a high probability that an incomplete tracrRNA is produced. Therefore, the present inventors considered a region (MS1) of the tracrRNA constituting stem 5, which comprises the penta-uridinylate (UUUUU) sequence, as a hotspot for gRNA engineering.

### Example 2-1. Engineered tracrRNA (M1-1): modification of region of tracrRNA constituting stem 5 which comprises penta-uridinylate (UUUUU) sequence

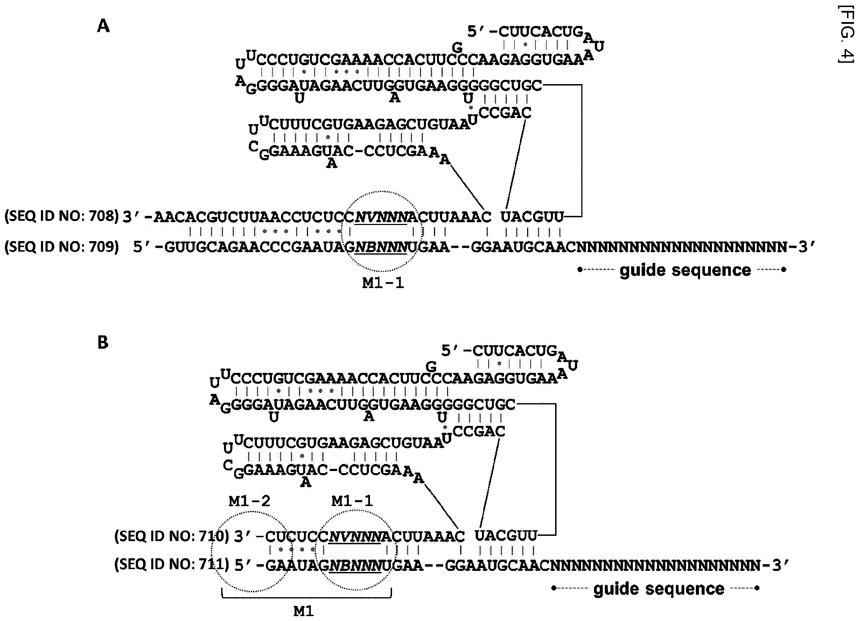

Hereinafter, when a region comprising UUUUU (U5) in a tracrRNA was modified, the modification was denoted as M1-1 or MS1 engineering. That is, when a tracrRNA is modified so as not to comprise a sequence of five or more consecutive uridines, the tracrRNA was denoted as an engineered tracrRNA comprising M1-1 modification or engineered tracrRNA (M1-1). In addition, based on the detailed description described above, the engineered tracrRNA (M1-1) generated by MS1 engineering may have the first sequence (which does not comprise a sequence of five consecutive uridines) described above in the section of "1-1) First sequence" in the description of 1. Engineered tracrRNA (FIG. 4A).

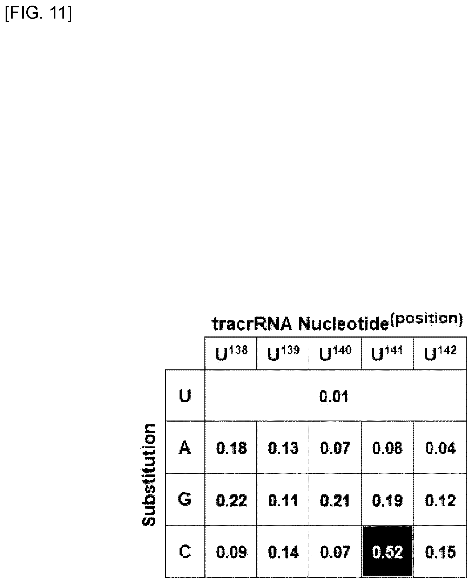

To remove a sequence of five or more consecutive uridines that may serve as a termination signal, an engineered tracrRNA was generated by substituting each uridine (U) with a nucleotide (A, C, or G) other than uridine (Table 4). The generated engineered tracrRNA was used to investigate indel efficiency for an endogenous target (Target 1) in HEK293T cells. As a result of deep sequencing analysis, it was confirmed that each substitution resulted in at least a 4-fold increase in indel efficiency; and in particular, substitution of the 141st uridine (U141) with cytidine (C) resulted in a significant increase of about 50-fold (FIG. 11). It is speculated that cytidine not only removed the termination signal, but also acted in a structurally suitable manner at its position.

**[Table 4]**

| Engineered tracrRNA with modification of MS1 that comprises sequence of five or more consecutive uridines in tracrRNA (MS1 in tracrRNA is underlined) | | |
|---|---|---|
| Label | Sequence (5' to 3') | SEQ ID NO |
| Wildtype tracrRNA (canonical tracrRNA) | CUUCACUGAUAAAGUGGAGAACCGCUUCACCAAA AGCUGUCCCUUAGGGGAUUAGAACUUGAGUGAA GGUGGGCUGCUUGCAUCAGCCUAAUGUCGAGAA GUGCUUUCUUCGGAAAGUAACCCUCGAAACAAAU | 1 |
| | UCAUUUUCCUCUCCAAUUCUGCACAA | |
| U138A | CUUCACUGAUAAAGUGGAGAACCGCUUCACCAAA AGCUGUCCCUUAGGGGAUUAGAACUUGAGUGAA GGUGGGCUGCUUGCAUCAGCCUAAUGUCGAGAA GUGCUUUCUUCGGAAAGUAACCCUCGAAACAAAU UCAAUUUUCCUCUCCAAUUCUGCACAA | 511 |
| U138G | CUUCACUGAUAAAGUGGAGAACCGCUUCACCAAA AGCUGUCCCUUAGGGGAUUAGAACUUGAGUGAA GGUGGGCUGCUUGCAUCAGCCUAAUGUCGAGAA GUGCUUUCUUCGGAAAGUAACCCUCGAAACAAAU UCAGUUUCCUCUCCAAUUCUGCACAA | 512 |
| U138C | CUUCACUGAUAAAGUGGAGAACCGCUUCACCAAA AGCUGUCCCUUAGGGGAUUAGAACUUGAGUGAA GGUGGGCUGCUUGCAUCAGCCUAAUGUCGAGAA GUGCUUUCUUCGGAAAGUAACCCUCGAAACAAAU UCACUUUUCCUCUCCAAUUCUGCACAA | 513 |
| U139A | CUUCACUGAUAAAGUGGAGAACCGCUUCACCAAA AGCUGUCCCUUAGGGGAUUAGAACUUGAGUGAA GGUGGGCUGCUUGCAUCAGCCUAAUGUCGAGAA GUGCUUUCUUCGGAAAGUAACCCUCGAAACAAAU UCAUAUUUCCUCUCCAAUUCUGCACAA | 514 |
| U139G | CUUCACUGAUAAAGUGGAGAACCGCUUCACCAAA AGCUGUCCCUUAGGGGAUUAGAACUUGAGUGAA | 515 |
| | GGUGGGCUGCUUGCAUCAGCCUAAUGUCGAGAA GUGCUUUCUUCGGAAAGUAACCCUCGAAACAAAU UCAUGUUUCCUCUCCAAUUCUGCACAA | |
| U139C | CUUCACUGAUAAAGUGGAGAACCGCUUCACCAAA AGCUGUCCCUUAGGGGAUUAGAACUUGAGUGAA GGUGGGCUGCUUGCAUCAGCCUAAUGUCGAGAA GUGCUUUCUUCGGAAAGUAACCCUCGAAACAAAU UCAUCUUUCCUCUCCAAUUCUGCACAA | 516 |
| U140A | CUUCACUGAUAAAGUGGAGAACCGCUUCACCAAA AGCUGUCCCUUAGGGGAUUAGAACUUGAGUGAA GGUGGGCUGCUUGCAUCAGCCUAAUGUCGAGAA GUGCUUUCUUCGGAAAGUAACCCUCGAAACAAAU UCAUUAUUCCUCUCCAAUUCUGCACAA | 517 |
| U140G | CUUCACUGAUAAAGUGGAGAACCGCUUCACCAAA AGCUGUCCCUUAGGGGAUUAGAACUUGAGUGAA GGUGGGCUGCUUGCAUCAGCCUAAUGUCGAGAA GUGCUUUCUUCGGAAAGUAACCCUCGAAACAAAU UCAUUGUUCCUCUCCAAUUCUGCACAA | 518 |
| U140C | CUUCACUGAUAAAGUGGAGAACCGCUUCACCAAA AGCUGUCCCUUAGGGGAUUAGAACUUGAGUGAA GGUGGGCUGCUUGCAUCAGCCUAAUGUCGAGAA GUGCUUUCUUCGGAAAGUAACCCUCGAAACAAAU UCAUUCUUCCUCUCCAAUUCUGCACAA | 519 |
| U141A | CUUCACUGAUAAAGUGGAGAACCGCUUCACCAAA AGCUGUCCCUUAGGGGAUUAGAACUUGAGUGAA GGUGGGCUGCUUGCAUCAGCCUAAUGUCGAGAA GUGCUUUCUUCGGAAAGUAACCCUCGAAACAAAU UCAUUUAUCCUCUCCAAUUCUGCACAA | 520 |
| U141G | CUUCACUGAUAAAGUGGAGAACCGCUUCACCAAA AGCUGUCCCUUAGGGGAUUAGAACUUGAGUGAA GGUGGGCUGCUUGCAUCAGCCUAAUGUCGAGAA GUGCUUUCUUCGGAAAGUAACCCUCGAAACAAAU UCAUUUGUCCUCUCCAAUUCUGCACAA | 521 |
| U141C | CUUCACUGAUAAAGUGGAGAACCGCUUCACCAAA AGCUGUCCCUUAGGGGAUUAGAACUUGAGUGAA GGUGGGCUGCUUGCAUCAGCCUAAUGUCGAGAA GUGCUUUCUUCGGAAAGUAACCCUCGAAACAAAU UCAUUUCUCCUCUCCAAUUCUGCACAA | 271 |
| U142A | CUUCACUGAUAAAGUGGAGAACCGCUUCACCAAA AGCUGUCCCUUAGGGGAUUAGAACUUGAGUGAA GGUGGGCUGCUUGCAUCAGCCUAAUGUCGAGAA GUGCUUUCUUCGGAAAGUAACCCUCGAAACAAAU UCAUUUUACCUCUCCAAUUCUGCACAA | 522 |
| U142G | CUUCACUGAUAAAGUGGAGAACCGCUUCACCAAA AGCUGUCCCUUAGGGGAUUAGAACUUGAGUGAA GGUGGGCUGCUUGCAUCAGCCUAAUGUCGAGAA | 523 |
| | GUGCUUUCUUCGGAAAGUAACCCUCGAAACAAAU UCAUUUUGCCUCUCCAAUUCUGCACAA | |
| U142C | CUUCACUGAUAAAGUGGAGAACCGCUUCACCAAA AGCUGUCCCUUAGGGGAUUAGAACUUGAGUGAA GGUGGGCUGCUUGCAUCAGCCUAAUGUCGAGAA GUGCUUUCUUCGGAAAGUAACCCUCGAAACAAAU UCAUUUUCCCUCUCCAAUUCUGCACAA | 524 |

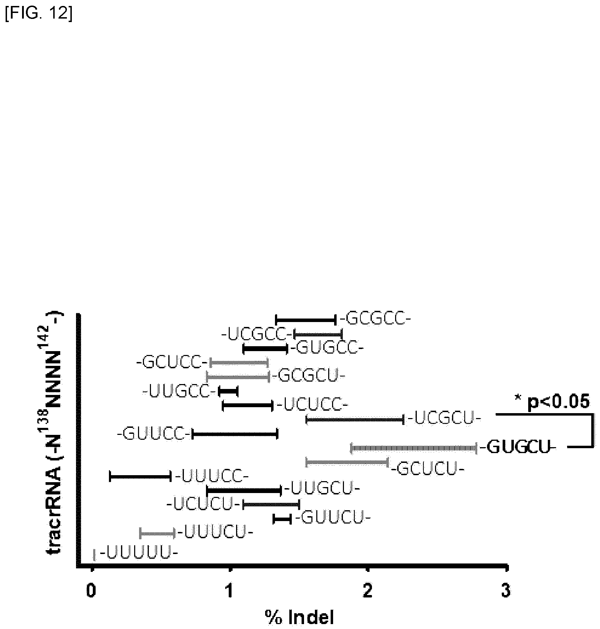

In addition, in order to confirm the effect of an additional substitution, comparison was performed on sixteen (16) engineered tracrRNAs which were made by fixing the 141st U to C while substituting the remaining uridines in various combinations based on FIG. 12 (Table 5). As a result of comparative analysis, most engineered tracrRNAs showed higher indel efficiency than the canonical tracrRNA (i.e., the wildtype tarcrRNA) comprising UUUUU. In particular, when UUUUU in the tracrRNA was replaced by 'GUGCU', indel efficiency was shown to be further increased (FIG. 12).

**[Table 5]**

| Engineered tracrRNA with modification of MS1 that comprises sequence of five or more consecutive uridines in tracrRNA (MS1 in tracrRNA is underlined) | | |
|---|---|---|
| Label | Sequence (5' to 3') | SEQ ID NO |
| UUUUU (Wildtype | CUUCACUGAUAAAGUGGAGAACCGCUUCACCAAA AGCUGUCCCUUAGGGGAUUAGAACUUGAGUGAA | 1 |
| tracrRNA) | GGUGGGCUGCUUGCAUCAGCCUAAUGUCGAGAA GUGCUUUCUUCGGAAAGUAACCCUCGAAACAAAU UCAUUUUCCUCUCCAAUUCUGCACAA | |
| UUUCU | CUUCACUGAUAAAGUGGAGAACCGCUUCACCAAA AGCUGUCCCUUAGGGGAUUAGAACUUGAGUGAA GGUGGGCUGCUUGCAUCAGCCUAAUGUCGAGAA GUGCUUUCUUCGGAAAGUAACCCUCGAAACAAAU UCAUUUCUCCUCUCCAAUUCUGCACAA | 271 |
| GUUCU | CUUCACUGAUAAAGUGGAGAACCGCUUCACCAAA AGCUGUCCCUUAGGGGAUUAGAACUUGAGUGAA GGUGGGCUGCUUGCAUCAGCCUAAUGUCGAGAA GUGCUUUCUUCGGAAAGUAACCCUCGAAACAAAU UCAGUUCUCCUCUCCAAUUCUGCACAA | 525 |
| UCUCU | CUUCACUGAUAAAGUGGAGAACCGCUUCACCAAA AGCUGUCCCUUAGGGGAUUAGAACUUGAGUGAA GGUGGGCUGCUUGCAUCAGCCUAAUGUCGAGAA GUGCUUUCUUCGGAAAGUAACCCUCGAAACAAAU UCAUCUCUCCUCUCCAAUUCUGCACAA | 526 |
| UUGCU | CUUCACUGAUAAAGUGGAGAACCGCUUCACCAAA AGCUGUCCCUUAGGGGAUUAGAACUUGAGUGAA GGUGGGCUGCUUGCAUCAGCCUAAUGUCGAGAA GUGCUUUCUUCGGAAAGUAACCCUCGAAACAAAU UCAUUGCUCCUCUCCAAUUCUGCACAA | 527 |
| UUUCC | CUUCACUGAUAAAGUGGAGAACCGCUUCACCAAA AGCUGUCCCUUAGGGGAUUAGAACUUGAGUGAA GGUGGGCUGCUUGCAUCAGCCUAAUGUCGAGAA GUGCUUUCUUCGGAAAGUAACCCUCGAAACAAAU UCAUUUCCCCUCUCCAAUUCUGCACAA | 528 |
| GCUCU | CUUCACUGAUAAAGUGGAGAACCGCUUCACCAAA AGCUGUCCCUUAGGGGAUUAGAACUUGAGUGAA GGUGGGCUGCUUGCAUCAGCCUAAUGUCGAGAA GUGCUUUCUUCGGAAAGUAACCCUCGAAACAAAU UCAGCUCUCCUCUCCAAUUCUGCACAA | 529 |
| GUGCU | CUUCACUGAUAAAGUGGAGAACCGCUUCACCAAA AGCUGUCCCUUAGGGGAUUAGAACUUGAGUGAA GGUGGGCUGCUUGCAUCAGCCUAAUGUCGAGAA GUGCUUUCUUCGGAAAGUAACCCUCGAAACAAAU UCAGUGCUCCUCUCCAAUUCUGCACAA | 270 |
| GUUCC | CUUCACUGAUAAAGUGGAGAACCGCUUCACCAAA AGCUGUCCCUUAGGGGAUUAGAACUUGAGUGAA GGUGGGCUGCUUGCAUCAGCCUAAUGUCGAGAA GUGCUUUCUUCGGAAAGUAACCCUCGAAACAAAU UCAGUUCCCCUCUCCAAUUCUGCACAA | 530 |
| UCGCU | CUUCACUGAUAAAGUGGAGAACCGCUUCACCAAA AGCUGUCCCUUAGGGGAUUAGAACUUGAGUGAA GGUGGGCUGCUUGCAUCAGCCUAAUGUCGAGAA | 531 |
| | GUGCUUUCUUCGGAAAGUAACCCUCGAAACAAAU UCAUCGCUCCUCUCCAAUUCUGCACAA | |
| UCUCC | CUUCACUGAUAAAGUGGAGAACCGCUUCACCAAA AGCUGUCCCUUAGGGGAUUAGAACUUGAGUGAA GGUGGGCUGCUUGCAUCAGCCUAAUGUCGAGAA GUGCUUUCUUCGGAAAGUAACCCUCGAAACAAAU UCAUCUCCCCUCUCCAAUUCUGCACAA | 532 |
| UUGCC | CUUCACUGAUAAAGUGGAGAACCGCUUCACCAAA AGCUGUCCCUUAGGGGAUUAGAACUUGAGUGAA GGUGGGCUGCUUGCAUCAGCCUAAUGUCGAGAA GUGCUUUCUUCGGAAAGUAACCCUCGAAACAAAU UCAUUGCCCCUCUCCAAUUCUGCACAA | 533 |
| GCGCU | CUUCACUGAUAAAGUGGAGAACCGCUUCACCAAA AGCUGUCCCUUAGGGGAUUAGAACUUGAGUGAA GGUGGGCUGCUUGCAUCAGCCUAAUGUCGAGAA GUGCUUUCUUCGGAAAGUAACCCUCGAAACAAAU UCAGCGCUCCUCUCCAAUUCUGCACAA | 534 |
| GCUCC | CUUCACUGAUAAAGUGGAGAACCGCUUCACCAAA AGCUGUCCCUUAGGGGAUUAGAACUUGAGUGAA GGUGGGCUGCUUGCAUCAGCCUAAUGUCGAGAA GUGCUUUCUUCGGAAAGUAACCCUCGAAACAAAU UCAGCUCCCCUCUCCAAUUCUGCACAA | 535 |
| GUGCC | CUUCACUGAUAAAGUGGAGAACCGCUUCACCAAA | 536 |
| | AGCUGUCCCUUAGGGGAUUAGAACUUGAGUGAA GGUGGGCUGCUUGCAUCAGCCUAAUGUCGAGAA GUGCUUUCUUCGGAAAGUAACCCUCGAAACAAAU UCAGUGCCCCUCUCCAAUUCUGCACAA | |
| UCGCC | CUUCACUGAUAAAGUGGAGAACCGCUUCACCAAA AGCUGUCCCUUAGGGGAUUAGAACUUGAGUGAA GGUGGGCUGCUUGCAUCAGCCUAAUGUCGAGAA GUGCUUUCUUCGGAAAGUAACCCUCGAAACAAAU UCAUCGCCCCUCUCCAAUUCUGCACAA | 537 |
| GCGCC | CUUCACUGAUAAAGUGGAGAACCGCUUCACCAAA AGCUGUCCCUUAGGGGAUUAGAACUUGAGUGAA GGUGGGCUGCUUGCAUCAGCCUAAUGUCGAGAA GUGCUUUCUUCGGAAAGUAACCCUCGAAACAAAU UCAGCGCCCCUCUCCAAUUCUGCACAA | 538 |

### Example 2-2. Engineered crRNA (M1-1): Modification of crRNA corresponding to engineered tracrRNA (M1-1)

In addition, in order to maximize indel efficiency, a crRNA corresponding to the engineered tracrRNA (M1-1) was engineered. Hereinafter, the crRNA modified according to the engineered tracrRNA (M1-1) was denoted as an engineered crRNA (M1-1) (FIG. 4A). In addition, based on the detailed description described above, the engineered crRNA (MS1-1) may have the sixth sequence (which does not comprise 5'-ACGAA-3') described above in the section "2-1) Sixth sequence" in the description of 2. Wildtype crRNA or engineered crRNA.

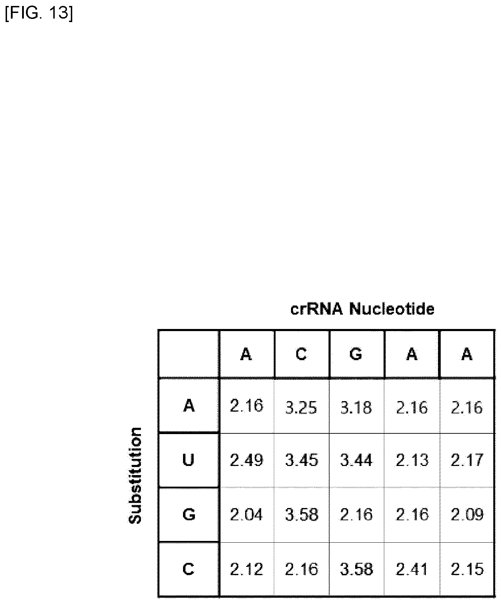

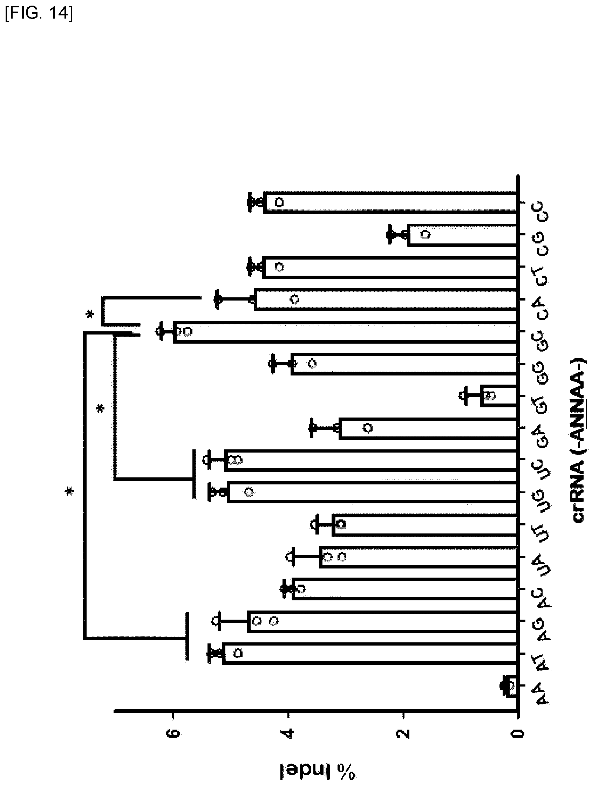

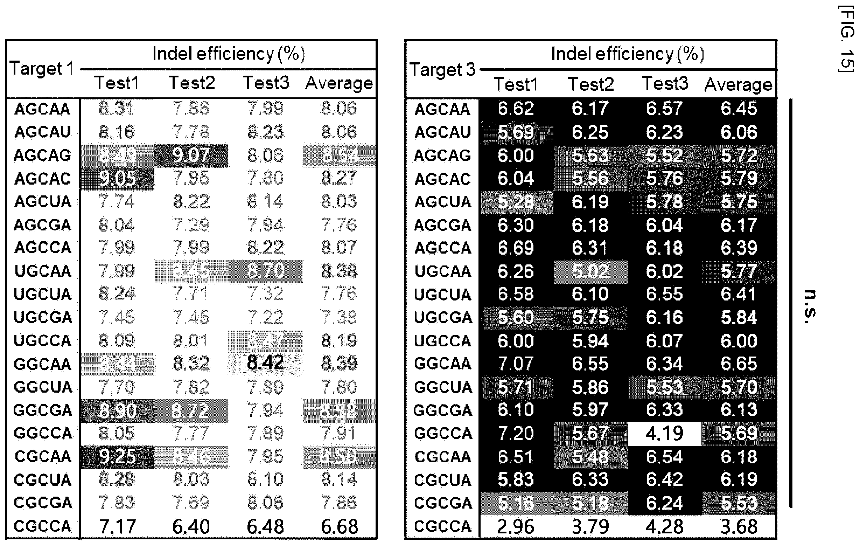

In order to find the optimal sequence of a crRNA corresponding to 5'-GUGCU-3' of the engineered tracrRNA (M1-1), effects of substitution by various sequences were analyzed using a method similar to the previous engineering method for the tracrRNA. Engineered crRNAs were generated by substituting respective nucleotides of 5'-ACGAA-3' of the crRNA, which corresponds to 5'-GUGCU-3' of the engineered tracrRNA (M1-1), with various nucleotides in various combinations. The generated engineered crRNAs were used to identify indel efficiency for three target genes in HEK293T cells. As a result, 5'-AGCAA-3' was found to be the optimal corresponding sequence to 5'-GUGCU-3' of the tracrRNA (FIGS. 13 to 15).

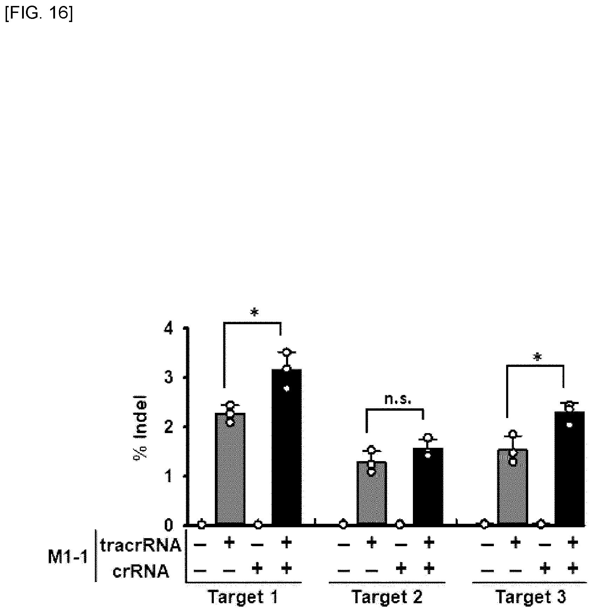

In addition, effects of the engineered gRNAs comprising MS1 modification were checked for three target genes in HEK293T cells. As a result, modification of the crRNA alone (i.e., the engineered crRNA (M1-1) comprises 5'-AGCAA-3', i.e., 5'-GUUGCAGAACCCGAAUAGAGCAAUGAAGGAAUGCAAC-3' (SEQ ID NO: 539)) did not affect indel activity caused by Cas12f1, but modification of the tracrRNA alone (i.e., the engineered tracrRNA (M1-1) is SEQ ID NO: 270 comprising 5'-GUGCU-3') resulted in significantly increased indel efficiency for all the target genes. In addition, when the tracrRNA and the crRNA were modified together, indel efficiency was further increased as compared with when the tracrRNA alone was modified (FIG. 16).

Briefly, as a result of engineering the tracrRNA and the crRNA, when the engineered tracrRNA (M1-1) comprises 5'-GUGCU-3' instead of 5'-UUUUU-3', and the engineered crRNA (M1-1) comprises 5'-AGCAA-3' instead of 5'-ACGAA-3', indel efficiency was found to be maximized. Hereinafter, the engineered gRNA comprising the engineered tracrRNA (M1-1) and the engineered crRNA (M1-1) was denoted as the engineered gRNA (M-1).

### Example 2-3. Engineered tracrRNA (M1-2) and engineered crRNA (M1-2): Truncation of tracrRNA-crRNA complementary sequence in stem 5

The engineered tracrRNA (M1-1) and the engineered crRNA (M1-1), which were generated by the preceding Examples 2-1 and 2-3, were further engineered. The gRNA for Cas14a1 consists of a tracrRNA and a crRNA and has a long sequence. Accordingly, in order to reduce a length of the gRNA without decreasing indel efficiency, the present inventors further modified the crRNA-tracrRNA complementarily binding region in the gRNA, that is, the region constituting stem 5 (MS1),. This was done in consideration of future clinical applications because chemical synthesis of a sgRNA having a length of 200 nts or higher may be a burden in a clinical environment. Hereinafter, when some base pairs are deleted from the tracrRNA-crRNA complementarily biding region, that is, the region constituting stem 5, this modification is denoted as M1-2. That is, when all or a part of the sequences, which are present at the 3' end of the tracrRNA and the 5' end of the crRNA and bind complementarily to each other, are deleted, the tracrRNA is denoted as an engineered tracrRNA (M1-2) and the crRNA is denoted as an engineered crRNA (M1-2), or the gRNA is denoted as an engineered sgRNA (M1-2). In addition, when all modifications of MS1 are included, that is, when both M1-1 and M1-2 are included, the tracrRNA is denoted as an engineered tracrRNA (M1) and the crRNA is denoted as an engineered crRNA (M1), or the gRNA is denoted as an engineered gRNA (M1) (FIG. 4B).

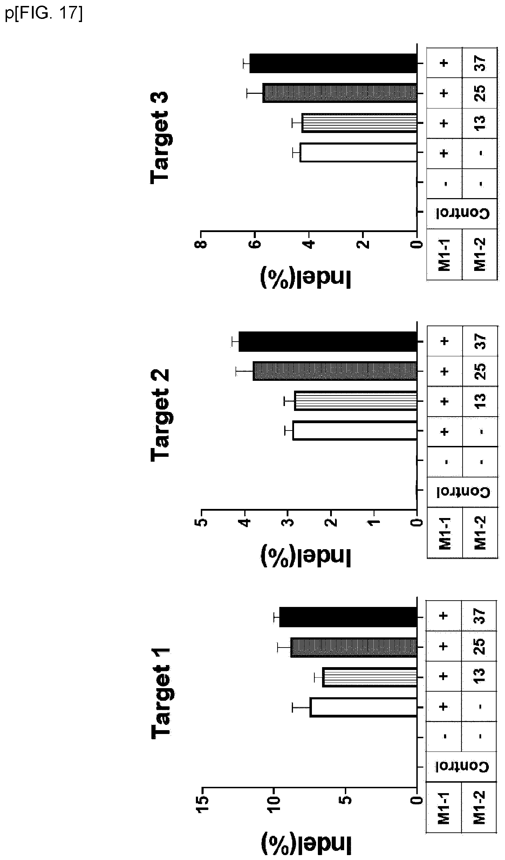

In order to identify the effect on indel efficiency, sgRNAs having various lengths of stem 5 (tracrRNA-crRNA complementarily binding region) were generated and tested (Table 6, FIG. 17). As a result, the sgRNA, from which the tracrRNA-crRNA complementarily binding region is deleted, almost retained its indel efficiency.

**[Table 6]**

| Sequences excluding guide sequence in engineered sgRNA (M1) from which some base pairs in tracrRNA-crRNA complementarily region are deleted (guide sequence is located at the 3' end of sequences listed below) | | |
|---|---|---|
| Label | Sequence (5' to 3') | SEQ ID NO |
| M1-1 (sgRNA) | CUUCACUGAUAAAGUGGAGAACCGCUUCACCAAA AGCUGUCCCUUAGGGGAUUAGAACUUGAGUGAA GGUGGGCUGCUUGCAUCAGCCUAAUGUCGAGAA GUGCUUUCUUCGGAAAGUAACCCUCGAAACAAAU UCAGUGCUCCUCUCCAAUUCUGCACAAGAAAGUU GCAGAACCCGAAUAGAGCAAUGAAGGAAUGCAAC | 540 |
| Δ6bp(Δ13nts) | CUUCACUGAUAAAGUGGAGAACCGCUUCACCAAA AGCUGUCCCUUAGGGGAUUAGAACUUGAGUGAA GGUGGGCUGCUUGCAUCAGCCUAAUGUCGAGAA | 541 |
| | GUGCUUUCUUCGGAAAGUAACCCUCGAAACAAAU UCAGUGCUCCUCUCCAAUUCGAAAGAACCCGAAU AGAGCAAUGAAGGAAUGCAAC | |
| Δ12bp(Δ25nts) | CUUCACUGAUAAAGUGGAGAACCGCUUCACCAAA AGCUGUCCCUUAGGGGAUUAGAACUUGAGUGAA GGUGGGCUGCUUGCAUCAGCCUAAUGUCGAGAA GUGCUUUCUUCGGAAAGUAACCCUCGAAACAAAU UCAGUGCUCCUCUCGAAAGAAUAGAGCAAUGAAG GAAUGCAAC | 542 |
| Δ18bp(Δ37nts) | CUUCACUGAUAAAGUGGAGAACCGCUUCACCAAA AGCUGUCCCUUAGGGGAUUAGAACUUGAGUGAA GGUGGGCUGCUUGCAUCAGCCUAAUGUCGAGAA GUGCUUUCUUCGGAAAGUAACCCUCGAAACAAAU UCAGUGCUGAAAGCAAUGAAGGAAUGCAAC | 543 |

### Example 3. Engineered guide RNA (MS1 and MS2): Truncation of tracrRNA-crRNA complementary sequence in stem 2 (engineered tracrRNA(M2))

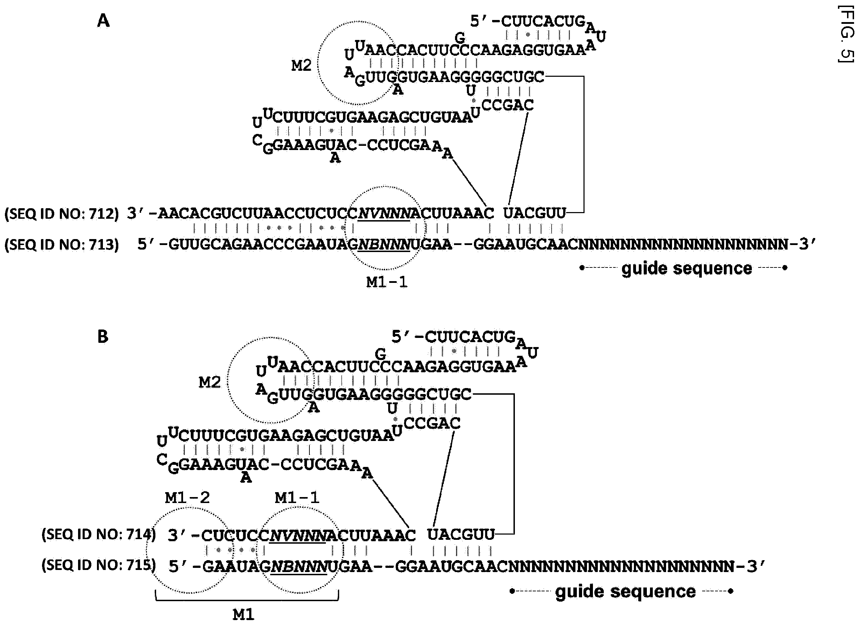

Cas14a1 has a very long gRNA due to a large size of the tracrRNA as compared with other class II CRISPR systems. Accordingly, the present inventors hypothesized that, given the compact size of Cas12f1, it is unlikely that the entire tracrRNA sequence will participate in interaction with Cas12f1. To confirm this hypothesis, the region (MS2) of the tracrRNA constituting stem 2 was further modified. This was done in consideration of future clinical applications because chemical synthesis of a sgRNA having a length of 200 nts or higher may be a burden in a clinical environment. Hereinafter, when some base pairs in the region constituting stem 2 are deleted, this modification was denoted as M2 or MS2 engineering. That is, when all or a part of the sequences, which are present at the 3' end of the tracrRNA and the 5' end of the crRNA and bind complementarily to each other, are deleted, the tracrRNA is denoted as an engineered tracrRNA (M2), or the gRNA is denoted as an engineered gRNA (M2). In addition, when a partial modification of MS1 is included, that is, when M1-1 is included, the tracrRNA is denoted as an engineered tracrRNA (M1-1/2), or the gRNA is denoted as an engineered gRNA (M1-1/2). In addition, when all modifications of MS1 are included, that is, when M1-1 and M1-2 are included, the tracrRNA is denoted as an engineered tracrRNA (M1/2) or the gRNA is denoted as an engineered gRNA (M1/2) (FIG. 5).

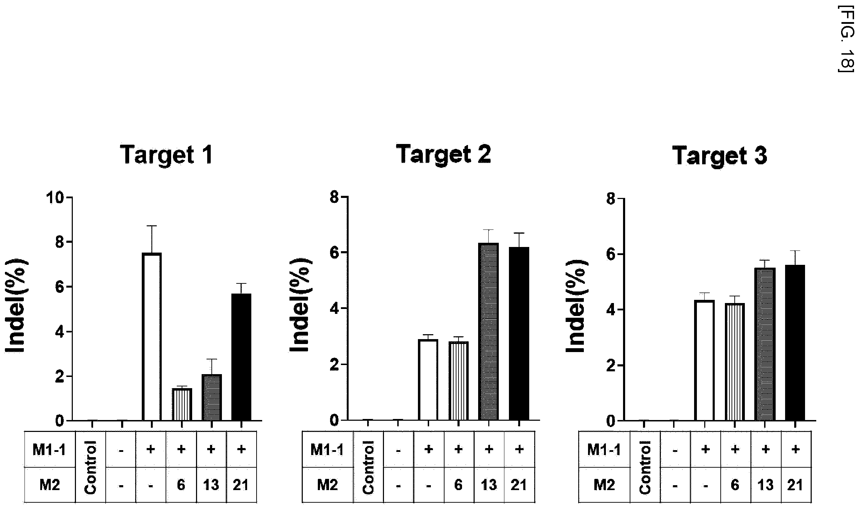

In order to confirm the effect on indel efficiency, sgRNAs having various lengths of stem 2 were generated and tested (Table 7, FIG. 18). As a result, the sgRNA, from which the tracrRNA-crRNA complementarily binding region is deleted, almost retained its indel efficiency.

**[Table 7]**

| Sequences excluding guide sequence in engineered gRNA (M1-1/2) from which some base pairs are deleted in region constituting stem 2 (guide sequence is located at 3' end of sequences listed below) | | |
|---|---|---|
| Label | Sequence (5' to 3') | SEQ ID NO |
| M1-1(sgRNA) | CUUCACUGAUAAAGUGGAGAACCGCUUCACCAAAAGC UGUCCCUUAGGGGAUUAGAACUUGAGUGAAGGUGGG CUGCUUGCAUCAGCCUAAUGUCGAGAAGUGCUUUCU UCGGAAAGUAACCCUCGAAACAAAUUCAGUGCUCCUC UCCAAUUCUGCACAAGAAAGUUGCAGAACCCGAAUAG AGCAAUGAAGGAAUGCAAC | 540 |
| Δ3bp(Δ6nts ) | CUUCACUGAUAAAGUGGAGAACCGCUUCACCAAAAGC UGUUUAGAUUAGAACUUGAGUGAAGGUGGGCUGCUU GCAUCAGCCUAAUGUCGAGAAGUGCUUUCUUCGGAAA GUAACCCUCGAAACAAAUUCAGUGCUCCUCUCCAAUU CUGCACAAGAAAGUUGCAGAACCCGAAUAGAGCAAUG AAGGAAUGCAAC | 544 |
| Δ6bp(Δ13nt s) | CUUCACUGAUAAAGUGGAGAACCGCUUCACCAAAAGC UUAGGAACUUGAGUGAAGGUGGGCUGCUUGCAUCAG CCUAAUGUCGAGAAGUGCUUUCUUCGGAAAGUAACCC UCGAAACAAAUUCAGUGCUCCUCUCCAAUUCUGCACA AGAAAGUUGCAGAACCCGAAUAGAGCAAUGAAGGAAU GCAAC | 546 |
| Δ10bp(Δ21 nts) | CUUCACUGAUAAAGUGGAGAACCGCUUCACCAAUUAG UUGAGUGAAGGUGGGCUGCUUGCAUCAGCCUAAUGU CGAGAAGUGCUUUCUUCGGAAAGUAACCCUCGAAACA AAUUCAGUGCUCCUCUCCAAUUCUGCACAAGAAAGUU | 547 |
| | GCAGAACCCGAAUAGAGCAAUGAAGGAAUGCAAC | |

### Example 4. Engineered guide RNA (MS1 and MS3): 5'-Truncation of tracrRNA (engineered tracrRNA (M3))

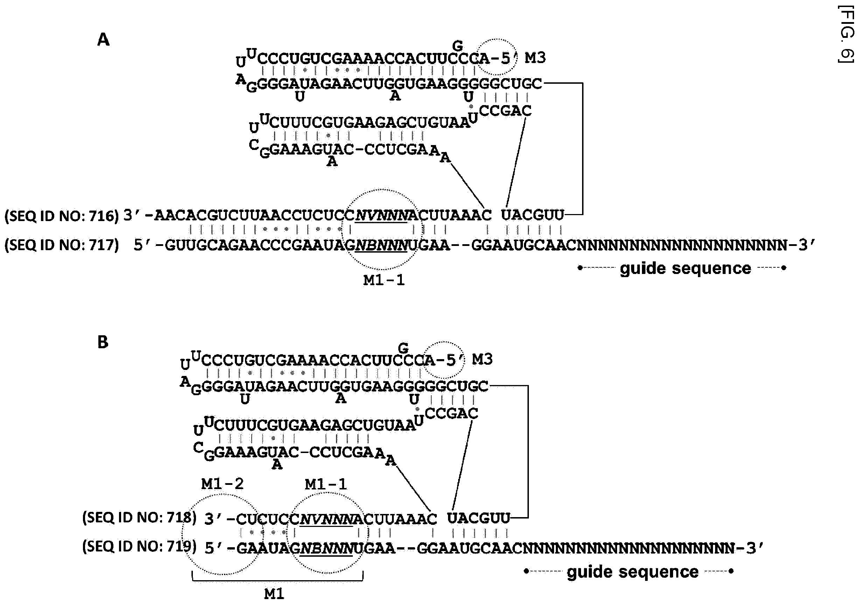

Cas14a1 has a very long gRNA due to a large size of the tracrRNA as compared with other class II CRISPR systems. Accordingly, the present inventors hypothesized that, given the compact size of Cas12f1, it is unlikely that the entire tracrRNA sequence will participate in interaction with Cas12f1. To confirm this hypothesis, a tracrRNA was designed so that the 5' end region of the tracrRNA (5' end region constituting stem 1) is deleted. Hereinafter, when a partial sequence at the 5' end of a tracrRNA is deleted, this modification was denoted as M3 or MS3 engineering. That is, when a partial sequence is deleted from the 5' end of the tracrRNA, the tracrRNA was denoted as an engineered tracrRNA comprising MS3 modification or an engineered tracrRNA (M3). In addition, when a partial modification of MS1 is included in the tracrRNA, that is, when M1-1 is included in the tracrRNA, the tracrRNA is denoted as an engineered tracrRNA (M1-1/3), or the gRNA is denoted as an engineered gRNA (M1-1/3). In addition, when all modifications of MS1 are included in the tracrRNA, that is, when M1-1 and M1-2 are included in the tracrRNA, the tracrRNA is denoted as an engineered tracrRNA (M1/3), or the gRNA is denoted as an engineered gRNA (M1/3) (FIG. 6).

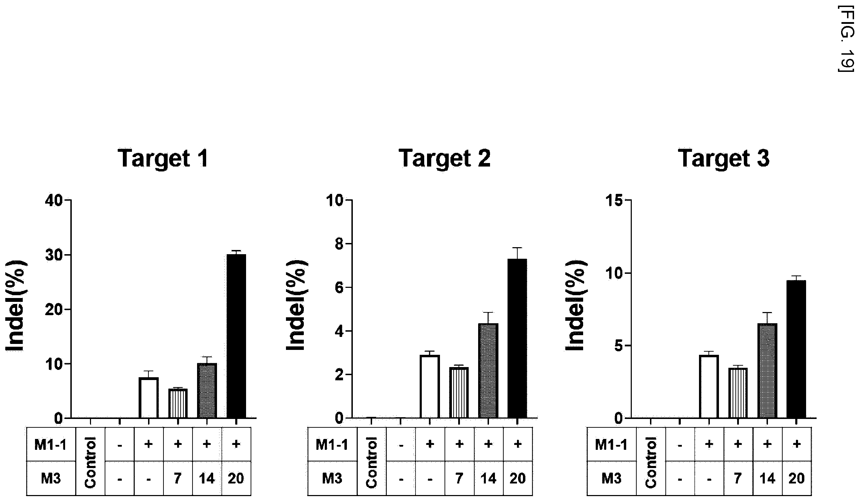

As a result of the experiment (Table 8) using the engineered tracrRNA (M1-1/3) designed so that the 5' end region of the tracrRNA is deleted, indel efficiency was found to be significantly increased when the tracrRNA with 20 nts deleted was used (FIG. 19).

**[Table 8]**

| Engineered tracrRNA (M1-1/3) designed so that 5' end region of tracrRNA is deleted | | |
|---|---|---|
| Label | Sequence (5' to 3') | SEQ ID NO |
| M1-1 | CUUCACUGAUAAAGUGGAGAACCGCUUCACCAAA AGCUGUCCCUUAGGGGAUUAGAACUUGAGUGAA GGUGGGCUGCUUGCAUCAGCCUAAUGUCGAGAA GUGCUUUCUUCGGAAAGUAACCCUCGAAACAAAU UCAGUGCUCCUCUCCAAUUCUGCACAA | 270 |
| -7nt | GAUAAAGUGGAGAACCGCUUCACCAAAAGCUGUC CCUUAGGGGAUUAGAACUUGAGUGAAGGUGGGC UGCUUGCAUCAGCCUAAUGUCGAGAAGUGCUUU CUUCGGAAAGUAACCCUCGAAACAAAUUCAGUGC UCCUCUCCAAUUCUGCACAA | 548 |
| -14nt | UGGAGAACCGCUUCACCAAAAGCUGUCCCUUAG GGGAUUAGAACUUGAGUGAAGGUGGGCUGCUUG CAUCAGCCUAAUGUCGAGAAGUGCUUUCUUCGG AAAGUAACCCUCGAAACAAAUUCAGUGCUCCUCU CCAAUUCUGCACAA | 549 |
| -20nt | ACCGCUUCACCAAAAGCUGUCCCUUAGGGGAUU | 308 |
| | AGAACUUGAGUGAAGGUGGGCUGCUUGCAUCAG CCUAAUGUCGAGAAGUGCUUUCUUCGGAAAGUAA CCCUCGAAACAAAUUCAGUGCUCCUCUCCAAUUC UGCACAA | |

### Example 5. Engineered guide RNAs (MS1 and MS4): Addition of 3'-polyuridinylates in crRNA (engineered crRNA (M4))

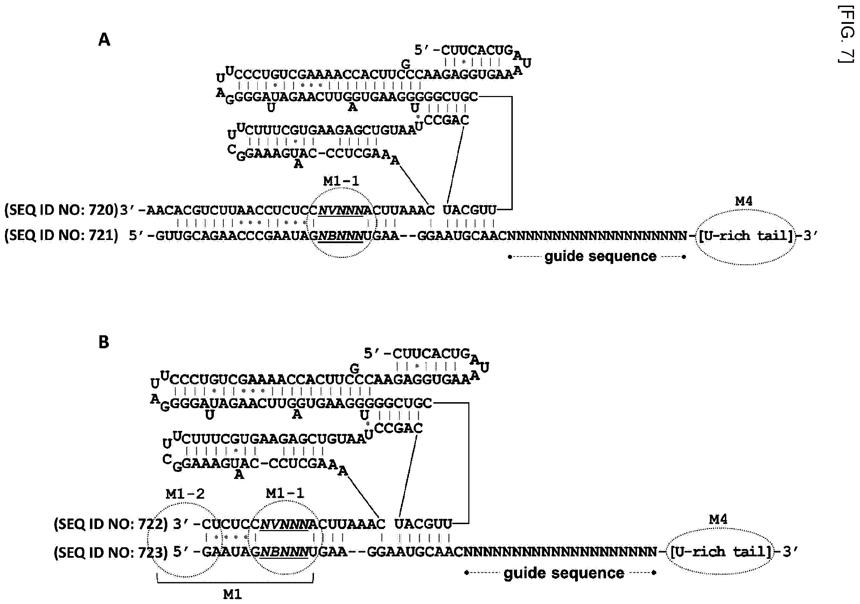

In a previous study, it was identified that when the CRISPR/Cas12f1 system was modified by addition of a U-rich tail sequence to the crRNA, improved indel efficiency was obtained (see PCT/KR2020/014961). Based on this, a modification was designed so that a U-rich tail sequence is added to the 3' end of the crRNA. When a U-rich tail sequence was included at the 3' end of the crRNA, this modification was denoted as M4 or MS4 engineering. That is, when a U-rich tail sequence was added to the 3' end of the crRNA, the crRNA was denoted as an engineered crRNA comprising MS4 modification or an engineered crRNA (M4). In addition, when a partial modification of MS1 is included in the crRNA, that is, when M1-1 is included in the crRNA, the crRNA is indicated as an engineered crRNA (M1-1/4), or the gRNA is denoted as an engineered gRNA (M1-1/4). In addition, when all modifications of MS1 are included in the crRNA, that is, when M1-1 and M1-2 are included in the crRNA, the crRNA is denoted as an engineered crRNA (M1/4), or the gRNA is denoted as an engineered gRNA (M1/4) (FIG. 7).

Indel efficiency of the engineered gRNAs (M1-1/4), which comprise the engineered tracrRNA (M1-1) of Example 2 and the engineered crRNA (M1-1/4) to which a U-rich tail sequence is added, was identified.

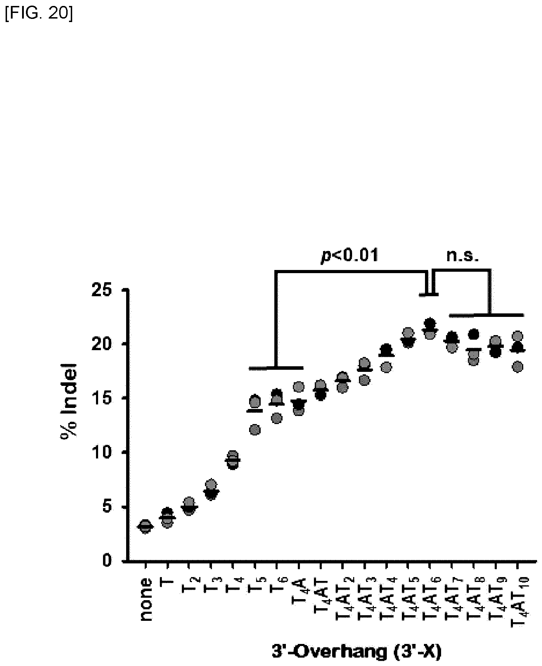

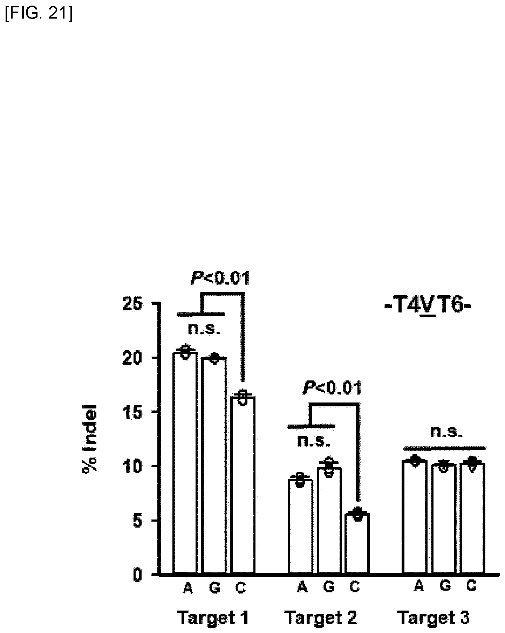

The engineered crRNAs (M1-1/4) were designed so that U-rich tail sequences of various lengths were included at the 3' end of the engineered crRNA (M1-1) for Cas12f1. Here, engineered crRNAs (M1-1/4) having U-rich tail sequences of various lengths were designed by gradually increasing a length of thymidines at the 3' end of the nucleic acid encoding the crRNA (Table 9). In particular, indel efficiency was further increased when the number of thymidines was 5 or more (FIG. 20). Here, A in the middle of a sequence of consecutive thymidines such as T₄A and T₄AT was designed to remove 5 consecutive thymidines (T5) that serve as a termination signal under a U6 promoter. In addition, C or G, instead of A, was added after TTTT and an effect thereof was identified (FIG. 21).

**[Table 9]**

| U-rich tail sequence added to 3' end of engineered crRNA (M1-1) | |
|---|---|
| Label | U-rich tail Sequence (5' to 3') |
| T | U |
| T₂ | UU |
| T₃ | UUU |
| T₄ | UUUU |
| T₅ | UUUUU |
| T₆ | UUUUUU |
| T₄A | UUUUA |
| T₄AT | UUUUAU |
| T₄AT₂ | UUUUAUU |
| T₄AT₃ | UUUUAUUU |
| T₄AT₄ | UUUUAUUUU |
| T₄AT₅ | UUUUAUUUUU (SEQ ID NO: 550) |
| T₄AT₆ | UUUUAUUUUUU (SEQ ID NO: 551) |
| T₄AT₇ | UUUUAUUUUUUU (SEQ ID NO: 552) |
| T₄AT₈ | UUUUAUUUUUUUU (SEQ ID NO: 553) |
| T₄AT₉ | UUUUAUUUUUUUUU (SEQ ID NO: 554) |
| T₄AT₁₀ | UUUUAUUUUUUUUUU (SEQ ID NO: 555) |

As a result, it was confirmed that indel efficiency increased as a length of thymidines increased. In particular, it was confirmed that when the engineered crRNA (M1-1/4) comprising U₄AU₄ and the engineered tracrRNA (M1-1) were used together, a greater synergistic effect was shown than when each of them was used alone, wherein indel efficiency was greatly improved up to 1,148-fold for Target 1.

### Example 6. Engineered guide RNAs (MS1, MS3 and MS4)

Based on the previous examples, an engineered tracrRNA (M1-1/3) (Table 9) comprising MS1 engineering, MS3 engineering, and MS4 engineering and an engineered crRNA (M1-1/4) (5'-[SEQ ID NO: 516]-[guide sequence]-[U₄AU₄]-3') were designed. As a result of the experiment using the engineered tracrRNAs and the engineered crRNAs, high indel efficiency was confirmed in all three targets. In particular, indel efficiency was about 3 to 6-fold higher in a case wherein the three combined modifications (M1-1/3/4) are used than a case where single modification (M1-1) or two combined modifications (M1-1/3 or M1-1/4) are used.

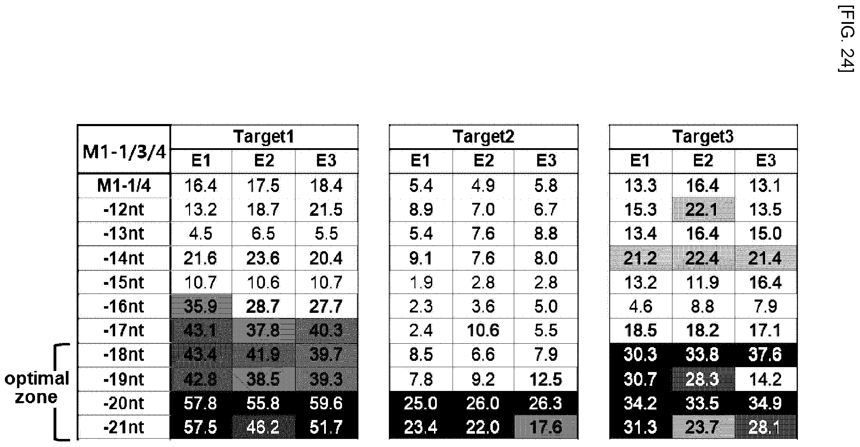

After confirming this effect, in order to optimize the sequence to be truncated at the 5' end region of the tracrRNA, a length of the 5' end of the tracrRNA was fine-tuned within a range of -12 nts to -21 nts and indel efficiency thereof for the three targets was compared (Table 10). As a result, it was confirmed that truncation in a range of 18 nts to 21 nts at the 5' end of the tracrRNA was most effective for indel efficiency (FIG. 24). In particular, the tracrRNA in which the sequence of 20 nts was truncated at the 5' end was most effective.

**[Table 10]**

| Engineered tracrRNA (M1-1/3) designed so that 5' end region of tracrRNA is truncated | | |
|---|---|---|
| Label | Sequence (5' to 3') | SEQ ID NO |
| M1-1 | CUUCACUGAUAAAGUGGAGAACCGCUUCACC AAAAGCUGUCCCUUAGGGGAUUAGAACUUGAGU | 270 |
| | GAAGGUGGGCUGCUUGCAUCAGCCUAAUGUCGA GAAGUGCUUUCUUCGGAAAGUAACCCUCGAAACA AAUUCAGUGCUCCUCUCCAAUUCUGCACAA | |
| -12nt | AGUGGAGAACCGCUUCACCAAAAGCUGUCCC UUAGGGGAUUAGAACUUGAGUGAAGGUGGGCUG CUUGCAUCAGCCUAAUGUCGAGAAGUGCUUUCU UCGGAAAGUAACCCUCGAAACAAAUUCAGUGCUC CUCUCCAAUUCUGCACAA | 556 |
| -13nt | GUGGAGAACCGCUUCACCAAAAGCUGUCCCU UAGGGGAUUAGAACUUGAGUGAAGGUGGGCUGC UUGCAUCAGCCUAAUGUCGAGAAGUGCUUUCUU CGGAAAGUAACCCUCGAAACAAAUUCAGUGCUCC UCUCCAAUUCUGCACAA | 557 |
| -14nt | UGGAGAACCGCUUCACCAAAAGCUGUCCCUU AGGGGAUUAGAACUUGAGUGAAGGUGGGCUGCU UGCAUCAGCCUAAUGUCGAGAAGUGCUUUCUUC GGAAAGUAACCCUCGAAACAAAUUCAGUGCUCCU CUCCAAUUCUGCACAA | 549 |
| -15nt | GGAGAACCGCUUCACCAAAAGCUGUCCCUUA GGGGAUUAGAACUUGAGUGAAGGUGGGCUGCUU GCAUCAGCCUAAUGUCGAGAAGUGCUUUCUUCG GAAAGUAACCCUCGAAACAAAUUCAGUGCUCCUC UCCAAUUCUGCACAA | 558 |
| -16nt | GAGAACCGCUUCACCAAAAGCUGUCCCUUAG GGGAUUAGAACUUGAGUGAAGGUGGGCUGCUUG CAUCAGCCUAAUGUCGAGAAGUGCUUUCUUCGG AAAGUAACCCUCGAAACAAAUUCAGUGCUCCUCU CCAAUUCUGCACAA | 559 |
| -17nt | AGAACCGCUUCACCAAAAGCUGUCCCUUAGG GGAUUAGAACUUGAGUGAAGGUGGGCUGCUUGC AUCAGCCUAAUGUCGAGAAGUGCUUUCUUCGGA AAGUAACCCUCGAAACAAAUUCAGUGCUCCUCUC CAAUUCUGCACAA | 560 |
| -18nt | GAACCGCUUCACCAAAAGCUGUCCCUUAGGG GAUUAGAACUUGAGUGAAGGUGGGCUGCUUGCA UCAGCCUAAUGUCGAGAAGUGCUUUCUUCGGAA AGUAACCCUCGAAACAAAUUCAGUGCUCCUCUCC AAUUCUGCACAA | 561 |
| -19nt | AACCGCUUCACCAAAAGCUGUCCCUUAGGGG AUUAGAACUUGAGUGAAGGUGGGCUGCUUGCAU CAGCCUAAUGUCGAGAAGUGCUUUCUUCGGAAA GUAACCCUCGAAACAAAUUCAGUGCUCCUCUCCA AUUCUGCACAA | 562 |
| -20nt | ACCGCUUCACCAAAAGCUGUCCCUUAGGGGA UUAGAACUUGAGUGAAGGUGGGCUGCUUGCAUC AGCCUAAUGUCGAGAAGUGCUUUCUUCGGAAAG | 308 |
| | UAACCCUCGAAACAAAUUCAGUGCUCCUCUCCAA UUCUGCACAA | |
| -21nt | CCGCUUCACCAAAAGCUGUCCCUUAGGGGAU UAGAACUUGAGUGAAGGUGGGCUGCUUGCAUCA GCCUAAUGUCGAGAAGUGCUUUCUUCGGAAAGU AACCCUCGAAACAAAUUCAGUGCUCCUCUCCAAU UCUGCACAA | 563 |

### Example 7. Engineered guide RNAs (MS1, MS3 and MS4)

Based on the previous examples, an engineered tracrRNA (M1/3) comprising MS1 engineering, MS3 engineering and MS4 engineering and an engineered crRNA (M1/4) were designed.

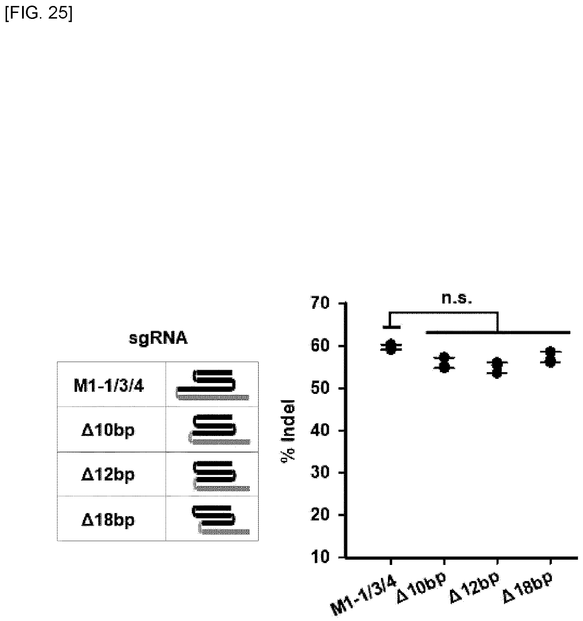

According to Example 6, it was confirmed that indel efficiency was increased in a case where the three combined modifications (M1-1/3/4) were used. However, the gRNA for Cas14a1 still has a long sequence even after truncation of the 5' end of the tracrRNA. Accordingly, the present inventors additionally designed the gRNA so that M1-2 modification was further included to optimize its length (Table 11). When an engineered sgRNA (M1/3/4) comprising the engineered tracrRNA (M1/3) and the engineered crRNA (M1/4) was used, indel efficiency was found to be slightly increased or similar to the indel efficiency obtained when the engineered tracrRNA (M1-1/3) and the engineered crRNA (M1-1/4) were used in Example 6 (FIG. 25).

**[Table 11]**

| Sequences excluding guide sequence and U-rich tail sequence in engineered sgRNA (M1/3/4) (guide sequence and U-rich tail are located at 3' end of sequences listed below wherein U-rich tail sequence in are is 5'-U₄AU₄-3') | | |
|---|---|---|
| Label | Sequence (5' to 3') | SEQ ID NO |
| M1-1/3/4 (sgRNA) | ACCGCUUCACCAAAAGCUGUCCCUUAGGGGA UUAGAACUUGAGUGAAGGUGGGCUGCUUGCAUC AGCCUAAUGUCGAGAAGUGCUUUCUUCGGAAAG UAACCCUCGAAACAAAUUCAGUGCUCCUCUCCAA UUCUGCACAAGAAAGUUGCAGAACCCGAAUAGAG CAAUGAAGGAAUGCAAC | 564 |
| Δ10bp (Δ21nts) | ACCGCUUCACCAAAAGCUGUCCCUUAGGGGA UUAGAACUUGAGUGAAGGUGGGCUGCUUGCAUC AGCCUAAUGUCGAGAAGUGCUUUCUUCGGAAAG UAACCCUCGAAACAAAUUCAGUGCUCCUCUCCAG AAACCGAAUAGAGCAAUGAAGGAAUGCAAC | 565 |
| Δ12bp (Δ25nts) | ACCGCUUCACCAAAAGCUGUCCCUUAGGGGA UUAGAACUUGAGUGAAGGUGGGCUGCUUGCAUC AGCCUAAUGUCGAGAAGUGCUUUCUUCGGAAAG UAACCCUCGAAACAAAUUCAGUGCUCCUCUCGAA AGAAUAGAGCAAUGAAGGAAUGCAAC | 566 |
| Δ18bp (Δ37nts) | ACCGCUUCACCAAAAGCUGUCCCUUAGGGGA UUAGAACUUGAGUGAAGGUGGGCUGCUUGCAUC AGCCUAAUGUCGAGAAGUGCUUUCUUCGGAAAG UAACCCUCGAAACAAAUUCAGUGCUGAAAGCAA UGAAGGAAUGCAAC | 567 |

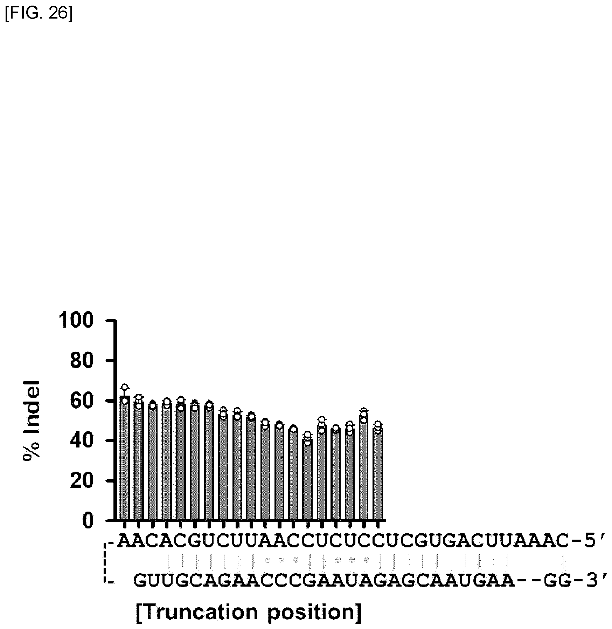

Based on these results, additional screening was performed to find a sgRNA having an optimal length without affecting interaction with Cas14a1. Various sgRNAs were designed so that they have a difference in length by 1 bp over the entire range of the tracrRNA-crRNA complementarily binding region (stem 5) and tested (Table 12, FIG. 26).

**[Table 12]**

| Sequences excluding guide sequence and U-rich tail sequence in engineered sgRNA (M1/3/4) (guide sequence and U-rich tail sequence are located at 3' end of sequences listed below wherein U-rich tail sequence is 5'-U₄AU₄-3') | | |
|---|---|---|
| Label | Sequence (5' to 3') | SEQ ID NO |
| M1-1/3/4 (sgRNA) | ACCGCUUCACCAAAAGCUGUCCCUUAGGGGAUUA GAACUUGAGUGAAGGUGGGCUGCUUGCAUCAGC CUAAUGUCGAGAAGUGCUUUCUUCGGAAAGUAAC CCUCGAAACAAAUUCAGUGCUCCUCUCCAAUUCU | 564 |
| | GCACAAGAAAGUUGCAGAACCCGAAUAGAGCAAU GAAGGAAUGCAAC | |
| -1nt | ACCGCUUCACCAAAAGCUGUCCCUUAGGGGAUUA GAACUUGAGUGAAGGUGGGCUGCUUGCAUCAGC CUAAUGUCGAGAAGUGCUUUCUUCGGAAAGUAAC CCUCGAAACAAAUUCAGUGCUCCUCUCCAAUUCU GCACAGAAAGUUGCAGAACCCGAAUAGAGCAAUG AAGGAAUGCAAC | 568 |
| -3nt | ACCGCUUCACCAAAAGCUGUCCCUUAGGGGAUUA GAACUUGAGUGAAGGUGGGCUGCUUGCAUCAGC CUAAUGUCGAGAAGUGCUUUCUUCGGAAAGUAAC CCUCGAAACAAAUUCAGUGCUCCUCUCCAAUUCU GCACGAAAUUGCAGAACCCGAAUAGAGCAAUGAA GGAAUGCAAC | 569 |
| -5nt | ACCGCUUCACCAAAAGCUGUCCCUUAGGGGAUUA GAACUUGAGUGAAGGUGGGCUGCUUGCAUCAGC CUAAUGUCGAGAAGUGCUUUCUUCGGAAAGUAAC CCUCGAAACAAAUUCAGUGCUCCUCUCCAAUUCU GCAGAAAUGCAGAACCCGAAUAGAGCAAUGAAGG AAUGCAAC | 570 |
| -7nt | ACCGCUUCACCAAAAGCUGUCCCUUAGGGGAUUA GAACUUGAGUGAAGGUGGGCUGCUUGCAUCAGC CUAAUGUCGAGAAGUGCUUUCUUCGGAAAGUAAC | 571 |
| | CCUCGAAACAAAUUCAGUGCUCCUCUCCAAUUCU GCGAAAGCAGAACCCGAAUAGAGCAAUGAAGGAA UGCAAC | |
| -9nt | ACCGCUUCACCAAAAGCUGUCCCUUAGGGGAUUA GAACUUGAGUGAAGGUGGGCUGCUUGCAUCAGC CUAAUGUCGAGAAGUGCUUUCUUCGGAAAGUAAC CCUCGAAACAAAUUCAGUGCUCCUCUCCAAUUCU GGAAACAGAACCCGAAUAGAGCAAUGAAGGAAUG CAAC | 572 |
| -11nt | ACCGCUUCACCAAAAGCUGUCCCUUAGGGGAUUA GAACUUGAGUGAAGGUGGGCUGCUUGCAUCAGC CUAAUGUCGAGAAGUGCUUUCUUCGGAAAGUAAC CCUCGAAACAAAUUCAGUGCUCCUCUCCAAUUCU GAAAAGAACCCGAAUAGAGCAAUGAAGGAAUGCA AC | 573 |
| -13nt | ACCGCUUCACCAAAAGCUGUCCCUUAGGGGAUUA GAACUUGAGUGAAGGUGGGCUGCUUGCAUCAGC CUAAUGUCGAGAAGUGCUUUCUUCGGAAAGUAAC CCUCGAAACAAAUUCAGUGCUCCUCUCCAAUUCG AAAGAACCCGAAUAGAGCAAUGAAGGAAUGCAAC | 574 |
| -15nt | ACCGCUUCACCAAAAGCUGUCCCUUAGGGGAUUA GAACUUGAGUGAAGGUGGGCUGCUUGCAUCAGC CUAAUGUCGAGAAGUGCUUUCUUCGGAAAGUAAC | 575 |
| | CCUCGAAACAAAUUCAGUGCUCCUCUCCAAUUGA AAAACCCGAAUAGAGCAAUGAAGGAAUGCAAC | |
| -17nt | ACCGCUUCACCAAAAGCUGUCCCUUAGGGGAUUA GAACUUGAGUGAAGGUGGGCUGCUUGCAUCAGC CUAAUGUCGAGAAGUGCUUUCUUCGGAAAGUAAC CCUCGAAACAAAUUCAGUGCUCCUCUCCAAUGAA AACCCGAAUAGAGCAAUGAAGGAAUGCAAC | 576 |
| -19nt | ACCGCUUCACCAAAAGCUGUCCCUUAGGGGAUUA GAACUUGAGUGAAGGUGGGCUGCUUGCAUCAGC CUAAUGUCGAGAAGUGCUUUCUUCGGAAAGUAAC CCUCGAAACAAAUUCAGUGCUCCUCUCCAAGAAA CCCGAAUAGAGCAAUGAAGGAAUGCAAC | 577 |
| -21nt | ACCGCUUCACCAAAAGCUGUCCCUUAGGGGAUUA GAACUUGAGUGAAGGUGGGCUGCUUGCAUCAGC CUAAUGUCGAGAAGUGCUUUCUUCGGAAAGUAAC CCUCGAAACAAAUUCAGUGCUCCUCUCCAGAAAC CGAAUAGAGCAAUGAAGGAAUGCAAC | 578 |
| -23nt | ACCGCUUCACCAAAAGCUGUCCCUUAGGGGAUUA GAACUUGAGUGAAGGUGGGCUGCUUGCAUCAGC CUAAUGUCGAGAAGUGCUUUCUUCGGAAAGUAAC CCUCGAAACAAAUUCAGUGCUCCUCUCCGAAACG AAUAGAGCAAUGAAGGAAUGCAAC | 579 |
| -25nt | ACCGCUUCACCAAAAGCUGUCCCUUAGGGGAUUA | 566 |
| | GAACUUGAGUGAAGGUGGGCUGCUUGCAUCAGC CUAAUGUCGAGAAGUGCUUUCUUCGGAAAGUAAC CCUCGAAACAAAUUCAGUGCUCCUCUCGAAAGAA UAGAGCAAUGAAGGAAUGCAAC | |
| -27nt | ACCGCUUCACCAAAAGCUGUCCCUUAGGGGAUUA GAACUUGAGUGAAGGUGGGCUGCUUGCAUCAGC CUAAUGUCGAGAAGUGCUUUCUUCGGAAAGUAAC CCUCGAAACAAAUUCAGUGCUCCUCUGAAAAUA GAGCAAUGAAGGAAUGCAAC | 580 |
| -29nt | ACCGCUUCACCAAAAGCUGUCCCUUAGGGGAUUA GAACUUGAGUGAAGGUGGGCUGCUUGCAUCAGC CUAAUGUCGAGAAGUGCUUUCUUCGGAAAGUAAC CCUCGAAACAAAUUCAGUGCUCCUCGAAAAUAGA GCAAUGAAGGAAUGCAAC | 581 |
| -31nt | ACCGCUUCACCAAAAGCUGUCCCUUAGGGGAUUA GAACUUGAGUGAAGGUGGGCUGCUUGCAUCAGC CUAAUGUCGAGAAGUGCUUUCUUCGGAAAGUAAC CCUCGAAACAAAUUCAGUGCUCCUGAAAUAGAGC AAUGAAGGAAUGCAAC | 582 |
| -33nt | ACCGCUUCACCAAAAGCUGUCCCUUAGGGGAUUA GAACUUGAGUGAAGGUGGGCUGCUUGCAUCAGC CUAAUGUCGAGAAGUGCUUUCUUCGGAAAGUAAC CCUCGAAACAAAUUCAGUGCUCCGAAAAGAGCAA | 583 |
| | UGAAGGAAUGCAAC | |
| -35nt | ACCGCUUCACCAAAAGCUGUCCCUUAGGGGAUUA GAACUUGAGUGAAGGUGGGCUGCUUGCAUCAGC CUAAUGUCGAGAAGUGCUUUCUUCGGAAAGUAAC CCUCGAAACAAAUUCAGUGCUCGAAAGAGCAAUG AAGGAAUGCAAC | 584 |
| -37nt | ACCGCUUCACCAAAAGCUGUCCCUUAGGGGAUUA GAACUUGAGUGAAGGUGGGCUGCUUGCAUCAGC CUAAUGUCGAGAAGUGCUUUCUUCGGAAAGUAAC CCUCGAAACAAAUUCAGUGCUGAAAAGCAAUGAA GGAAUGCAAC | 585 |

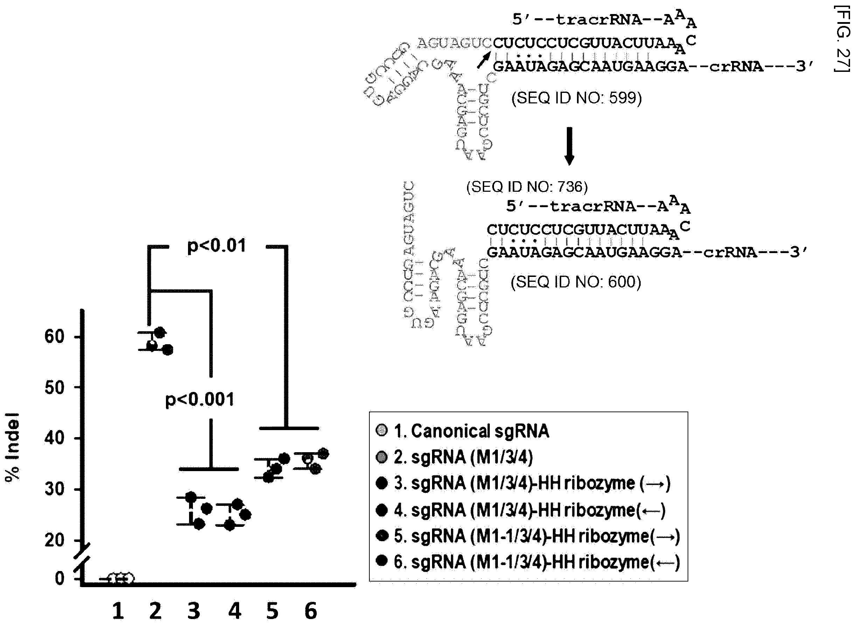

Meanwhile, further modification was attempted on the sgRNA using a linker sequence. Generally, GAAA is used as a linker for the sgRNA. Here, the gRNA acts as a single RNA molecule. On the other hand, when the sequence GAAA is replaced with a hammerhead ribozyme sequence, the sgRNA is produced at the expression stage and is self-cleaved by the hammerhead ribozyme to generate a double guide RNA with an overhang in the tracrRNA or crRNA. That is, binding of a hammerhead ribozyme to the tracrRNA and the crRNA may generate a 3'-truncated tracrRNA and a 5'-elongated crRNA (FIG. 27). Alternatively, on the contrary, when incorporation of the hammerhead ribozyme sequence occurs in a reverse direction, a 5'-elongated tracrRNA and a 3'-truncated crRNA are generated. Structural modification of the gRNA using characteristics of the hammerhead ribozyme sequence was tested to investigate whether such modification can further improve genome editing As a result, none of the gRNAs, which were designed to have a hammerhead ribozyme sequence, showed increased efficiency as compared with the sgRNA in which 25 nucleotides in total were truncated (i.e., 12 nts and 13 nts were truncated in the crRNA and the tracrRNA, respectively) (FIG. 27).

### Example 8. Various engineered guide RNAs: MS1, MS2, MS3 and/or MS4

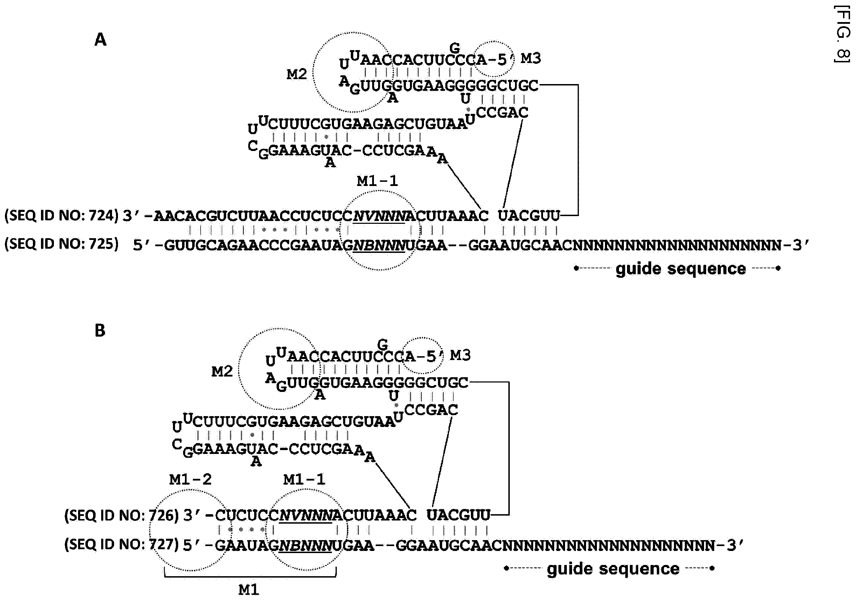

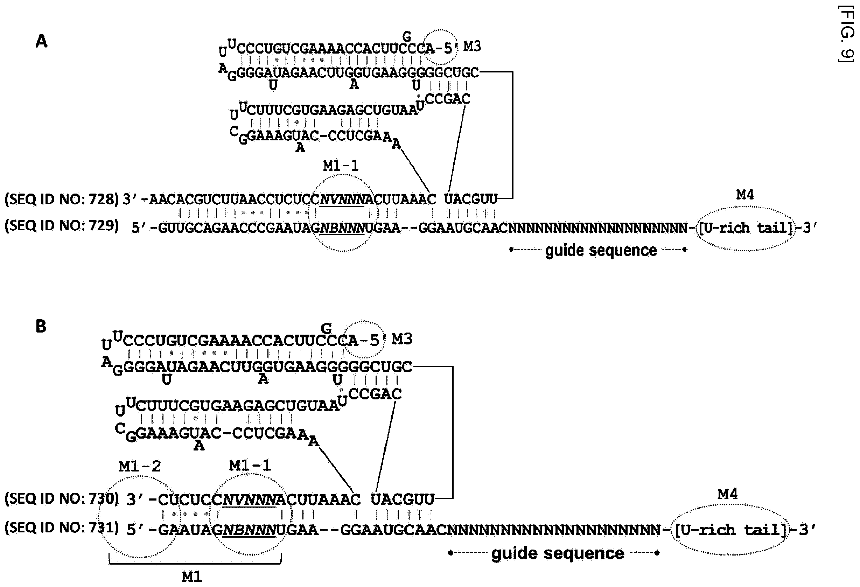

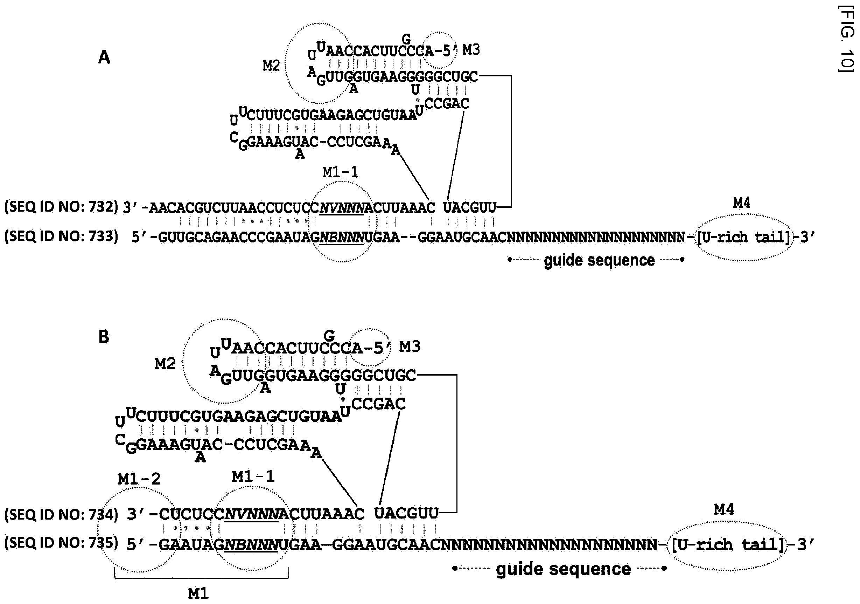

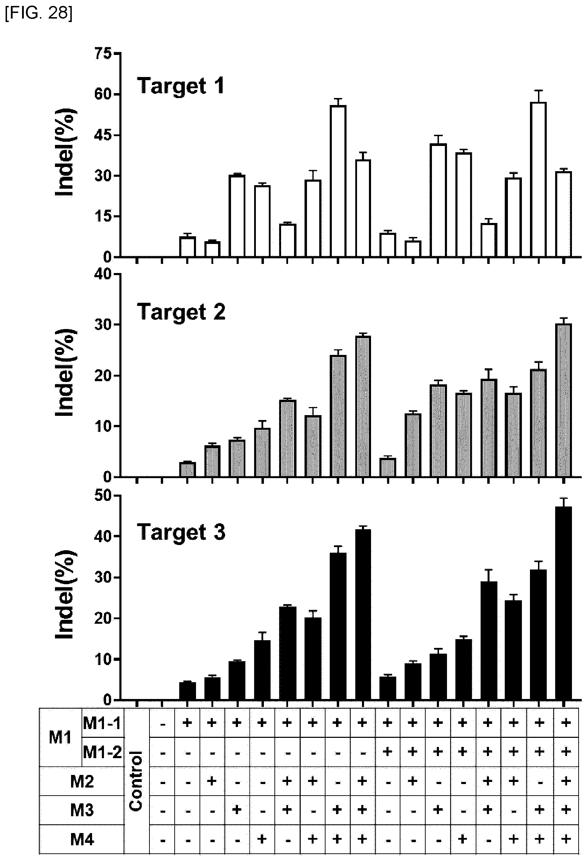

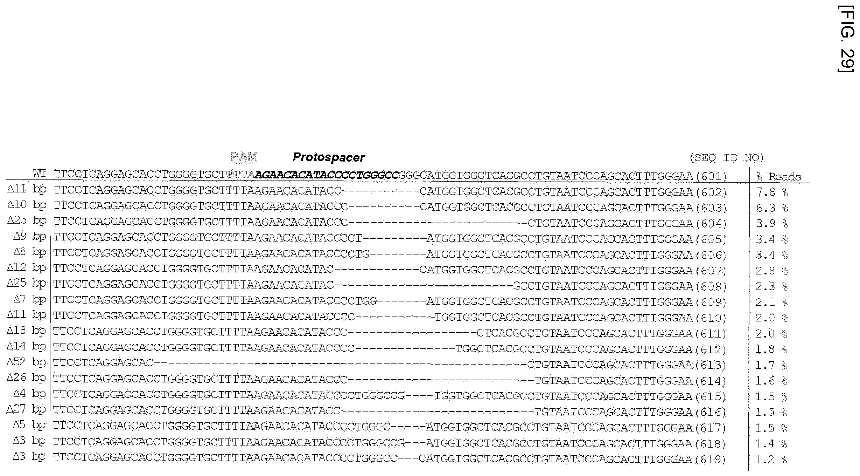

Based on the various gRNA engineerings identified in Examples 2 to 5, various engineered gRNAs were designed by various combinations of MS1, MS2, MS3 and MS4 engineerings, and effects thereof were identified (FIGS. 4 to 10 and Table 13). As a result, various gRNA engineerings improved indel efficiency (FIG. 28). In particular, the CRISPR/Cas14a1 systems, in whichgRNAs engineered by M1/3/4 combination and M1/2/3/4 combination, showed the best genome editing performance. Hereinafter, the engineered CRISPR/Cas14a1 system comprising the gRNA engineered by M1/3/4 combination is denoted as geCas14a1_3.0 system. In addition, indel patterns caused by the geCas14a1_3.0 system were identified in HEK293T cells (FIG. 29). To summarize, a powerful and more compact CRISPR/Cas12f1 system was developed by extensive gRNA engineering and this system enables high-efficiency genome editing in eukaryotic cells which could not be achieved with the existing CRISPR/Cas12f1 system (i.e., the wildtype CRISPR/Cas12f1 system).

**[Table 13]**

| Sequences excluding guide sequence in various engineered sgRNAs (guide sequence is located at 3' end of sequences listed below), wherein when M4 is included, 5'-UUUUAUUUUU-3' is added to 3' end of guide sequence. | | |
|---|---|---|
| Label | Sequence (5' to 3') (SEQ ID NO:) | Inclusion of 5'-UUUUAUUUU-3' |
| Canonical (Wildtype sgRNA) | CUUCACUGAUAAAGUGGAGAACCGCUUCACC AAAAGCUGUCCCUUAGGGGAUUAGAACUUGA GUGAAGGUGGGCUGCUUGCAUCAGCCUAAUG UCGAGAAGUGCUUUCUUCGGAAAGUAACCCU CGAAACAAAUUCAUUUUUCCUCUCCAAUUCUG CACAAGAAAGUUGCAGAACCCGAAUAGACGAA UGAAGGAAUGCAAC (SEQ ID NO: 586) | not included |
| M1-1 | CUUCACUGAUAAAGUGGAGAACCGCUUCACC AAAAGCUGUCCCUUAGGGGAUUAGAACUUGA GUGAAGGUGGGCUGCUUGCAUCAGCCUAAUG UCGAGAAGUGCUUUCUUCGGAAAGUAACCCU CGAAACAAAUUCAGUGCUCCUCUCCAAUUCU GCACAAGAAAGUUGCAGAACCCGAAUAGAGC AAUGAAGGAAUGCAAC (SEQ ID NO: 540) | not included |
| M1 | CUUCACUGAUAAAGUGGAGAACCGCUUCACC AAAAGCUGUCCCUUAGGGGAUUAGAACUUGA GUGAAGGUGGGCUGCUUGCAUCAGCCUAAUG UCGAGAAGUGCUUUCUUCGGAAAGUAACCCU | not included |
| | CGAAACAAAUUCAGUGCUCCUCUCGAAAGAA UAGAGCAAUGAAGGAAUGCAAC (SEQ ID NO: 542) | |
| M1-1/2 | CUUCACUGAUAAAGUGGAGAACCGCUUCACC AAUUAGUUGAGUGAAGGUGGGCUGCUUGCAU CAGCCUAAUGUCGAGAAGUGCUUUCUUCGGA AAGUAACCCUCGAAACAAAUUCAGUGCUCCU CUCCAAUUCUGCACAAGAAAGUUGCAGAACC CGAAUAGAGCAAUGAAGGAAUGCAAC (SEQ ID NO: 547) | not included |
| M1/2 | CUUCACUGAUAAAGUGGAGAACCGCUUCACC AAUUAGUUGAGUGAAGGUGGGCUGCUUGCAU CAGCCUAAUGUCGAGAAGUGCUUUCUUCGGA AAGUAACCCUCGAAACAAUUCAGUGCUCCU CUCGAAAGAAUAGAGCAAUGAAGGAAUGCAA C (SEQ ID NO: 587) | not included |
| M1-1/3 | ACCGCUUCACCAAAAGCUGUCCCUUAGGGGA UUAGAACUUGAGUGAAGGUGGGCUGCUUGCA UCAGCCUAAUGUCGAGAAGUGCUUUCUUCGG AAAGUAACCCUCGAAACAAAUUCAGUGCUCCU CUCCAAUUCUGCACAAGAAAGUUGCAGAACC CGAAUAGAGCAAUGAAGGAAUGCAAC (SEQ ID NO: 564) | not included |
| M1/3 | ACCGCUUCACCAAAAGCUGUCCCUUAGGGGA UUAGAACUUGAGUGAAGGUGGGCUGCUUGCA UCAGCCUAAUGUCGAGAAGUGCUUUCUUCGG AAAGUAACCCUCGAAACAAAUUCAGUGCUCCU CUCGAAAGAAUAGAGCAAUGAAGGAAUGCAA C (SEQ ID NO: 566) | not included |
| M1-1/4 | CUUCACUGAUAAAGUGGAGAACCGCUUCACC AAAAGCUGUCCCUUAGGGGAUUAGAACUUGA GUGAAGGUGGGCUGCUUGCAUCAGCCUAAUG UCGAGAAGUGCUUUCUUCGGAAAGUAACCCU CGAAACAAAUUCAGUGCUCCUCUCCAAUUCU GCACAAGAAAGUUGCAGAACCCGAAUAGAGC AAUGAAGGAAUGCAAC (SEQ ID NO: 540) | included |
| M1/4 | CUUCACUGAUAAAGUGGAGAACCGCUUCACC AAAAGCUGUCCCUUAGGGGAUUAGAACUUGA GUGAAGGUGGGCUGCUUGCAUCAGCCUAAUG UCGAGAAGUGCUUUCUUCGGAAAGUAACCCU CGAAACAAAUUCAGUGCUCCUCUCGAAAGAA UAGAGCAAUGAAGGAAUGCAAC (SEQ ID NO: 542) | included |
| M1-1/2/3 | ACCGCUUCACCAAUUAGUUGAGUGAAGGUGG GCUGCUUGCAUCAGCCUAAUGUCGAGAAGUG CUUUCUUCGGAAAGUAACCCUCGAAACAAAU | not included |
| | UCAGUGCUCCUCUCCAAUUCUGCACAAGAAA GUUGCAGAACCCGAAUAGAGCAAUGAAGGAA UGCAAC (SEQ ID NO: 588) | |
| M1/2/3 | ACCGCUUCACCAAUUAGUUGAGUGAAGGUGG GCUGCUUGCAUCAGCCUAAUGUCGAGAAGUG CUUUCUUCGGAAAGUAACCCUCGAAACAAAU UCAGUGCUCCUCUCGAAAGAAUAGAGCAAUG AAGGAAUGCAAC (SEQ ID NO: 589) | not included |
| M1-1/2/4 | CUUCACUGAUAAAGUGGAGAACCGCUUCACC AAUUAGUUGAGUGAAGGUGGGCUGCUUGCAU CAGCCUAAUGUCGAGAAGUGCUUUCUUCGGA AAGUAACCCUCGAAACAAAUUCAGUGCUCCU CUCCAAUUCUGCACAAGAAAGUUGCAGAACC CGAAUAGAGCAAUGAAGGAAUGCAAC (SEQ ID NO: 547) | included |
| M1/2/4 | CUUCACUGAUAAAGUGGAGAACCGCUUCACC AAUUAGUUGAGUGAAGGUGGGCUGCUUGCAU CAGCCUAAUGUCGAGAAGUGCUUUCUUCGGA AAGUAACCCUCGAAACAAAUUCAGUGCUCCU CUCGAAAGAAUAGAGCAAUGAAGGAAUGCAA C (SEQ ID NO: 587) | included |
| M1-1/3/4 | ACCGCUUCACCAAAAGCUGUCCCUUAGGGGA UUAGAACUUGAGUGAAGGUGGGCUGCUUGCA | included |
| | UCAGCCUAAUGUCGAGAAGUGCUUUCUUCGG AAAGUAACCCUCGAAACAAAUUCAGUGCUCCU CUCCAAUUCUGCACAAGAAAGUUGCAGAACC CGAAUAGAGCAAUGAAGGAAUGCAAC (SEQ ID NO: 564) | |
| M1/3/4 (geCas14a1_ 3.0) | ACCGCUUCACCAAAAGCUGUCCCUUAGGGGA UUAGAACUUGAGUGAAGGUGGGCUGCUUGCA UCAGCCUAAUGUCGAGAAGUGCUUUCUUCGG AAAGUAACCCUCGAAACAAAUUCAGUGCUCCU CUCGAAAGAAUAGAGCAAUGAAGGAAUGCAA C (SEQ ID NO: 566) | included |
| M1-1/2/3/4 | ACCGCUUCACCAAUUAGUUGAGUGAAGGUGG GCUGCUUGCAUCAGCCUAAUGUCGAGAAGUG CUUUCUUCGGAAAGUAACCCUCGAAACAAAU UCAGUGCUCCUCUCCAAUUCUGCACAGAAAG UUGCAGAACCCGAAUAGAGCAAUGAAGGAAU GCAAC (SEQ ID NO: 588) | included |
| M1/2/3/4 | ACCGCUUCACCAAUUAGUUGAGUGAAGGUGG GCUGCUUGCAUCAGCCUAAUGUCGAGAAGUG CUUUCUUCGGAAAGUAACCCUCGAAACAAAU UCAGUGCUCCUCUCGAAAGAAUAGAGCAAUG AAGGAAUGCAAC (SEQ ID NO: 589) | included |

### Example 9. Effects of gRNA engineering

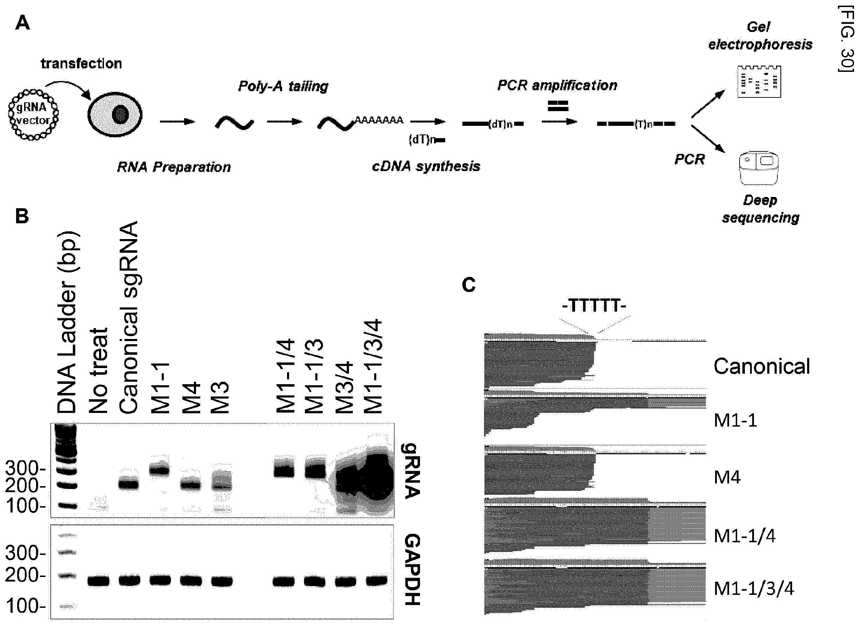

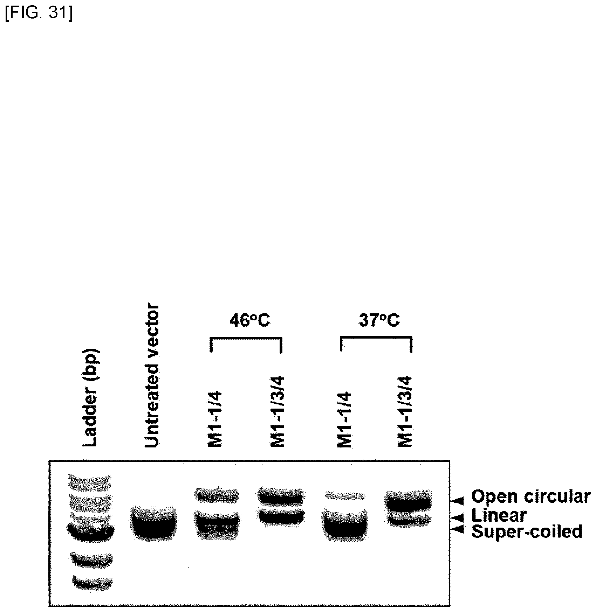

In addition, the present inventors investigated the molecular mechanism underlying effects of gRNA engineering (M1-M4) on increased indel efficiency. As a result, as expected, it was identified that M1-1 sharply increased the expression of gRNA as a complete sequence as assessed by target RNA-seq analysis (FIG. 30). In contrast, M3 and M4 were not associated with changes in gRNA expression. Regarding M4, the U-rich tail sequence induced efficient RNP formation. However, specific information about the effect of M3 could not be identified. However, it was found that through in vitro double-stranded DNA (dsDNA) digestion analysis, a double-strand break occurs better in the engineered gRNA to which MS3 engineering was added. Although a specific mechanism is not yet known, it is predicted that as a partial sequence is removed from the 5' end of the gRNA by MS3 engineering, the Cas12f1's accessibility to the target strand is increased, and it is thought that MS3 engineering improves low cleavage efficiency of the wildtype CRISPR/Cas12f1 system for the target strand (FIG. 31). In other words, the wildtype CRISPR/Cas12f1 system has significantly low indel efficiency because the wildtype CRISPR/Cas12f1 system leads to low-efficiency cleavage of the target strand and thus generates a nick that mainly cleaves only a single strand. However, the MS3 engineering allowed the CRISPR/Cas12f1 system to exert significantly increased cleavage activity on the target strand without changing the cleavage pattern of the CRISPR/Cas12f1 system, and as a result, significantly increased indel efficiency was observed due to double-strand cleavage.

### Example 10. Large-scale validation of engineered CRISPR/Cas12f1 as efficient genome editor

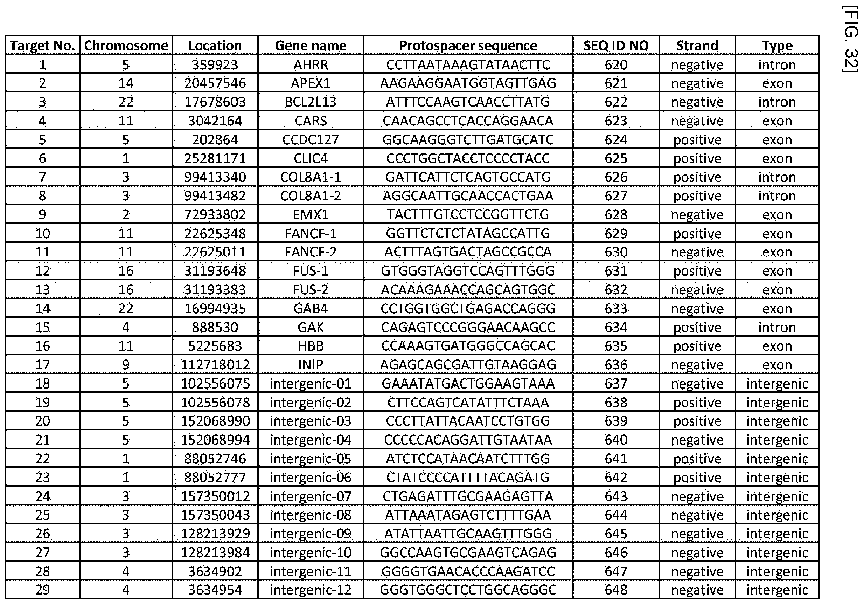

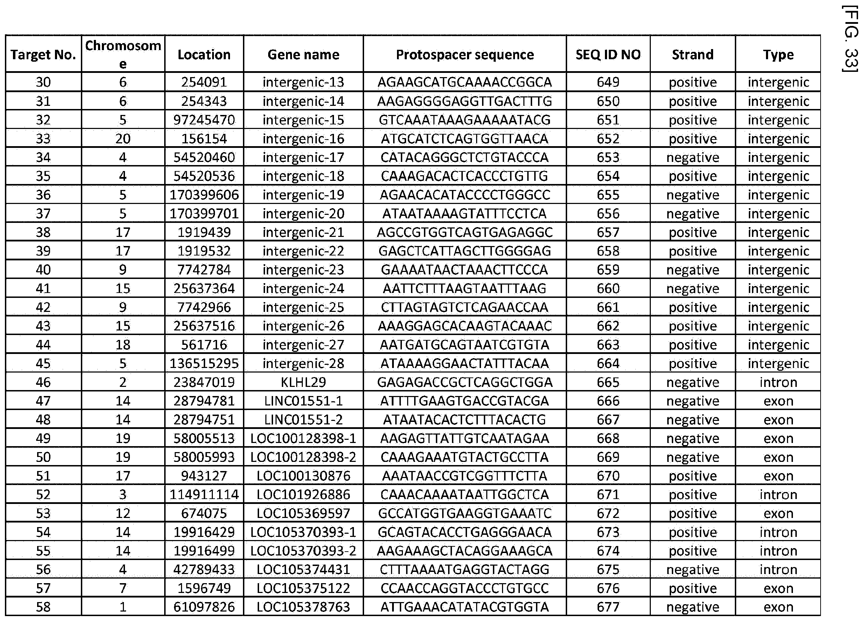

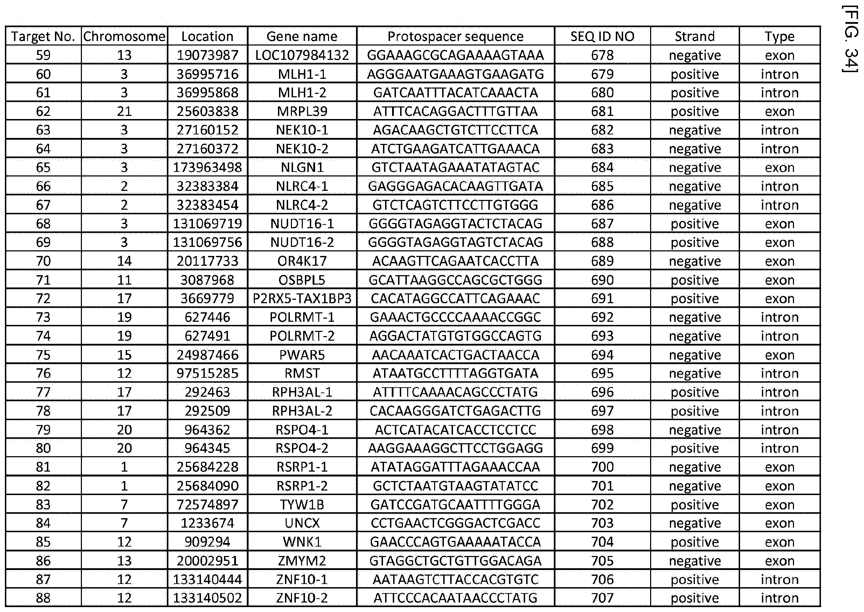

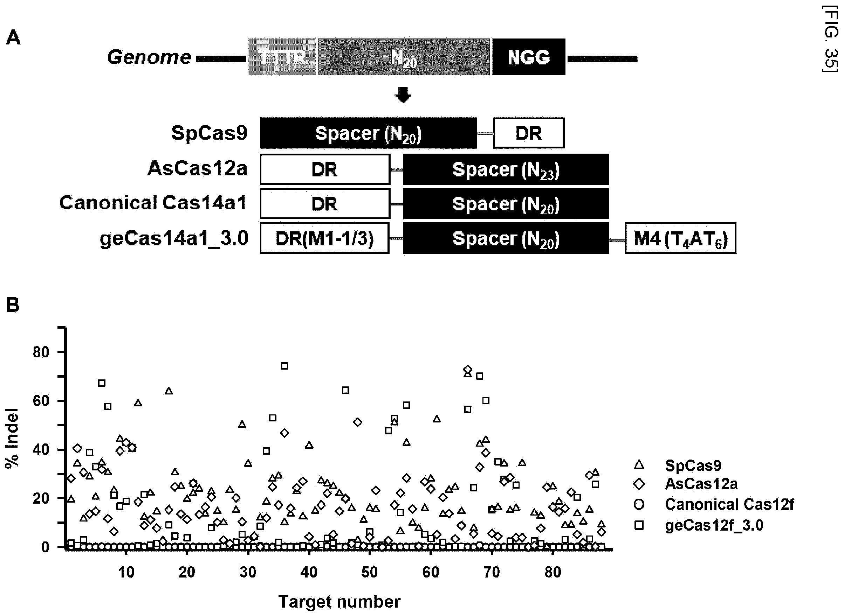

Since the above-described gRNA engineering was implemented by monitoring changes in indel efficiency for only three endogenous targets, experiments were conducted to see whether the engineered CRISPR/Cas12f1 system (hereinafter, used interchangeably with the geCas14a1 system) is capable of exerting genome editing activity on a wide range of targets. For this purpose, endogenous targets having 5'-TTTR-N20-NGG-3', which can be edited with Cas9, Cas12a and Cas12f1, were searched in silico, and 88 endogenous loci were randomly selected(FIGS. 32 to 34). After transfection of HEK293-T cells with the CRISPR/SpCas9 system, the CRISPR/AsCas12a system, the canonical CRISPR/Cas14a1 system or the geCas14a1_3.0 system, indel efficiency at the selected 88 endogenous loci was identified through deep sequencing analysis (FIG. 35). As a result, the canonical CRISPR/Cas14a1 system showed indel efficiency of less than 0.1% in 91% (80/88) of the targets. However, the geCas14a1_3.0 system showed greatly improved indel efficiency for a wide range of targets (FIG 35). The average indel efficiency of the geCas14a1_3.0 system was nearly comparable to that of AsCas12a (p> 0.05) and slightly lower than that of SpCas9.

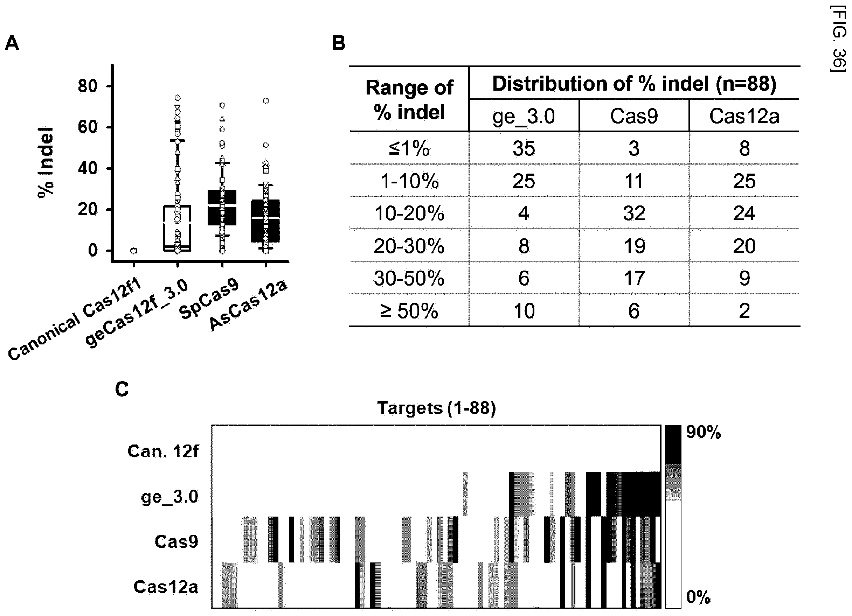

A box-and-whisker plot (FIG. 36) of this large-scale analysis led to the following conclusions: 1) the canonical CRISPR/Cas14a1 system itself shows average indel efficiency of less than 0.1% in human cells, and this makes it difficult to use the system as a genome editor; 2) an average increase in indel efficiency caused by gRNA engineering is 540-fold; 3) like the CRISPR/SpCas9 system and the CRISPR/AsCas12a system, the geCas14a1_3.0 system can be used as a genome editor in eukaryotic cells, in particular, the geCas14a1_3.0 system showed higher indel efficiency for some targets (26%, 23/88) than the CRISPR/SpCas9 system and the CRISPR/AsCas12a system; 4) unlike the CRISPR/SpCas9 system and the CRISPR/AsCas12a system,the geCas14a1_3.0 system did not show Gaussian distribution, and rather the targets were more evenly distributed over the entire range of indel efficiency (FIG. 36).

### Example 11. Comparison of cleavage patterns between canonical gRNA and engineered gRNA

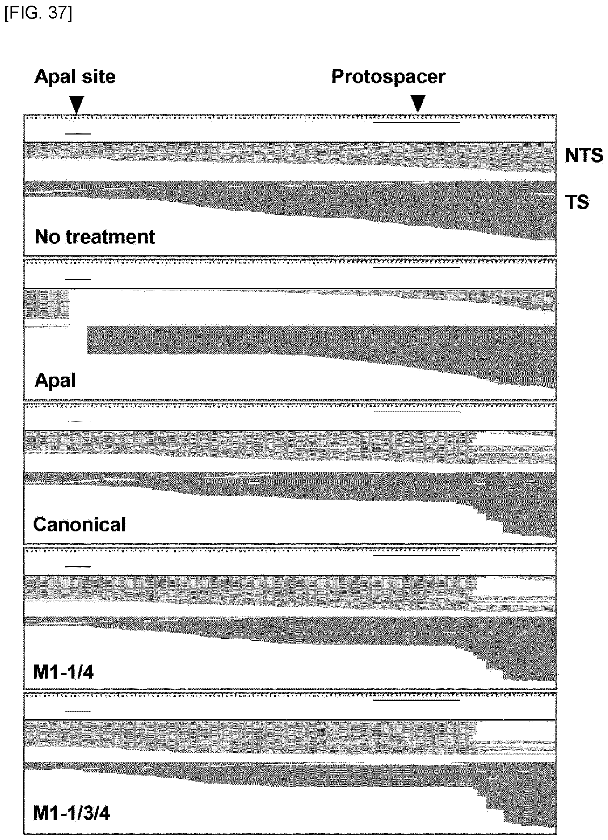

Using the canonical gRNA and the engineered gRNA, their cleavage patterns for the target strand (TS) and non-target strand (NTS) were analyzed by next-generation sequencing (NGS). As a result, it was confirmed that the canonical gRNA caused relatively less cleavage of NTS. On the other hand, it was confirmed that the engineered gRNA caused gradually increased cleavage of NTS as the gRNA became engineered. In addition, it was confirmed that the engineered gRNA also caused slightly increased cleavage of TS. Through these results, it can be understood that the engineered gRNA reduces the difference in cleavage between NTS and TS, and thus increases the double-strand cleavage(FIG. 37).

## Claims

1. An engineered guide RNA for a CRISPR/Cas12f1 (Cas14a1) system, comprising an engineered trans-activating CRISPR RNA (tracrRNA) and a CRISPR RNA (crRNA),
wherein the engineered tracrRNA is modified not to comprise a sequence of five or more consecutive uridines, and comprises a first sequence, a second sequence, a third sequence, a fourth sequence and a fifth sequence sequentially in a 3' to 5' direction,
the first sequence is 5'-CAAAUUCANNNVNCCUCUCCAAAUUCUGCACAA-3' (SEQ ID NO: 194) or a part of SEQ ID NO: 194, wherein N is each independently A, C, G or U, and V is A, C or G, and wherein the part of SEQ ID NO: 194 comprises 5'-CAAAUUCANNNVN-3' (SEQ ID NO: 193) and does not comprise at least one nucleotide from the 3' end of SEQ ID NO: 194,
the second sequence is 5'-AUGUCGAGAAGUGCUUUCUUCGGAAAGUAACCCUCGAAA-3' (SEQ ID NO: 211) or a sequence having sequence identity or sequence similarity of at least 70% or more to SEQ ID NO: 211,
the third sequence is 5'-GGCUGCUUGCAUCAGCCUA-3' (SEQ ID NO: 212) or a sequence having sequence identity or sequence similarity of at least 70% or more to SEQ ID NO: 212,
the fourth sequence is 5'-CCGCUUCACCAAAAGCUGUCCCUUAGGGGAUUAGAACUUGAGUGAA GGUG-3' (SEQ ID NO: 213) or a part of SEQ ID NO: 213, wherein the part of SEQ ID NO: 213 is a sequence obtained by deleting at least one pair of nucleotides forming a complementary base pair and/or at least one nucleotide not involved in forming a complementary base pair from SEQ ID NO: 213, and
the fifth sequence is 5'-CUUCACUGAUAAAGUGGAGAA-3' (SEQ ID NO: 248) or a part of SEQ ID NO: 248, wherein the part of SEQ ID NO: 248 is a sequential partial sequence at the 3' end of SEQ ID NO: 248; and
the crRNA is a wildtype crRNA or an engineered crRNA,
wherein the wildtype crRNA comprises a wildtype repeat sequence and a guide sequence sequentially in a 5' to 3' direction, and
the engineered crRNA comprises a sixth sequence, a seventh sequence and a guide sequence sequentially in a 5' to 3' direction,
wherein the sixth sequence is 5'-GUUGCAGAACCCGAAUAGNBNNNUGAAGGA-3' (SEQ ID NO: 373) or a part of SEQ ID NO: 373, wherein N is each independently A, C, G or U, and B is U, C or G; and the part of SEQ ID NO: 373 is a sequence that comprises 5'-NBNNNUGAAGGA-3' (SEQ ID NO: 372) and does not comprise at least one nucleotide from the 3' end of SEQ ID NO: 373,
the seventh sequence is 5'-AUGCAAC-3' or a sequence having sequence identity or sequence similarity of at least 70% or more to 5'-AUGCAAC-3', and
the guide sequence is a sequence capable of hybridizing with a target sequence or a sequence forming a complementary bond with the target sequence and has 15 to 30 nucleotides (nts).

2. The engineered guide RNA of claim 1, wherein the first sequence is 5'-CAAAUUCANNNCNCCUCUCCAAUUCUGCACAA-3' (SEQ ID NO: 111) or a part of SEQ ID NO: 111,
wherein the part of SEQ ID NO: 111 is
5'-CAAAUUCANNNCNCCUCUCCAAUUCUGCACA-3' (SEQ ID NO: 273),
5'-CAAAUUCANNNCNCCUCUCCAAUUCUGCAC-3' (SEQ ID NO: 274),
5'-CAAAUUCANNNCNCCUCUCCAAUUCUGCA- 3' (SEQ ID NO: 275),
5'-CAAAUUCANNNCNCCUCUCCAAUUCUGC-3' (SEQ ID NO: 276),
5'-CAAAUUCANNNCNCCUCUCCAAUUCUG-3' (SEQ ID NO: 277),
5'-CAAAUUCANNNCNCCUCUCCAAUUCU-3' (SEQ ID NO: 278),
5'-CAAAUUCANNNCNCCUCUCCAAUUC-3' (SEQ ID NO: 279),
5'-CAAAUUCANNNCNCCUCUCCAAUU-3' (SEQ ID NO: 280),
5'-CAAAUUCANNNCNCCUCUCCAAU-3' (SEQ ID NO: 281),
5'-CAAAUUCANNNCNCCUCUCCAA-3' (SEQ ID NO: 282),
5'-CAAAUUCANNNCNCCUCUCCA-3' (SEQ ID NO: 283),
5'-CAAAUUCANNNCNCCUCUCC-3' (SEQ ID NO: 284),
5'-CAAAUUCANNNCNCCUCUC-3' (SEQ ID NO: 285),
5'-CAAAUUCANNNCNCCUCU-3' (SEQ ID NO: 286),
5'-CAAAUUCANNNCNCCUC-3' (SEQ ID NO: 287),
5'-CAAAUUCANNNCCU-3' (SEQ ID NO: 288),
5'-CAAAUUCANNNCNCC-3' (SEQ ID NO: 289),
5'-CAAAUUCANNNCNC-3' (SEQ ID NO: 290), or
5'-CAAAUUCANNNCN-3' (SEQ ID NO: 272),
wherein N is each independently A, C, G or U.

3. The engineered guide RNA of claim 1, wherein the part of SEQ ID NO: 213 is 
5'-
CCGCUUCACCAAAAGCUGUCCUUAGGGAUUAGAACUUGAGUGAAGGUG-
3' (SEQ ID NO: 214),
 
5'-
CCGCUUCACCAAAAGCUGUCUUAGGAUUAGAACUUGAGUGAAGGUG-3'
(SEQ ID NO: 215),
 
5'-CCGCUUCACCAAAAGCUGUUUAGAUUAGAACUUGAGUGAAGGUG-
3' (SEQ ID NO: 216),
5'-CCGCUUCACCAAAAGCUGUUAGUUAGAACUUGAGUGAAGGUG-3' (SEQ ID NO: 217),
5'-CCGCUUCACCAAAAGCUGUUAGUAGAACUUGAGUGAAGGUG-3' (SEQ ID NO: 231),
5'-CCGCUUCACCAAAAGCUUUAGAGAACUUGAGUGAAGGUG-3' (SEQ ID NO: 232),
5'-CCGCUUCACCAAAAGCUUAGGAACUUGAGUGAAGGUG-3' (SEQ ID NO: 233),
5'-CCGCUUCACCAAAAGUUAGAACUUGAGUGAAGGUG-3' (SEQ ID NO: 234),
5'-CCGCUUCACCAAAAUUAGACUUGAGUGAAGGUG-3' (SEQ ID NO: 235),
5'-CCGCUUCACCAAAUUAGCUUGAGUGAAGGUG-3' (SEQ ID NO: 236),
5'-CCGCUUCACCAAUUAGUUGAGUGAAGGUG-3' (SEQ ID NO: 237),
5'-CCGCUUCACCAUUAGUGAGUGAAGGUG-3' (SEQ ID NO: 238),
5'-CCGCUUCACCUUAGGAGUGAAGGUG-3' (SEQ ID NO:239),
5'-CCGCUUCACUUAGAGUGAAGGUG-3' (SEQ ID NO: 240),
5'-CCGCUUCACUUAGGUGAAGGUG-3' (SEQ ID NO: 241),
5'-CCGCUUCAUUAGUGAAGGUG-3' (SEQ ID NO: 242),
5'-CCGCUUCUUAGGAAGGUG-3' (SEQ ID NO: 243),
5'-CCGCUUUUAGAAGGUG-3' (SEQ ID NO: 244),
5'-CCGCUUUAGAGGUG-3' (SEQ ID NO: 245),
5'-CCGCUUAGGGUG-3' (SEQ ID NO: 246),
5'-CCGUUAGGUG-3' (SEQ ID NO: 247),
5'-CCUUAGGUG-3',
5'-CUUAGUG-3',
5'-CUUAGG-3' or
5'-UUAG-3',
wherein 5'-UUAG-3' included in the part of SEQ ID NO: 213 is optionally substituted with 5'-GAAA-3'.

4. The engineered guide RNA of claim 1, wherein the part of SEQ ID NO: 248 is 5'-A-3', 5'-AA-3', 5'-GAA-3', 5'-AGAA-3', 5'- GAGAA-3', 5'-GGAGAA-3', 5'-UGGAGAA-3', 5'-GUGGAGAA-3', 5'-AGUGGAGAA-3', 5'-AAGUGGAGAA-3' (SEQ ID NO: 249), 5'-AAAGUGGAGAA-3' (SEQ ID NO: 250), 5'-UAAAGUGGAGAA-3' (SEQ ID NO: 251), 5'-AUAAAGUGGAGAA-3' (SEQ ID NO: 252), 5'-GAUAAAGUGGAGAA-3' (SEQ ID NO: 253), 5'-UGAUAAAGUGGAGAA-3' (SEQ ID NO: 254), 5'-CUGAUAAAGUGGAGAA-3' (SEQ ID NO: 255), 5'-ACUGAUAAAGUGGAGAA-3' (SEQ ID NO: 256), 5'-CACUGAUAAAGUGGAGAA-3' (SEQ ID NO: 257), 5'-UCACUGAUAAAGUGGAGAA-3' (SEQ ID NO: 258), or 5'-UUCACUGAUAAAGUGGAGAA-3' (SEQ ID NO: 259).

5. The engineered guide RNA of claim 1, wherein the sixth sequence is 5'-GUUGCAGAACCCGAAUAGNGNNNUGAAGGA-3' (SEQ ID NO: 408) or a part of SEQ ID NO: 408,
wherein the part of SEQ ID NO: 408 is 5'-UUGCAGAACCCGAAUAGNGNNNUGAAGGA-3' (SEQ ID NO: 413), 5'-UGCAGAACCCGAAUAGNGNNNUGAAGGA-3' (SEQ ID NO: 414), 5'-GCAGAACCCGAAUAGNGNNNUGAAGGA- 3' (SEQ ID NO: 415), 5'-CAGAACCCGAAUAGNGNNNUGAAGGA-3' (SEQ ID NO: 416), 5'-AGAACCCGAAUAGNGNNNUGAAGGA-3' (SEQ ID NO: 417), 5'-GAACCCGAAUAGNGNNNUGAAGGA-3' (SEQ ID NO: 418), 5'-AACCCGAAUAGNGNNNUGAAGGA-3' (SEQ ID NO: 419), 5'-ACCCGAAUAGNGNNNUGAAGGA-3' (SEQ ID NO: 420), 5'-CCCGAAUAGNGNNNUGAAGGA-3' (SEQ ID NO: 421), 5'-CCGAAUAGNGNNNUGAAGGA-3' (SEQ ID NO: 422), 5'-CGAAUAGNGNNNUGAAGGA-3' (SEQ ID NO: 423), 5'-GAAUAGNGNNNUGAAGGA-3' (SEQ ID NO: 424), 5'-AAUAGNGNNNUGAAGGA-3' (SEQ ID NO: 425), 5'-AUAGNGNNNUGAAGGA-3' (SEQ ID NO: 426), 5'-UAGNGNNNUGAAGGA-3' (SEQ ID NO: 427), 5'-AGNGNNNUGAAGGA-3' (SEQ ID NO: 428), or 5'-NGNNNUGAAGGA-3' (SEQ ID NO: 412), wherein N is each independently A, C, G or U.

6. The engineered guide RNA of claim 1, wherein the crRNA further comprises a U-rich tail sequence located at the 3' end of the crRNA.

7. The engineered guide RNA of claim 6, wherein the U-rich tail sequence is 5'-(UₐN)_{d}Uₑ-3', 5'-UₐVUₐVUₑ-3', or 5'-UₐVUₐVUₐVUₑ-3', wherein N is A, C, G or U; each V is independently A, C or G; a is an integer of 0 to 4; d is an integer of 0 to 3; and e is an integer of 0 to 10.

8. The engineered guide RNA of claim 1, wherein the engineered guide RNA is a dual guide RNA or a single guide RNA,
wherein, when the engineered guide RNA is a single guide RNA, the engineered guide RNA further comprises a linker sequence, and the linker sequence is located between the engineered tracrRNA and the crRNA.

9. The engineered guide RNA of claim 1, wherein the engineered tracrRNA comprises 5'-CUUCACUGAUAAAGUGGAGAACCGCUUCACCAAAAGCUGUCCCUUAGGG GAUUAGAACUUGAGUGAAGGUGGGCUGCUUGCAUCAGCCUAAUGUCGA GAAGUGCUUUCUUCGGAAAGUAACCCUCGAAACAAAUUCANNNCNCCUC UCCAAUUCUGCACAA-3' (SEQ ID NO: 269), 5'-ACCGCUUCACCAAAAGCUGUCCCUUAGGGGAUUAGAACUUGAGUGAAGG UGGGCUGCUUGCAUCAGCCUAAUGUCGAGAAGUGCUUUCUUCGGAAAG UAACCCUCGAAACAAAUUCANNNCNCCUCUCCAAUUCUGCACAA-3' (SEQ ID NO: 304), 5'-CUUCACUGAUAAAGUGGAGAACCGCUUCACCAAUUAGUUGAGUGAAGGU GGGCUGCUUGCAUCAGCCUAAUGUCGAGAAGUGCUUUCUUCGGAAAGU AACCCUCGAAACAAAUUCANNNCNCCUCUCCAAUUCUGCACAA-3' (SEQ ID NO: 590), 5'-ACCGCUUCACCAAUUAGUUGAGUGAAGGUGGGCUGCUUGCAUCAGCCU AAUGUCGAGAAGUGCUUUCUUCGGAAAGUAACCCUCGAAACAAAUUCAN NNCNCCUCUCCAAUUCUGCACAA-3' (SEQ ID NO: 301), 5'-CUUCACUGAUAAAGUGGAGAACCGCUUCACCAAAAGCUGUCCCUUAGGG GAUUAGAACUUGAGUGAAGGUGGGCUGCUUGCAUCAGCCUAAUGUCGA GAAGUGCUUUCUUCGGAAAGUAACCCUCGAAACAAAUUCANNNCNCCUC UC-3' (SEQ ID NO: 592), 5'-ACCGCUUCACCAAAAGCUGUCCCUUAGGGGAUUAGAACUUGAGUGAAGG UGGGCUGCUUGCAUCAGCCUAAUGUCGAGAAGUGCUUUCUUCGGAAAG UAACCCUCGAAACAAAUUCANNNCNCCUCUC-3' (SEQ ID NO: 305), 5'-CUUCACUGAUAAAGUGGAGAACCGCUUCACCAAUUAGUUGAGUGAAGGU GGGCUGCUUGCAUCAGCCUAAUGUCGAGAAGUGCUUUCUUCGGAAAGU AACCCUCGAAACAAAUUCANNNCNCCUCUC-3' (SEQ ID NO: 593), or 5'-ACCGCUUCACCAAUUAGUUGAGUGAAGGUGGGCUGCUUGCAUCAGCCU AAUGUCGAGAAGUGCUUUCUUCGGAAAGUAACCCUCGAAACAAAUUCAN NNCNCCUCUC-3' (SEQ ID NO: 300), wherein N is each independently A, C, G or U.

10. The engineered guide RNA of claim 9, wherein the engineered crRNA comprises 5'-GUUGCAGAACCCGAAUAGNGNNNUGAAGGAAUGCAAC-3' (SEQ ID NO: 591) and a guide sequence; or comprises 5'-GAAUAGNGNNNUGAAGGAAUGCAAC-3' (SEQ ID NO: 594) and a guide sequence, wherein N is each independently A, C, G or U.

11. The engineered guide RNA of claim 2, wherein the 5'-NNNCN-3' present in the first sequence is 5'-UUUCU-3', 5'-GUUCU-3', 5'-UCUCU-3', 5'-UUGCU-3', 5'-UUUCC-3', 5'-GCUCU-3', 5'-GUUCC-3', 5'-UCGCU-3', 5'-UCUCC-3', 5'-UUGCC-3', 5'-GCGCU-3', 5'-GCUCC-3', 5'-GUGCC-3', 5'-UCGCC-3', 5'-GCGCC-3', or 5'-GUGCU-3'.

12. The engineered guide RNA of claim 5, wherein the 5'-NGNNN-3' present in the sixth sequence is 5'-AGGAA-3', 5'-AGCAA-3', 5'-AGAAA-3', 5'-AGCAU-3', 5'-AGCAG-3', 5'-AGCAC-3', 5'-AGCUA-3', 5'-AGCGA-3', 5'-AGCCA-3', 5'-UGCAA-3', 5'-UGCUA-3', 5'-UGCGA-3', 5'-UGCCA-3', 5'-GGCAA-3', 5'-GGCUA-3', 5'-GGCGA-3', 5'-GGCCA-3', 5'-CGCAA-3', 5'-CGCUA-3', 5'-CGCGA-3', or 5'-CGCCA -3'.

13. The engineered guide RNA of claim 11, wherein the engineered tracrRNA comprises 5'-CUUCACUGAUAAAGUGGAGAACCGCUUCACCAAAAGCUGUCCCUUAGGG GAUUAGAACUUGAGUGAAGGUGGGCUGCUUGCAUCAGCCUAAUGUCGA GAAGUGCUUUCUUCGGAAAGUAACCCUCGAAACAAAUUCAGUGCUCCUC UCCAAUUCUGCACAA-3' (SEQ ID NO: 270), 5'-ACCGCUUCACCAAAAGCUGUCCCUUAGGGGAUUAGAACUUGAGUGAAGG UGGGCUGCUUGCAUCAGCCUAAUGUCGAGAAGUGCUUUCUUCGGAAAG UAACCCUCGAAACAAAUUCAGUGCUCCUCUCCAAUUCUGCACAA-3' (SEQ ID NO: 308), 5'-CUUCACUGAUAAAGUGGAGAACCGCUUCACCAAUUAGUUGAGUGAAGGU GGGCUGCUUGCAUCAGCCUAAUGUCGAGAAGUGCUUUCUUCGGAAAGU AACCCUCGAAACAAAUUCAGUGCUCCUCUCCAAUUCUGCACAA-3' (SEQ ID NO: 595), 5'-ACCGCUUCACCAAUUAGUUGAGUGAAGGUGGGCUGCUUGCAUCAGCCU AAUGUCGAGAAGUGCUUUCUUCGGAAAGUAACCCUCGAAACAAAUUCAG UGCUCCUCUCCAAUUCUGCACAA-3' (SEQ ID NO: 306), 5'-CUUCACUGAUAAAGUGGAGAACCGCUUCACCAAAAGCUGUCCCUUAGGG GAUUAGAACUUGAGUGAAGGUGGGCUGCUUGCAUCAGCCUAAUGUCGA GAAGUGCUUUCUUCGGAAAGUAACCCUCGAAACAAAUUCGUGCUCCUCU C-3' (SEQ ID NO: 596), 5'-ACCGCUUCACCAAAAGCUGUCCCUUAGGGGAUUAGAACUUGAGUGAAGG UGGGCUGCUUGCAUCAGCCUAAUGUCGAGAAGUGCUUUCUUCGGAAAG UAACCCUCGAAACAAAUUCAGUGCUCCUCU C-3' (SEQ ID NO: 309), 5'-CUUCACUGAUAAAGUGGAGAACCGCUUCACCAAUUAGUUGAGUGAAGGU GGGCUGCUUGCAUCAGCCUAAUGUCGAGAAGUGCUUUCUUCGGAAAGU AACCCUCGAAACAAAUUCAGUGCUCCUCUC-3' (SEQ ID NO: 597), or 5'-ACCGCUUCACCAAUUAGUUGAGUGAAGGUGGGCUGCUUGCAUCAGCCU AAUGUCGAGAAGUGCUUUCUUCGGAAAGUAACCCUCGAAACAAAUUCAG UGCUCCUCUC-3' (SEQ ID NO: 307).

14. The engineered guide RNA of claim 13, wherein the engineered crRNA comprises 5'-GUUGCAGAACCCGAAUAGAGCAAUGAAGGAAUGCAAC-3' (SEQ ID NO: 539) and a guide sequence; or comprises 5'-GAAUAGAGCAAUGAAGGAAUGCAAC-3' (SEQ ID NO: 598) and a guide sequence.

15. A vector for a CRISPR/Cas12f1 (Cas14a1) system, comprising a nucleic acid encoding an engineered guide RNA,
wherein the engineered guide RNA comprises an engineered trans-activating CRISPR RNA (tracrRNA) and a CRISPR RNA (crRNA),
wherein the engineered tracrRNA is modified not to comprise a sequence of five or more consecutive uridines, and comprises a first sequence, a second sequence, a third sequence, a fourth sequence and a fifth sequence sequentially in a 3' to 5' direction,
wherein the first sequence is 5'-CAAAUUCANNNVNCCUCUCCAAAUUCUGCACAA-3' (SEQ ID NO: 194) or a part of SEQ ID NO: 194 wherein N is each independently A, C, G or U, and V is A, C or G, and the part of SEQ ID NO: 194 is a sequence that comprises 5'-CAAAUUCANNNVN-3' (SEQ ID NO: 193) and does not comprise at least one nucleotide from the 3' end of SEQ ID NO: 194,
the second sequence is 5'-AUGUCGAGAAGUGCUUUCUUCGGAAAGUAACCCUCGAAA-3' (SEQ ID NO: 211) or a sequence having sequence identity or sequence similarity of at least 70% or more to SEQ ID NO: 211,
the third sequence is 5'-GGCUGCUUGCAUCAGCCUA-3' (SEQ ID NO: 212) or a sequence having sequence identity or sequence similarity of at least 70% or more to SEQ ID NO: 212,
the fourth sequence is 5'-CCGCUUCACCAAAAGCUGUCCCUUAGGGGAUUAGAACUUGAGUGAAGGU G-3' (SEQ ID NO: 213) or a part of SEQ ID NO: 213 wherein the part of SEQ ID NO: 213 is a sequence obtained by deleting at least one pair of nucleotides forming a complementary base pair and/or at least one nucleotide not involved in forming a complementary base pair from SEQ ID NO: 213, and
the fifth sequence is 5'-CUUCACUGAUAAAGUGGAGAA-3' (SEQ ID NO: 248) or a part of SEQ ID NO: 248 wherein the part of SEQ ID NO: 248 is a sequential partial sequence at the 3' end of SEQ ID NO: 248; and
the crRNA is a wildtype crRNA or an engineered crRNA,
wherein the wildtype crRNA comprises a wildtype repeat sequence and a guide sequence sequentially in a 5' to 3' direction, and
the engineered crRNA comprises a sixth sequence, a seventh sequence and a guide sequence sequentially in a 5' to 3' direction,
wherein the sixth sequence is 5'-GUUGCAGAACCCGAAUAGNBNNNUGAAGGA-3' (SEQ ID NO: 373) or a part of SEQ ID NO: 373, wherein N is each independently A, C, G or U, and B is U, C or G; and the part of SEQ ID NO: 373 is a sequence that comprises 5'-NBNNNUGAAGGA-3' (SEQ ID NO: 372) and does not comprise at least one nucleotide from the 3' end of SEQ ID NO: 373,
the seventh sequence is 5'-AUGCAAC-3' or a sequence having sequence identity or sequence similarity of at least 70% or more to 5'-AUGCAAC-3', and
the guide sequence is a sequence capable of hybridizing with a target sequence or a sequence forming a complementary bond to the target sequence, and has 15 to 30 nucleotides (nts).

16. The vector of claim 15, further comprising a promoter for the nucleic acid encoding the engineered guide RNA.

17. The vector of claim 16, wherein the promoter is a U6 promoter, an H1 promoter or a 7SK promoter.

18. The vector of claim 15, wherein the vector is a plasmid, a PCR amplicon or a viral vector.

19. A composition comprising an engineered CRISPR/Cas12f1 (Cas14a1) system,
wherein the engineered CRISPR/Cas12f1 (Cas14a1) system comprises:
an engineered guide RNA or a nucleic acid encoding the engineered guide RNA; and
a Cas12f1 (Cas14a1) protein or a nucleic acid encoding the Cas12f1 protein, wherein the engineered guide RNA comprises an engineered trans-activating CRISPR RNA (tracrRNA) and a CRISPR RNA (crRNA),
wherein the engineered tracrRNA is modified not to comprise a sequence of five or more consecutive uridines, and comprises a first sequence, a second sequence, a third sequence, a fourth sequence and a fifth sequence sequentially in a 3' to 5' direction,
wherein the first sequence is 5'-CAAAUUCANNNVNCCUCUCCAAAUUCUGCACAA-3' (SEQ ID NO: 194) or a part of SEQ ID NO: 194, wherein N is each independently A, C, G or U, and V is A, C or G; and the part of SEQ ID NO: 194 is a sequence that comprises 5'-CAAAUUCANNNVN-3' (SEQ ID NO: 193) and does not comprise at least one nucleotide from the 3' end of SEQ ID NO: 194,
the second sequence is 5'-AUGUCGAGAAGUGCUUUCUUCGGAAAGUAACCCUCGAAA-3' (SEQ ID NO: 211) or a sequence having sequence identity or sequence similarity of at least 70% or more to SEQ ID NO: 211,
the third sequence is 5'-GGCUGCUUGCAUCAGCCUA-3' (SEQ ID NO: 212) or a sequence having sequence identity or sequence similarity of at least 70% or more to SEQ ID NO: 212,
the fourth sequence is 5'-CCGCUUCACCAAAAGCUGUCCCUUAGGGGAUUAGAACUUGAGUGAAGGU G-3' (SEQ ID NO: 213) or a part of SEQ ID NO: 213, wherein the part of SEQ ID NO: 213 is a sequence obtained by deleting at least one pair of nucleotides forming a complementary base pair and/or at least one nucleotide not involved in forming a complementary base pair from SEQ ID NO: 213, and
the fifth sequence is 5'-CUUCACUGAUAAAGUGGAGAA-3' (SEQ ID NO: 248) or a part of SEQ ID NO: 248, wherein the part of SEQ ID NO: 248 is a sequential partial sequence at the 3' end of SEQ ID NO: 248; and
the crRNA is a wildtype crRNA or an engineered crRNA,
wherein the wildtype crRNA comprises a wildtype repeat sequence and a guide sequence sequentially in a 5' to 3' direction, and
the engineered crRNA comprises a sixth sequence, a seventh sequence and a guide sequence sequentially in a 5' to 3' direction,
wherein the sixth sequence is 5'-GUUGCAGAACCCGAAUAGNBNNNUGAAGGA-3' (SEQ ID NO: 373) or a part of SEQ ID NO: 373, wherein N is each independently A, C, G or U, and B is U, C or G; and the part of SEQ ID NO: 373 is a sequence that comprises 5'-NBNNNUGAAGGA-3' (SEQ ID NO: 372) and does not comprise at least one nucleotide from the 3' end of SEQ ID NO: 373,
the seventh sequence is 5'-AUGCAAC-3' or a sequence having sequence identity or sequence similarity of at least 70% or more to 5'-AUGCAAC-3', and
the guide sequence is a sequence capable of hybridizing with a target sequence or a sequence forming a complementary bond to the target sequence, and is a sequence of 15 to 30 nucleotides (nts).

20. The composition of claim 19, wherein the composition comprises the nucleic acid encoding the engineered guide RNA and the nucleic acid encoding the Cas12f1 (Cas14a1) protein in one vector.

21. The composition of claim 19, further comprising a promoter for the nucleic acid encoding the engineered guide RNA and a promoter for the nucleic acid encoding the Cas12f1 (Cas14a1) protein.

22. The composition of claim 21, wherein the promoter is a U6 promoter, an H1 promoter, a 7SK promoter, a CMV promoter, an LTR promoter, an Ad MLP promoter, an HSV promoter, an SV40 promoter, a CBA promoter, or an RSV promoter.

23. The composition of claim 19, wherein the vector is a plasmid, mRNA (transcript), a PCR amplicon, or a viral vector.

24. The composition of claim 19, wherein the composition is in a form of nucleic acid-protein mixture or engineered ribonucleoprotein mixture formed with the engineered guide RNA and the Cas14a1 protein.
